# EUROPEAN PATENT APPLICATION

(11) **EP 1 669 370 A1**
(43) Date of publication of application: **14.06.2006**
(21) Application number: 04292378.9
(22) Date of filing: 06.10.2004
(51) Int. Cl.: C07K 14/445, G01N 33/50, C30B 7/00, G01N 23/00, G06F 17/50

(54) **Identification of regions of apical membrane antigen 1 of apicomplexan parasites suitable for targeting by vaccines and drugs by derivation and analysis of the three-dimensional structure**

(71) Applicant: INSTITUT PASTEUR, 75015 Paris (FR); CENTRE NATIONAL DE LA RECHERCHE SCIENTIFIQUE (CNRS), 75016 Paris (FR)
(72) Inventor: Bentley, Graham, 91191 Gif sur Yvette (FR); Vulliez-Le-Normand, Brigitte, 75020 Paris (FR); Pizarro, Juan Carlos, Seattle, WA 98119 (US); Blackman, Michael J., Hitchin Herts SC5 1SN (GB); Collins, Christine R., Mill Hill NW7 1JJ (GB); Kocken, Clemens H.M., 2751 DZ Moerkapelle (NL); Thomas, Alan, 2771 JB Boskoop (NL)
(74) Representative: Warcoin, Jacques

(57) **Abstract**

The present invention relates to a crystal of the ectoplasmic domain of Apical Membrane Protein 1 (AMA1) from *Plasmodium* and a method of crystallisation of such a protein. The invention is also directed to the crystal structure of this AMA1 domain and to the three-dimensional structure of the domain itself, particularly of AMA1 ectodomain from *Plasmodium vivax* and *Plasmodium falciparum*. The present invention also provides methods for identifying ligands of this AMA1 ectodomain using the said crystal structure, and to design pharmaceutical compounds capable of interacting or binding to that domain, particularly to certain amino-acid residues of domain I and II identified as contributing to a relevant epitope of that domain, or for identifying vaccine or drug candidates that could be used to inhibit erythocytes invasion by, and/or growth inhibition of, Plasmodial parasites and that can used in pharmaceutical compositions for the prevention or treatment of malaria. The present invention also concerns peptidic fragments on that AMA1 ectodomain, their coding nucleic acid and their uses for the preparation of immunogens or vaccines for the treatment or the prevention of Plasmodial infection.

## Description

The present invention relates to a crystal of the ectoplasmic domain of Apical Membrane Protein 1 (AMA1) from *Plasmodium* and a method of crystallisation of such a protein. The invention is also directed to the crystal structure of this AMA1 domain and to the three-dimensional structure of the domain itself, particularly of AMA1 ectodomain from *Plasmodium vivax* and *Plasmodium falciparum.* The present invention also provides methods for identifying ligands of this AMA1 ectodomain using the said crystal structure, and to design pharmaceutical compounds capable of interacting or binding to that domain, particularly to certain amino-acid residues of domain I and II identified as contributing to a relevant epitope of that domain, or for identifying vaccine or drug candidates that could be used to inhibit erythocytes invasion by, and/or growth inhibition of, Plasmodial parasites and that can used in pharmaceutical compositions for the prevention or treatment of malaria. The present invention also concerns peptidic fragments on that AMA1 ectodomain, their coding nucleic acid and their uses for the preparation of immunogens or vaccines for the treatment or the prevention of Plasmodial infection.

Apical Membrane Antigen 1 (AMA1) is a type 1 transmembrane protein originally identified in *Plasmodium knowlesi* (Deans et al., 1982; Deans et al., 1984) and subsequently found in a wide range of Plasmodial species (Waters et al, 1990) and other members of the Apicomplexan phylum, such as *Toxoplasma* (Donahue et al., 2000; Hehl et al., 2002) and *Babesia* (Gaffar et al., 2004). The protein is present in the microneme organelles of the developing merozoite of the malaria parasite prior to schizont rupture (Healer et al., 2002, Bannister et al., 2003). At or around the time of schizont rupture, it relocalises to the merozoite surface (Narum & Thomas, 1994). Antibodies raised to AMA1 can inhibit erythrocyte invasion (Thomas et al., 1984; Kocken et al., 1998; Hodder et al., 2001), strongly suggesting that this surface antigen plays a critical, although as yet uncharacterised, role during the blood stage of the parasite's life cycle. Indeed, vaccination with recombinant AMA1 confers protective immunity to malaria in animal model systems, and the antigen from *Plasmodium falciparum* is a leading vaccine candidate that is currently undergoing clinical trials.

AMA1 exhibits a primary structure that is well conserved between different species of *Plasmodium*. The gene encodes a signal sequence and prosequence in the N-terminal region of the protein, followed in succession by an ectoplasmic region, a transmembrane region and a small cytoplasmic domain. The ectoplasmic region carries a characteristic signature of 16 cysteine residues that form a defined set of intramolecular disulphide bridges in the native protein (Hodder et al., 1996). The pattern of disulphide bonding has suggested the division of the primary structure into three discrete regions termed domains I, II and III, respectively. Sequence identity within the ectoplasmic region between *P. falciparum* and *P. vivax*, the two dominant human malaria parasites, is 59%. Unlike many other Plasmodial surface antigens, AMA1 has no sequence repeats; nevertheless, it is highly polymorphic, with much of the variability residing in domain I (Thomas et al., 1990; Marshall et al., 1996; Polley & Conway, 2001). Since the ectoplasmic region of AMA1 is a promising malaria vaccine candidate, it is important to relate the location of protecting epitopes and the distribution of polymorphic sites to the three-dimensional structure of the molecule.

Therefore, there is a need in the art to provide a crystal structure and to elucidate the three-dimensional structures of the ectoplasmic region of AMA1 protein of Apicomplexan parasites such as Plasmodial parasites and to use such structures or model structures in therapeutic strategies, such as drug design and/or to identify anti-*Plasmodium* vaccine candidates capable of inhibiting erythrocytes infection. This is the object of the present invention.

The inventors have determined the crystal structure of a recombinant protein corresponding to ectoplasmic region of AMA1 from *Plasmodium vivax* (PvAMA1). Owing to the conserved nature of AMA1 primary structure, the crystal structure of PvAMA1 allows the prediction of three-dimensional structure of AMA1 from other Plasmodial species and other Apicomplexa. It also allows the partial prediction of the structure of other Apicomplexan molecules that show homology to AMA1, such as MAEBL (Kappe et al. 1998) From analysis of the three-dimensional structure of AMA1, alternative constructions that might optimise the design of both vaccine candidates and potentially novel drugs against Apicomplexan parasites become evident. In particular, the inventors have identified a region of the protein that is potentially important for its biological function by correlating the crystal structure of AMA1 with the epitope mapping of an invasion-inhibiting monoclonal antibody.

So, in a first aspect, the present invention is directed to a crystal of a protein wherein the said protein comprises or consists of the ectoplasmic domain of Apical Membrane Protein 1 (AMA1 of Apicomplexan parasites, preferably selected from the group consisting of *Plasmodium, Toxoplama* and *Babesia.*

In a preferred embodiment the said crystallized protein according to the present invention does not contain the signal sequence, the pro-sequence of the N-terminal region, the transmembrane region and the cytoplasmic domain of the AMA1 protein.

In another preferred embodiment the said crystal according to the present invention has a monoclinic form or an orthorhombic form.

In another preferred embodiment the said crystal according to the present invention, wherein the said *Plasmodium* is selected from the group consisting of *Plasmodium vivax* or *Plasmodium falciparum.*

In another preferred embodiment the said crystal according to the present invention, wherein the said *Plasmodium vivax* is the Sa1 I strain.

In another preferred embodiment the said crystal according to the present invention, wherein the said crystal effectively diffracts X-rays for the determination of the atomic coordinates of the crystallized protein.

In another preferred embodiment the said crystal of a protein according to the present invention, wherein the ectoplasmic domain of the AMA1 protein from *Plasmodium* is a recombinant protein, preferably expressed in *Pichia pastoris.*

In another preferred embodiment the said crystal of a protein according to the present invention, wherein the ectoplasmic domain of AMA1 protein from *Plasmodium* is a recombinant protein which has been purified by a method comprising a step of purification on an anion-exchange column and/or a step of metallo-affinity purification.

In another preferred embodiment the said crystal of a protein according to the present invention, characterized in that the said ectoplasmic domain of the AMA1 protein is a recombinant protein wherein the potentiel N-glycosylation sites of the wild-type AMA1 protein have been mutated.

In another preferred embodiment the said crystal of a protein according to the present invention, wherein the buffer used for the step of crystallisation comprises or consists of 10-12 % (w/v) PEG3350, 100-200 mM imidazole, pH 7.0, 5-10 % (v/v) isopropanol and 1 % (v/v) DMF or 3% (v/v) t-butanol.

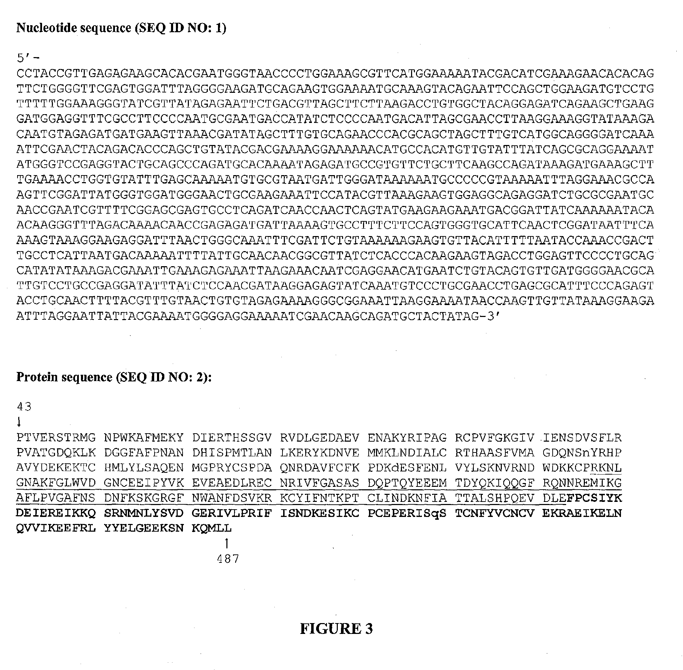

In another preferred embodiment the said crystal of a protein according to the present invention, wherein the said crystallized protein comprises or consists of:
a) the protein having the sequence of the ectoplasmic domain of AMA1 protein from *Plasmodium vivax* strain Sa1 I depicted in the figure 3 or encoded by the nucleic sequence depicted in figure 3, or a protein having an identity of sequence of at least 50 %, preferably, 55 %, 59 %, 60 %, 65 %, 70, %, 80 %, 85 %, 90 %, 95 %, 98 % or 99 %, with the ectoplasmic domain of AMA1 protein from *Plasmodium vivax* strain Sa1 I depicted in the figure 3 ;
b) the protein having the sequence of the ectoplasmic domain of AMA1 protein from *Plasmodium falciparum,* the said sequence consisting of the residues between Glu98 and Met545 of the amino-acid sequence depicted in GenBank under the Accession Number U84348, or a protein having a sequence with an identity of at least 50 %, preferably, 55 %, 59 %, 60 %, 65 %, 70, %, 80 %, 85 %, 90 %, 95 %, 98 % or 99 %, with the sequence of the ectoplasmic domain of *Plasmodium falciparum* AMA1 protein.

By protein having an identity sequence of at least 50 %, preferably, 55 %, 59 %, 60 %, 65 %, 70, %, 80 %, 85 %, 90 %, 95 %, 98 % or 99 %, with the ectoplasmic domain of AMA1 protein from *Plasmodium vivax* strain Sa1 I or from *Plasmodium falciparum* (Accession Number U84348), it is intented to particularly designated the sequences of ectoplasmic domain of AMA1 wild type protein from other *Plasmodium vivax* or *Plasmodium falciparum* strains having natural polymorphisms compared with these two sequences and wherein, optionally, the potential N-glycosylation sites of the wild type AMA1 protein have been mutated.

Two amino-acids or nucleotidic sequences are said to be "identical" if the sequence of amino-acids or nucleotidic residues in the two sequences is the same when aligned for maximum correspondence. Sequence comparisons between two peptides or polynucleotides are typically performed by comparing sequences of two optimally aligned sequences. Optimal alignment of sequences for comparison may be conducted by the local homology algorithm of Smith and Waterman, Ad. App. Math 2: 482 (1981), by the homology alignment algorithm of Neddleman and Wunsch, J. Mol. Biol. 48:443 (1970), by the search for similarity method of Pearson and Lipman, Proc. Natl. Acad. Sci. (U.S.A.) 85:2444 (1988), by computerized implementation of these algorithms (GAP, BESTFIT, FASTA, and TFASTA in the Wisconsin Genetics Software Package, Genetics Computer Group (GCG), 575 Science Dr., Madison, WI), or by visual inspection.

"Percentage of sequence identity" ( or identity) is determined by comparing two optimally aligned sequences over a comparison window, where the portion of the peptide or polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical amino-acid residue or nucleic acid base occurs in both sequences to yield the number of matched positions, dividing the number of matched positions by the total number of positions in the window of comparison and multiplying the result by 100 to yield the percentage of sequence identity. The definition of sequence identity given above is the definition that would use one of skill in the art. The definition by itself does not need the help of any algorithm, the said algorithms being helpful only to achieve the optimal alignments of sequences, rather than the calculation of sequence identity.

From the definition given above, it follows that there is a well defined and only one value for the sequence identity between two compared sequences which value corresponds to the value obtained for the best or optimal alignment.
In the BLAST N or BLAST P " BLAST 2 sequence" (Tatusova et al., "Blast 2 sequences - a new tool for comparing protein and nucleotide sequences", FEMS Microbiol. Lett. 174:247-250) software that is available in the web site http://www.ncbi.nlm.nih.gov/gorf/bl2.html, and habitually used by the inventors and in general by the skilled man for comparing and determining the identity between two sequences. The "open gap penalty" and « extension gap penalty » parameters that depends on the substitution matrix selected regarding the nature and the length of the sequence to be compared is directly selected by the software (i.e "5" and "2" respectively for substitution matrix BLOSUM-62). The identity percentage between the two sequences to be compared is directly calculated by the software.

In another preferred embodiment the said crystal of a protein according to the present invention, wherein the said crystal has the followings parameters, or which substantially conforms to these parameters, for the native crystal:
a) Space group: P2₁2₁2₁,
   Dimensions of the unit cell:
   a (Å): 54.10,
   b (Å): 76.10,
   c (Å):103.93, wherein a variability of ±2.5% in the said dimensions can be anticipated depending on the conditions under which the crystal is manipulated, and
   Resolution (Å): better than 3.0 Å,
   when the crystallized protein is the ectoplasmic domain of AMA1 protein from *Plasmodium vivax* strain Sa1 I that has been crystallized in the orthorhombic form; or
   b) Space group: C2,
   Dimensions of the unit cell:
   a (Å): 150.03,
   b (Å): 58, 83,
   c (Å): 60.29,
   β (°): 113.2 , wherein a variability of ±2.5% in the said dimensions can be anticipated depending on the conditions under which the crystal is manipulated, and
   Resolution (Å): better than 3.0 Å,
   when the crystallized protein is the ectoplasmic domain of AMA1 protein from *Plasmodium vivax* strain Sa1 I which has been crystallized in the monoclinic form.

In another preferred embodiment the said crystal of a protein according to the present invention has the characteristics as set forth in the Table 3.

One embodiment of the present invention is a method to derive a model of the three-dimensional structure of a target structure of the ectoplasmic domain of AMA1 protein from Plasmodial parasite species other than *Plasmodium vivax*, the method comprising the steps of:
a) providing the amino acid sequence of the target ectoplasmic domain of AMA1 protein from Plasmodial parasite species other than *Plasmodium vivax*;
b) identifying structurally conserved regions shared between the amino acid sequence of the ectoplasmic domain of AMA1 protein from *Plasmodium vivax*, such as the amino acid sequence depicted in figure 3, and the amino acid sequence of said target ectoplasmic domain of AMA1 protein;
c) determining the atomic coordinates for the target ectoplasmic domain of AMA1 protein by assigning the said structurally conserved regions of the target ectoplasmic domain of AMA1 protein to a three-dimensional structure using a three-dimensional structure of the ectoplasmic domain of AMA1 protein from *Plasmodium vivax* based on atomic coordinates that substantially conform to the atomic coordinates as set forth in the Table 6 or 7, to derive a model of the three-dimensional structure of the target ectoplasmic domain of AMA1 protein.

The characteristics of a derived model according to the present invention has been described in Table 8 for the three-dimensional structure of the ectoplasmic domain of AMA1 protein of *Plasmodium falciparum* having the amino acid sequence as depicted in GenBank, Accession Number U84348.

In another preferred embodiment the said crystal of a protein according to the present invention, wherein the said crystal has:
- the three-dimensional structure that substantially conforms to the atomic coordinates as set forth in the Table 6 when the crystallized protein is the ectoplasmic domain of AMA1 protein from *Plasmodium vivax* strain Sa1 I which has been crystallized in the orthorhombic form;
- the three-dimensional structure that substantially conforms to the atomic coordinates as set forth in the Table 7 when the crystallized protein is the ectoplasmic domain of AMA1 protein from *Plasmodium vivax* strain Sa1 I which has been crystallized in the monoclinic form.

In another prefered embodiment, a model of a protein is derived by homology modelling from a crystal of a protein, wherein the said model has the characteristics of the ectoplasmic domain of the AMA1 protein from *Plasmodium falciparum* strain FVO and the said crystal is the ectoplasmic domain of the AMA1 protein from *Plasmodium vivax* strain SalI.

In a second aspect, the present invention is directed to a method for obtaining a crystal of a protein having a monoclinic or an orthorhombic form wherein the said protein comprises or consists of a recombinant ectoplasmic domain of AMA1 from *Plasmodium* comprising the step of:
a) purifying the said recombinant ectoplasmic domain of AMA1 by a method comprising a step of purification on an anion-exchange column and/or a step of metallo-affinity purification;
b) growing the crystal comprising or consisting of the said recombinant ectoplasmic domain of AMA1 protein by hanging drop technique wherein the crystallising buffer used for the step of crystallisation comprises or consists of 10-12% (w/v) PEG3350, 100-200 mM imidazole, pH 7.0, 5-10 % (v/v) isopropanol and 1 % DMF (v/v) or 3 % (v/v) t-butanol.

In another embodiment, the invention relates to a method for preparing a crystal for diffraction measurements, the said crystal comprising or consisting of a recombinant ectoplasmic domain of AMA1 protein of *Plasmodium,* characterized in that the said method comprises the step of:
a) obtaining the said crystal by a method for obtaining a crystal of a protein having a monoclinic or an orthorhombic form according to the present invention; and
b) flash-freezing the said crystal in liquid nitrogen after a brief transfer to a cryo-protecting buffer comprising or consisting of 30 % (w/v), 100-200 mM imidazole, pH 7.0, 5-10 % (v/v) isopropanol and 1 % DMF (v/v) or 3 % (v/v) t-butanol.

In another embodiment, the invention relates to a method for obtaining a platinium derivative of a crystal a protein wherein the said protein comprises or consists of a recombinant ectoplasmic domain of AMA1 protein from *Plasmodium,* characterized in that the said method comprises the step of:
a) obtaining the said crystal by a method for obtaining a crystal of a protein having a monoclinic or an orthorhombic form according to the present invention; and
b) soaking the said crystal in a solution of potassium tetrachloroplatinate(II) at a concentration of 2 mg/ml in a buffer of 30 % (w/v) PEG 3350, 100 mM sodium cacodylate, 10 % (v/v) isopropanol, pH 7.0, for 24 hours; and
c) flash-freezing the crystals in liquid nitrogen without back-washing, using a cryo-buffer containing PEG 3350 at 30 % (v/v).

In a preferred embodiment, in the said methods for obtaining a crystal of a protein having a monoclinic or an orthorhombic form according to the present invention or for preparing a crystal for diffraction measurements according to the present invention or for obtaining a platinium derivative of a crystal according to the present invention:
- the crystallized protein does not contain the signal sequence, the pro-sequence of the N-terminal region, the transmembrane region and the cytoplasmic domain of the AMA1 protein; and/or
- the said *Plasmodium* is selected from the group consisting of *Plasmodium vivax* or *Plasmodium falciparum*; and/or
- wherein the said *Plasmodium* is *Plasmodium vivax* strain Sal I
- wherein the said ectoplasmic domain of the AMA1 recombinant protein from *Plasmodium* is a recombinant protein expressed in *Pichia pastoris*; and/or
- characterized in that the said ectoplasmic domain of the AMA1 recombinant protein is a recombinant protein wherein the potentiel N-glycosylation sites of the wild-type AMA1 protein have been mutated; and/or
- wherein the said crystallized recombinant protein has the sequence of the ectoplasmic domain of AMA1 protein from *Plasmodium vivax* strain Sa1 I depicted in the figure 3 or encoded by the nucleic acid sequence depicted in figure 3, or a protein having an identity of sequence of at least 50 % with the ectoplasmic domain of AMA1 protein from *Plasmodium vivax* strain Sal I depicted in the figure 3, or the protein having the sequence of the ectoplasmic domain of AMA1 protein from *Plasmodium falciparum,* the said sequence consisting of the residues between Glu98 and Met545 of the sequence depicted in GenBank under the Accession Number U84348, or a protein having a sequence with an identity of at least 50 % with the sequence of this ectoplasmic domain of *Plasmodium falciparum* AMA1 protein.

Preferably, a crystal of the ectoplasmic domain of AMA1 protein from *Plasmodium* is produced using the crystal formation method described above, in particular according to the method disclosed in Example 3. An ectoplasmic domain of AMA1 protein crystal of the present invention can comprise any crystal structure and preferably precipitates as an orthorhombic or a monoclinic crystal.

The crystal which has been obtained or obtainable by the methods according to the invention forms also part of the present invention.

In another aspect, the present invention relates to a computer-readable data storage material encoded with computer-readable data comprising the atomic structure coordinates of the crystal according to the present invention, preferably the three-dimensional structure of the ectoplasmic domain of AMA1 protein from *Plasmodium vivax*, orthorhombic and monoclinic form, and the three-dimensional structure of the ectoplasmic domain of AMA1 protein from *Plasmodium falciparum* which substantially conforms to the atomic coordinates represented by the Tables 6-8 respectively.

According to the present invention, the crystal of the ectoplasmic domain of AMA1 protein from *Plasmodium* can be used to determine the ability of a compound to bind to the said ectoplasmic domain of AMA1 protein from *Plasmodium,* wherein this ability is predicted by a structure-based, drug-design method that is based on the three-dimensional structure of the ectoplasmic domain of AMA1 protein from *Plasmodium* of the present invention. The predicted binding of the chemical compound to the crystal of the present invention is determined by methods standard in the art.

Potential compounds which can bind the ectoplasmic domain of AMA1 protein from *Plasmodium* and so can inhibit the erythrocyte invasion by, or growth of, *Plasmodium* can be designed using structure-based drug design. Until the discovery of the three-dimensional structure of the present invention, no information was available for structure-based development of therapeutic compounds based on the structure of the ectoplasmic domain of AMA1 protein from *Plasmodium.* Such rational development could not be executed *de novo* from available linear amino acid sequence information. Structure-based drug design refers to the use of computer simulation to predict a conformational interaction between the three-dimensional AMA1 ectodomain structure of the present invention and a potential therapeutic compound. For example, generally, for a protein to effectively interact with a therapeutic compound, it is necessary that the three-dimensional structure of the therapeutic compound assume a compatible conformation that allows the compound to bind to the protein in such a manner that a desired result is obtained upon binding. Knowledge of the three-dimensional structure of the protein enables a skilled artisan to design a therapeutic compound having such compatible conformation.

So, in another aspect, the present invention relates to a method for identifying or an assay for screening a potential inhibitor for inhibiting the erythrocyte invasion of mammal, preferably a human, by *Plasmodium* comprising:
a) selecting a potential inhibitor by performing rational drug design with the three-dimensional structure determined for the crystal according to the present invention, or by using the computer-readable data storage material according to the present invention, said selecting being optionally performed in conjunction with computer modeling.

In a preferred embodiment, the method for identifying or an assay for screening a potential inhibitor according to the present invention, further comprises:
b) growing a supplemental crystal comprising a protein-ligand complex formed between the ectoplasmic domain of AMA1 protein from *Plasmodium* and the said potential inhibitor from step (a), wherein the supplemental crystal effectively diffracts X-rays for the determination of the atomic coordinates of the protein-ligand complex to a suitable resolution; and
c) determining the three-dimensional structure of the supplemental crystal.

In a preferred embodiment, the method for identifying or an assay for screening a potential inhibitor according to the present invention, further comprises:
d) determining from the three-dimensional structure of the supplemental crystal obtained in step c) whether this potential inhibitor is bound to said ectoplasmic domain AMA1 protein.

Preferably, eight individual sites and sites contained within the 25-residue deletion of the ectoplasmic domain AMA1 protein are targets for structure-based drug design. These sites include the amino acid residues situated in position aa280, aa348, aa351, aa352, aa356, aa364 aa388, aa389, and within the segment aa353-377 of the ectoplasmic domain of the *Plasmodium falciparum* AMA1 protein, which corresponds to the position aa225, aa293, aa296, aa297, aa301, aa309, aa333, aa334 and residues within the segment aa298 to 322 of the AMA1 ectoplasmic domain of *Plasmodium vivax.* The residues of AMA1 protein from *P*. *vivax* are numbering by reference to the sequence of Accession Number AAC16731 (SEQ ID NO: 4). The residues of AMA1 protein from *P*. *falciparum* are numbering by reference to the sequence of Accession Number AAD03790 (SEQ ID NO: 5).

In a more preferred embodiment, the method for identifying or an assay for screening a potential inhibitor according to the present invention, wherein in step d), it is determined whether this potential inhibitor is bound to at least one the amino-acid residue of said ectoplasmic domain AMA1 protein selecting from the group of residues consisting of:
- the amino acid residues situated in position aa225, aa293, aa296, aa297, aa301, aa309, aa333, aa334 and residues within the segment aa298 to 322 of the ectoplasmic domain of the *Plasmodium vivax* strain Sa1 I AMA1 protein when the protein of the said protein-ligand complex is derivated from *Plasmodium vivax* AMA1 protein; and
- the amino acid residues situated in position aa280, aa348, aa351, aa352, aa356, aa364 aa388, aa389, and within the segment aa353-377 of the ectoplasmic domain of the *Plasmodium falciparum* AMA1 protein when the protein of said protein-ligand complex is derived from *Plasmodium falciparum* AMA1 protein; and
- the amino acid residues situated at a position corresponding to the position aa280, aa348, aa351, aa352, aa356, aa364 aa388, aa389, and within the segment aa353-377 of the ectoplasmic domain of the *Plasmodium falciparum* AMA1 protein, when the ectoplasmic domain AMA1 protein of the said protein-ligand complex is derived from another *Plasmodium* species than *Plasmodium falciparum* or *Plasmodium vivax*.

In an also more preferred embodiment, the method for identifying or an assay for screening a potential inhibitor according to the present invention, further comprising:
e) administrating the potential inhibitor to a non-human animal model which has been infected by *Plasmodium* or contacting *in vitro* the potential inhibitor with a cell model by using a cellular-based assay capable of reproducing the invasion of erythrocytes by *Plasmodium;* and
f) detecting the ability of the potential inhibitor for inhibiting the erythrocyte invasion.

It is also preferred the method for identifying or an assay for screening a potential inhibitor according to the present invention, wherein in step (a) the selection or the design of a potential inhibitor is performed by a rational drug design according to the three-dimensional structure of the ectoplasmic domain of AMA1 protein from *Plasmodium vivax,* orthorhombic or monoclinic form, or according to the three-dimensional structure of the ectoplasmic domain of AMA1 protein from *Plasmodium falciparum* which substantially conforms to the atomic coordinates represented by the Tables 6-8 respectively, optionally in conjunction with computer modeling.

In the method for identifying or an assay for screening a potential inhibitor according to the present invention, the said potential inhibitor is designed *de novo* or designed from a known inhibitor.

In another aspect, the present invention also includes a therapeutic composition that, when administered to a non-human mammal animal or a human, is capable of preventing or treating Plasmodial parasite infection, prefeably *Plasmodium vivax* or *Plasmodium falciparum* infection, the said therapeutic composition comprising a compound that binds to the ectoplasmic domain of AMA1 protein from that Plasmodial parasite, the said compound being identified by the method comprising:
a) using the three-dimensional structure of the ectoplasmic domain of AMA1 protein from that Plasmodial parasite obtained from a crystal of that protein according to the present invention to design a chemical compound that binds to the ectoplasmic domain of that AMA1 protein;
b) chemically synthesizing the said compound or obtaining it from a provider if this compound exists; and
c) evaluating the ability of the said compound to inhibit the erythrocyte invasion by the said Plasmodial parasite.

According to the invention, the protein having the sequence of the domain I and II of the ectoplasmic domain of Apical Membrane Protein 1 (AMA1) from *Plasmodium,* is recognized by the monoclonal antibody 4G2, particularly the epitope corresponding to the sites including the positions aa280, aa348, aa351, aa352, aa356, aa364 aa388, aa389, and within the segment aa353-377 of the ectoplasmic domain of the *Plasmodium falciparum* AMA1 protein when the protein is derived from *Plasmodium falciparum* AMA1 protein. Therefore the polypeptide including these sites, particularly the polypeptide including the positions aa348, aa351, aa352, aa356, aa364 aa388, aa389, and within the segment aa353-377 (or their equivalent in the AMA1 ectoplamic domain of other *Plasmodium* species, are suitable for testing as a vaccine against malaria infection in mammals.

So, in another aspect, the present invention comprises a purified or isolated polypeptide selected from the group of polypeptides constisting of the following polypeptides:
a) - the polypeptide (reference polypeptide) having the sequence of the domains I and II of the ectoplasmic domain of Apical Membrane Protein 1 (AMA1) from *Plasmodium,* wherein the signal sequence, the pro-sequence of the N-terminal region, the transmembrane region and the cytoplasmic domain of the AMA1 protein have been deleted, or
   - a homologous polypeptide thereof having a sequence presenting an identity of at least 50 % with that domains I and II sequence; and
b) a fragment of polypeptide as defined in a), the said fragment having at least 10 amino-acid residues and comprising at least one amino-acid residue of the said ectoplasmic domain AMA1 protein selecting from the group of residues consisting of:
   - the amino acid residues situated in position aa225, aa293, aa296, aa297, aa301, aa309, aa333, aa334 and residues within the segment aa298 to 322 of the ectoplasmic domain of the *Plasmodium vivax* AMA1 protein when the AMA1 protein is derived from *Plasmodium vivax* AMA1 protein; and
   - the amino acid residues situated in position aa280,aa348, aa351, aa352, aa356, aa364 aa388, aa389, and within the segment aa353-377 of the ectoplasmic domain of the *Plasmodium falciparum* AMA1 protein when the protein is derivated from *Plasmodium falciparum* AMA1 protein; and
   - the amino acid residues situated at a position corresponding to the position aa280, aa348, aa351, aa352, aa356, aa364 aa388, aa389, and within the segment aa353-377 of the ectoplasmic domain of the *Plasmodium falciparum* AMA1 protein, when the ectoplasmic domain AMA1 protein is derived from another *Plasmodium* species than *Plasmodium falciparum* or *Plasmodium vivax.*

In a preferred embodiment, the polypeptide according to the present invention is characterized in that it is a recombinant protein, and, optionally, wherein at least one of the potentiel N-glycosylation sites of the wild-type AMA1 protein ectoplasmic domains I and II have been mutated.

In a more preferred embodiment, the polypeptide according to the present invention is characterized in that it is a fragment of the ectoplasmic domains I and II and wherein the said fragment includes at least one or more of:
- the amino acid residues situated in position aa293, aa296, aa297, aa301, aa309, aa333, aa334 and residues within the segment aa298 to 322 and, optionally, aa225, of the ectoplasmic domain of a *Plasmodium vivax* AMA1 protein when the AMA1 protein is derived from *Plasmodium vivax* AMA1 protein; or
- the amino acid residues situated in position aa348, aa351, aa352, aa356, aa364 aa388, aa389, and within the segment aa353-377, and, optionally, aa280, of the ectoplasmic domain of a *Plasmodium falciparum* AMA1 protein when the protein of said protein-ligand complex is derived from *Plasmodium falciparum* AMA1 protein; or
- the amino acid residues situated at a position corresponding to the position, aa348, aa351, aa352, aa356, aa364 aa388, aa389, and within the segment aa353-377, and, optionally, aa280, of the ectoplasmic domain of the *Plasmodium falciparum* AMA1 protein, when the ectoplasmic domain AMA1 protein of said protein-ligand complex is derived from another *Plasmodium* species than *Plasmodium falciparum* or *Plasmodium vivax*.

Are preferred the recombinant polypeptides according to the present invention.

For the production of the polypeptides of the present invention, it is possible to have recourse to genetic engineering procedures making use of micro-organisms transformed by a specific nucleic acid encoding the polypeptide.

Consequently, forms also part of the present invention the purified or isolated nucleic acid, such as DNA or RNA, encoding the polypeptide according to the invention.

Are also included in the present invention, cloning or expression vectors encoding the polypeptide according to invention, and the host cells transformed by said vectors.

A vector of the present invention can be either prokaryotic or eukaryotic vector, such as a plasmid or a virus. Expression vectors of the present invention include any DNA or RNA vectors that direct the expression of the recombinant polypeptide of the invention in the host cell, including bacterial, fungal, yeast, insect, other animal such as mammal, and plant cells.

An expression vector of the present invention can be transformed into any suitable host cell to form a recombinant cell. A suitable host cell of the present invention includes any cell capable of expressing a nucleic acid of the present invention inserted into the expression vector. For example, a prokaryotic expression vector can be transformed into a bacterial host cell.

In another aspect, the present invention relates to the polypeptide or recombinant polypeptide according to invention as a medicament, as an immunogen or as a vaccine.

Antibodies, monoclonal, polyclonal, chimeric or humanized antibodies, which are specifically directed against the polypeptide of the present invention are comprised in the invention.

In another aspect, the present invention relates to composition for stimulating an immune response against infection by a Plasmodium species in a mammal, said composition comprising a polypeptide or an acid nucleic according to the invention.

The said *Plasmodium* species can be selected from the group consisting of *Plasmodium falciparum*, *Plasmodium vivax*, *Plasmodium malariae*, *and Plasmodium ovale.*

Preferably, the said polypeptide or the said nucleic acid according to the invention are formulated as a vaccine in a pharmaceutically effective carrier.

The present invention is also directed to a method of protecting a mammalian subject against infection by malaria caused by *Plasmodium* species selected from the group consisting of *Plasmodium falciparum* and *Plasmodium vivax,* the said method comprising administering to the said mammal the polypeptide or the nucleic acid according to the invention.

The invention also relates to compositions comprising the polypeptides according to the invention with carrier molecules (natural or synthetic), physiologically acceptable and non-toxic, such as for example tetanus toxoid, ovalbumin, serum albumin, hemocyanins.

Is also comprises in the present invention, the use of the polypeptide according to the invention for the manufacture of a composition for the prevention or the treatment of malaria.

These polypeptides or immunogens according to the invention are capable of triggering in vivo the synthesis of specific immunoglobulins, particularly directed against the antigenic epitope of the 4G2 antibody and are capable of inducing in vivo the neutralization of the merozoite present in the blood.

In another aspect, the crystal structure of PvAMA1 according to the present invention allows or can be used in a method allowing:
- a precise definition of the structural limits of the three domains in terms of organisation of the polypeptide chain and the segments of secondary structure such as helices and beta-strands. Domains and/or sub-domains of the ectodomain that preserve the native conformation of AMA1 can be defined and chosen as potential vaccine constructions.
- modelling by homology of the ectodomain of AMA1 from Plasmodium falciparum, from other *Plasmodium* species, from other Apicomplexan parasites.
- modelling by homology of other Apicomplexan molecules, such as MAEBL, that share significant amino acid sequence identity with AMA1.

The crystal structure of PvAMA1 according to the present invention and other homology-modelled structures of AMA1 according to the invention can be used in a method to define the three-dimensional distribution of polymorphic residues of the ectodomain.

The crystal structure of PvAMA1 and homology-modelled structures of related proteins according to the present invention can be used in a method to define regions of AMA1 and homology-related proteins that are surface-exposed and therefore suitable for development as vaccines.

The crystal structure of PvAMA1 and homology-modelled structures of related proteins according to the present invention can be used in a method to define regions of AMA1 and homology-related proteins that are surface exposed and therefore potential targets for the development of drugs that block the function of that protein.

The crystal structure of PvAMA1 proteins according to the present invention can be used in a method to search for proteins and protein domains of known three-dimensional structure to identify potential receptors and/or ligands that might bind to AMA1.

The crystal structure of PvAMA1 and homology-modelled structures of related proteins according to the present invention can be used in a method to define the three-dimensional distribution of conserved residues of the ectodomain.

The crystal structure of PvAMA1 and homology-modelled structures of related proteins according to the present invention can be used in a method to define regions of Domain II that induce antibodies and, although having no effect on the protein's function, block access of antibodies to the functionally important epitope region of AMA1 defined by mAb 4G2.

The crystal structure of AMA1 proteins according to the present invention can be used in a method to find the three-dimensional disposition of epitopes on AMA1 against which immune rersponses that inhibit AMA1 function can be targeted.

Epitope mapping of the invasion-inhibiting monoclonal antibody 4G2 has defined a functionally important region located on both Domain I and Domain II of AMA1 from *P. falciparum* that can be related to the crystal structure of PvAMA1, important polypeptidic region, as defined in the present description, which is comprised in the invention.

Epitope and domain mapping of the invasion-inhibiting monoclonal antibody 4G2 has defined a functionally important region on and/or near the Domain II loop of AMA1 that requires association with (regions of) Domain I to create the 4G2 epitope.

The present invention relates to a vaccine comprising as immunogen the polypeptide having the sequence of the domains I and II of the AMA1 protein, or fragment thereof as defined in the set of claims.

The following figures and their legends are illustrative of embodiments of the present invention and are not meant to limit the scope of the invention as encompassed by the claims.

### Description of the Figures

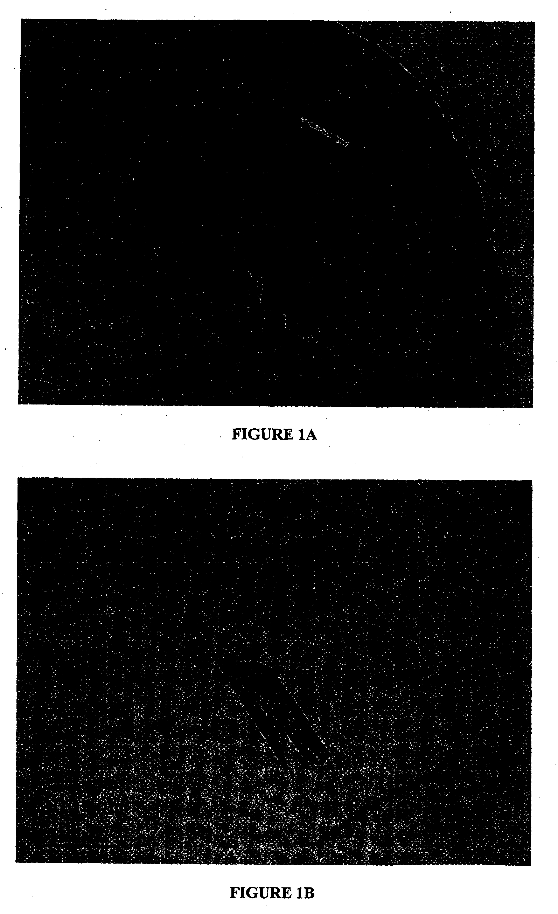

**Figures 1A and 1B.** Crystals of ectoplasmic domain of PvAMA1
Figure 1A: Orthorhombic form.
Figure 1B: Monoclinic form. (A Scale is shown in each case)
**Figures 2A and 2B.** Phasing statistics for the platinum derivative of PvAMA1 crystals.

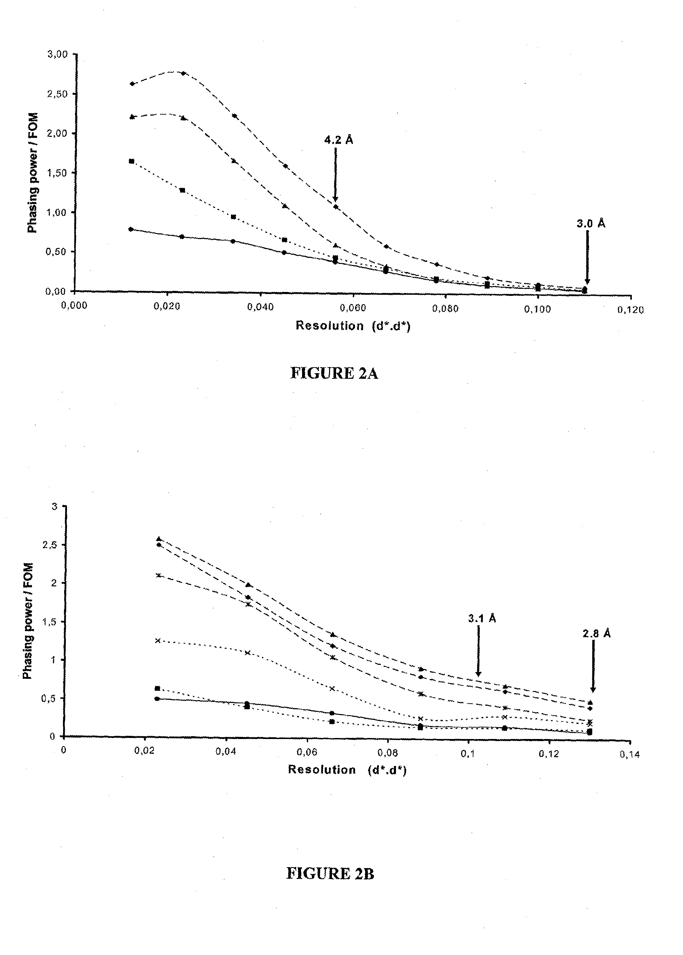

Figure 2A: Orthorhombic form. Phasing power (< |F_{H}(calc)| >/< phase integrated lack of closure >) as a function of resolution (d*²): inflection anomalous (▲), remote anomalous (◆), remote isomorphous (■); figure of merit: (•). Resolution limits for the heavy atom parameter refinement (4.2 Å) and phase calculation (3.0 Å) are indicated by arrows. d* is the reciprocal of the crystallographic Bragg spacing.
Figure 2B: Monoclinic form. Phasing power as a function of resolution (d^{*2}): inflection anomalous (◆), peak anomalous (▲), remote anomalous ( 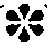 ), peak isomorphous (■), remote isomorphous (**x**); figure of merit, (•). Resolution limits for the heavy atom parameter refinement (3.1 Å) and phase calculation (2.8 Å) are indicated by arrows.
**Figure 3.** Nucleotide (SEQ ID NO: 1) and protein sequence ((SEQ ID NO: 2) of recombinant ectoplasmic domain of PvAMAl.
   The expressed PvAMA1 sequence in *Pichia pastoris* is from residue 43 to 487, inclusive, for the nucleotide and protein sequences. These sequences are identical to those of the Genbank entry Y16950. Three potential N-glycosylation sites have been mutagenised: Ser178Asn, Asn226Asp and Asn441Gln. The expressed sequence also includes the 22-residue sequence encoded by the pPICZaC expression vector (c-myc and hexa-histidine markers, LEQKLISEEDLNSAVDHHHHHH (SEQ ID NO: 3)) C-terminal to the PvAMA1 sequence, and the di-peptide segment Ser-Ile N-terminal to the PvAMA1 sequence. Domain I is indicated in normal font, domain II is indicated by underling and domain III is indicted in bold type. The three potential glycosylation sites that were mutated are indicted by small lettering.
   Note that the first residue of the amino-acid sequence showed in figure 3 correspond to the amino-acid residue locared in position 43 of the complete AMA1 protein (comprising both the sequence signal and the pro-sequence)

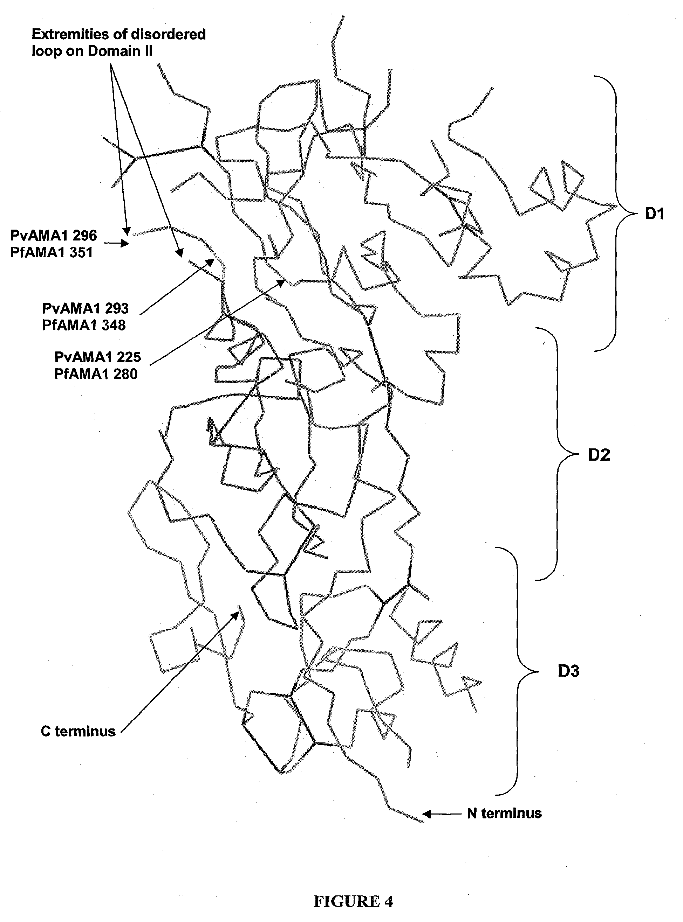

   **Figure 4.** α-carbon skeleton of PvAMA1 of the monoclinic crystal structure
   Approximate limits of Domains I, II and III are indicated by D1, D2 and D3, respectively. The positions of PvAMA1 residues corresponding to PfAMA1 mutations abolishing the binding of the monoclonal antibody 4G2 are indicated, giving the PvAMA1 and PfAMA1 residue numbering, respectively. The other PfAMA1 mutations that abolish 4G2 binding occur in a disordered region of the PvAMA1 crystal structure that could not be modelled; these occur in the 40-residue segment in Domain II (residues 297, 333 and 334).

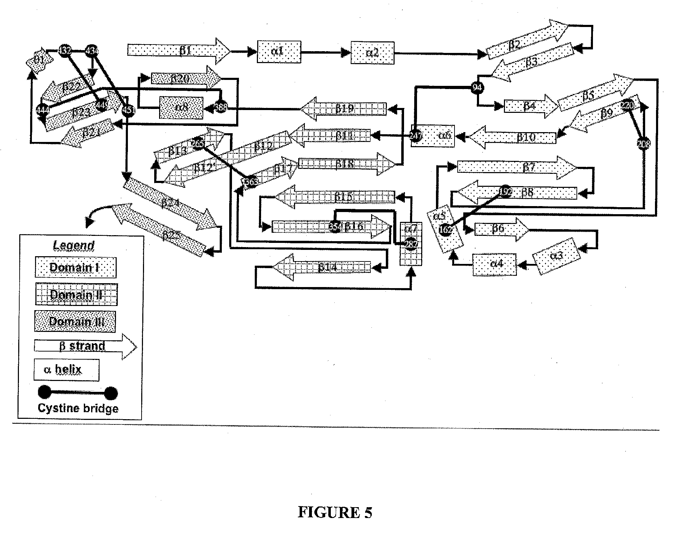

   **Figure 5.** Domain organisation and secondary structure of PvAMA1

### Example 1: Functional domain mapping of PfAMA1

### 1.1. Expression of P. falciparum AMA1 domains in Pichia Pastoris

The *P. falciparum* FVO AMA1 nucleotide sequence was used to develop a synthetic gene utilising the codon usage of *P. pastoris* with the aid of the CODOP programme as described previously (Kocken et al., 2002). N-glycosylation sites were removed following a strategy exploiting the lack of conservation of N-glycosylation sites in AMA1 *of Plasmodium* (Kocken et al., 1999, 2002).

For secreted, methanol-inducible expression in *P. pastoris* strain KM71H (Mut^{s} phenotype) vector pPICZαA (Invitrogen, Groningen, The Netherlands) was used. PCR products were cloned in frame with the vector encoded N-terminal secretion signal and C-terminal myc epitope and hexaHis tag (Kocken et al., 2002). The following clones were constructed that all produce high levels of rec protein in the culture supernatant (Table 1).

**Table 1. Pichia expression of PfAMA1 domains**

| **Clone** | **PfAMA1 residues** | **PfAMA1 domains** |
|---|---|---|
| Pf3mH | 25-442 | Pro-I-II |
| Pf4mH | 25-544 | Pro-I-II-III |
| Pf15mH | 97-442 | I-II |
| Pfl4-0 (lacking myc-His tail) | 97-545 | I-II-III |
| Pf8mH | 303-442 | II |
| Pf9mH | 303-544 | II-III |
| Pf10mH | 419-544 | III |

Clones to express domain I on its own or in combination with the prosequence did not express detectable protein levels, suggesting that domain I by itself is not a stable moiety of the protein and needs domain II for attaining its proper stable structure.

### 1.2. Monoclonal antibody 4G2 reactivity

MAb 4G2 is reactive with a conformation dependent, disulphide bond stabilised epitope and is inhibiting invasion of red blood cells by merozoites (Kocken et al., 1998). Non-reducing Western blot analysis of the expressed domains for reactivity with mAb 4G2 showed that 4G2 is only reactive when domains I and II are present (Pf3mH, Pf4mH, Pf15mH, Pfl4-0). Domain II (Pf8mH), domain III (Pf10mH) and the combination of domain II and III (Pf9mH) are not reactive with 4G2. As expected, under reducing conditions none of the proteins were reactive with 4G2. This means that the epitope for 4G2 requires either domain I alone or the combination of domains I and II.

### 1.3. Reactivity of domain-specific antisera

Antisera were raised in rabbits against Pf3mH, Pf4mH, Pf8mH, Pf9mH and Pf10mH using protein purified to homogeniety by Nickel-chelate chromatography (Kocken et al., 2002). High-titer antisera were obtained with end-point ELISA titers on Pf4mH ranging from 1.2 to 2.5 x 10⁶, and immunofluorescence titers (IFA) ranging from 128 to 256 x 10³. One rabbit immunised with Pf10mH responded lower as tested by IFA to an end-point titer of 32 x 10³.

IgG isolated from rabbit sera was tested for its capacity to inhibit invasion of red blood cells by *P*. *falciparum* merozoites, essentially as described (Kocken et al., 2002). When tested at 1.5 mg/ml total IgG concentration, IgG directed against Pf4mH and Pf3mH showed 55-75% inhibition of invasion of the homologous *P*. *falciparum* strain. IgG directed against Pf8mH, Pf9mH and Pf10mH showed <10% invasion inhibition. This indicates that domains II and III on their own, as well as the combination of domains II and III are not very potent inducers of parasite-inhibitory antibodies. By contrast, the complete ectodomain and domains Pro-I-II (the ectodomain lacking domain III) are equally potent inducers of parasite-inhibitory antibodies. Given that these proteins are also reactive with the inhibitory mAb 4G2 and the minimum epitope for 4G2 has been mapped to domains I-II, it is likely that domains I-II contain the functional region of AMA1 that can be blocked by suitable antibodies.

### 1.4. Blocking of 4G2 binding to AMA ectodomain

To determine whether antibodies against PfAMA1 that were not inhibitory to merozoite invasion of red cells could (presumably through steric hindrance) block the binding of 4G2 to PfAMA1 the following experiment was performed. 4G2 was diluted to yield an OD of 1.0 in an ELISA when tested against recombinant PfAMA1 ectodomain (comprising Prosequence and domains I, II and III). When 4G2 at this dilution was mixed with serum from rabbits (at 1 in 200 dilution) directed against domain III there was no reduction in 4G2 binding to the ectodomain. When mixed with rabbit antibodies (1:200 dilution) directed against domain II alone, or against a domain II/III fusion, 4G2 binding was reduced to OD's of 0.4 and 0.3 respectively. Thus domain II contains epitopes recognised by antibodies that block access to the 4G2 epitope, while these antibodies have themselves no significant anti-parasitic effect. Resolution of the three dimensional structure of AMA1 when combined with this information allows the design of AMA1 molecules that lack non-critical domain II regions, thereby potentially avoiding the induction of non-effector antibodies that blockade the 4G2 epitope.

### Example 2: Epitope mapping of monoclonal antibody 4G2

All expression studies made use of the synthetic *Plasmodium falciparum ama-1* gene called FVO *ama-1syn* described previously (Kocken et al., 2002).

### 2.1. Antibody reagents

Four sets of antibodies were used for this work, as follows:
*1*. *Monoclonal antibody 4G2* (Kocken et al., 1998). This was produced and purified using standard protocols.
*2. Polyclonal mouse antiserum specific for denatured PfAMA1 (antiserum R1).* This serum was raised against highly purified recombinant PfAMA1 (rPfAMA1) ectodomain (domains I+II+III) produced by expression of the corresponding region of the synthetic gene in Pichia pastoris (Kocken et al., 2002). Purified protein was denatured in 8 M urea and alkylated with iodoacetamide, then dialysed extensively prior to being used to immunise mice. In Western blot analysis the resulting serum is strongly reactive with parasite-derived or rPfAMA1 when it has been subjected to SDS PAGE under either reducing or non-reducing conditions.
*3. Polyclonal mouse antiserum specific for 'folded' PfAMA1 (antiserum N1)*. This was raised against the same highly purified rPfAMA1 ectodomain (domains I+II+III) as used above, but without prior reduction and alkylation of the antigen. Following collection the antiserum was adsorbed extensively against reduced and alkylated antigen in order to produce a reagent which predominantly recognises epitopes unique to folded protein. In Western blots the resulting antiserum reacts with parasite-derived or rPfAMA1 only when it has been subjected to SDS PAGE under non-reducing conditions. It does not react with reduced PfAMA1.
*4. Polyclonal mouse antiserum specific for the cytoplasmic domain of PfAMA1 (mouse anti- AMA1cyt).* This was raised against a recombinant fusion protein corresponding to Arg569-Tyr622 of PfAMA1. Expression was carried out in *Escherichia coli* and the recombinant protein purified to homogeneity by nickel chelate chromatography and gel filtration before immunisation of mice. The resulting antibodies strongly recognise parasite-derived PfAMA1₈₃ and PfAMA1₆₆ on Western blots (Howell et al., 2001). They show no reactivity with shed PfAMA1_{48/44} (Howell et al., 2003) or rPfAMA1 domains I+II+III, all of which lack the transmembrane and cytoplasmic domains.

### 2.2. The 4G2 epitope is contained within domains I and II of AMA1

In preliminary experiments, expression of domains I+II only (Ile97-His439) of PfAMA1 in *Pichia pastoris* resulted in secretion of a ~30 kDa protein which was recognised on Western blots by mAb 4G2, indicating that the epitope is contained within this part of the molecule. Subsequent work focused on fine mapping of the epitope.

### 2.3. Expression of PfAMA1 in a correctly-folded form at the surface of COS-7 cells

FVO ama-1syn sequence encoding Ile97-Tyr622 (i.e. the entire mature ectodomain of the protein as well as its cognate transmembrane and cytoplasmic domains) was cloned into the mammalian expression vector pSecTag2a (Invitrogen) in frame with the vector-encoded Igκ secretory signal sequence and under the control of the constitutive cytomegalovirus promoter. The resulting construct, called pSec/sgAMA1 was transfected into COS-7 cells and the cells harvested 48 h later for expression studies. Immunofluorescence analysis (IFA) of unfixed cells using mAb 4G2 or polyclonal mouse antiserum N1 showed that the recombinant protein was expressed at the surface of the transfected COS-7 cells in a correctly folded form. Western blot analysis of cell extracts with antisera N1 and R1, and mouse anti-AMAcyt showed that the PfAMA1 was expressed as an ~60 kDa protein (~66 kDa under reducing conditions) with an intact cytoplasmic domain.

### 2.4. Site-directed mutagenesis

The COS-7-based expression system was exploited to identify residues involved in the 4G2 epitope, using the following two mutagenesis-based approaches:
1. In the first approach, a screen was developed based on transient expression of a library of random mutants. Sequence encoding domains I+II of the FVO *ama-1syn* gene was amplified by PCR under conditions designed to promote a high frequency of errors (approximately 5 substitutions per kb). PCR products were cloned back into pSec/sgAMA1 using flanking PstI and ClaI sites to produce a library of mutant clones. These were transformed into E. coli, expanded by growth in liquid culture, and the resulting plasmid library transfected into COS-7 cells. Forty-eight hours following transfection cells were lightly trypsinised to produce a single-cell suspension then subjected to an antibody-based screen. First, cells expressing surface protein reactive with mAb 4G2 were depleted using mAb 4G2 and anti-mouse IgG-coated magnetic beads (Dynabeads). The remaining cell population was then put through a positive screen by binding to magnetic beads coated with antiserum N1. Plasmid was isolated from the bound cells, amplified in E. coli and subjected to up to two further rounds of screening in the COS-7 assay. Finally, selected plasmid clones were screened individually by expression in COS-7 cells and Western blot to confirm loss of the 4G2 epitope, then sequenced to identify mutations responsible for loss of the epitope.
2. In a second strategy the primary sequence of domains I+II was analysed using the Jnet (Cuff and Barton, 2000) and Emini (Emini et al., 1985) algorithms to identify residues predicted to be solvent accessible. These were sorted according to their predicted relative solvent accessibility then individually modified by site-directed mutagenesis using the QuikChange system (Stratagene). Codons were replaced either in contiguous groups of three or individually with Ala codons or, in the case of existing Ala codons, with the equivalent codon from another *Plasmodium* AMA1 orthologue. Mutants were screened individually by expression in COS-7 cells.

To date these combined approaches have identified a series of 8 positions and a segment of 25 residues within domains I+II which contribute to the 4G2 epitope (Table 2). Note that some but not all of these residues are conserved between *Plasmodium* AMA1 orthologues.

**Table 2. AMA1 residues identified which contribute to the mAb 4G2 epitope.**

| **PfAMA1 residue**^{**a**} | **Corresponding PvAMA1 residue**^{**b**} | **Conservation across *Plasmodium* AMA1 orthologues**^{**c**} |
|---|---|---|
| Lys280 | Lys225 | yes |
| Asp348 | Asp293 | yes |
| Lys351 | Thr296 | no |
| Gln352 | Gln297 | yes |
| His356 | Glu301 | no |
| Lys364 | Gln309 | no |
| Asp388 | Asp333 | no |
| Arg389 | Asn 334 | no |
| Deletion segment: aa 353 to 377 | Deletion segment: aa 298 to 322 | no |
| Deletion segment: aa 353 to 368 | Deletion segment: aa 298 to 313 | no |

| | | |
|---|---|---|
| ^{a} substitution with Ala in each case disrupts the 4G2 epitope except His356 and Lys364 which were mutated to Pro. | | |
| ^{b} numbering as in (Cheng and Saul, 1994). | | |
| ^{c} see (Polley and Conway, 2001; Polley et al., 2003) for recent analyses of polymorphism within PfAMA1 | | |

### Example 3. Expression, crystallisation and structural analysis of the ectoplasmic domain of Apical Membrane Protein 1 from Plasmodium vivax.

### 3.1. Cloning and expression of PvAMA1

Cloning of the ectoplasmic region of PvAMA1 from the Sal I strain (GeneBank entry AF063138), comprising residues 43 to 487 (numbering commencing from the first residue of the signal sequence), was performed essentially as reported for a similar construction of the same antigen (Kocken et al., 1999). The *Pichia pastoris* pPICZα/KM71H expression kit (Invitrogen, Leek, The Netherlands) was used to prepare recombinant clones expressing secreted soluble product carrying a c-myc marker and hexa-histidine tag at the C-terminal end of the recombinant protein. Potential N-glycosylation sites were mutated as follows: Ser178→Asn, Asn226→Asp and Asn441→Gln. Residue replacements for Ser178 and Asn226 were chosen from the P. falciparum homologue, while the replacement for Asn441 is from the P. chabaudi sequence (Kochen et al, 1999). Expression from the wildtype PvAMA1 gene in P. pastoris, incidentally, did not present the same problems as did the P. falciparum homologue (Kocken et al., 2002), largely because the codon usage of the former is better adapted to this expression system; the AT content of PvAMA1 is 56%, which is close to that of P. pastoris (54%), whereas that of PfAMA1 is 70%.

A 20 ml preculture of recombinant P. pastoris was cultured in BMGY (1% yeast extract, 2% peptone, 1.34% yeast nitrogen base, 1% glycerol, 0.4 mg of biotin per litre, 0.1 M potassium phosphate, pH 6.0) for 65 hours at 30°C. One litre of BMMY (BMGY with glycerol replaced by 0.5% methanol) was inoculated with this culture and then grown for 24 hours at 30°C with vigorous shaking. Cells were subsequently harvested by centrifugation at 3000 g for 5 minutes, then resuspended in 200 ml of BMMY and grown as induction culture at 30°C for 72 hours. Methanol was added to a final concentration of 0.5% every 24 hours. Cells were harvested by centrifugation and the supernatant was filtered on 0.22 µm Millex units.

### 3.2. Protein purification

### Procedure (a):

The protein was purified by a metallo-affinity procedure as follows, making use of the C-terminal His-tag. Culture supernatant was directly applied in batch mode to ProBond resin (Invitrogen, 4 ml slurry for 50 ml of supernatant in a Falcon tube) previously equilibrated in 20 mM sodium phosphate, 500 mM NaCl, pH 7.4, and incubated for 3 hours at room temperature. The resin was then separated from the supernatant by centrifugation and treated with wash buffer (20 mM sodium phosphate, 500 mM NaCl, pH 6.0) in successive volumes of 25 ml until the absorbance of the supernatant was close to background. The protein was then eluted with the same wash buffer to which had been added 500mM imidazole.

### Procedure (b):

Since procedure (a) did not give the expected yields of PvAMA1 from the total recombinant protein, purification on an anion-exchange column was also followed. The supernatant was precipitated with 70% ammonium sulphate and the resulting pellet was redissolved in 10 mM Tris/HCl buffer, pH 8.0, and applied to an Econo-Pac 10DG column (Biorad), either with or without a preliminary passage over a metallo-affinity column as described in procedure (a). The desalted protein was then purified on a monoQ column (Pharmacia) using a NaCl gradient in 20 mM Tris/HCl buffer, pH 8.0. The protein fraction not absorbed by the metallo-affinity resin reacted neither with an anti-His-tag monoclonal antibody nor an anti-c-myc monoclonal antibody in a Western blot, showing that C-tenninal end of the expressed protein was labile towards proteolysis. Indeed, the molecular mass of the purified product was 54,150 ± 50 from procedure (a) and 51,600 ± 50 from procedure (b), as determined by SELDI-TOF MS (Surface-Enhanced Laser-Desorption-Ionisation time-of-flight Mass Spectrometry) retentate chromatography. The former value agrees with the expected molecular mass of the full-length product (54,175), whereas the latter corresponds well to that excepted for the recombinant product cleaved at the junction of the AMA1 C-terminus and the c-myc marker (51,567). These results, moreover, confirm that there is no O-glycosylation. On average, the yield of total purified protein was 60 mg per litre of supernatant, with the full-length and truncated products obtained in about equal proportions.

### 3.3. Crystallisation and heavy-atom derivatives

Crystals were grown by vapour diffusion using the hanging drop technique at 18°C. Initial crystallisation conditions were screened by the sparse-matrix sampling approach (Jancarik & Kim, 1991); micro-crystals were obtained in conditions 40 and 41 described by these authors. Optimisation was then pursued with modifications of the buffer components and the addition of various additives. The best crystals were obtained with a crystallisation mixture that typically consisted of 10-12 % (w/v) PEG3350, 100-200 mM imidazole, pH 7.0, 5-10 % (v/v) isopropanol, and 1% (v/v) DMF or 3% (v/v) t-butanol. The protein solution was diluted by 40 - 60% with the crystallisation buffer, giving a final concentration of 4-6 mg. ml⁻¹ in suspended drops of volume 2 - 3 µl. The crystals were monoclinic for metallo-affinity-purified protein (procedure (a)) and orthorhombic for monoQ-purified protein (procedure (b)) (Figure 1). The crystalline form obtained thus appears to be determined by the presence or absence of the C-terminal segment containing the c-myc and hexa-histidine markers.

Orthorhombic and monoclinic crystals of the native protein were prepared for diffraction measurements by flash-freezing in liquid nitrogen after a brief transfer to a cryo-protecting buffer consisting of the original crystallising buffer in which the PEG 3350 concentration had been increased to 30 % (w/v). A platinum derivative of both crystal forms was prepared by soaking crystals in a solution of potassium tetrachloroplatinate(II) (Aldrich) at a concentration of 2 mg. ml-1 in a buffer of 30 % (w/v) PEG 3350, 100 mM sodium cacodylate, 10 % (v/v) isopropanol, pH 7.0, for 24 hours. Crystals were mounted by flash-freezing in liquid nitrogen without back-washing, using the same solution as a cryo-protector.

### 3.4. Data collection and preliminary structure analysis

Data were collected at the E.S.R.F., Grenoble, using the beam-lines BM14, ID 14-2 and ID 14-4. Crystals were maintained at 100 K in a stream of cold nitrogen gas during measurement but they proved to be very sensitive to radiation damage, thus limiting the total exposure that could be tolerated in the beam. The orthorhombic crystals usually grew no larger than about 50x50x200 µm³, whereas the monoclinic form could grow to maximum dimensions of about 100x100x400 µm³. The larger monoclinic crystals, however, were prone to damage upon freezing, and smaller sizes were therefore favoured for diffraction measurements. Diffraction intensities were integrated and scaled using the programs DENZO and SCALEPACK, respectively (Otwinowski & Minor, 1997). Details of measurements and data-processing statistics are given in Table 3.

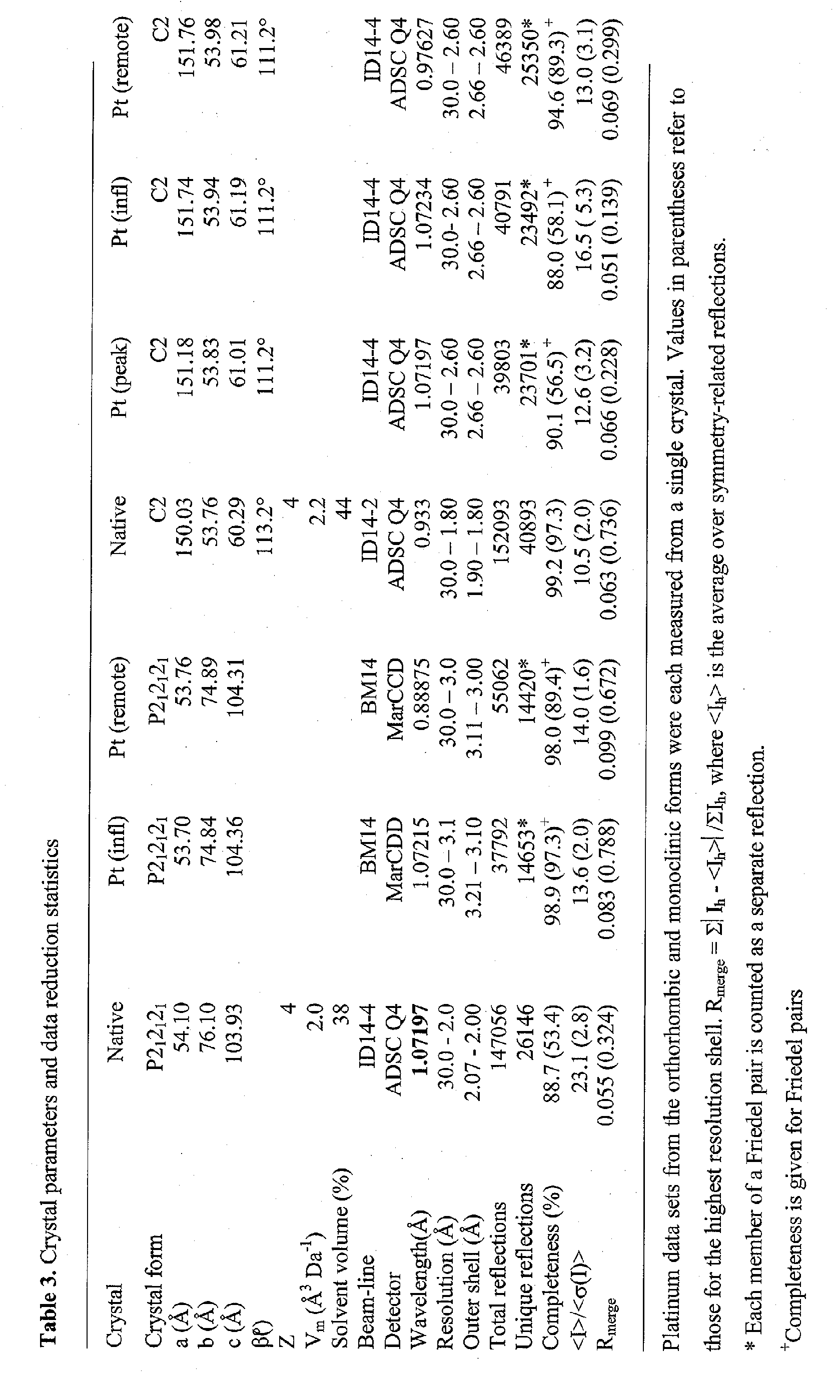

Since the orthorhombic crystals were obtained first, structure analysis began with this form. Diffraction data for the platinum derivative were measured at the beamline BM14 at the E.S.R.F., Grenoble, using a single crystal. Measurements were made at two wavelengths, 1.0722 Å and 0.8887 Å, corresponding to the inflection and remote points of the fluorescence spectrum, respectively. Data could not be measured at more than two wavelengths from the same crystal because of sensitivity to radiation damage and the comparable size of the sample to the X-ray beam on BM14. There was also a large variability in the unit cell dimensions of cryo-frozen crystals, which excluded the possibility of using data sets from different crystals in the phasing calculations. Analysis was therefore restricted to data from the best crystal of the platinum derivative. An initial heavy atom search made with the program SHELXD (Schneider & Sheldrick, 2002) found two sites, which were subsequently refined using the program SHARP (de la Fortelle & Bricogne, 1997). Heavy atom parameters were refined at 4.2 Å resolution, the limit beyond which the correlation between the anomalous differences of the inflection and remote data sets fell below 0.3. Two additional platinum sites were subsequently found, giving an overall figure of merit (FOM) of 0.36. All four platinum atoms showed high temperature factors. Phases were then calculated to 3.0 Å using these heavy atom parameters (FOM of 0.22) and refined by density modification using the programs SOLOMON (Abrahams, 1997) and DM (The CCP4 Suite, 1994; Cowtan, 1994). In spite of the poor resolution, the resulting electron density map showed clear indications of the polypeptide main chain in certain regions, and a partial model could be built. This gave a preliminary polyalanine model that included 244 of the 467 residues comprising the recombinant protein, which were distributed over seven peptide segments.

At this stage, the monoclinic form of AMA1 had been crystallised. A derivative was prepared with K2PtCl4 under the same conditions as for the orthorhombic form and diffraction intensities were measured at three wavelengths on ID14-4 at the E.S.R.F.: 1.07197 Å (peak), 1.07234 Å (inflection) and 0.97627 Å (remote). These three data sets could be measured from the same crystal because the smaller beam size on ID14-4 and the larger size of the monoclinic crystals allowed successive translations to previously unexposed regions of the sample for each wavelength.

The partial model from the orthorhombic form was then used as a search model in a molecular replacement calculation to place the molecule in the monoclinic unit cell. The highest peak in the rotation function (peak height of 0.24, second highest peak at 0.22) gave a clear solution in the translation function (highest peak at 0.32, second highest peak at 0.25). An anomalous difference map from the peak data, phased by molecular replacement solution, showed two very clear peaks that could be related, via the search model, to the two major platinum sites in the orthorhombic crystal form. The heavy atom parameters were refined with SHARP using data to 3.1 Å (a limit based on the fall-off of correlation between anomalous differences from the three MAD data sets), leading to the inclusion of three additional platinum sites that could be related to peaks in the anomalous difference map. This gave a FOM of 0.28. Phases were then calculated to 2.8 Å with these platinum parameters (FOM of 0.26) and refined by density modification with SOLOMON and DM. As with the orthorhombic form, all platinum atoms had high temperature factors. Nonetheless, the quality of the phases was superior for the monoclinic data and the definition of the resulting electron density allowed easier interpretation. Phasing statistics for both crystal forms are presented in Table 4.

**Table 4. Heavy atom refinement**

| **Space group** | **orthorhombic** | | **monoclinic** | | |
|---|---|---|---|---|---|
| Data set | inflection | remote | peak | inflection | remote |
| f' | -20.9 | -6.8 | -22.8 | -18.3 | -7.6 |
| f" | 7.6 | 11.9 | 9.6 | 14.2 | 8.9 |
| Resolution (Å)* | | 30.0-4.2 | | | 30.0 - 3.1 |
| FOM (centric) | | 0.360 | | | 0.282 |
| FOM (acentric) | 0.554 | | | | 0.431 |

| | | | | | |
|---|---|---|---|---|---|
| *Resolution of heavy atom refinement | | | | | |

The partial orthorhombic model, placed in the monoclinic unit cell by molecular replacement, followed the MAD-phased electron density of the latter crystal form very well. This showed that the heavy-atom phasing results were coherent between the two crystal forms, and were thus correct. Additional residues could be modelled in the monoclinic map as polyalanine, and the electron density for side chains in the better defined regions eventually permitted the sequence to be assigned to most of the polypeptide segments comprising the partial model. At this stage, model comprised seven separate peptide segments with a total of 268 residues. Native data sets of both crystal forms have also been measured to high resolution (see Table 3), and these are currently being used to refine the atomic model of AMA1 with the program ARP/WARP (Morris et al., 2002) and REFMAC (Murshudov et al., 1997).

Apical Membrane Antigen 1 (AMA1), a type 1 transmembrane protein produced in the microneme organelles of Plasmodium, is a leading malaria vaccine candidate. The ectoplasmic region of AMA1 from Plasmodium vivax has been expressed in Pichia pastoris and crystallised in two different forms: an orthorhombic form (P2₁2₁2₁, a = 54.1 Å, b = 76.1 Å, c = 103.9 Å) and a monoclinic form (C2, a = 150.0 Å, b = 53.8 Å, c = 60.3 Å, β = 113.2°). Native data have been collected to 2.0 Å resolution for the orthorhombic form and 1.8 Å for the monoclinic form. A platinum derivative was prepared for the orthorhombic and monoclinic crystals using K₂PtCl₄ and data were collected at several wavelengths to obtain phases by the Multiwavelength Anomalous Diffraction (MAD) technique. A partial model has been built from the electron density maps of both forms and refinement is in progress.

### Example 4. Refinement of the structure of Apical Membrane Antigen 1 from Plasmodium vivax (PvAMA1)

These models of PvAMA1 in the monoclinic and orthorhombic forms, obtained as described in the example 3 were refined using the programs REFMAC (Murshudov et al., 1997) and ARP/WARP (Morris et al., 2002). Details of the final refinement statistics are given in Table 5.

**Table 5. Refinement statistics**

| | **PvAMA1 monoclinic** | **PvAMA1 orthorhombic** |
|---|---|---|
| Resolution range | 20. - 2.01 Å | 20.0 - 1.80 Å |
| No. of Bragg intensities | 24739 | 38819 |
| R-factor | 0.1976 | 0.2217 |
| R-free r.m.s. deviations | 0.2592 | 0.2688 |
| Bond lengths | 0.018 Å | 0.020 Å |
| Bond angles | 1.7° | 1.9° |
| No. of protein atoms | 2944 | 2932 |
| No. of Solvent atoms | 293 | 218 |

PvAMA1 is an elongated molecule with approximate dimensions of 70Å x 45Å x 35Å. The structure of the antigen is very similar in the orthorhombic and monoclinic crystals; the most notable differences, occurring only in certain loops, can be attributed to different intermolecular environments between the two crystalline forms. In the monoclinic crystal structure, the polypeptide chain could be built from Pro43, the first PvAMA1 residue in the recombinant protein, to Glu475. Nonetheless, breaks in the main-chain trace occur in several places where the electron density became absent or too poorly defined model the structure. The atomic model of the orthorhombic structure begins at the N-terminus of the recombinant protein, thus including the dipeptide segment, Ser-Ile, that arises from insertion of the PvAMA1 gene into the plasmid, and extends to Glu474. As with the monoclinic structure, there were several breaks in the continuity of the electron density defining the main chain of the molecule. The model is more complete for the orthorhombic structure since intermolecular contacts offer slightly more favourable stabilisation of the flexible regions, which may correlate with the slightly smaller Vm for this crystal form (See Tables 6 and 7 giving the atomic coordinates of the ectoplasmic domain of AMA1 protein from *Plasmodium vivax* strain Sa1 I which has been crystallized respectively in the orthorhombic form and in the monoclinic form).

### Example 5. Orthorhombic crystallisation modelling of the three dimensional structure of Apical Membrane Antigen 1 from Plasmodium falciparum (PfAMA1) and polymorphism in PfAMA1

The structure of PfAMA1 of the FVO strain (GenBank entry U84348) has been modelled by introducing the 135 amino acid changes that exist in the ordered regions of the PvAMA1 crystal structure (an additional 48 changes occur in disordered regions). These are distributed over the entire ectoplasmic region: domain I carries 87 differences from a total of 206 residues (42 %), 47 differences occur within the 138 residues (34 %) comprising domain II, and 49 of the 101 (49 %) residues differ in domain III. Conformation angles of the side chains of changed residues were adjusted using the mean force field algorithm (Kohl & Delarue, JMB, 1994). The five sequence differences occurring at sites inaccessible by solvent in the PfAMA1 model are conservative changes involving hydrophobic aliphatic side chains (Val, Leu and Ile). The remaining sequence differences are evenly distributed over the surface of the molecule. The side chain conformation of these residues could be easily adapted to the PvAMA1 structure, implying that the structure of the two homologues should be very similar.

Using this model of PfAMA1, the 3-dimensional distribution of polymorphic sites has been examined. Taking 356 PfAMA1 sequences that were available on Medline,it has been considered a site to be polymorphic if at least two sequences differed from the consensus amino acid at that position. All polymorphic sites are surface exposed; a total of 29 are located in domain I (14 %), 10 in domain II (7 %) and 8 in domain III (9 %), thus showing domain I to be the most polymorphic region of PfAMA1. The presence of a particular amino acid at certain polymorphic sites was found in some cases to be highly correlated with the sequence occurring at other polymorphic sites. The relationship between such sequence-correlated sites is not clear, but since a number of these are well separated from each other in space, it does not appear that their occurrence is directly caused by structural factors in the protein itself. Unlike the distribution of PvAMA1/PfAMA1 sequence differences, that of the PfAMA1 polymorphic sites is highly biased to one side of the molecule. This observation suggests that the less polymorphic side of the ectoplasmic region could be in direct contact with the parasite surface, thus exposing the vast majority of the polymorphic residues to provide an antigenic camouflage ((See Table 8 giving the atomic coordinates of the ectoplasmic domain of AMA1 protein from *Plasmodium falciparum* which has been modelled in the orthorhombic form).

Regions of the polypeptide chain of PvAMA1 that have been modelled are indicated on the sequence of the recombinant construction shown in Figure 3. The α-carbon skeleton of the molecule showing the domain structure is shown in Figure 4. PfAMA1 mutations that abolish binding of the invasion-inhibition monoclonal antibody 4G2 have been mapped onto the PvAMA1 structure by homology modelling (see Example 1, Table 1): three of these residues occur on ordered regions of the structure and are indicated in Figure 4, and three additional sites occur in a large loop in Domain II, which is disordered (residues 296 to 334) and thus could not be modelled from the electron density maps.

The ectoplasmic region of Apical Membrane Antigen 1 from *Plasmodium faliciparum* (PfAMA1) and constructions containing different combinations of the three domains that comprise this part of the protein have been cloned and expressed in Pichia pastoris. 4G2, a monoclonal antibody specific for the ectoplasmic region of PfAMA1, inhibits erythrocyte invasion by *P. falciparum.* Among the different domain combinations that have been expressed, the fusion construct Domain I-Domain II was found to be the minimum recombinant product that retained full reactivity towards mAb 4G2. Fine epitope mapping for 4G2, carried out by site-directed mutagenesis on the ectoplasmic region of PfAMA1, has implicated a number of residues from these two domains as being important for recognition by this mAb. These residues have been mapped onto the crystal structure of PvAMA1, the homologue from *P*. *vivax*, revealing a functionally important region of the protein.

PvAMA1 is an elongated molecule with approximate dimensions of 70Å x 45Å x 35Å. The crystal structure confirms the hypothesis that the molecule can be divided into three structural domains. Each domain is based on a central beta-sheet, around which additional beta-sheets, beta-hairpin turns and/or alpha-helices are arranged. The polypeptide chain has been modelled from Pro43, the first PvAMA1 residue in the recombinant protein, to Glu474 (orthorhombic) or Glu475 (monoclinic); however, breaks in the main-chain trace occur in several places where the electron density becomes too poorly defined or absent to permit modelling of the structure, indicating the presence of several highly flexible regions. Indeed, the atomic model accounts for only 80% of the PvAMA1 sequence present in the recombinant construct.

The sequence of AMA1 is highly conserved between different Plasmodial species: the sequence identity between PvAMA1 and PfAMA1 is 59% in the ectodomain, with the latter protein containing one 3-residue insertion in a region corresponding to a flexible loop in the crystal structure of PvAMA1 and an additional one-residue insertion close to the C-terminus of the recombinant ectodomain. Both insertions occur in regions that could not be modelled in the PvAMA1 structure because of flexibility in the polypeptide chain. The crystal structure shows that many conserved residues, in addition to the cystines, are involved in intramolecular interactions that are important in defining the overall conformation of the molecule. Homology modelling of the PfAMA1 structure from the crystal structure of PvAMA1 can be therefore be achieved with high confidence. Comparison of PvAMA1 with other known 3-dimensional structures using the program DALI (Holm, L., Sander, C. 1993) revealed that domains I and II are structurally similar to leech anti-platelet protein (LAPP) (Huizinga et al., 2001), which belongs to the PAN module superfamily (Tordai et al., 1999). For domain I of PvAMA1, the region comprising residues 92 to 238 (147 amino acids) superimpose upon residues 46 to 122 of LAPP (77 amino acids, PDB entry 1i8n) with a r.m.s. difference of 2.3 Å between 70 structurally equivalent Cα positions (Z-score of 4.0). Similarly, residues 249 to 376 from domain II of PvAMA1 (128 amino acids) superimpose upon the region from residues 47 to 122 of LAPP (76 amino acids) with a r.m.s. difference of 2.6 Å between the 70 structurally equivalent Cα atoms (Z-score of 5.1). When comparing the PvAMA1 domains with each other, domain I from residues 93 to 243 can be optimally positioned onto domain II from residues 249 to 381 with a r.m.s. difference of 2.3 Å between 77 equivalent Cα positions (Z-score of 6.0). The DALI algorithm did not identify any previously determined polypeptide fold corresponding to the 3-dimensional structure of domain III. The structure of PvAMA1 we describe here suggests a potential receptor-binding role for this molecule. Although structural folds had not been previously attributed to the AMA1 domains, the crystal structure shows that both domains I and II are based on the PAN motif. PAN modules are found in proteins with diverse adhesion functions, binding to protein or carbohydrate receptors (Tordai et al., 1999). Moreover, certain of the micronemal proteins from apicomplexan parasites have been shown to include PAN domains in their overall structural organisation (Tomley & Soldati, 2001; Brown et al., 2001). The PAN motif is organised around a 5-stranded anti-parallel β-sheet with the strands arranged in the order β1-β5-β3-β4-β2. An α-helix, connecting β2 to β3, lies against one face of the sheet. The other face of the β-sheet is covered by two polypeptide segments, one connecting β1 to β2 and the other connecting β4 to β5. This organisation is found in the N-terminal domain of hepatocyte growth factor (HGF) (Zhou et al., 1998) and LAPP, except that the latter has an additional β-strand, β0, joined to the β1 edge of the sheet to give a 6-stranded anti-parallel arrangement. The helix of LAPP and the N-domain of HGF both carry two half-cystine residues separated by one turn and which form disulphide bridges to the region immediately before β3 and within β4, respectively. For the HGF N-domain, the cystine bridges are formed in the order Cys1-Cys4 and Cys2-Cys3. LAPP has an additional cystine bridge joining the N-and C-terminal regions of the polypeptide chain to give a disulphide-bonding pattern Cysl-Cys6, Cys2-Cys5 and Cys3-Cys4; this arrangement of three disulphide bridges forms the Apple domain motif within the PAN module superfamily (Brown et al., 2001). In LAPP and the N-domain of HGF, the two segments packed against the face of the β-sheet opposite the α-helix cross each other such that the β1-β2 joining segment passes between the face of the β-sheet and the outer β4-β5 joining segment. We thus refer to these two segments as the inner and outer crossing segments, respectively. The crossing segments form a double anti-parallel β-strand between them for part of their trajectories. PAN modules generally show low sequence identity within the superfamily and have been recognised largely by the pattern of cystines and the observed or predicted secondary structure (Tordai et al., 1999). The crystal structure shows that the conformation of domains I and II is based on the PAN folding motif. Strands β4-β10-β7-β8-β6 of domain I and β11-β18-β15-β16-β14 of domain II form the central 5-stranded β-sheet motif of the PAN module, while α5 and α7 correspond to the helical component in the two respective domains. The inner and outer crossing segments, occurring between β4 and β6, and β8 and β10, respectively, in domain I, and between β11 and β14, and β16 and β18, respectively, in domain II, also conform to the PAN module organisation. The pattern of cystine bridge formation is less well preserved in AMA1; only Cys162-Cys192 in domain I and Cys282-Cys354 in domain II, corresponding to disulphide bond between β4 and the helix in LAPP (Huizinga et al. 2001) and the N-domain of HGF (Lietha et al., 2001), conforms to the PAN motif. Thus, the other cystine bridges in AMA1 do not appear to be critical for maintaining the essential features of this protein fold.

The largest missing region in the atomic model of the PvAMA1 crystal structure is the 40-residue segment between Pro295 and Asn324, inclusive, in Domain II. From secondary structure prediction algorithms, about two-thirds of this segment are suggested to be alpha-helical; it could therefore have a well defined structure that is connected to the rest of the molecule by flexible hinge regions at the N- and C-terminal ends of this region. Interestingly, this segment contains no known polymorphic sites even though it is expected to be very exposed.

The monoclonal antibody 4G2 [14], specific to PfAMA1, inhibits erythrocyte invasion by the *P. falciparum* merozoite. The epitope recognised by 4G2 is not present when PfAMA1 is reduced; furthermore immunisation with reduced AMA1 does not induce antibodies that inhibit merzoite invasion. Thus, surface-exposed epitopes recognised by antibodies that inhibit AMA1 function are a dependant on the three-dimensional structure of AMA1. Recombinant expression of domains of PfAMA1 has shown that 4G2 does not react with Domains II or III when expressed individually or as a Domain II/III fusion. 4G2 reactivity was only evident in a fusion of Domains I and II. Immunisation of rabbits with recombinant proteins has shown that domains II and III, either individually or in combination, induce only very low amounts of antibody capable of inhibiting merozoite invasion in vitro. By contrast, the fusion of Domains I and II induces levels of inhibitory activity comparable to immunisations with the entire ectodomain. Although rabbit antibodies to Domain II have very little invasion-blocking activity, they do inhibit the binding of 4G2 to the PfAMA1 ectoplasmic region. Thus epitopes in Domain II induce antibodies that themselves block the binding of antibodies the inhibit AMA1 function. To refine the epitope further, alanine mutations have been introduced in the recombinant ectodomain of PfAMA1. By mapping onto the crystal structure of PvAMA1 those residues that eliminate the binding of 4G2, we have located a region within Domain I and at the base of the missing Domain II loop described in the preceding paragraph as important for recognition by 4G2. These results identify a potentially important protecting epitope and, taken together with the absence of any known polymorphic sites within the Domain II loop, suggest that this region of the protein could be critical for the biological function of AMA1.

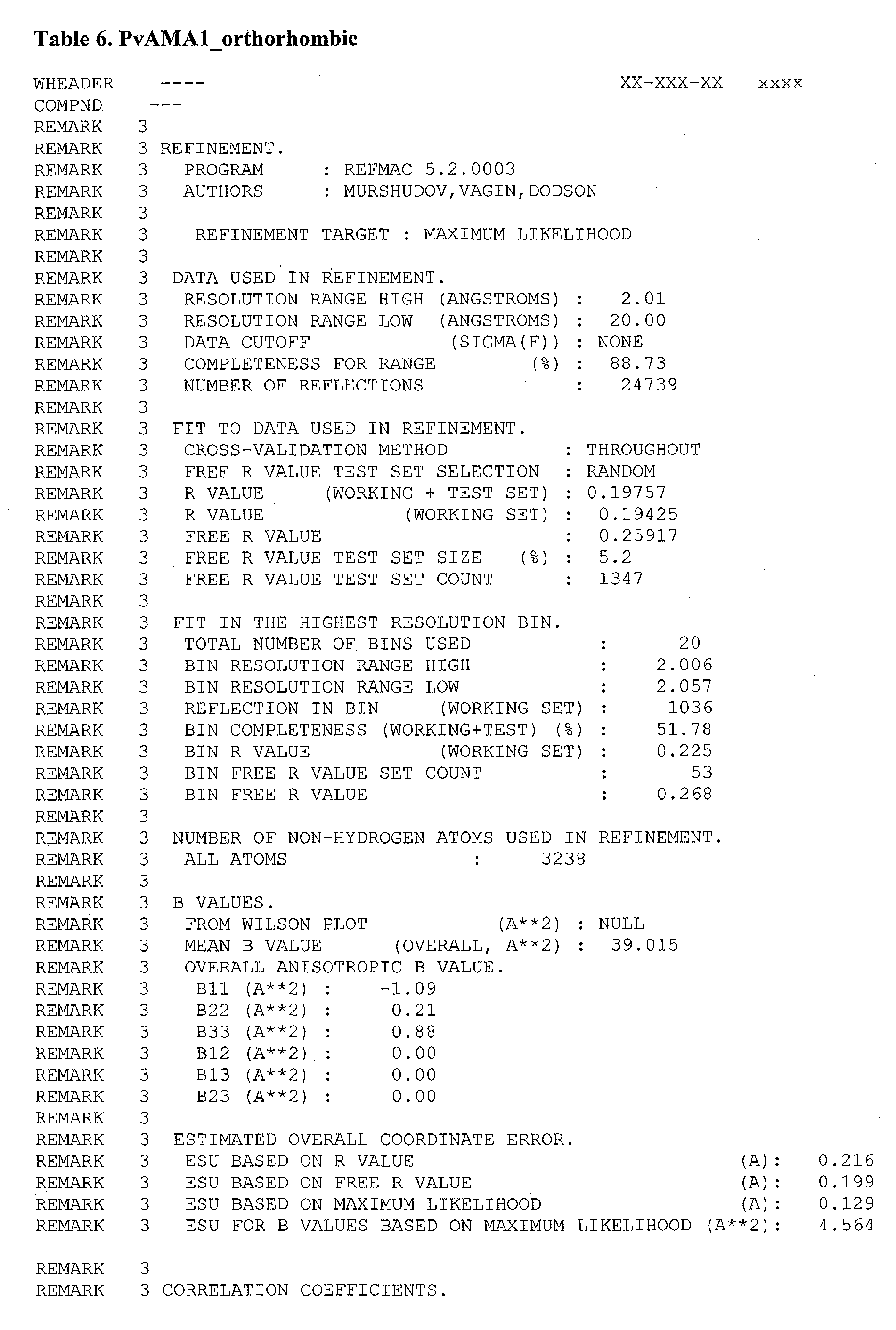

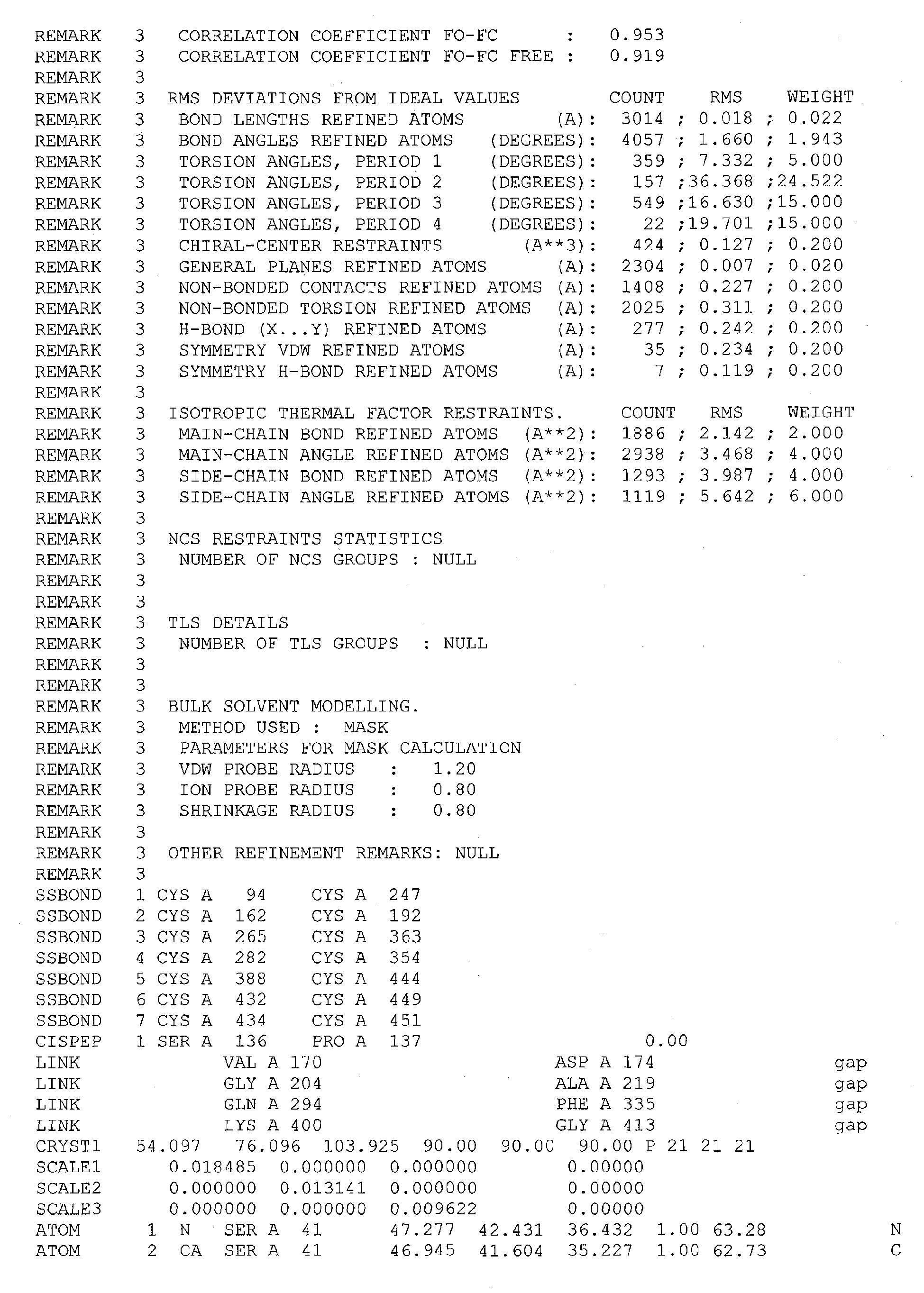

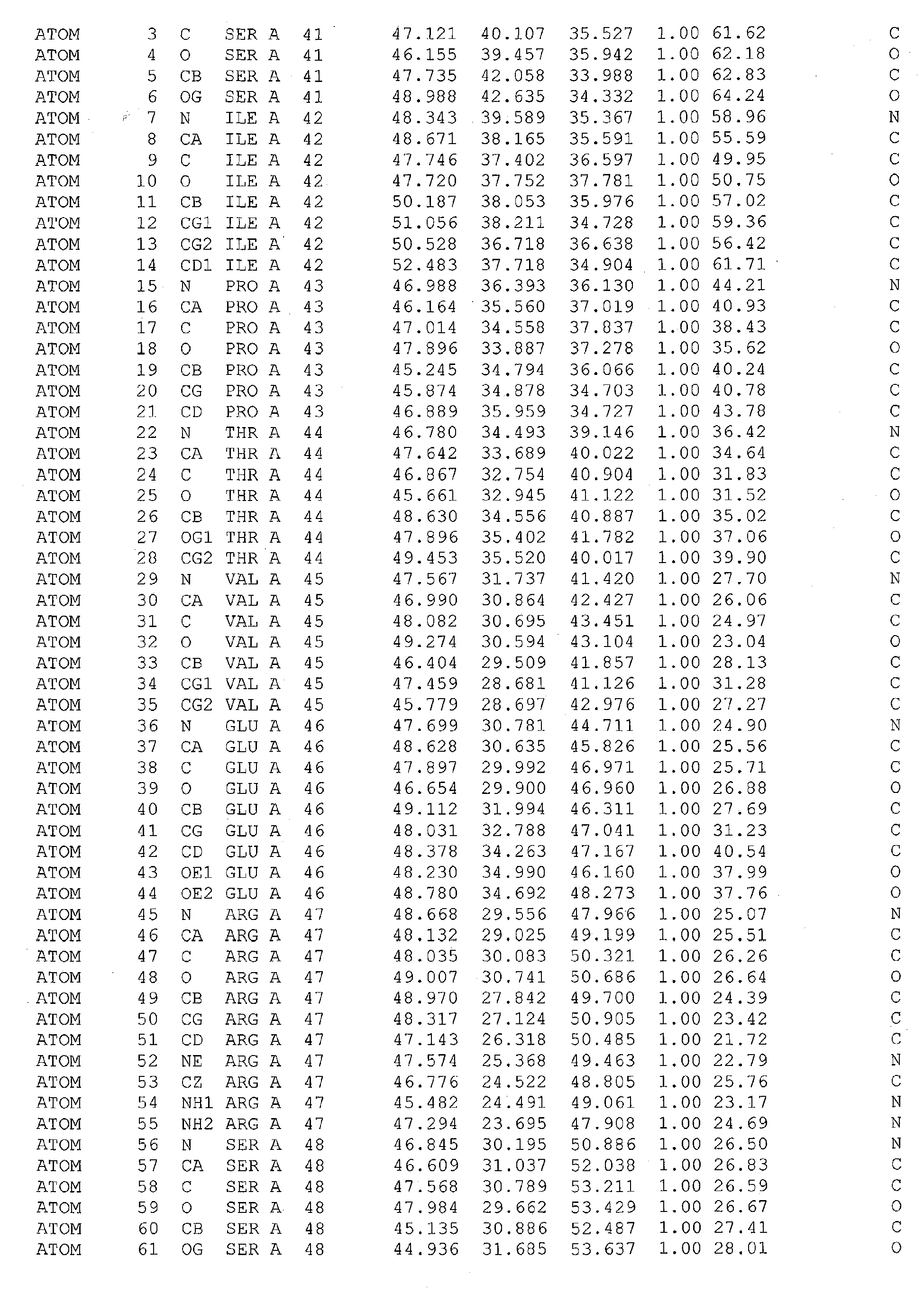

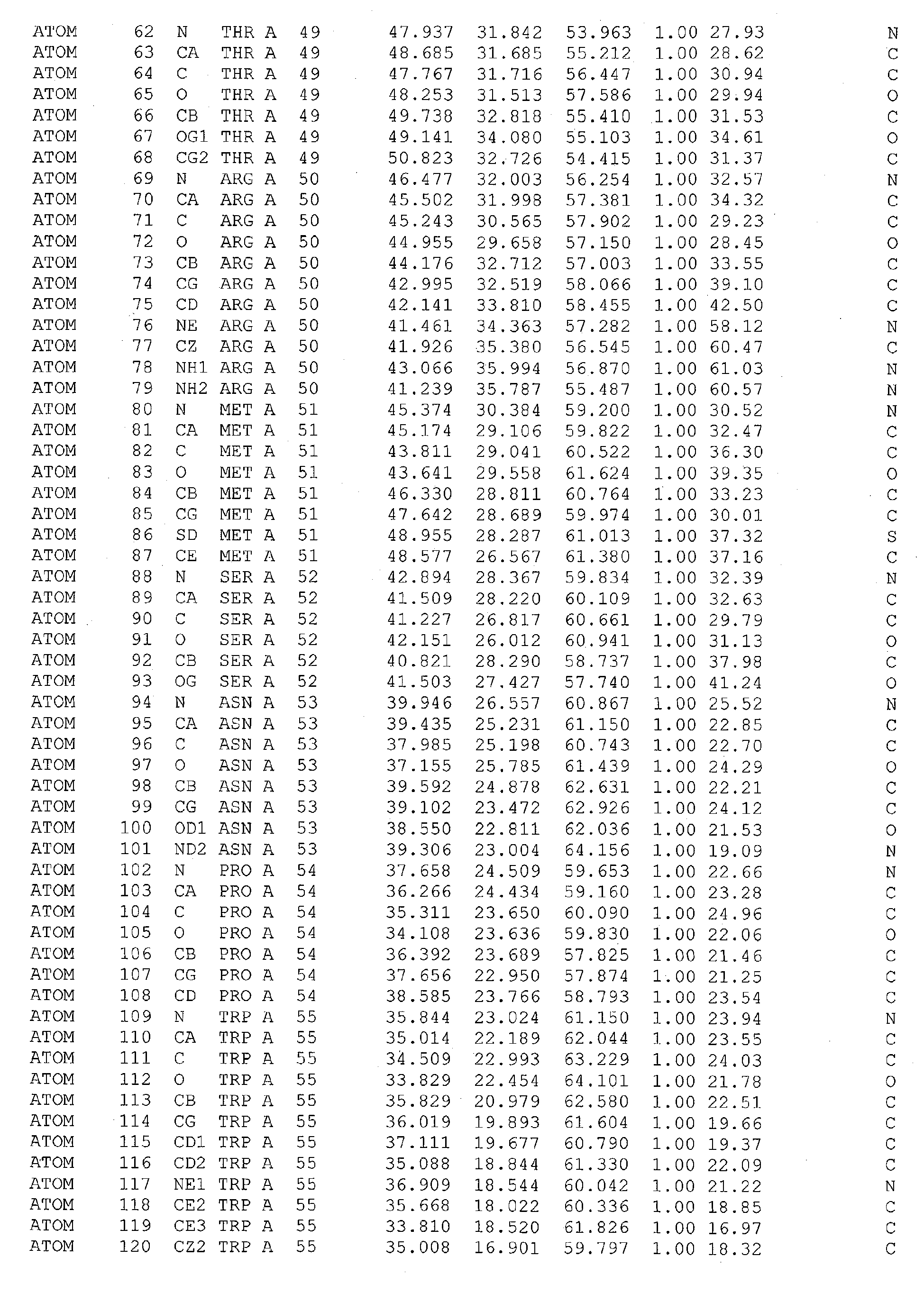

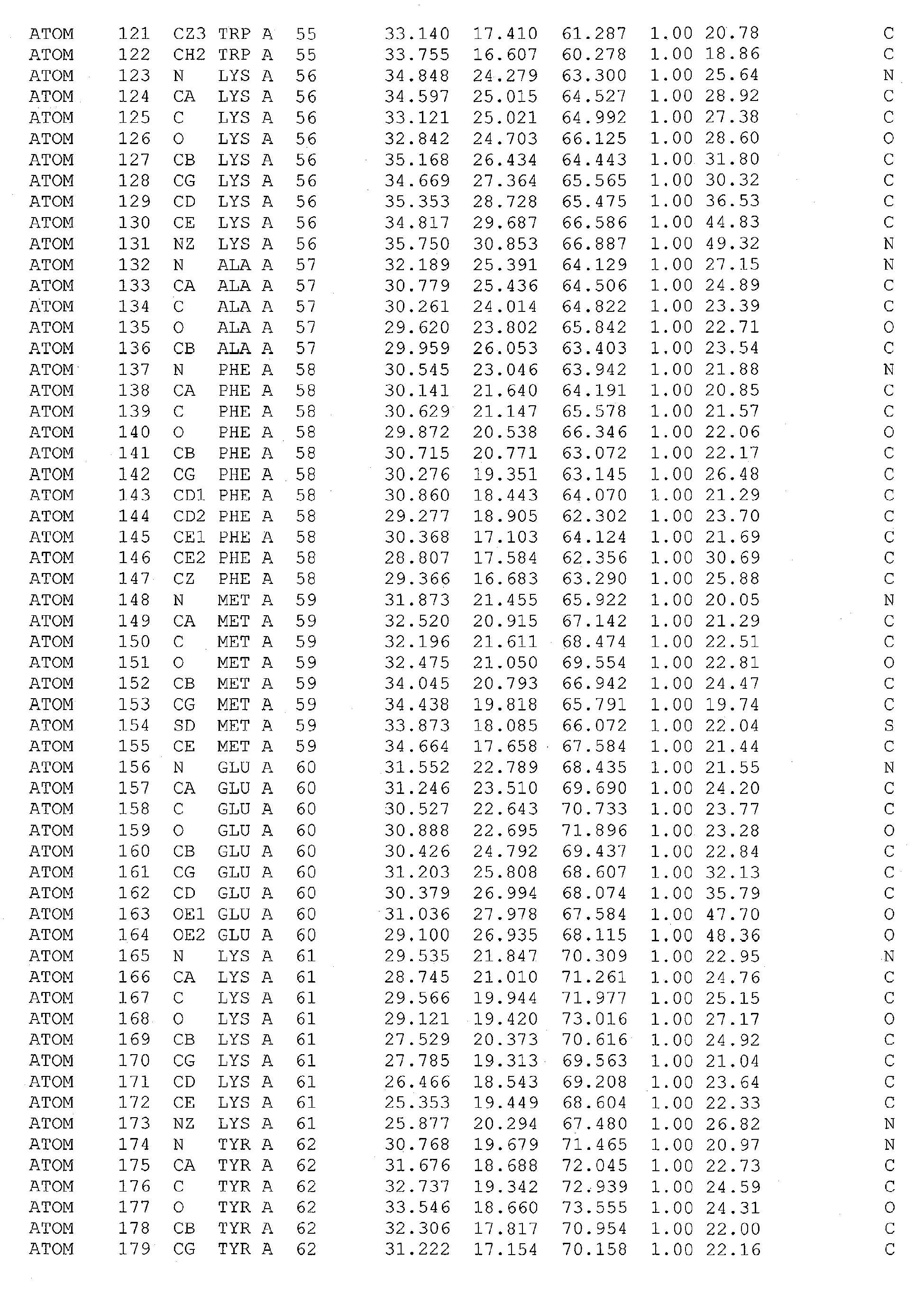

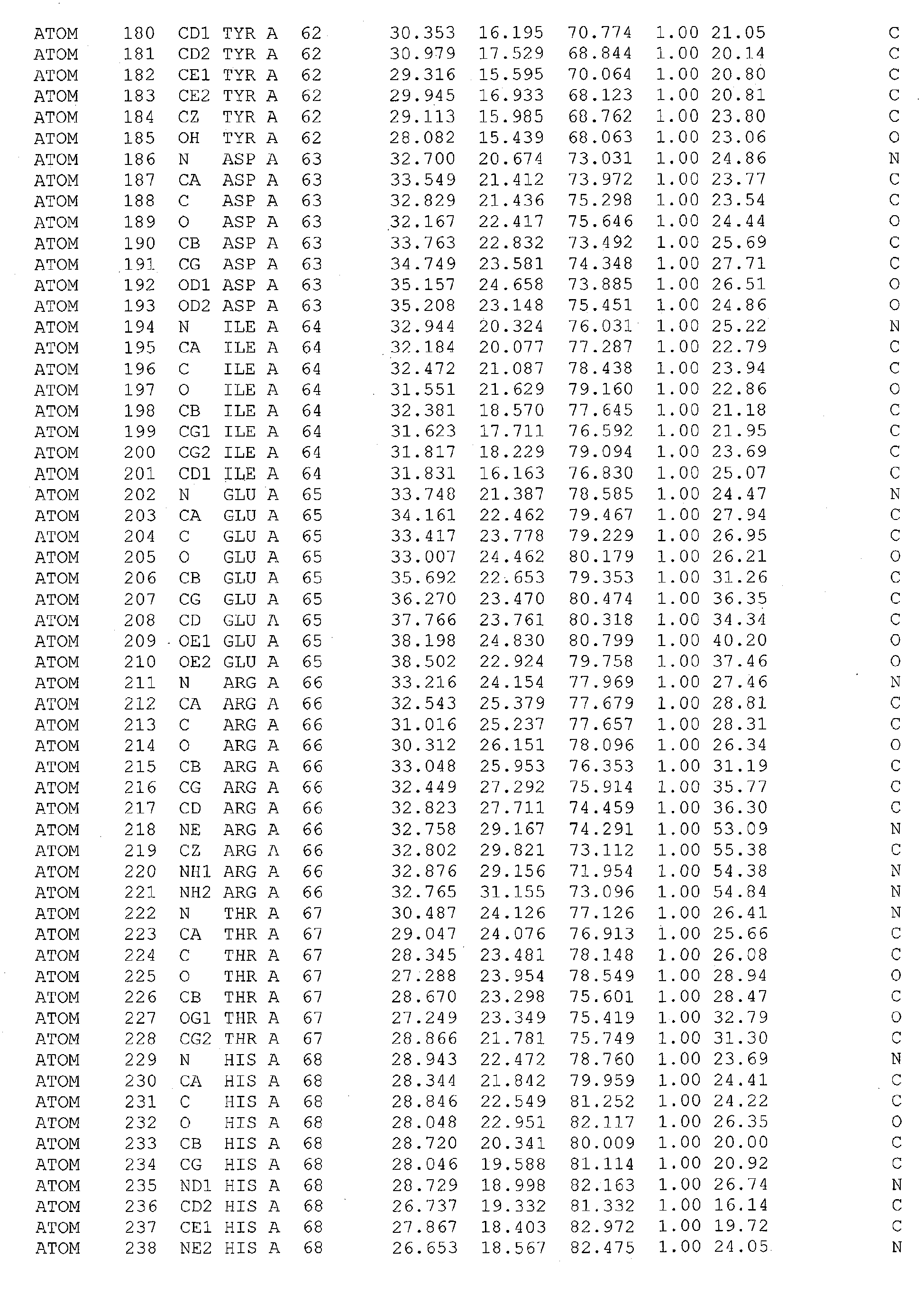

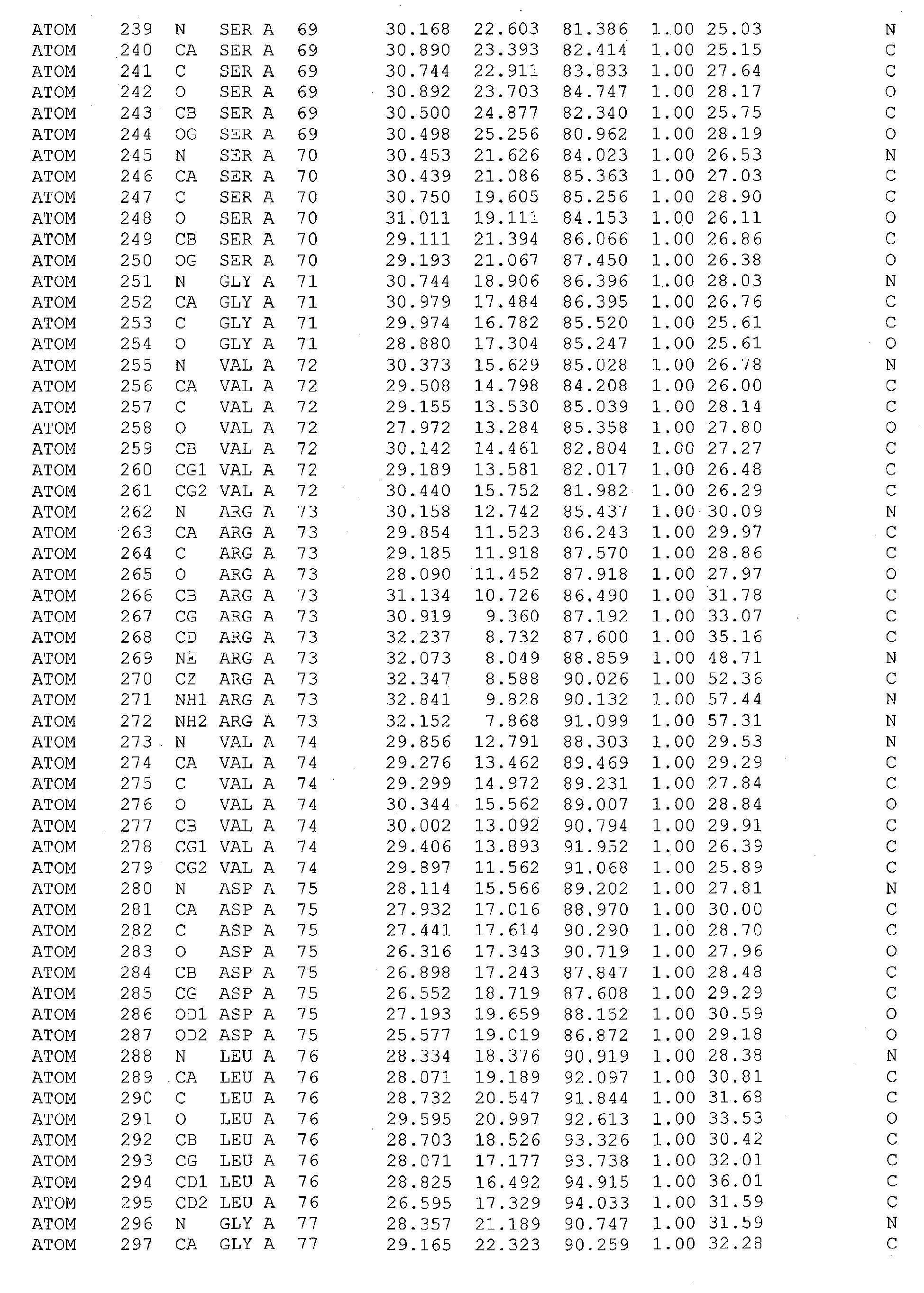

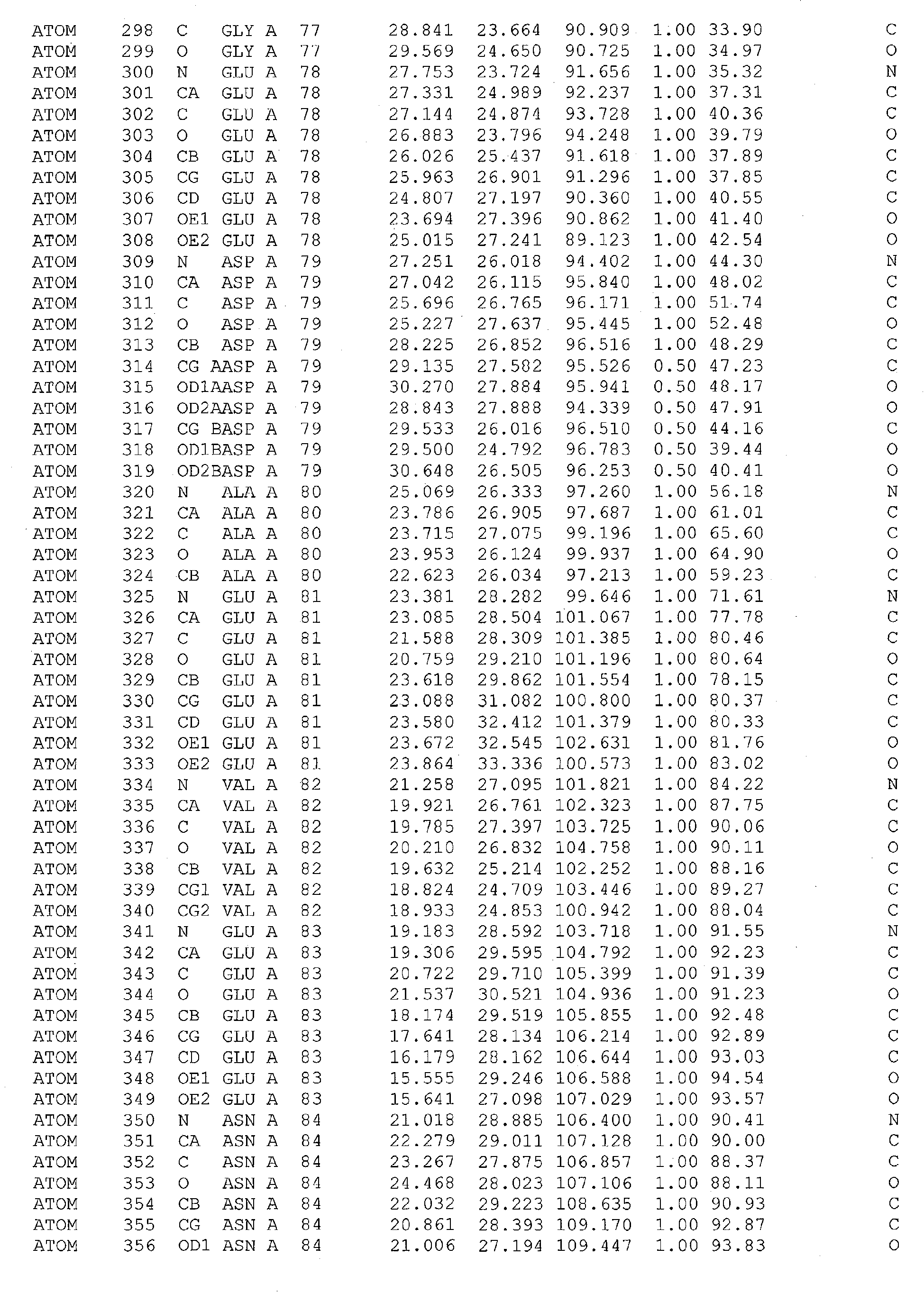

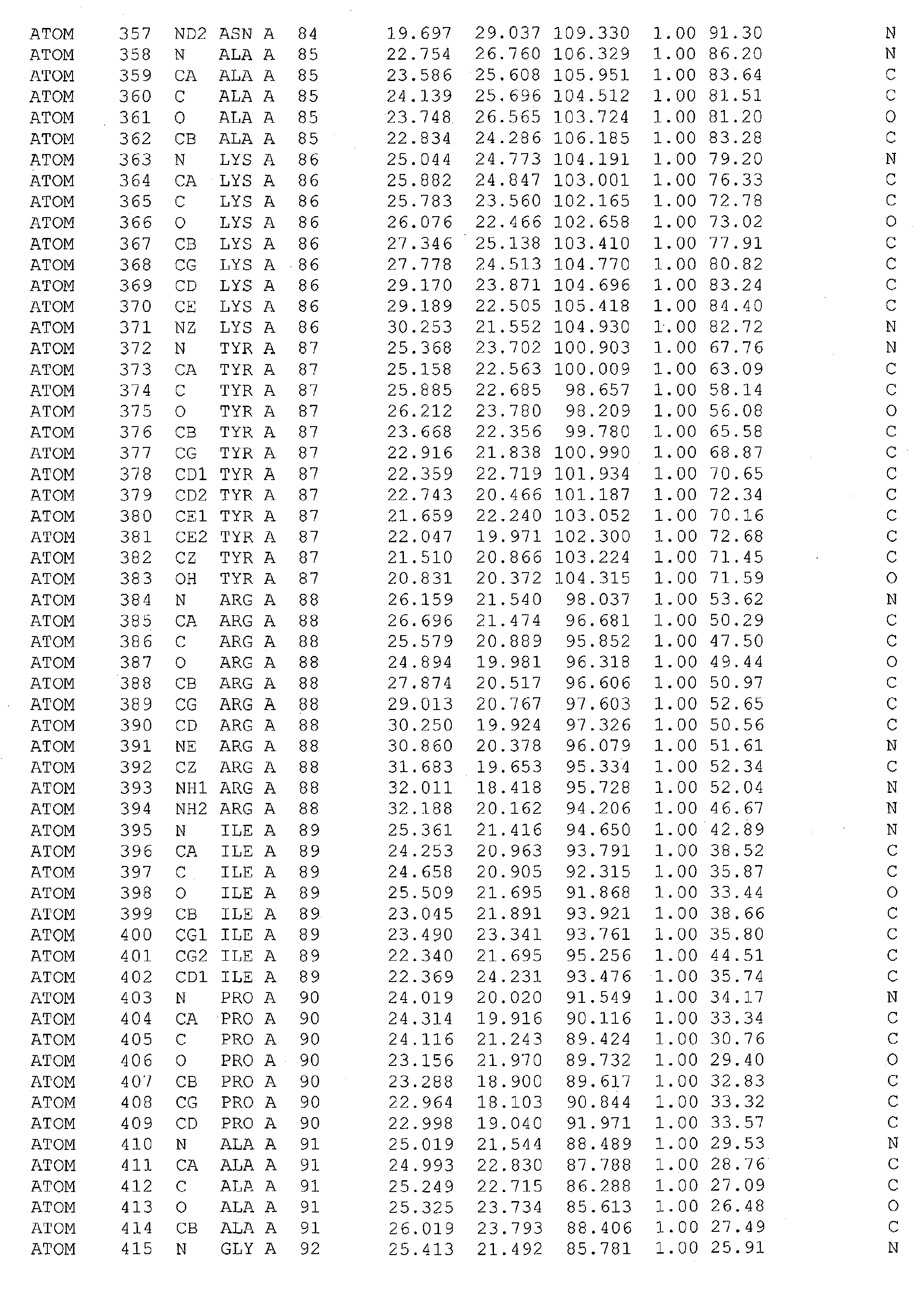

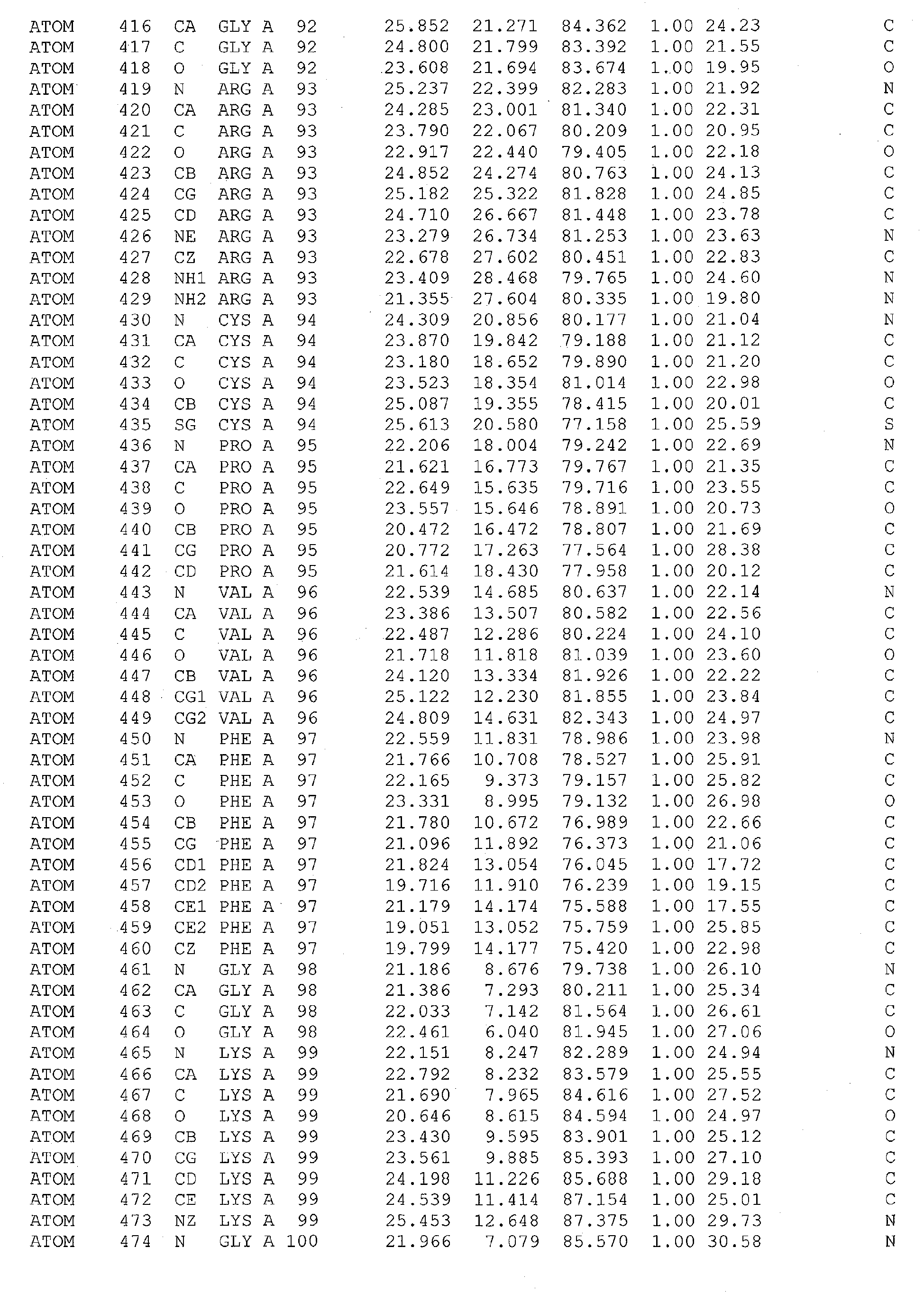

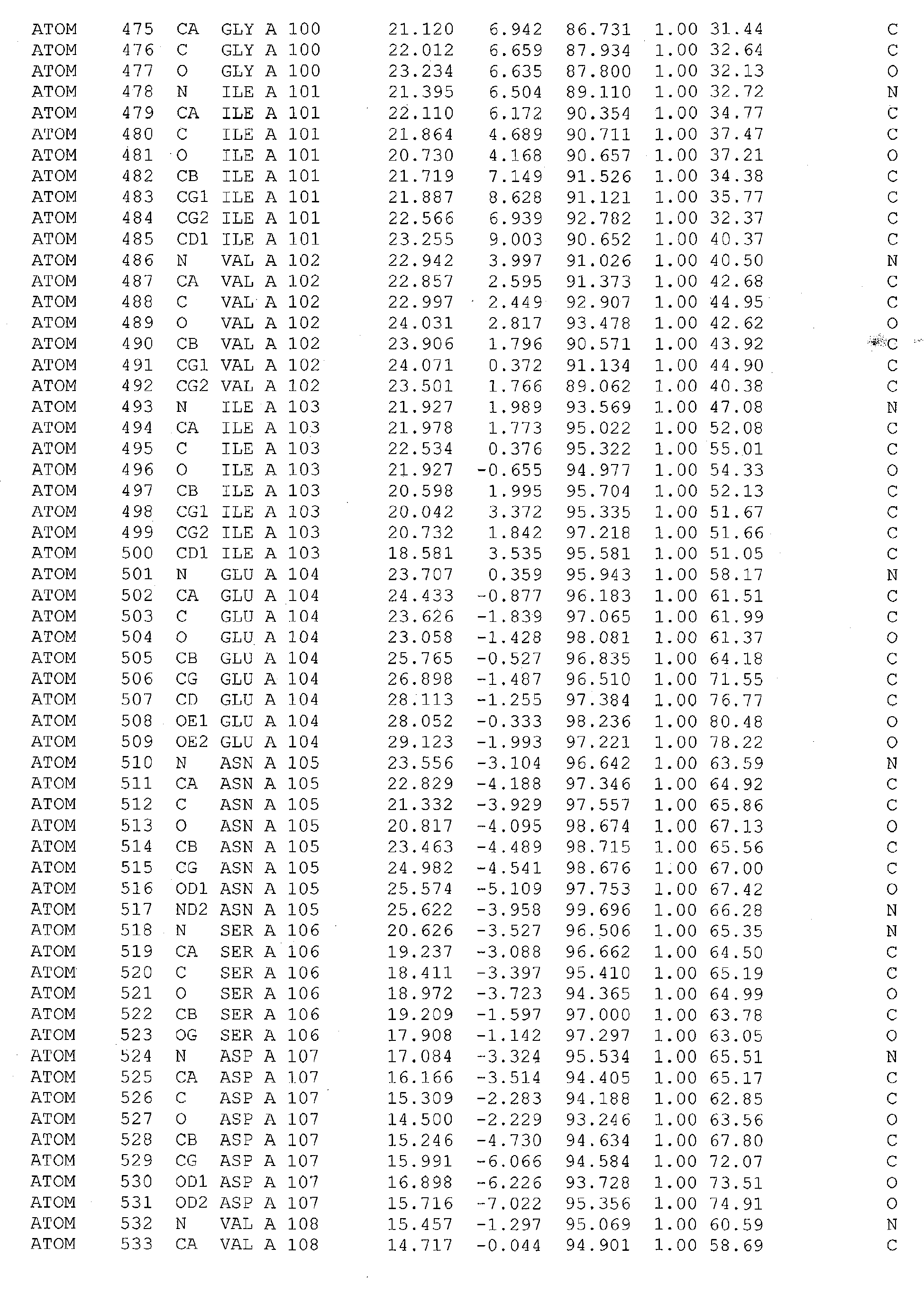

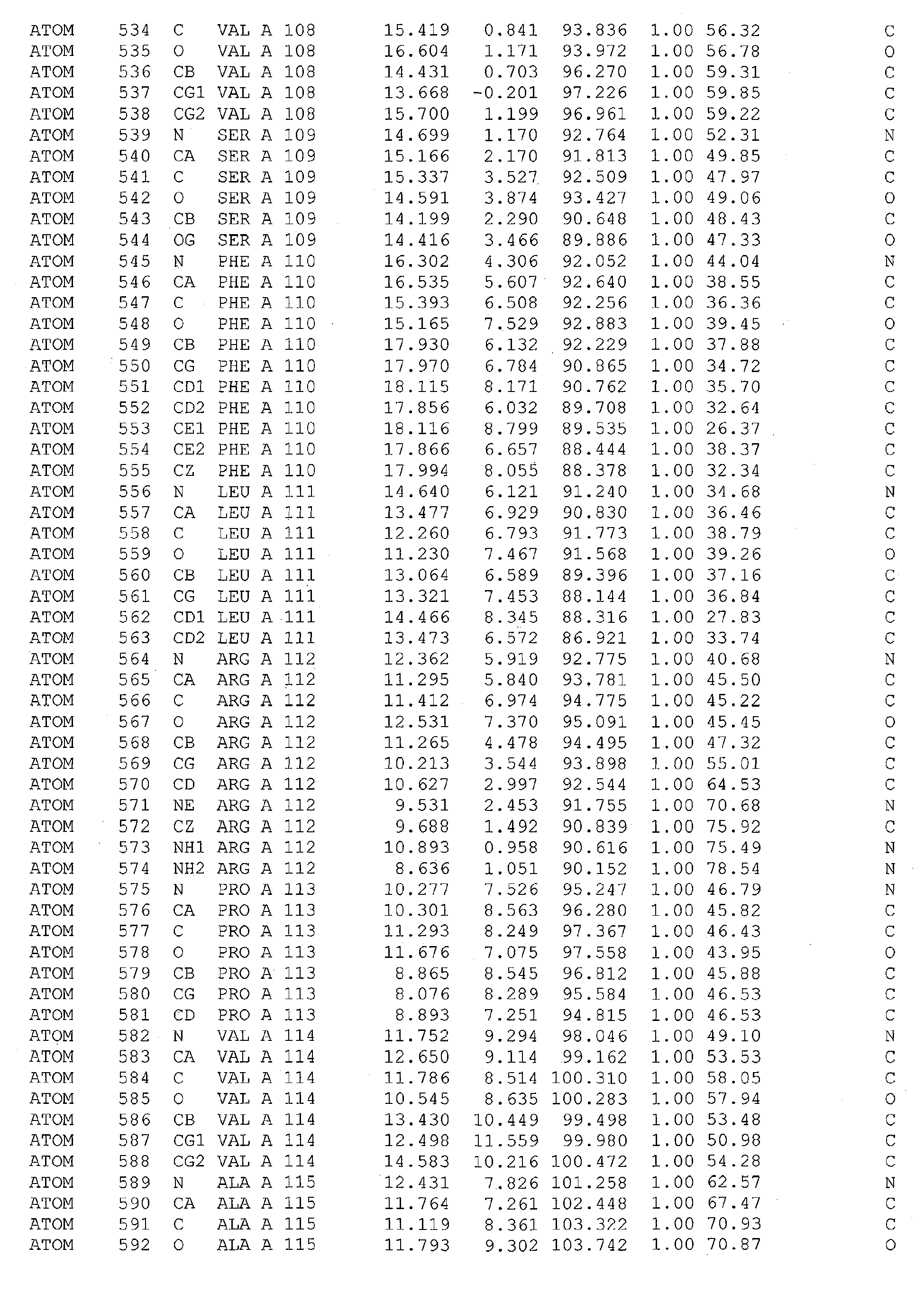

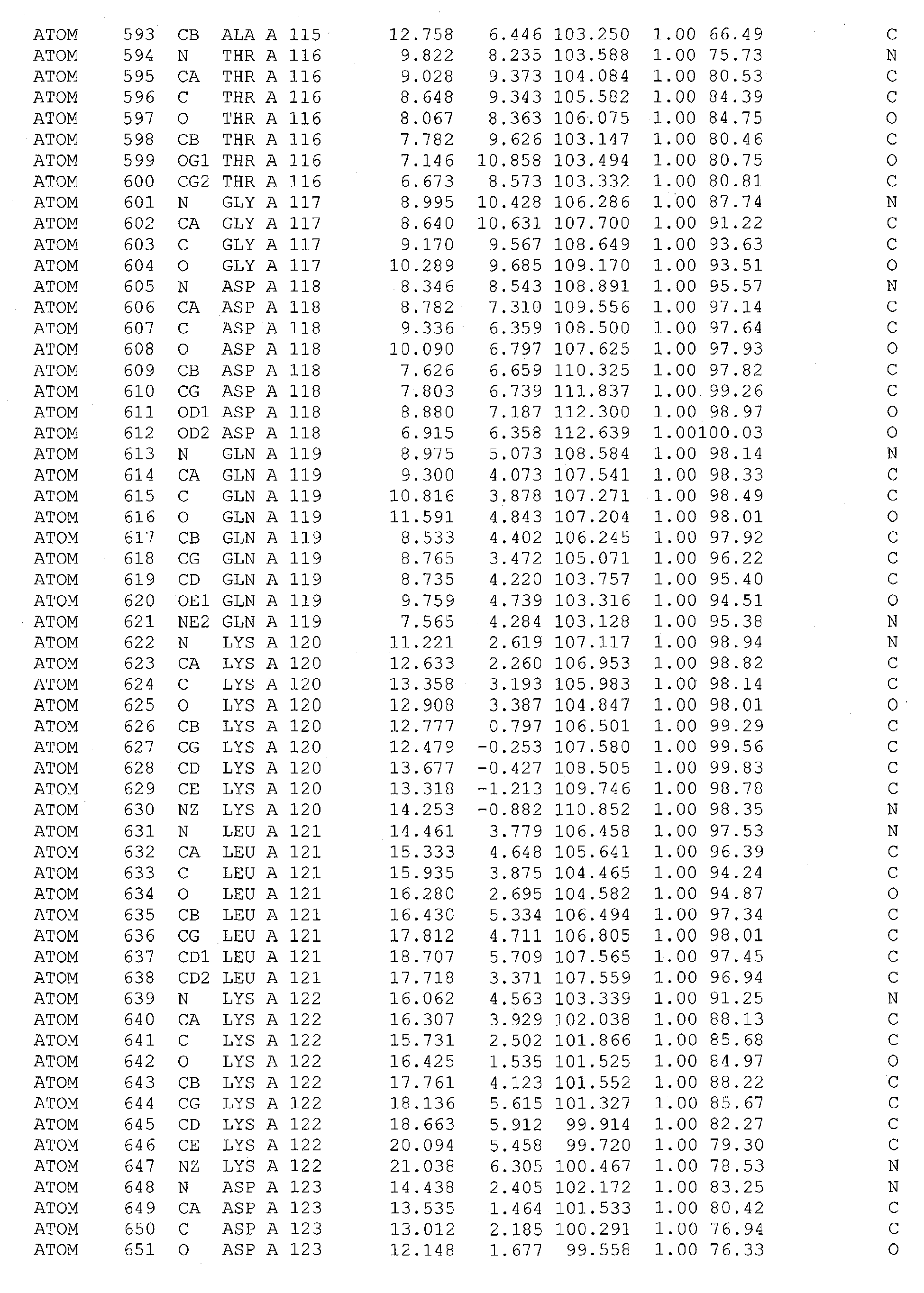

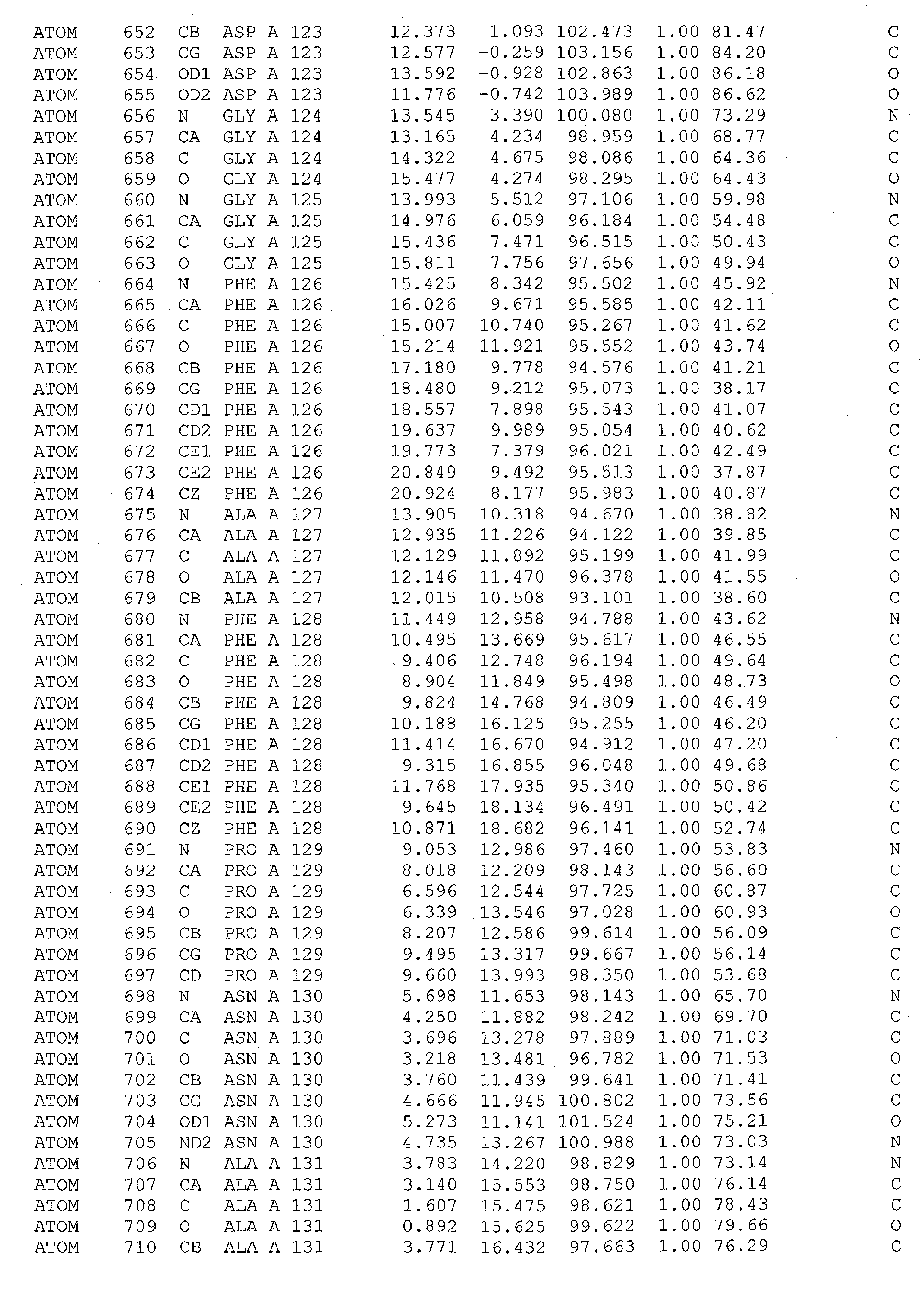

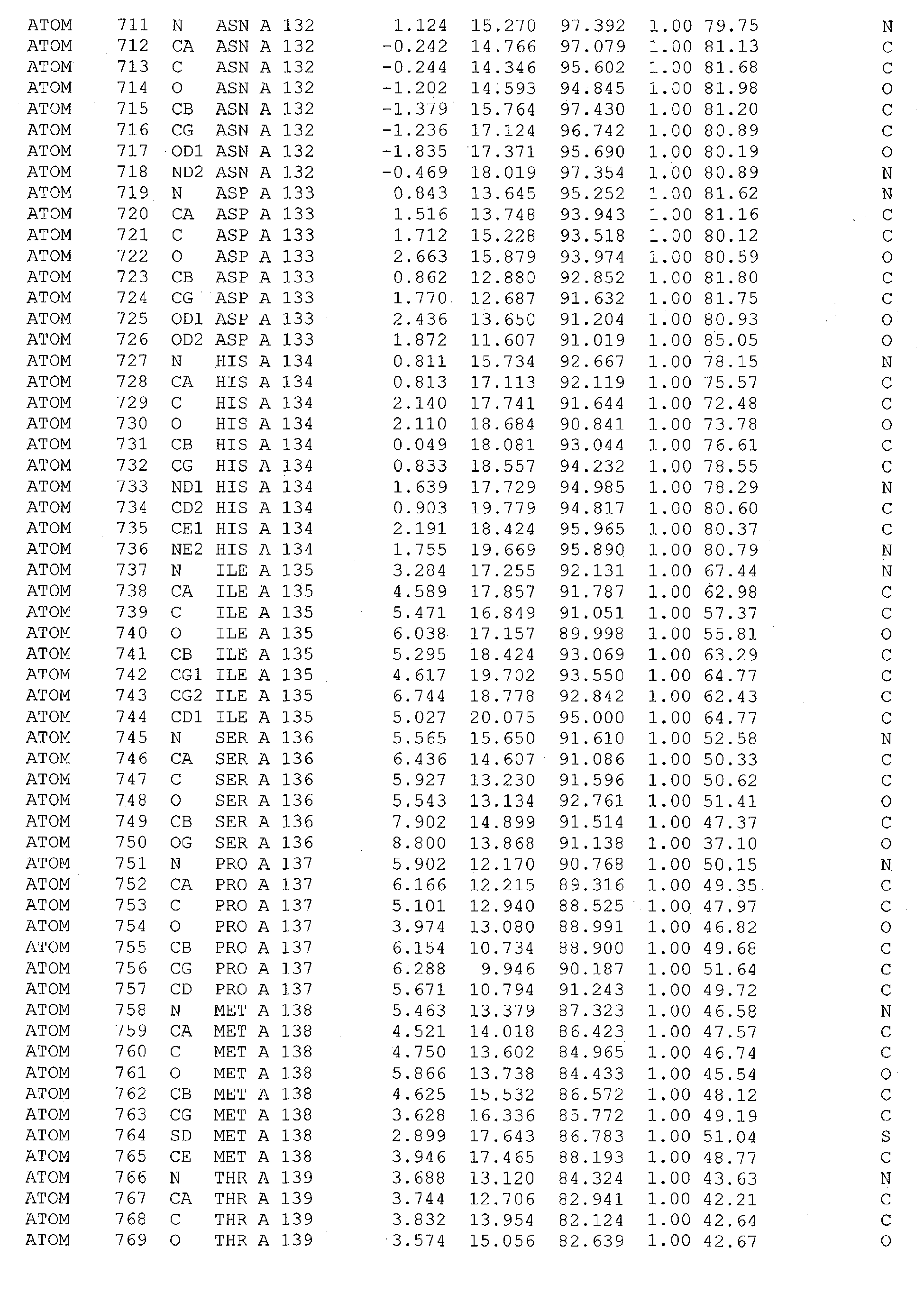

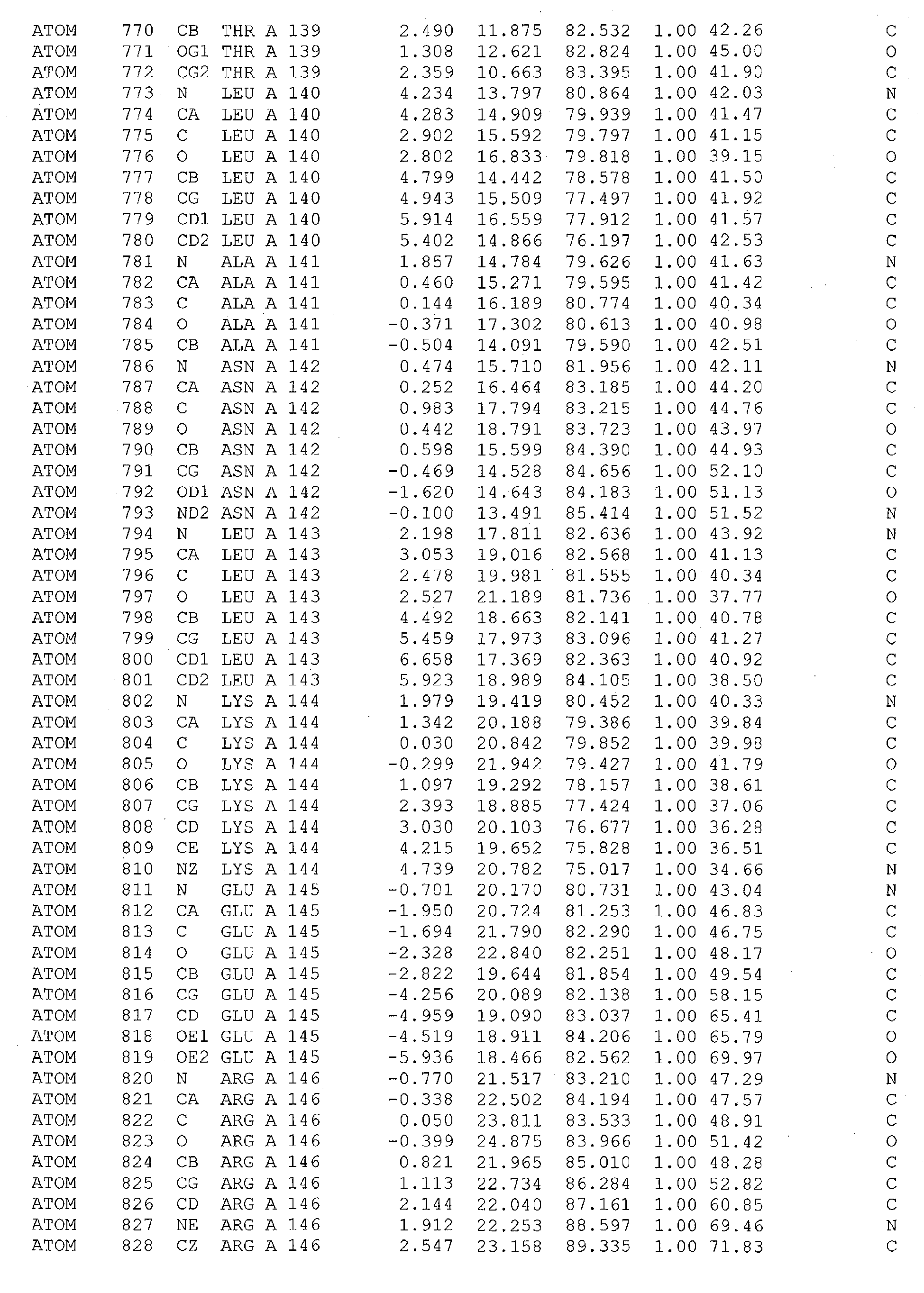

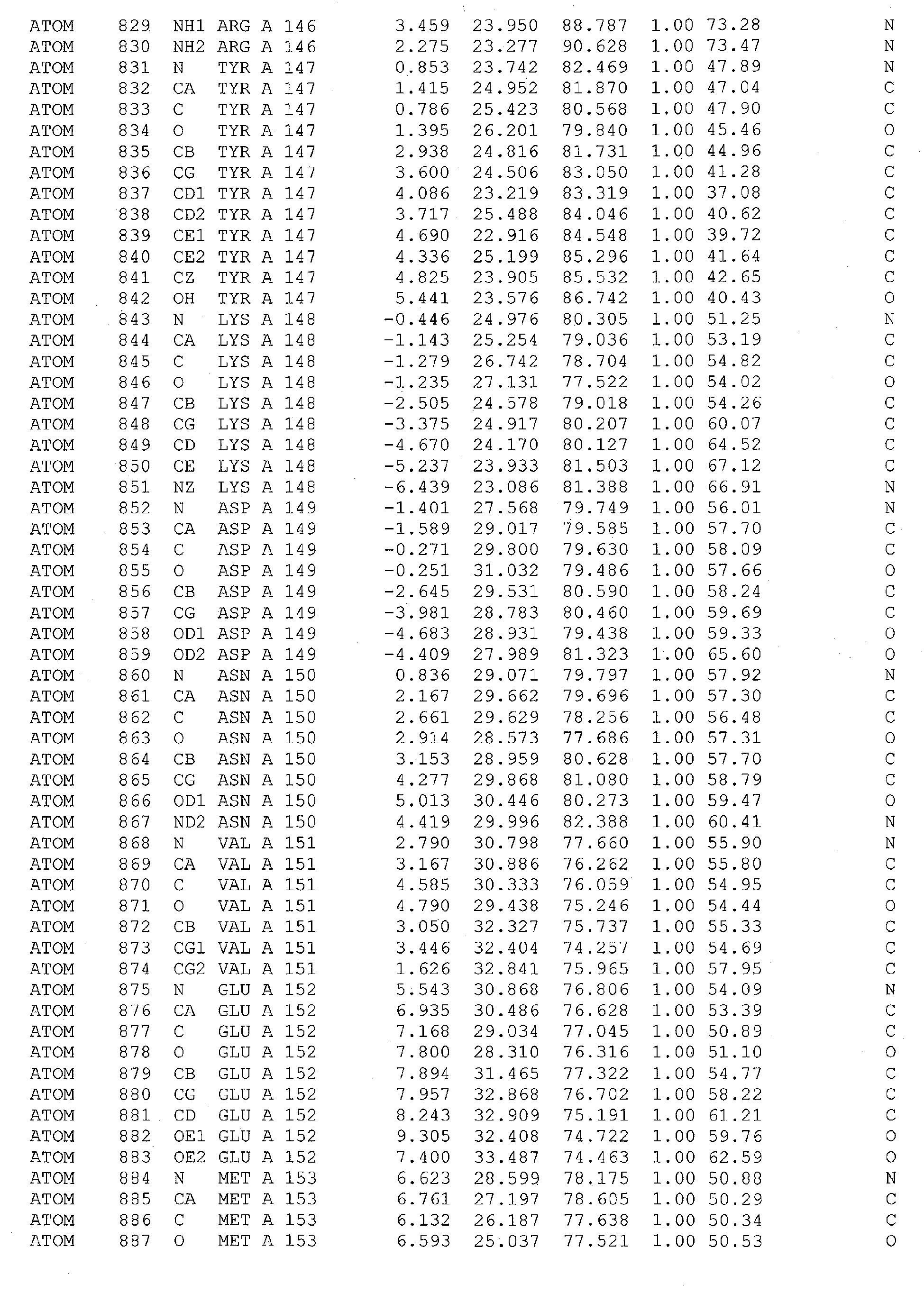

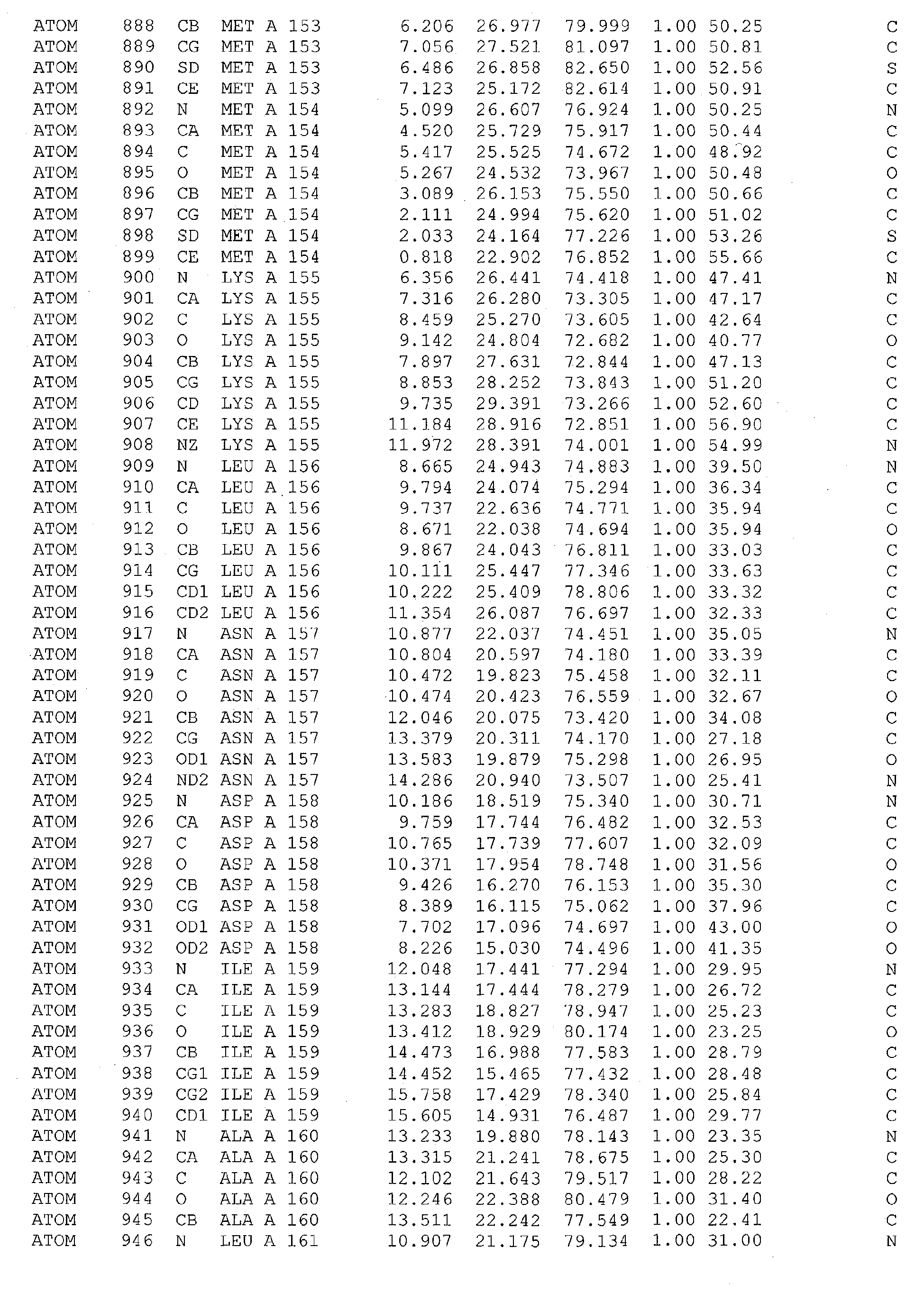

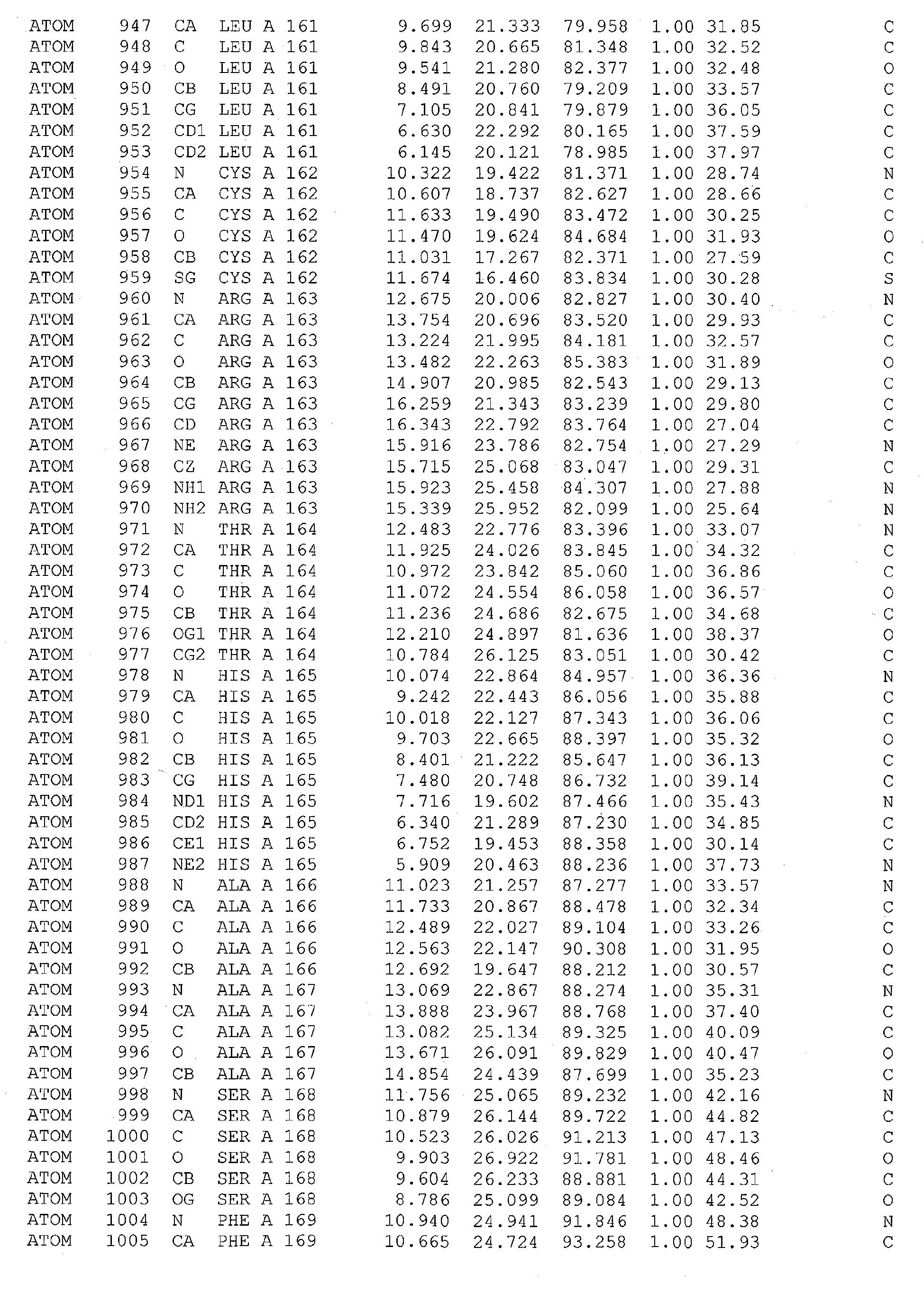

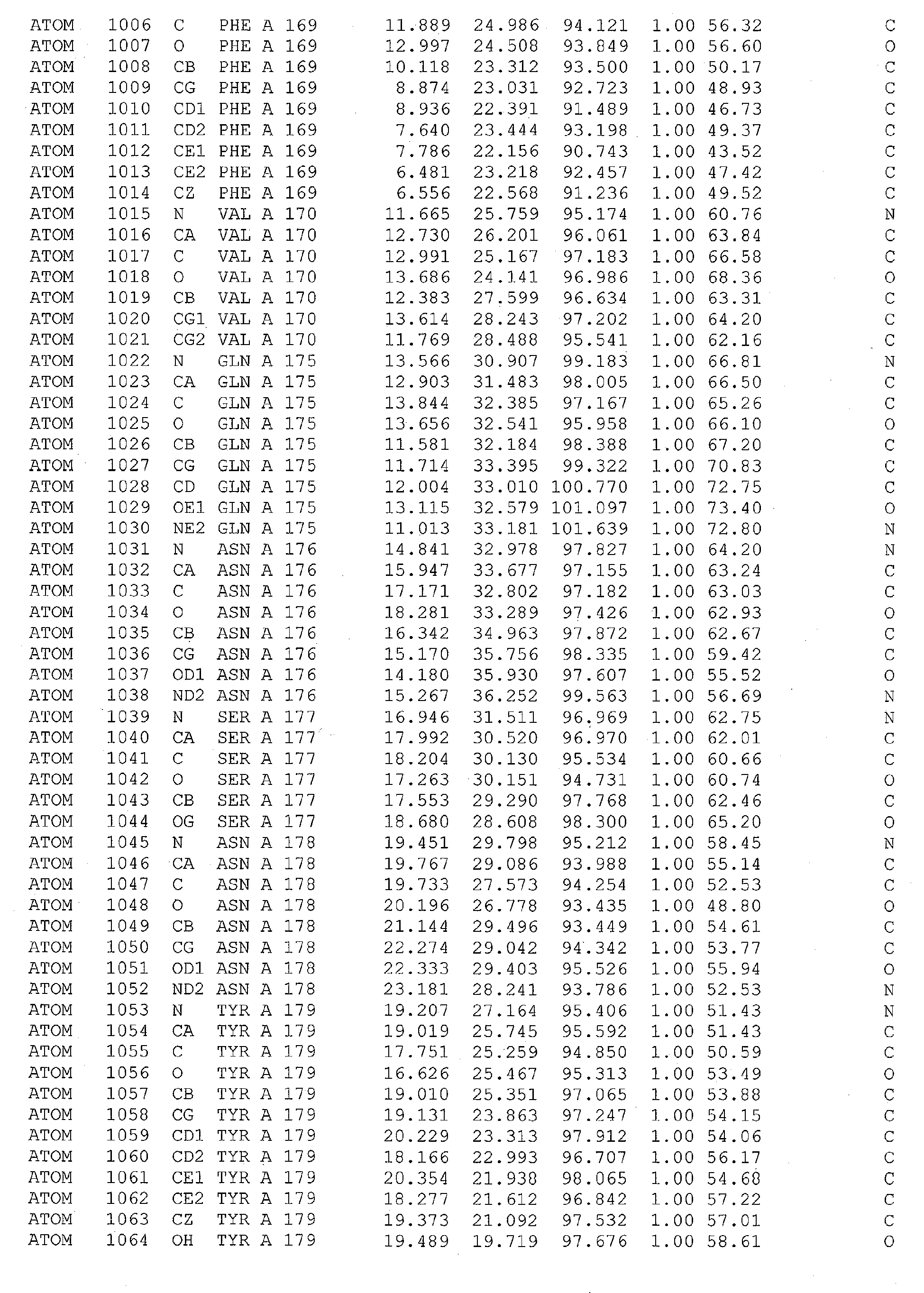

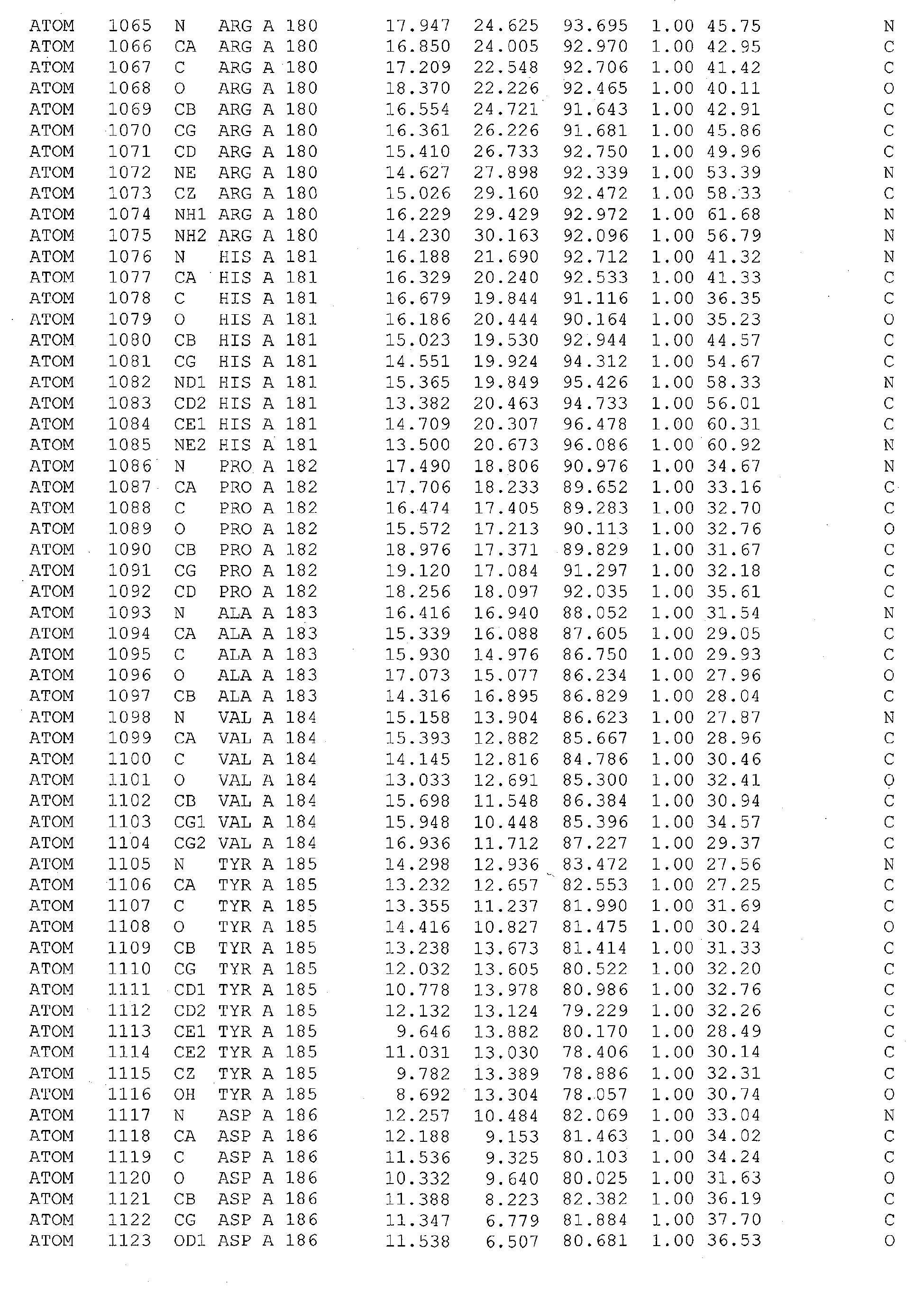

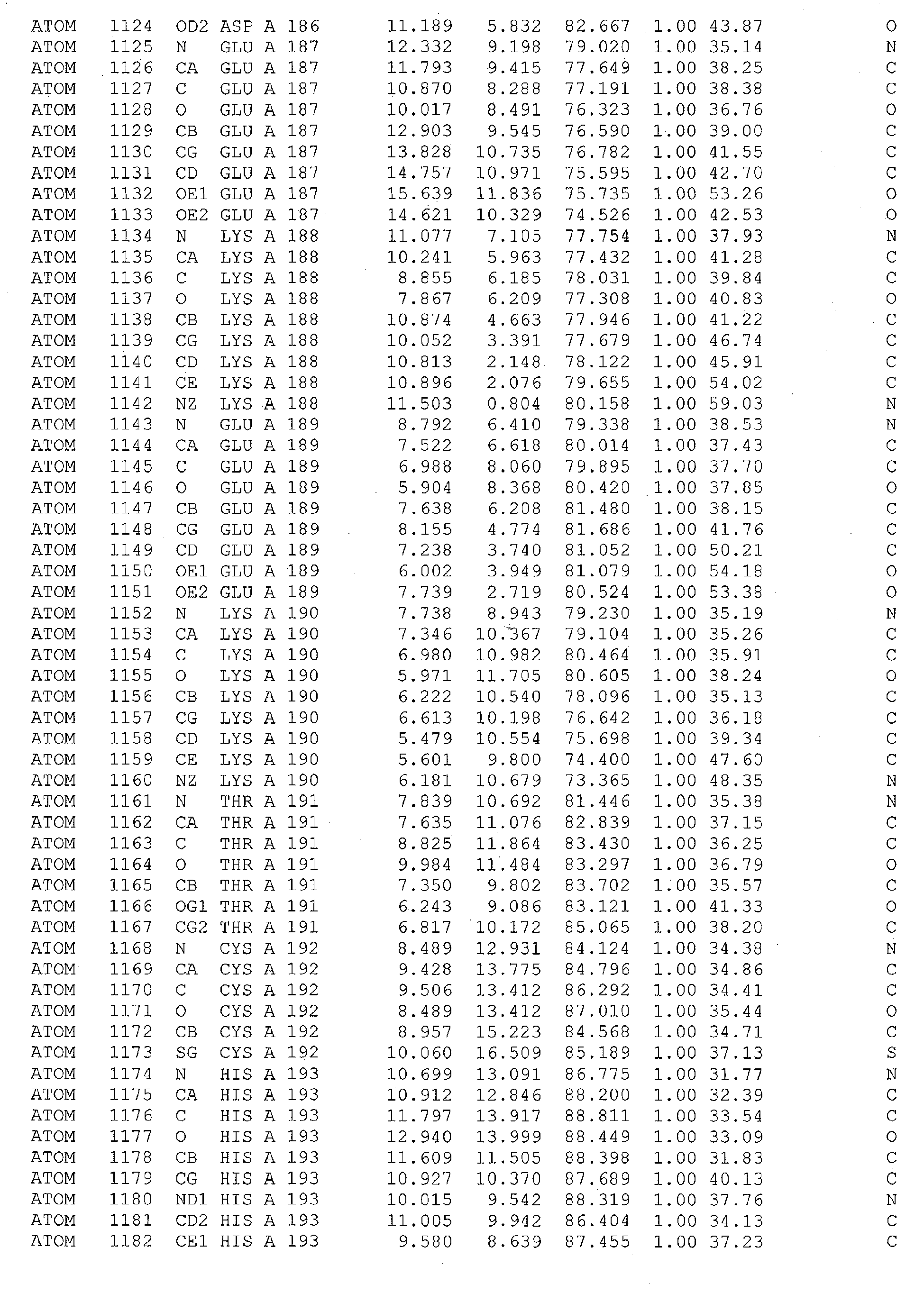

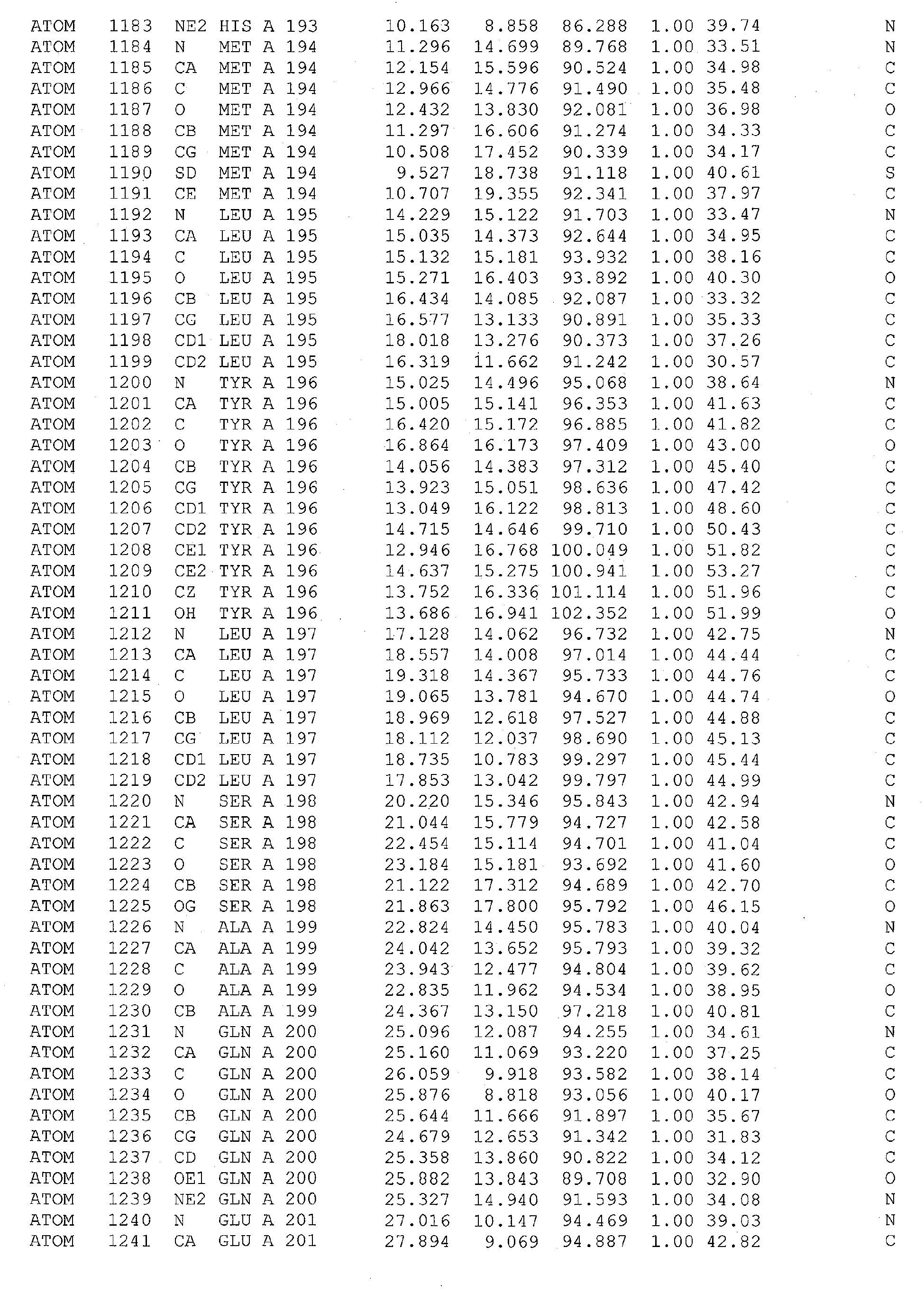

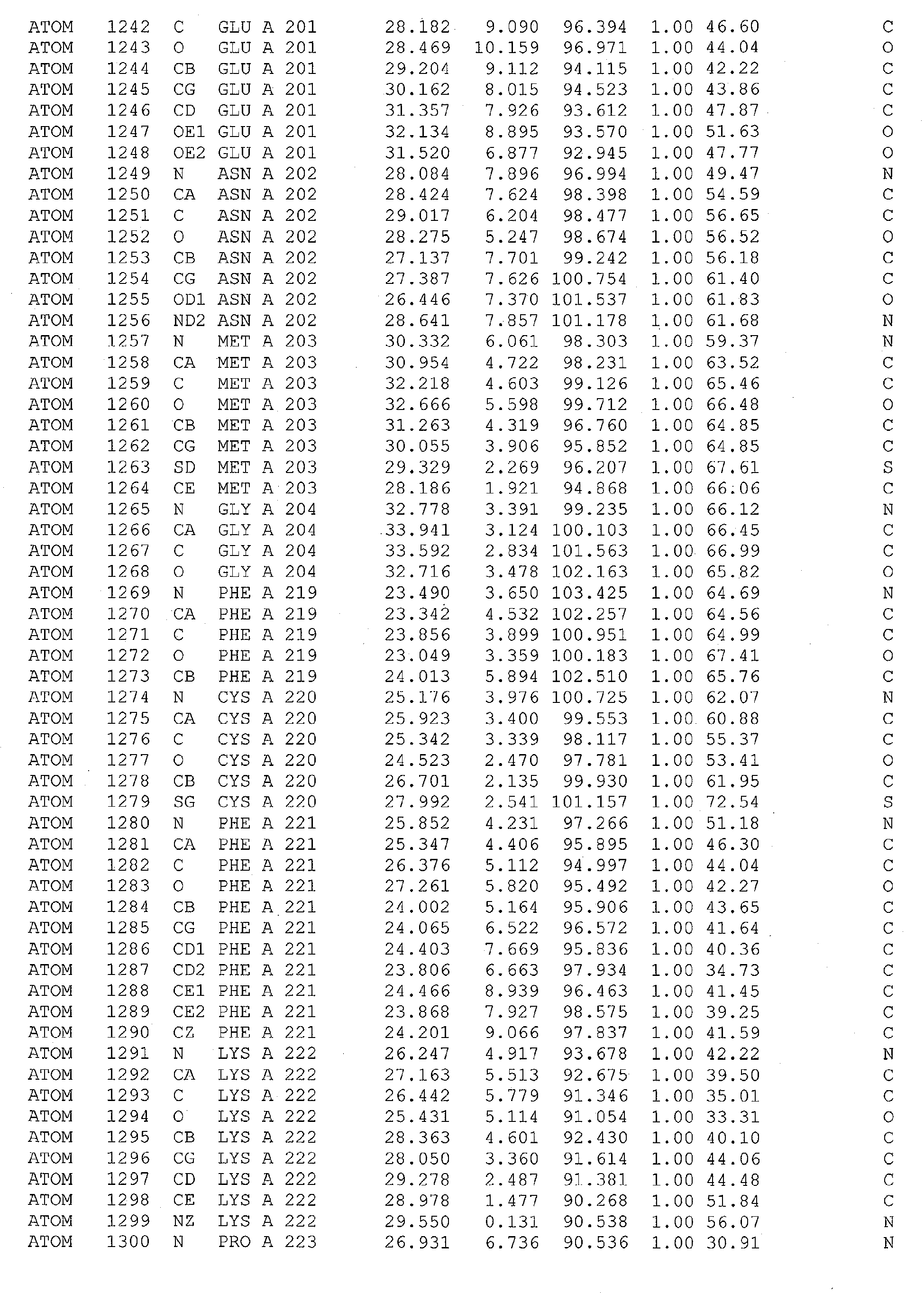

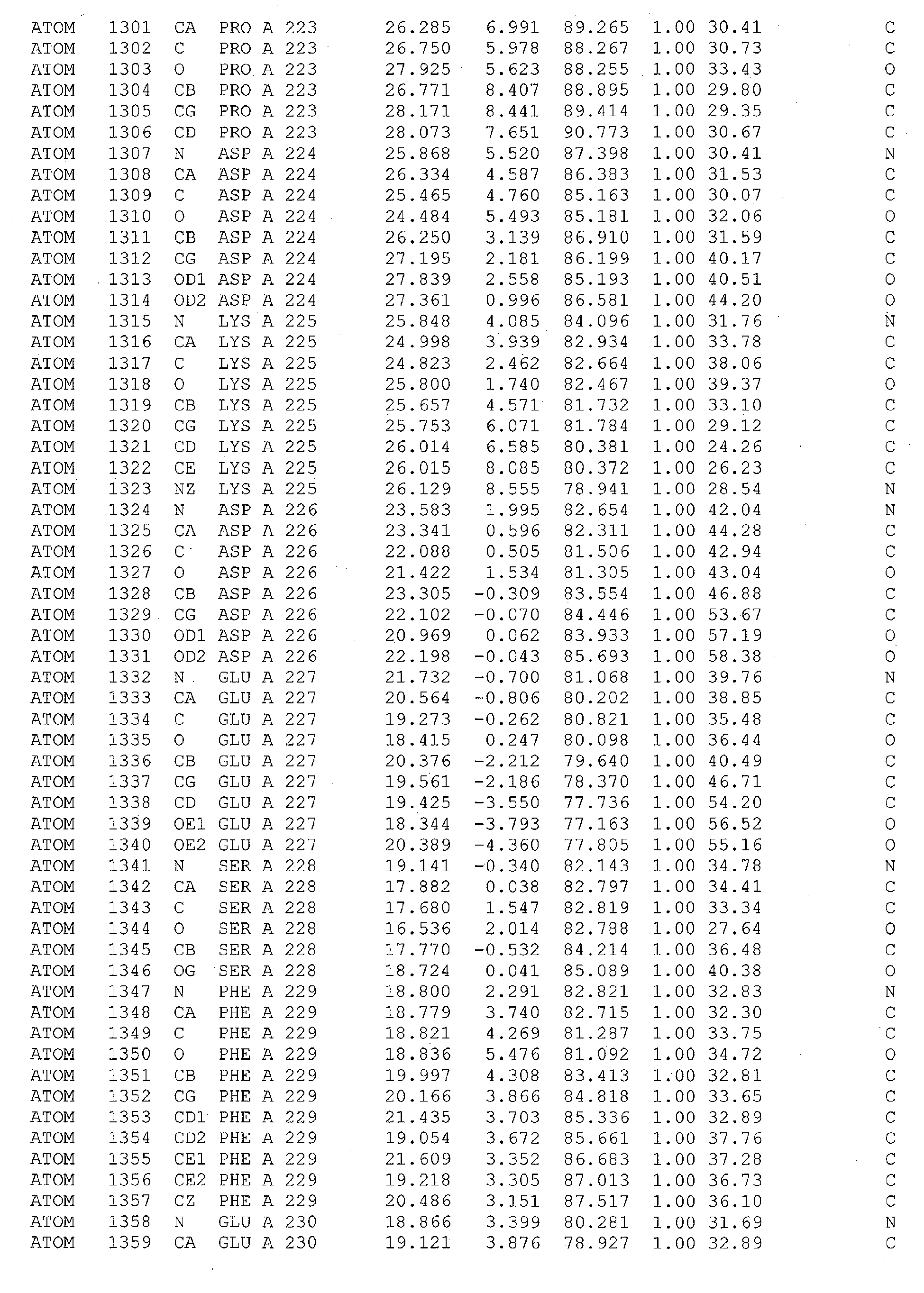

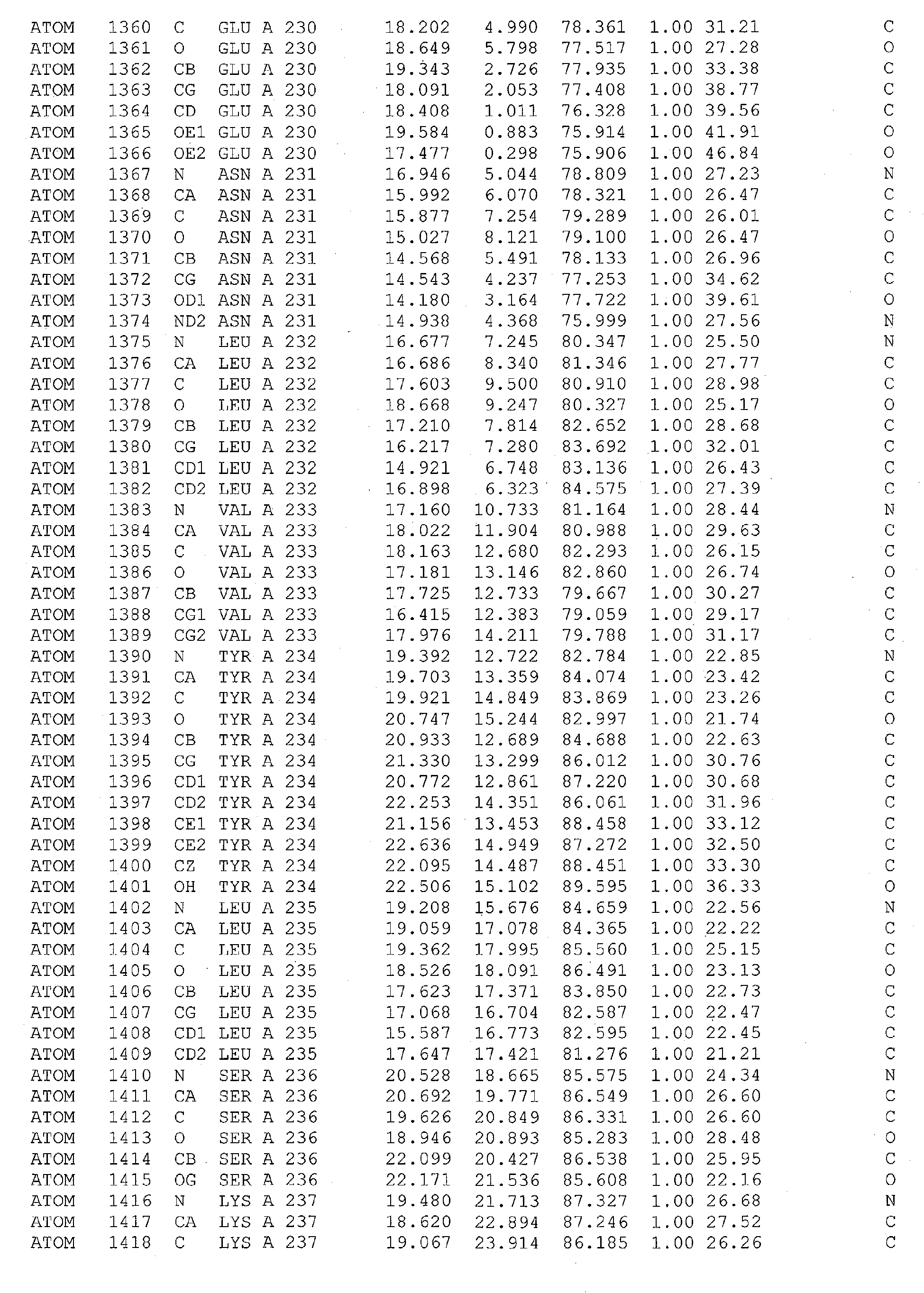

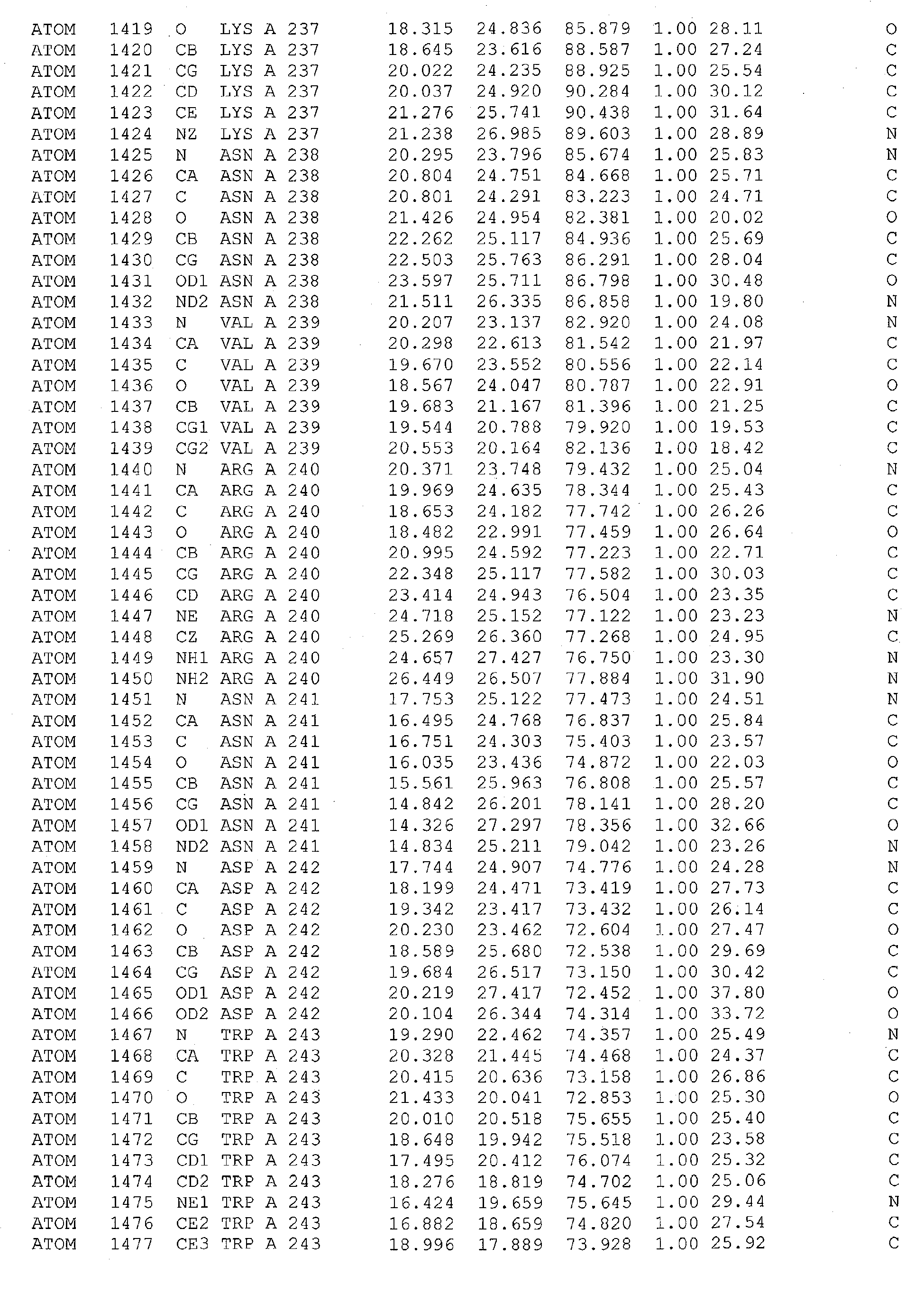

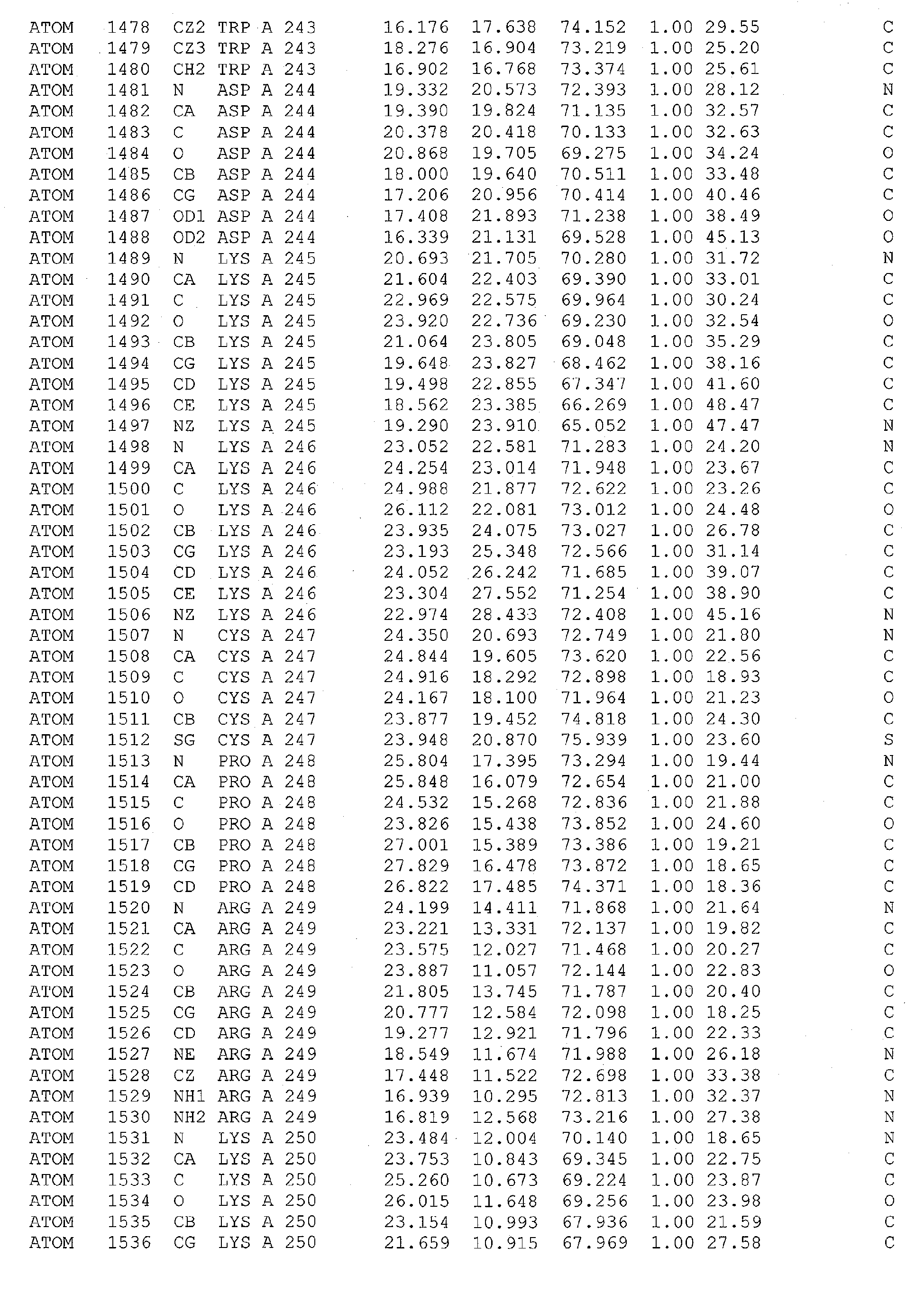

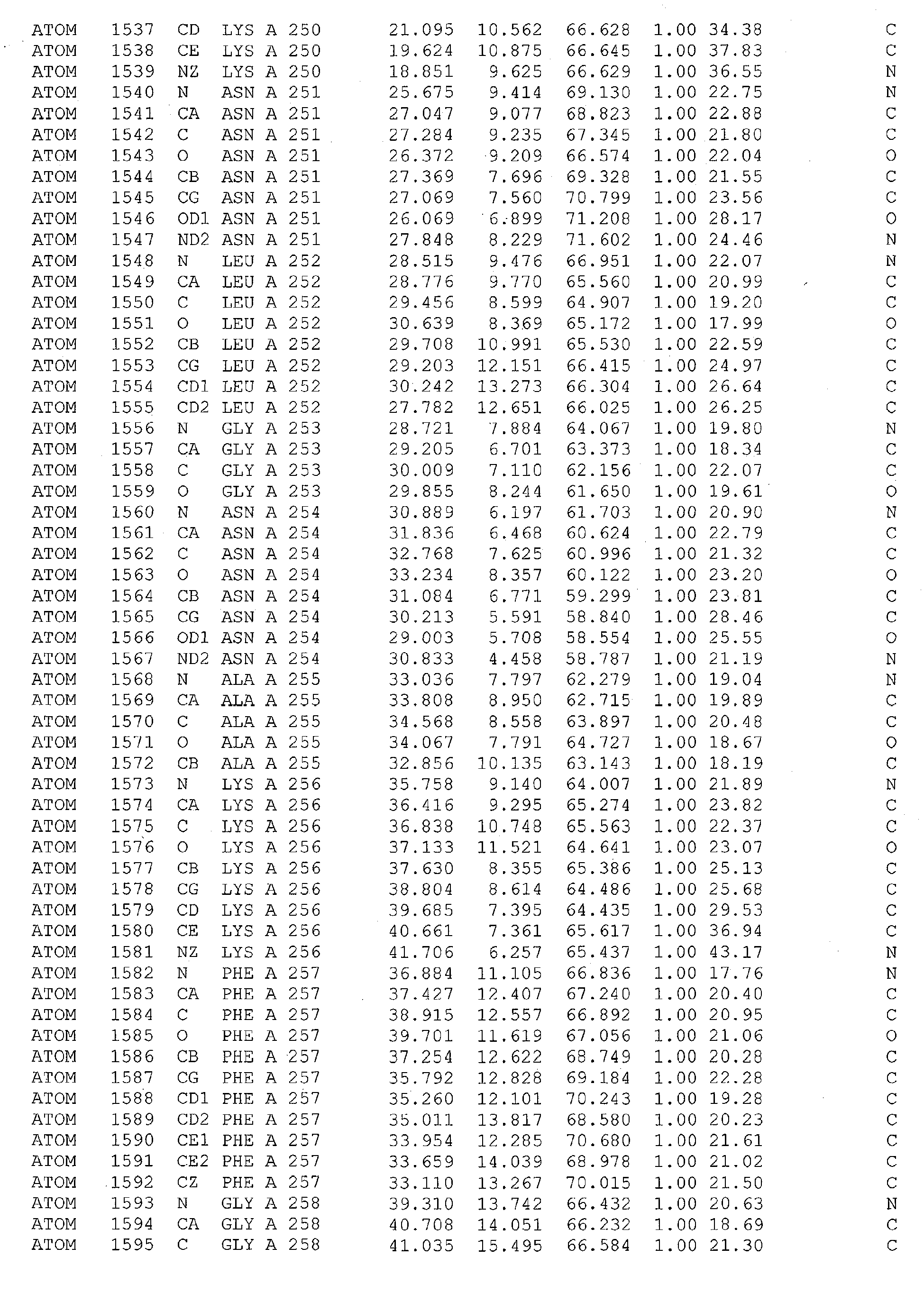

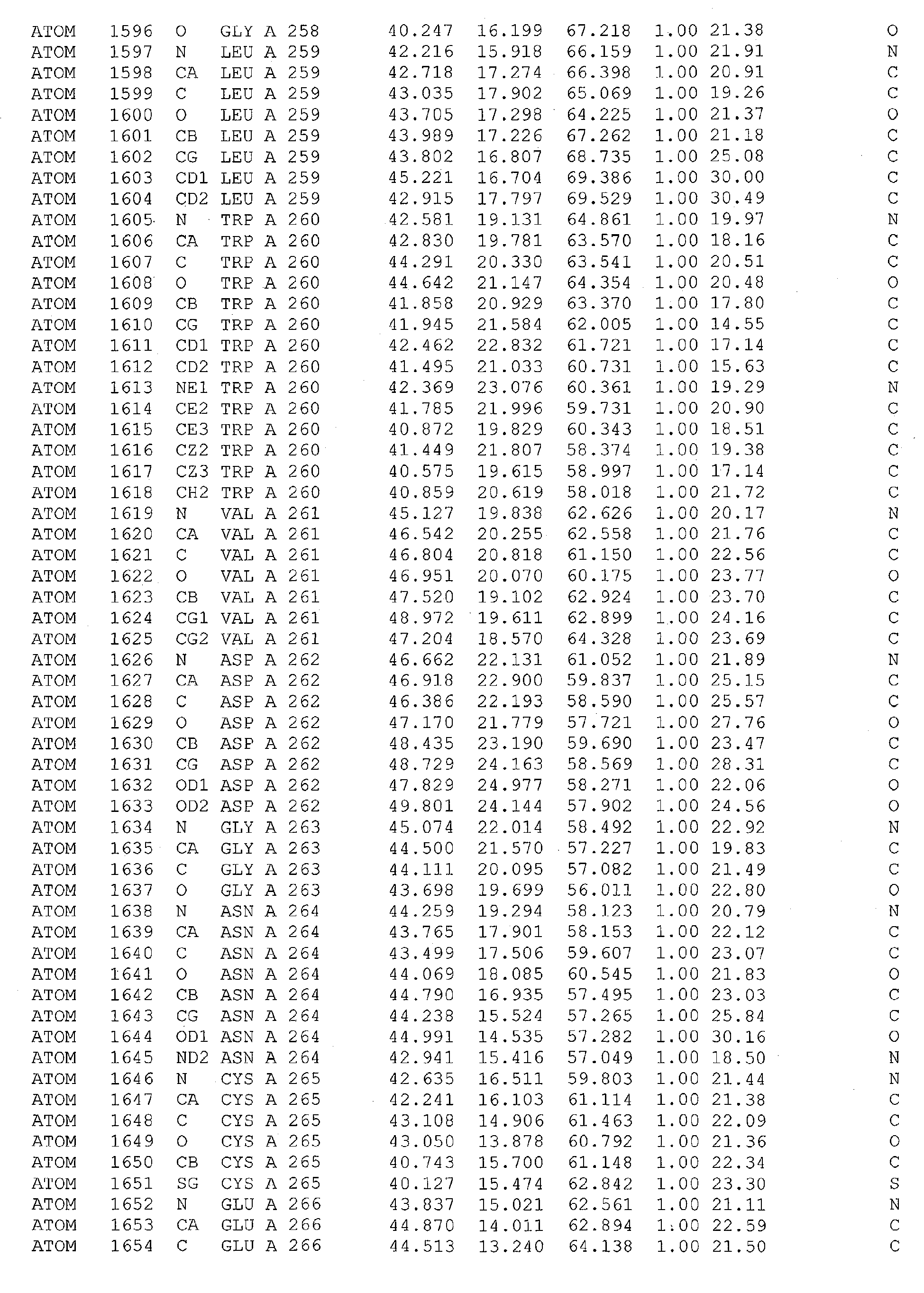

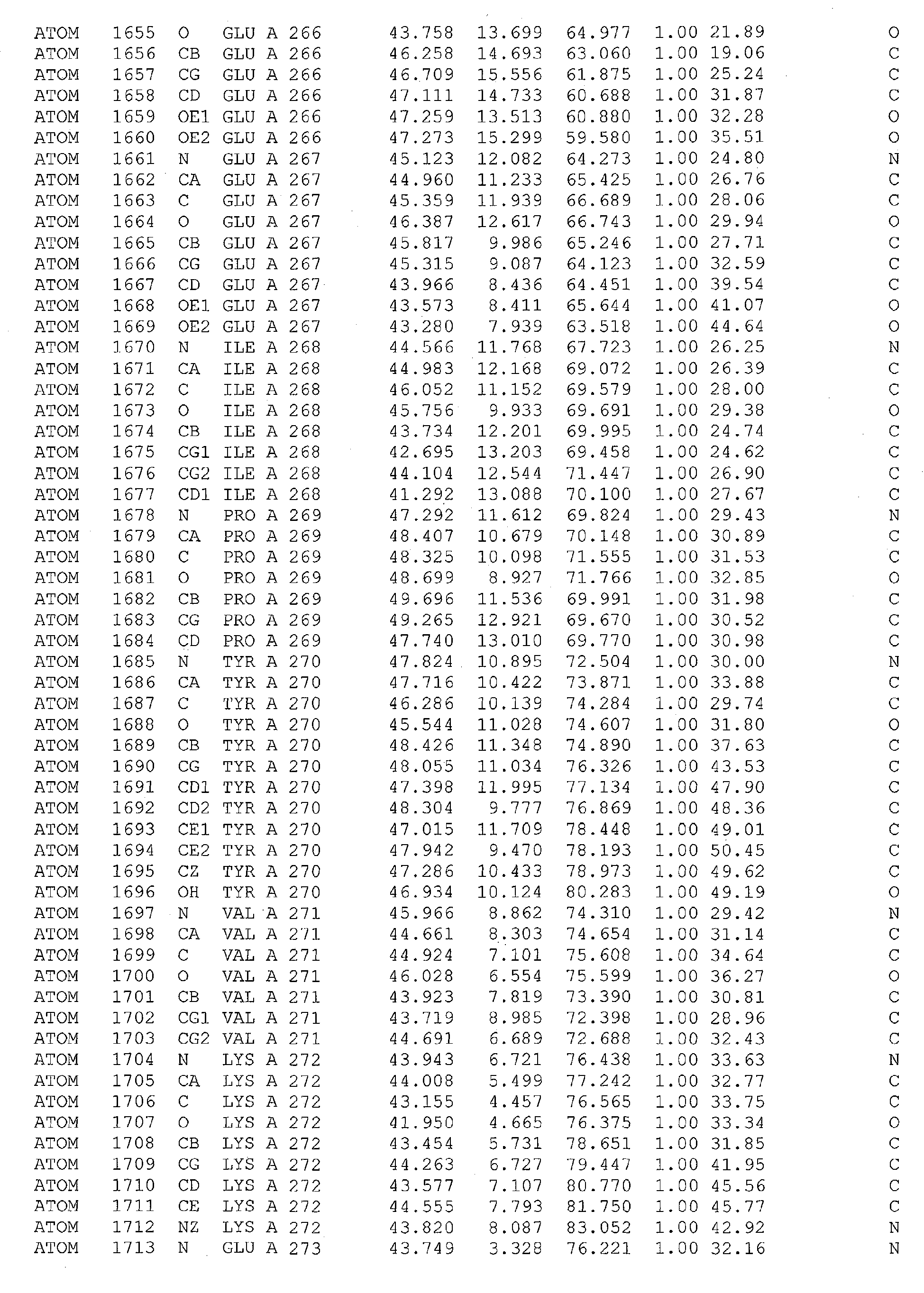

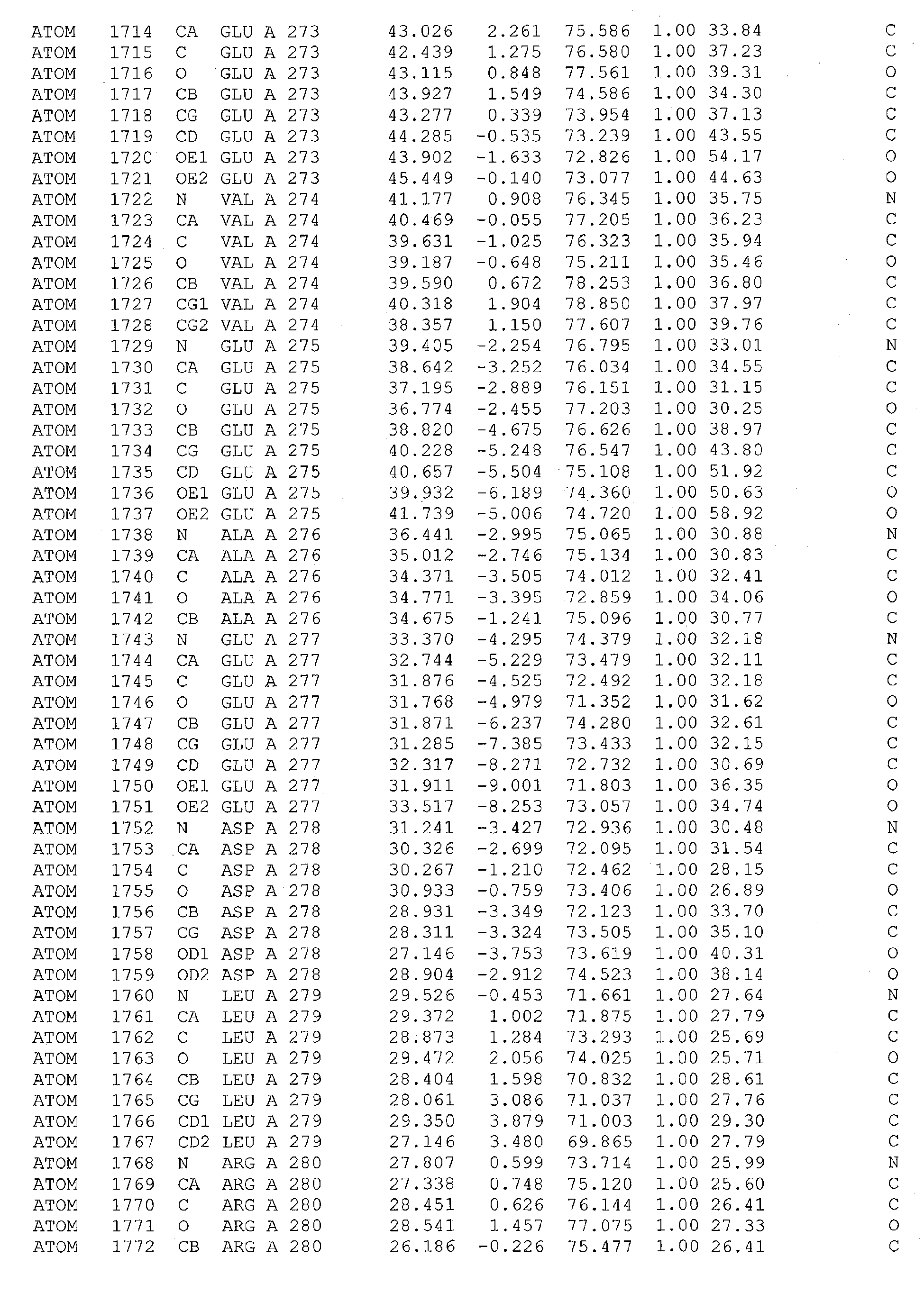

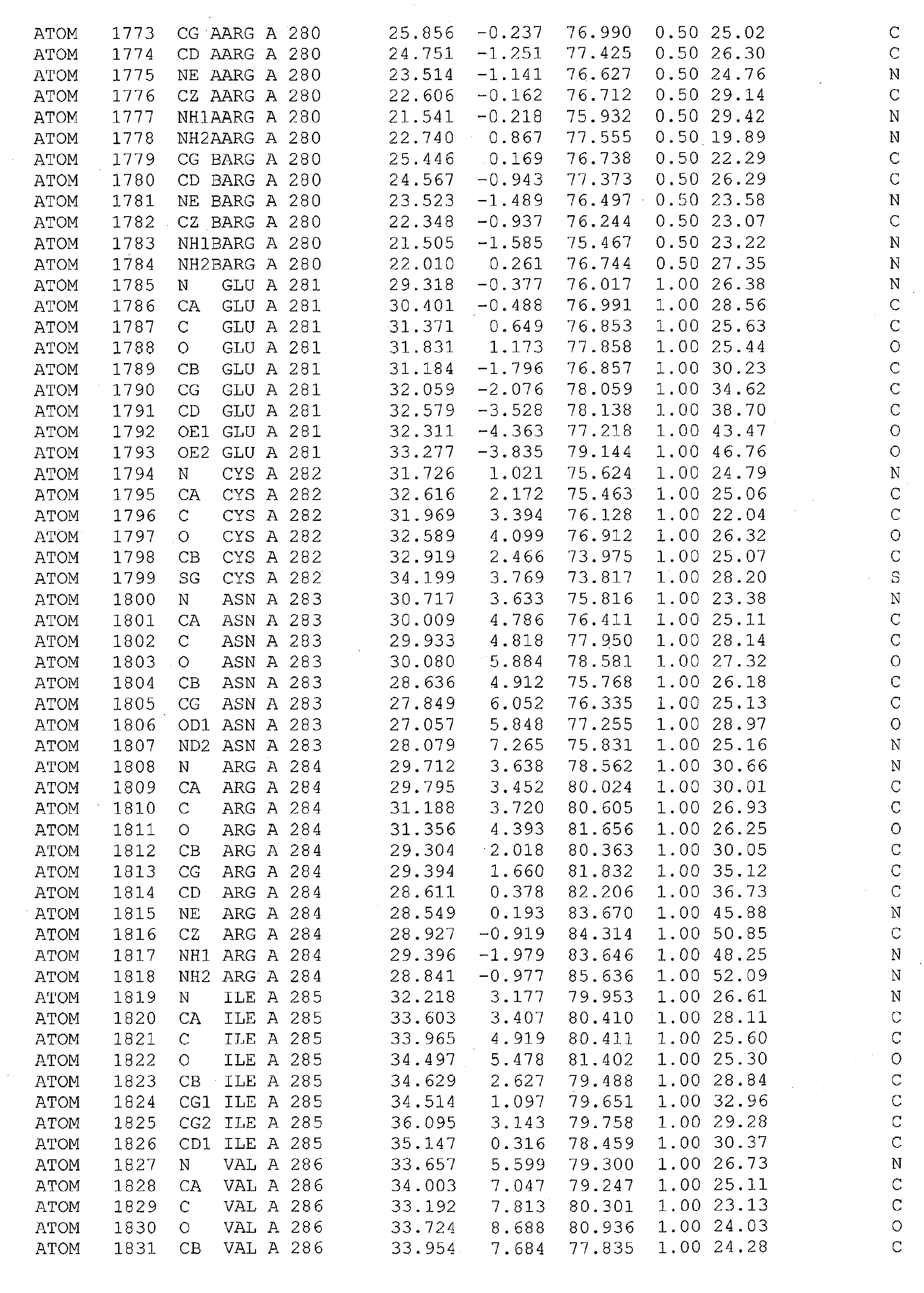

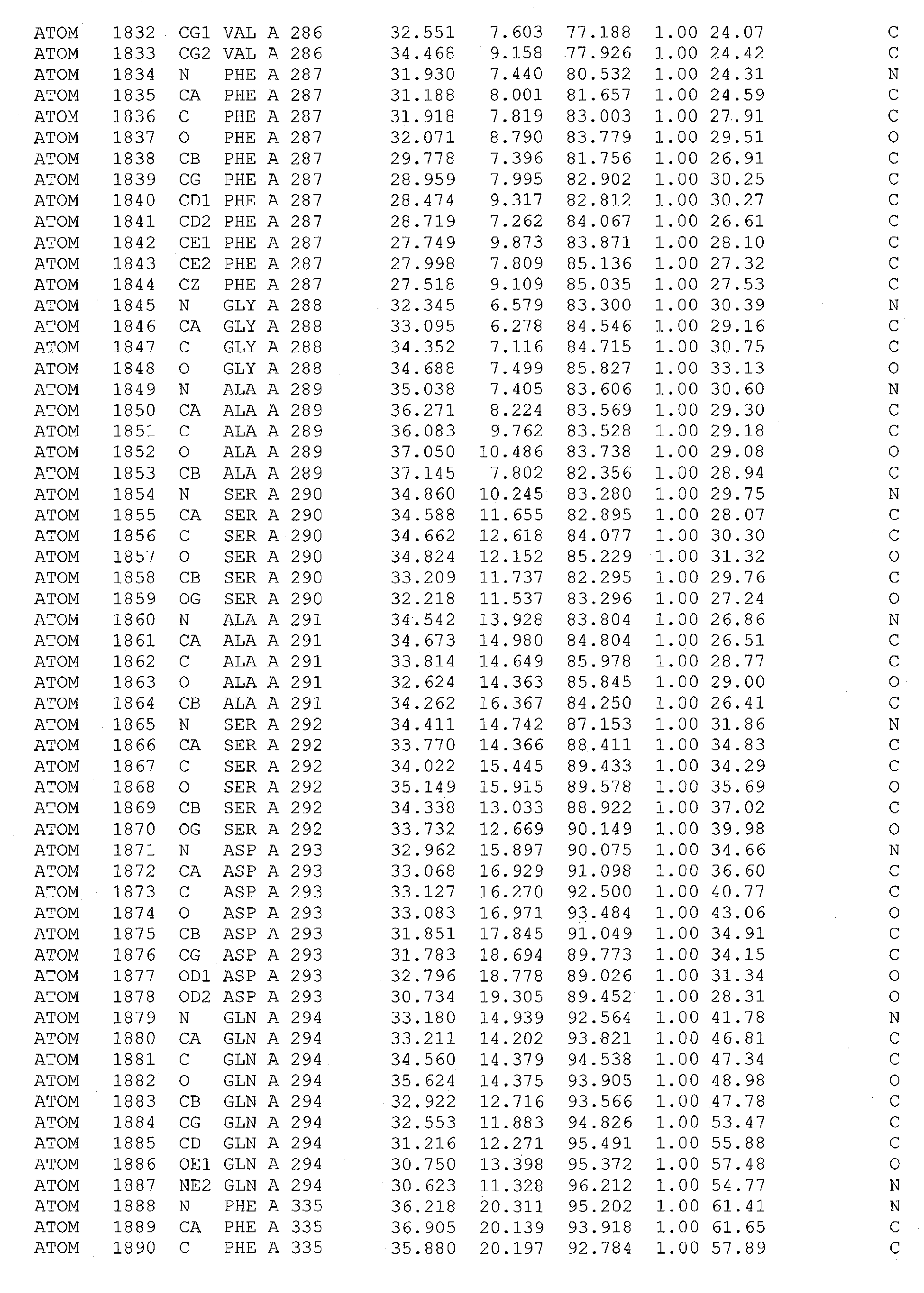

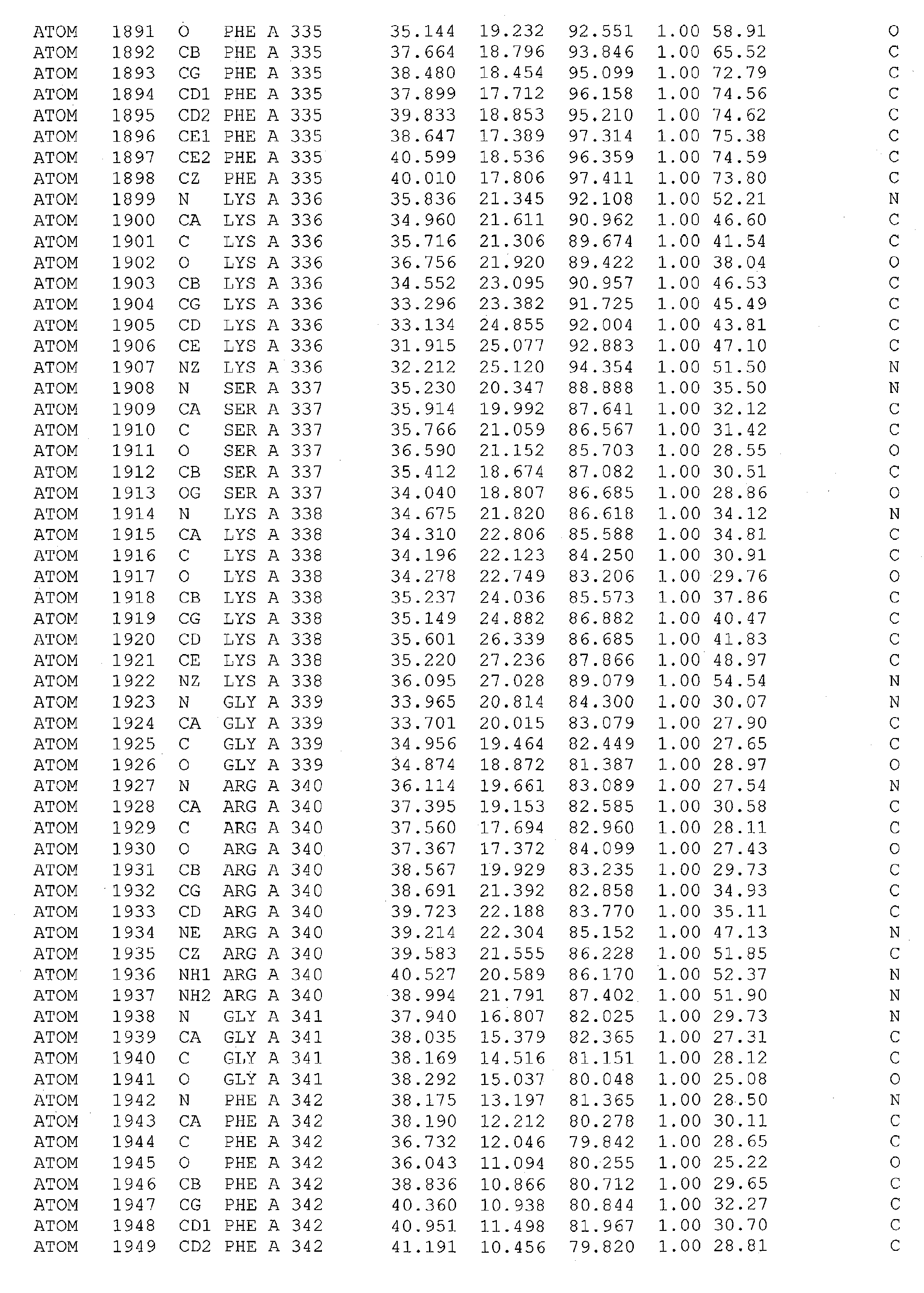

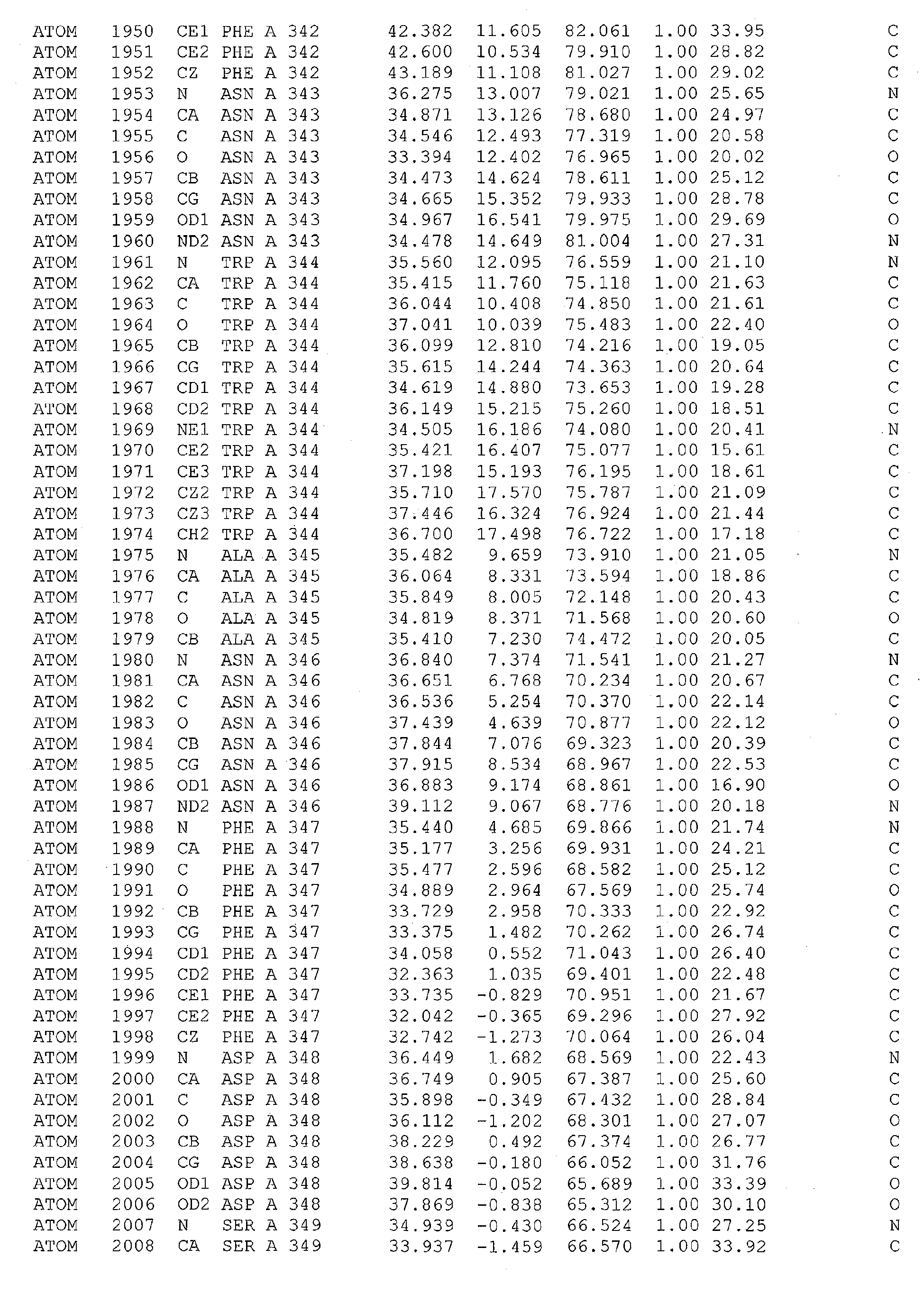

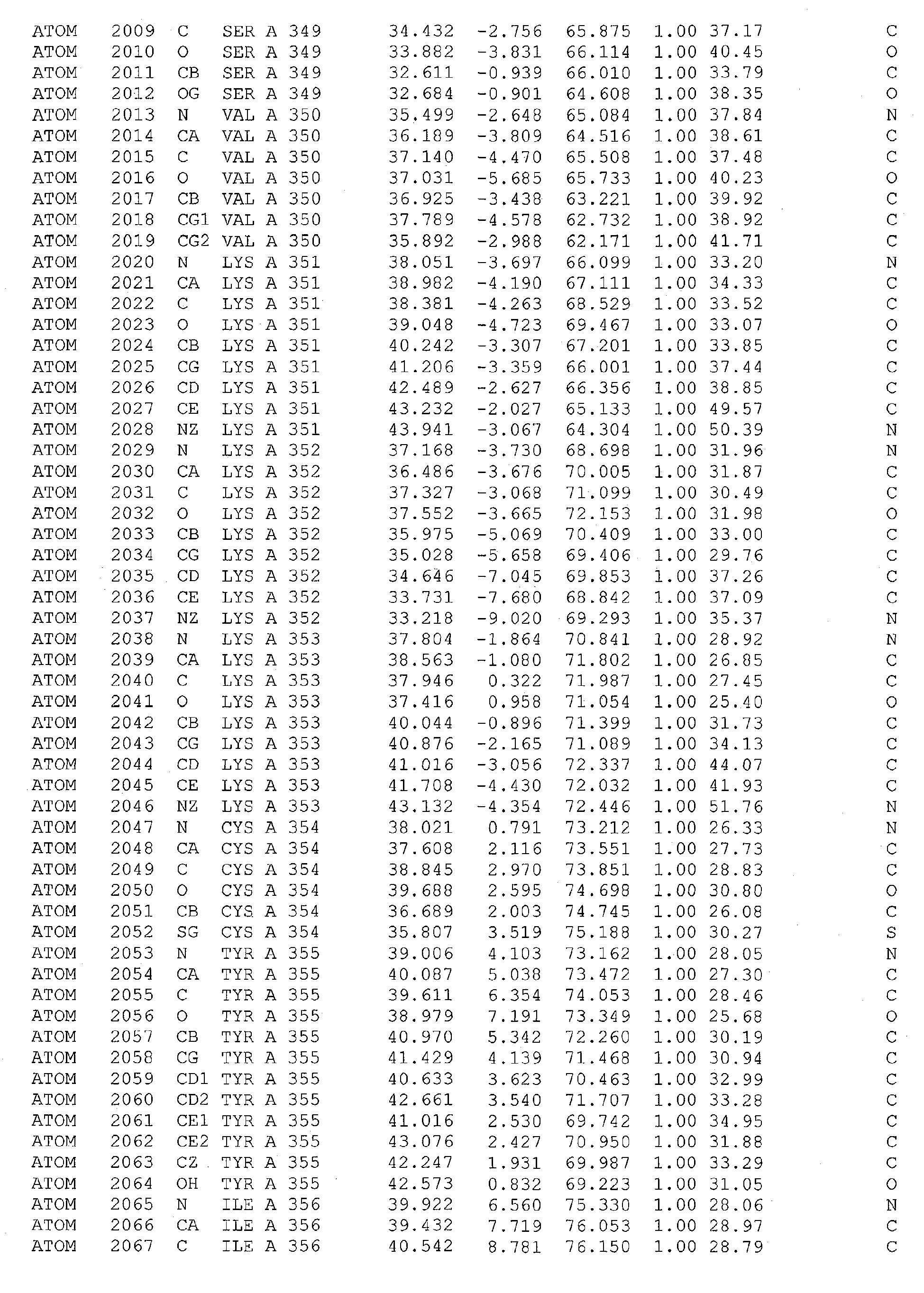

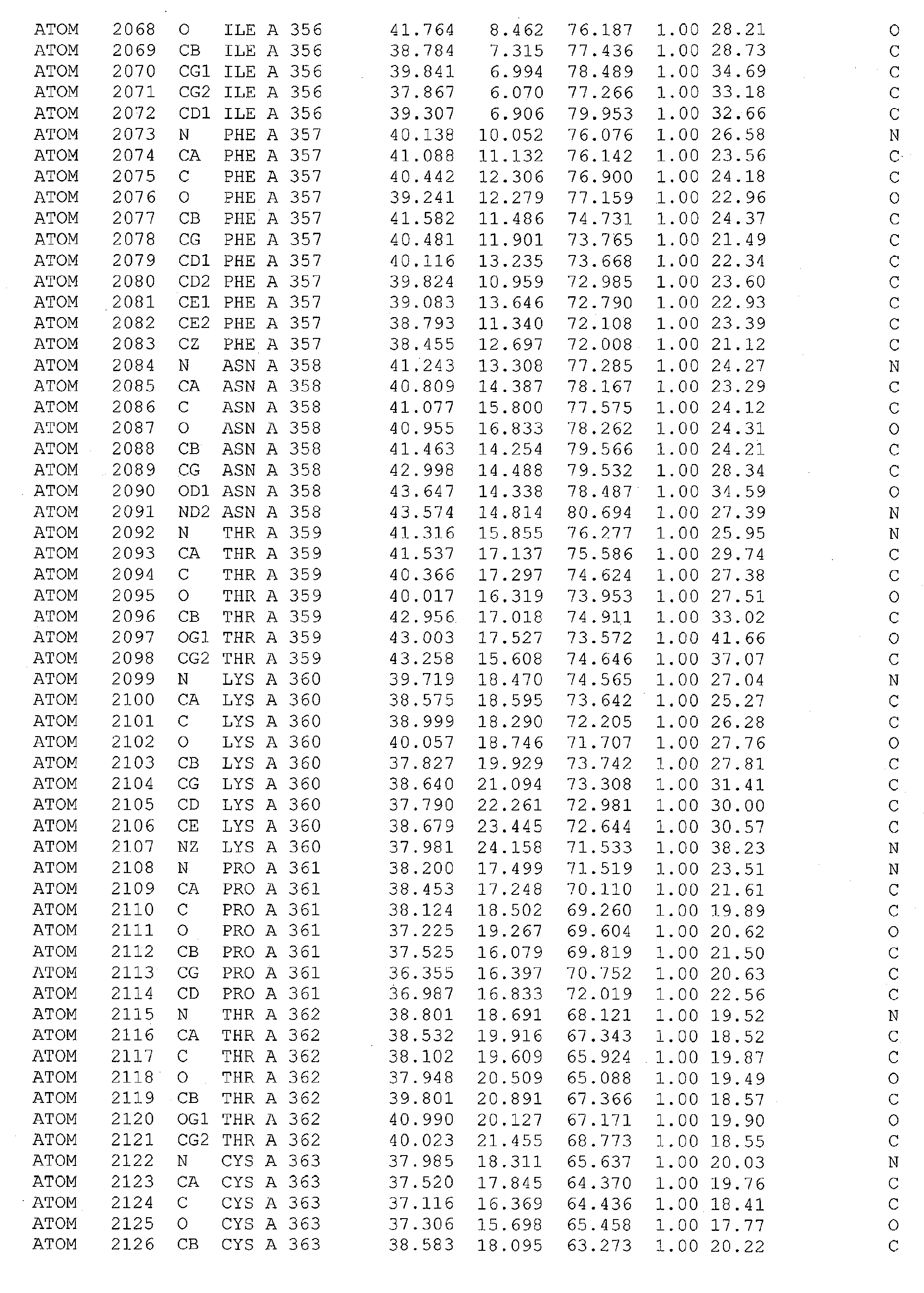

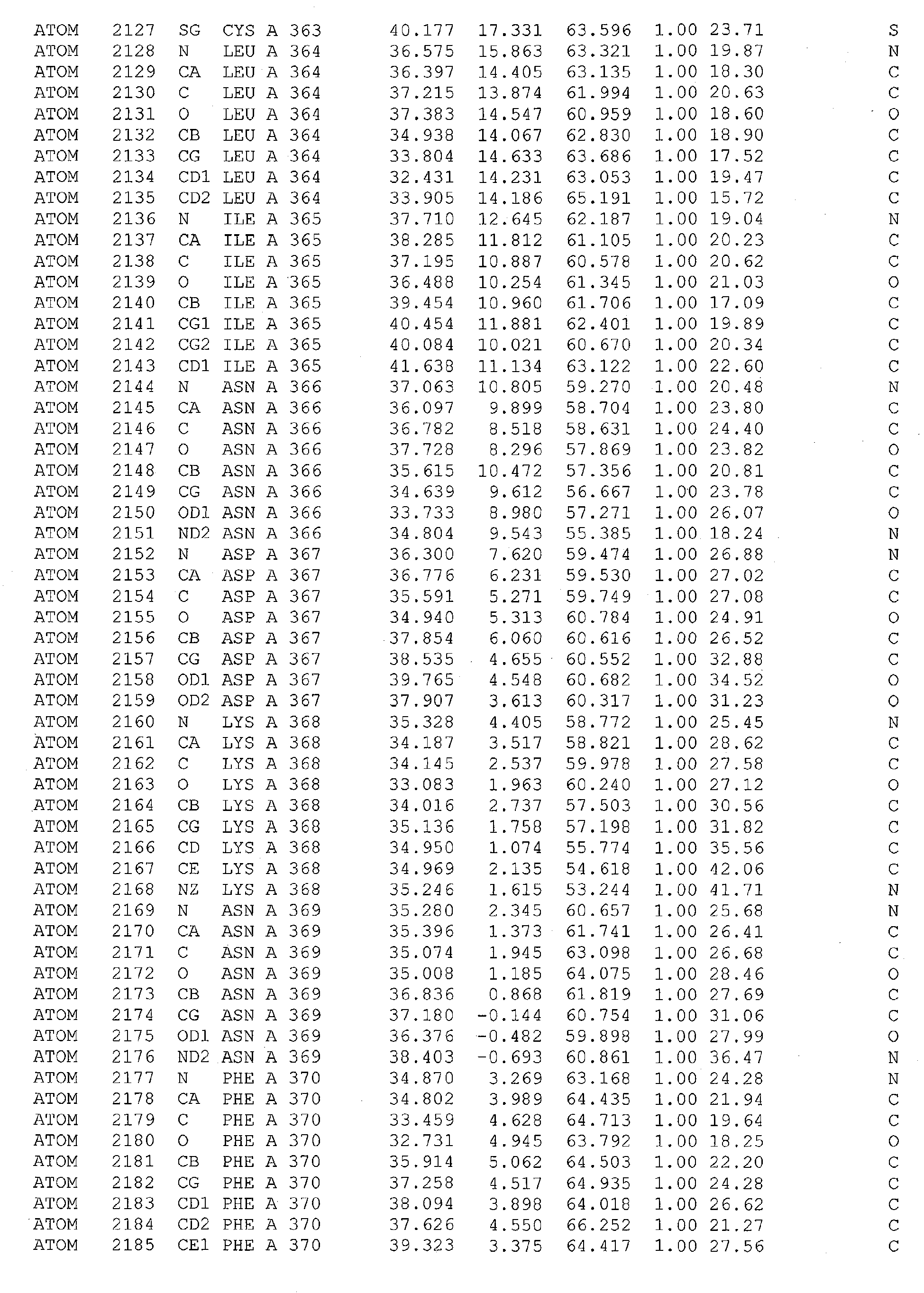

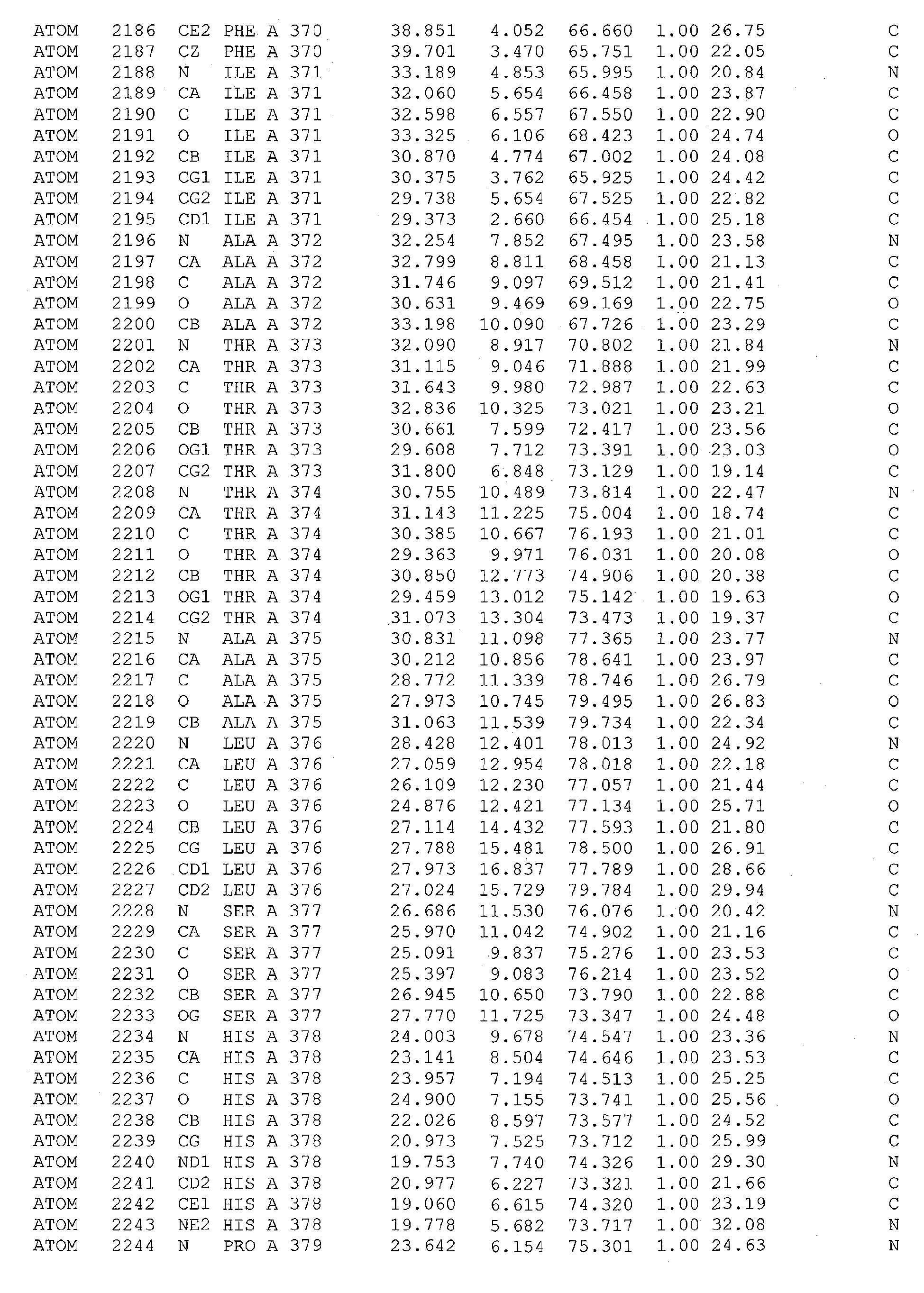

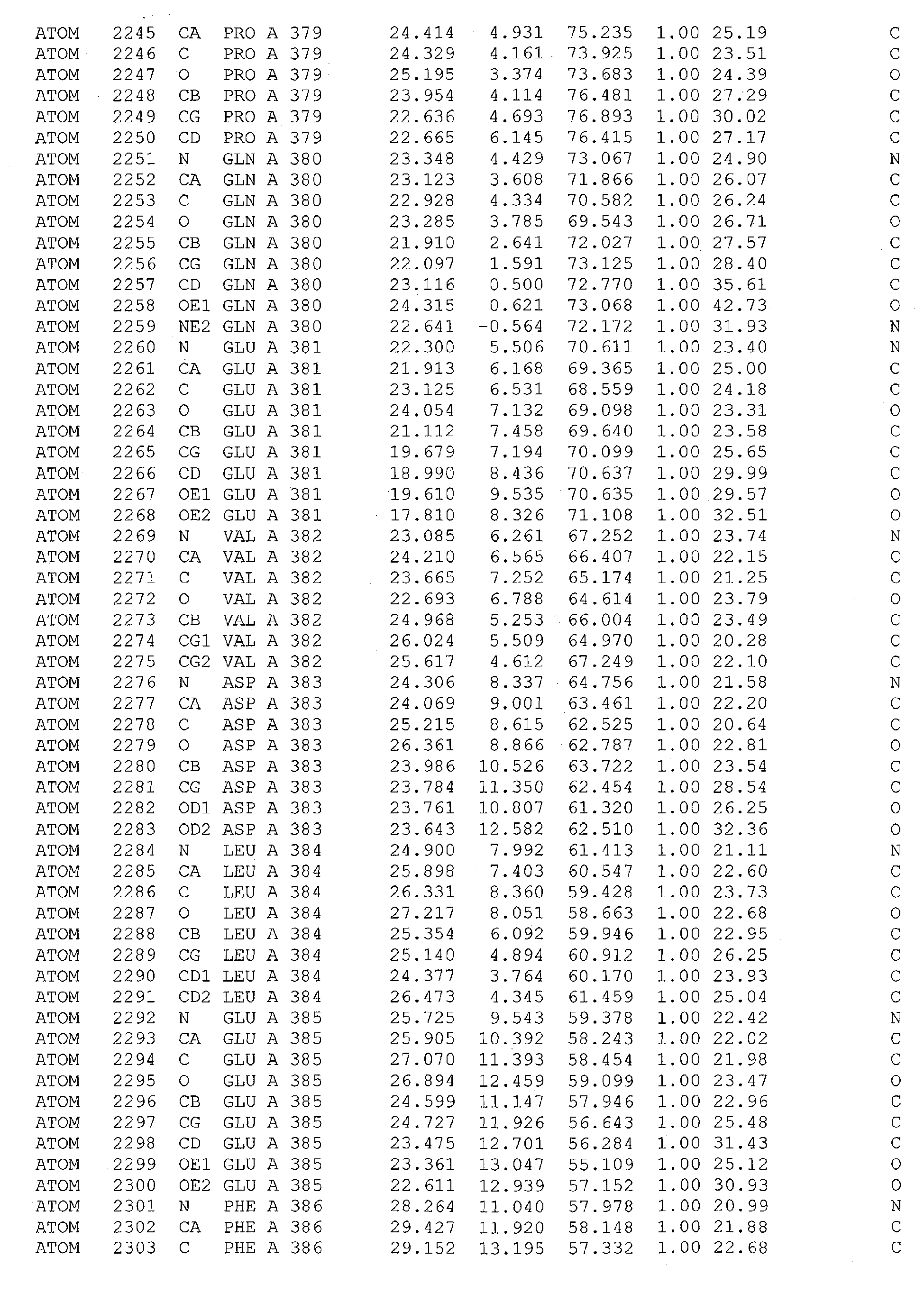

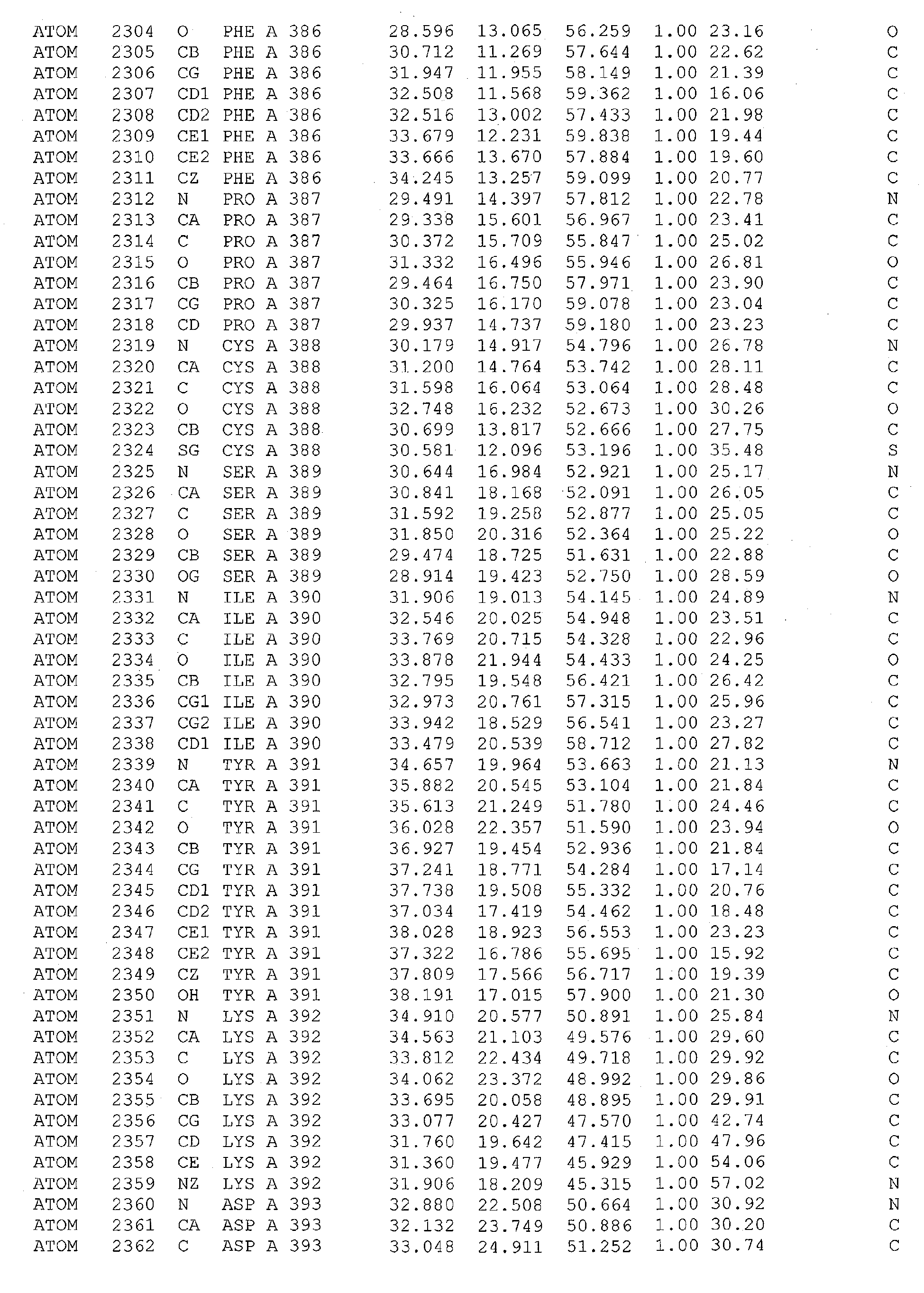

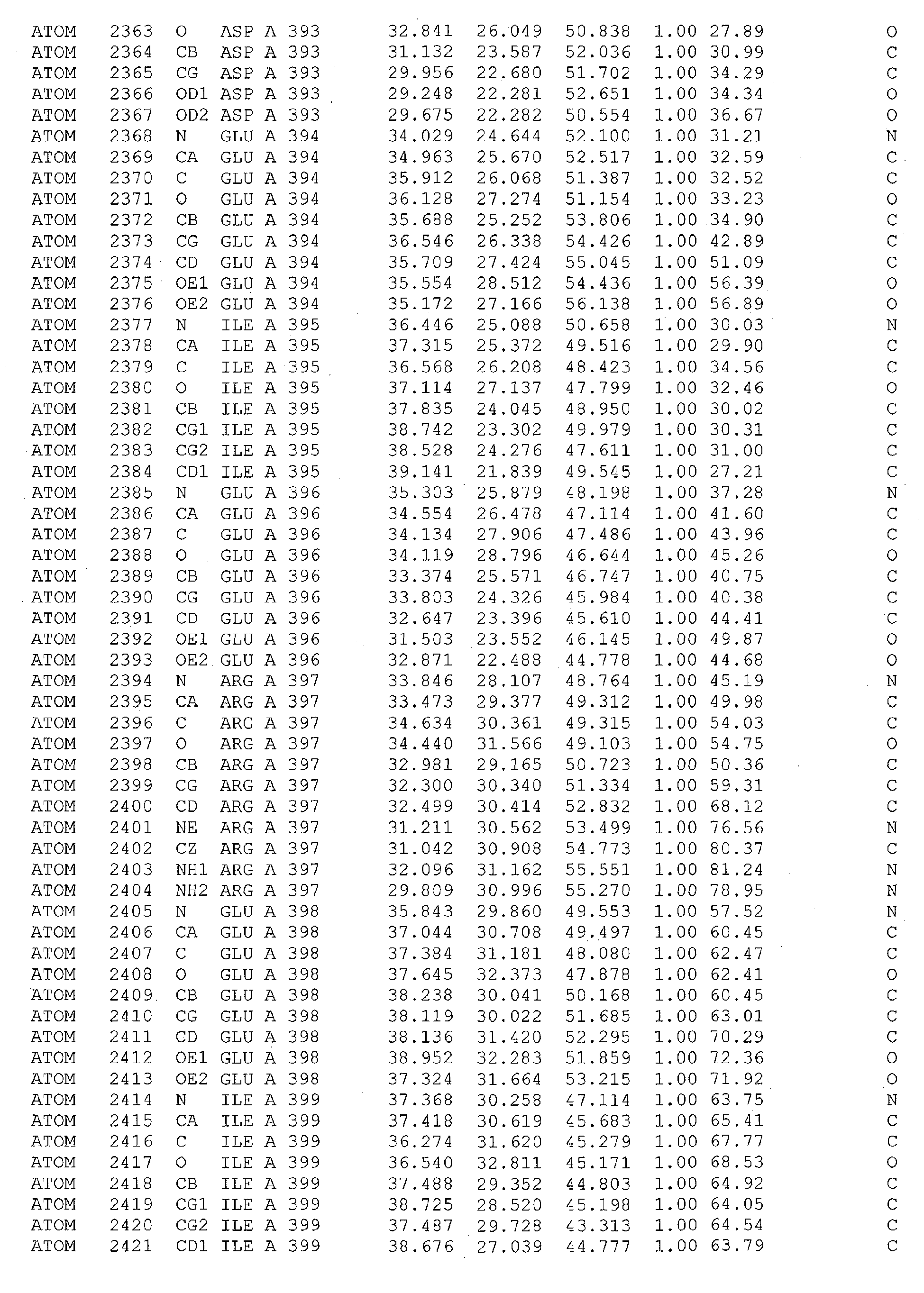

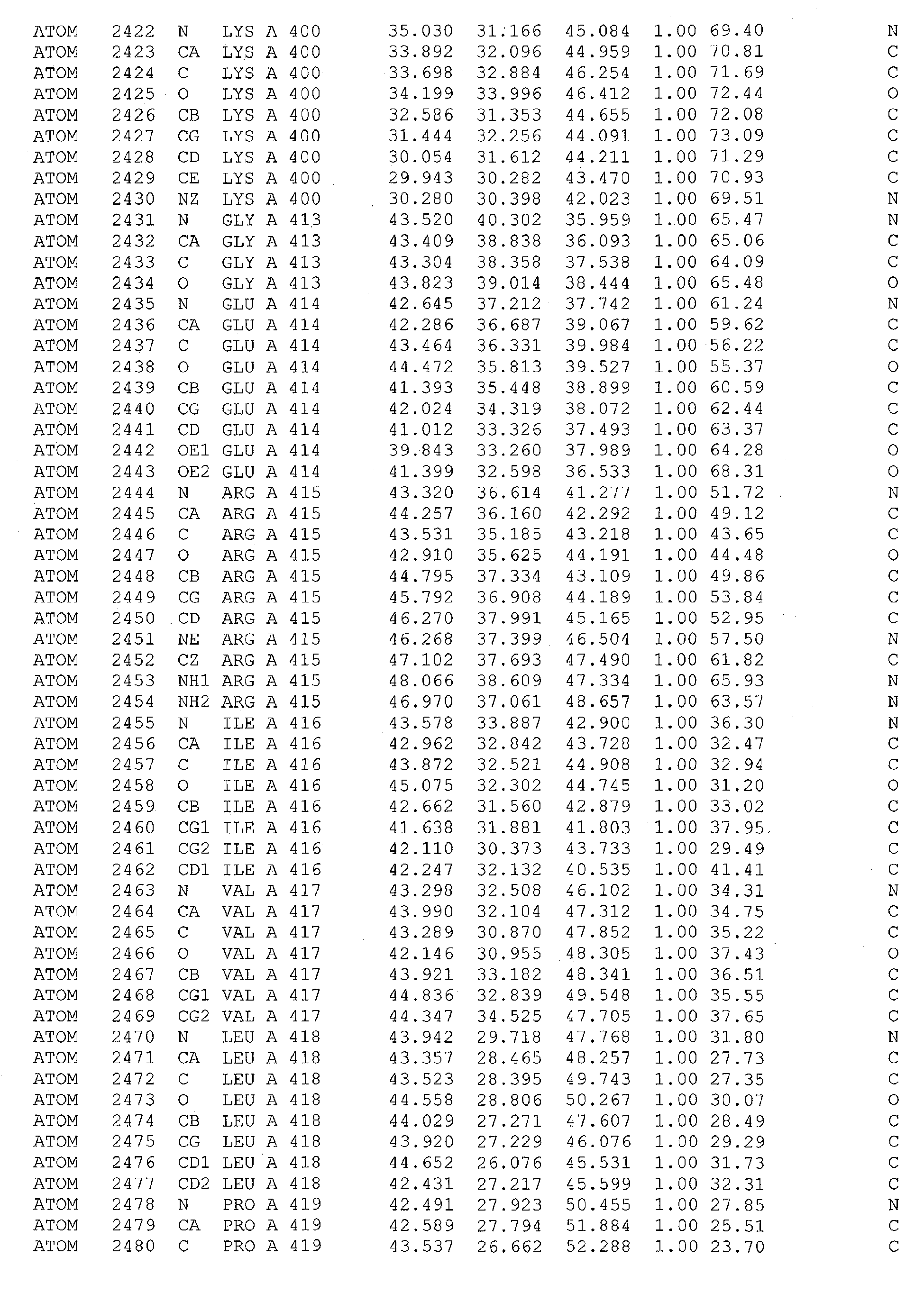

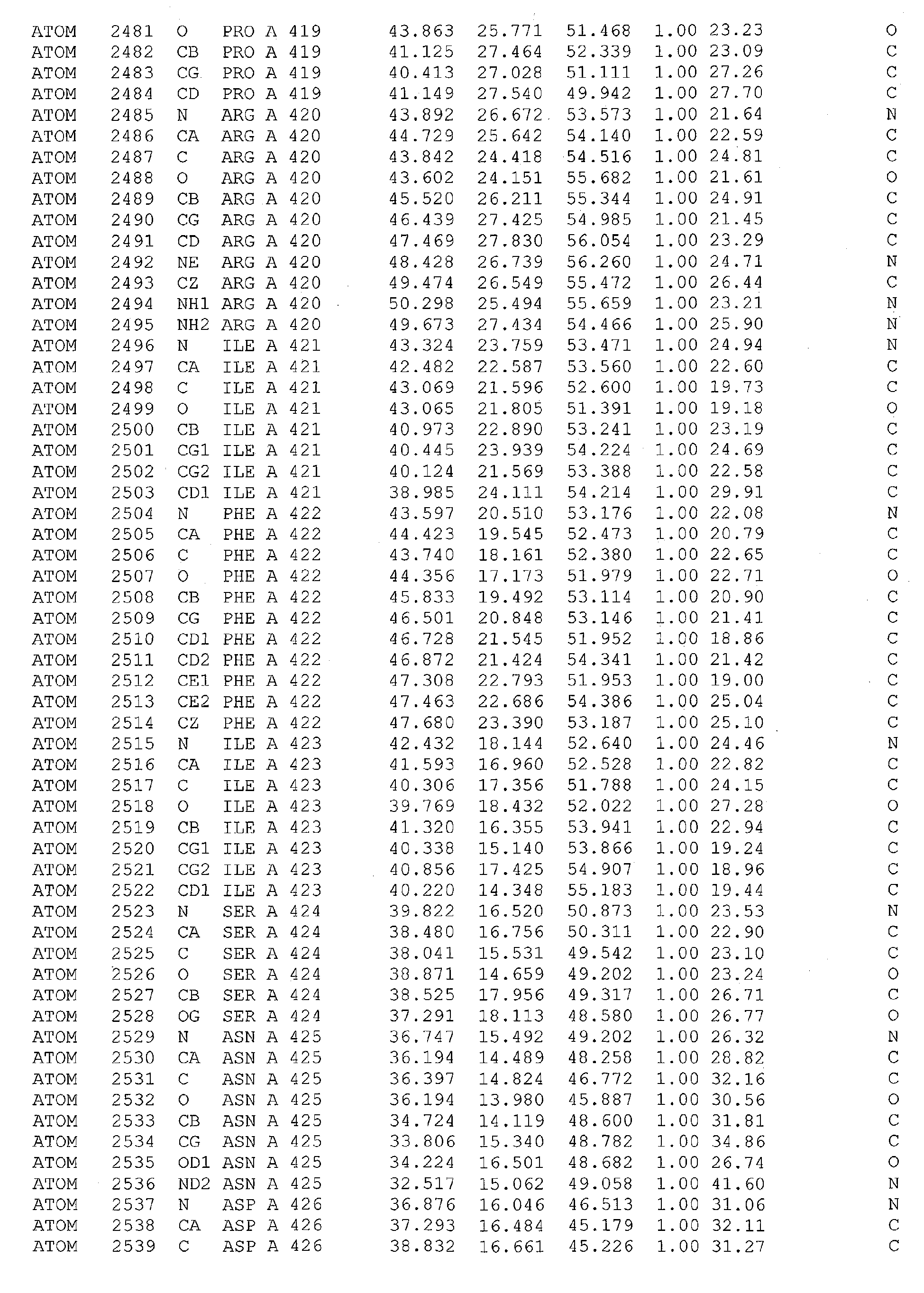

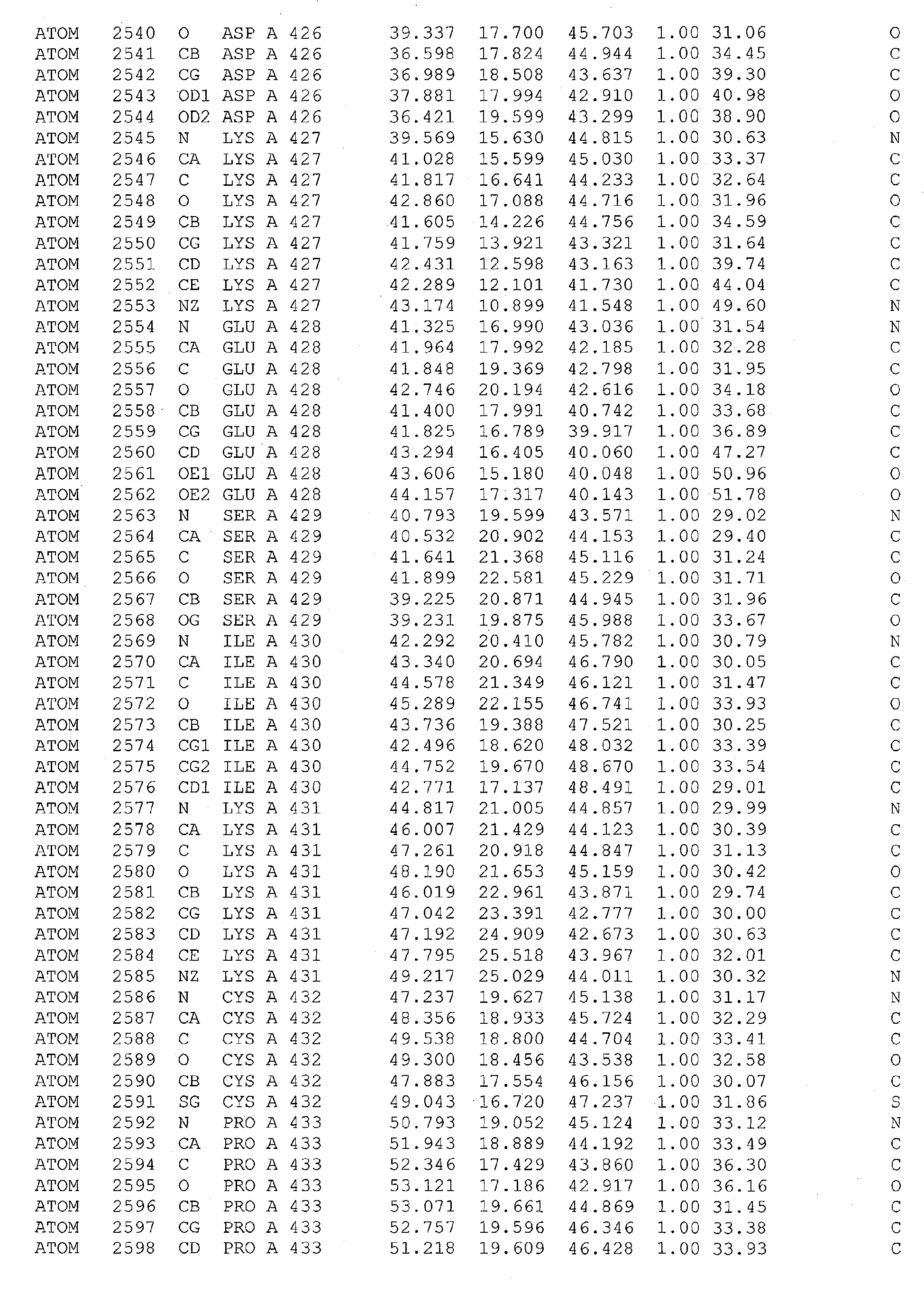

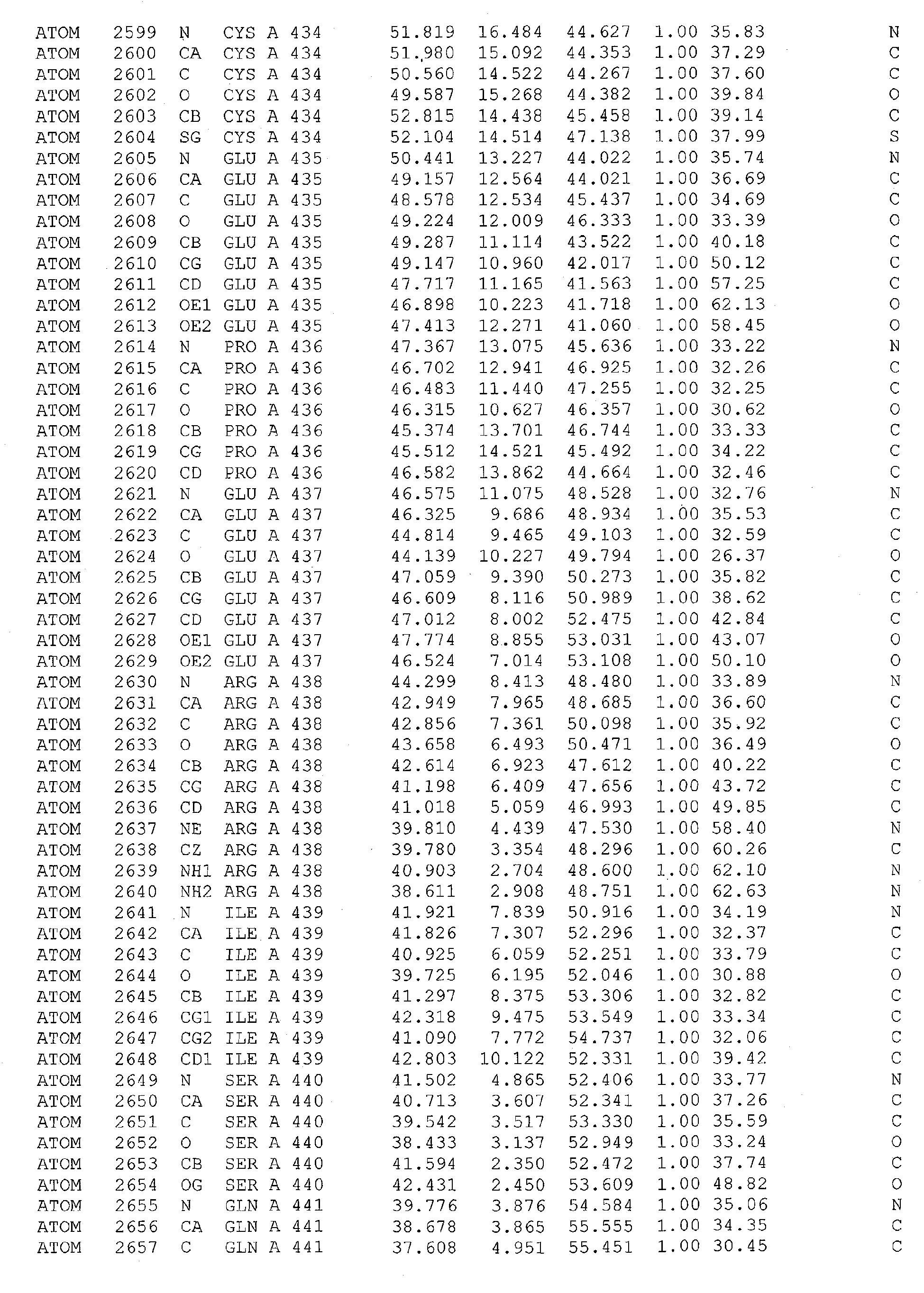

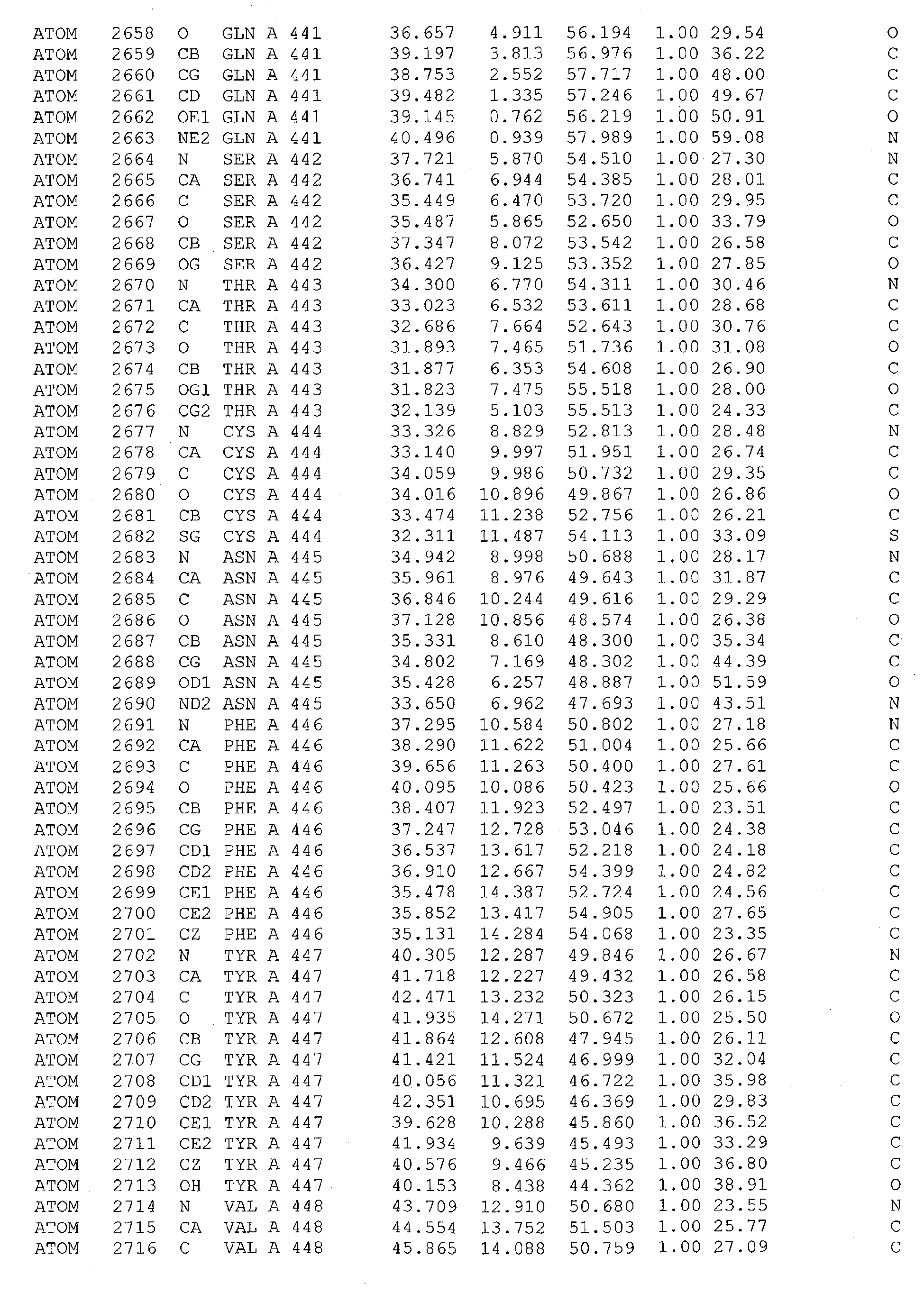

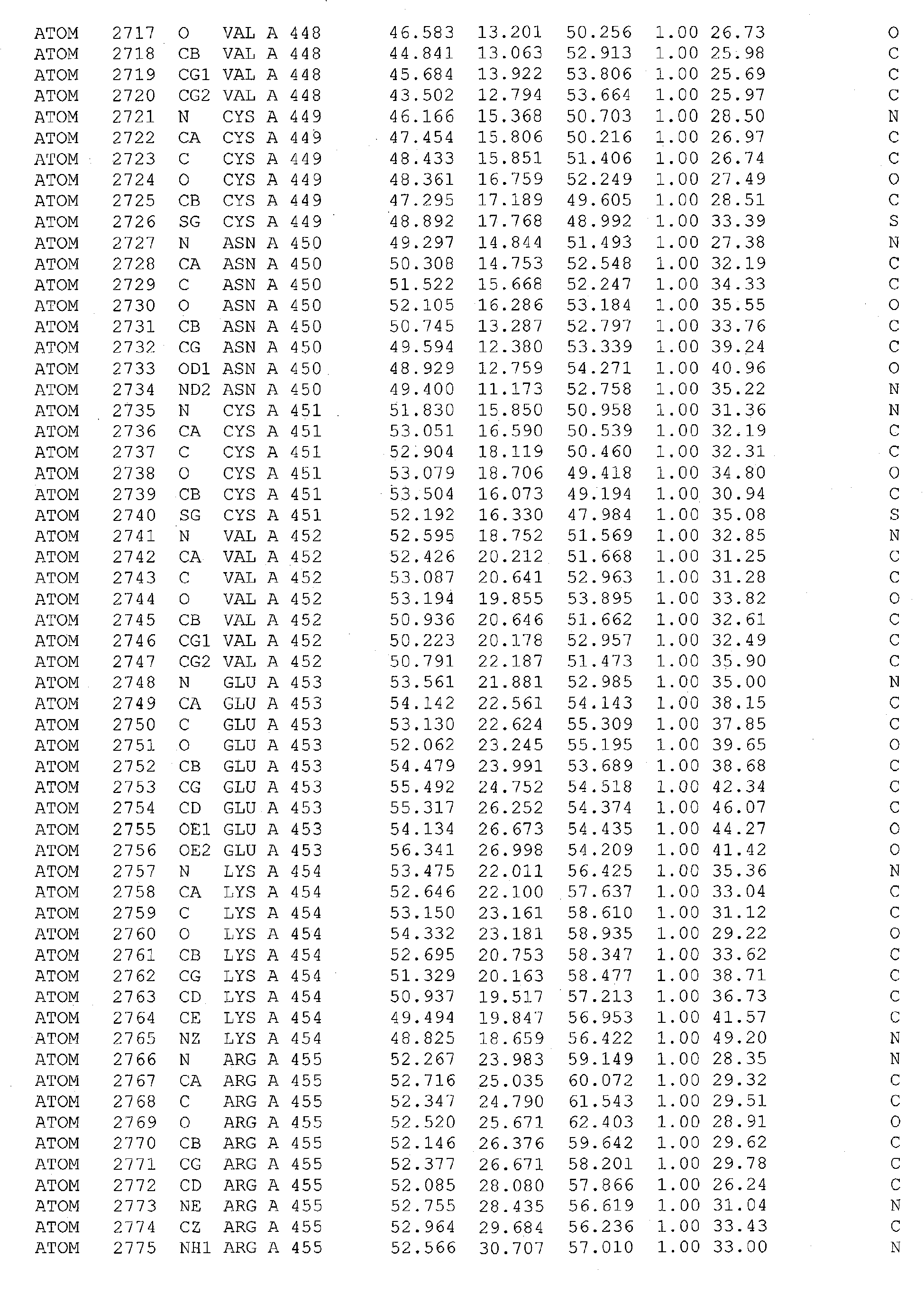

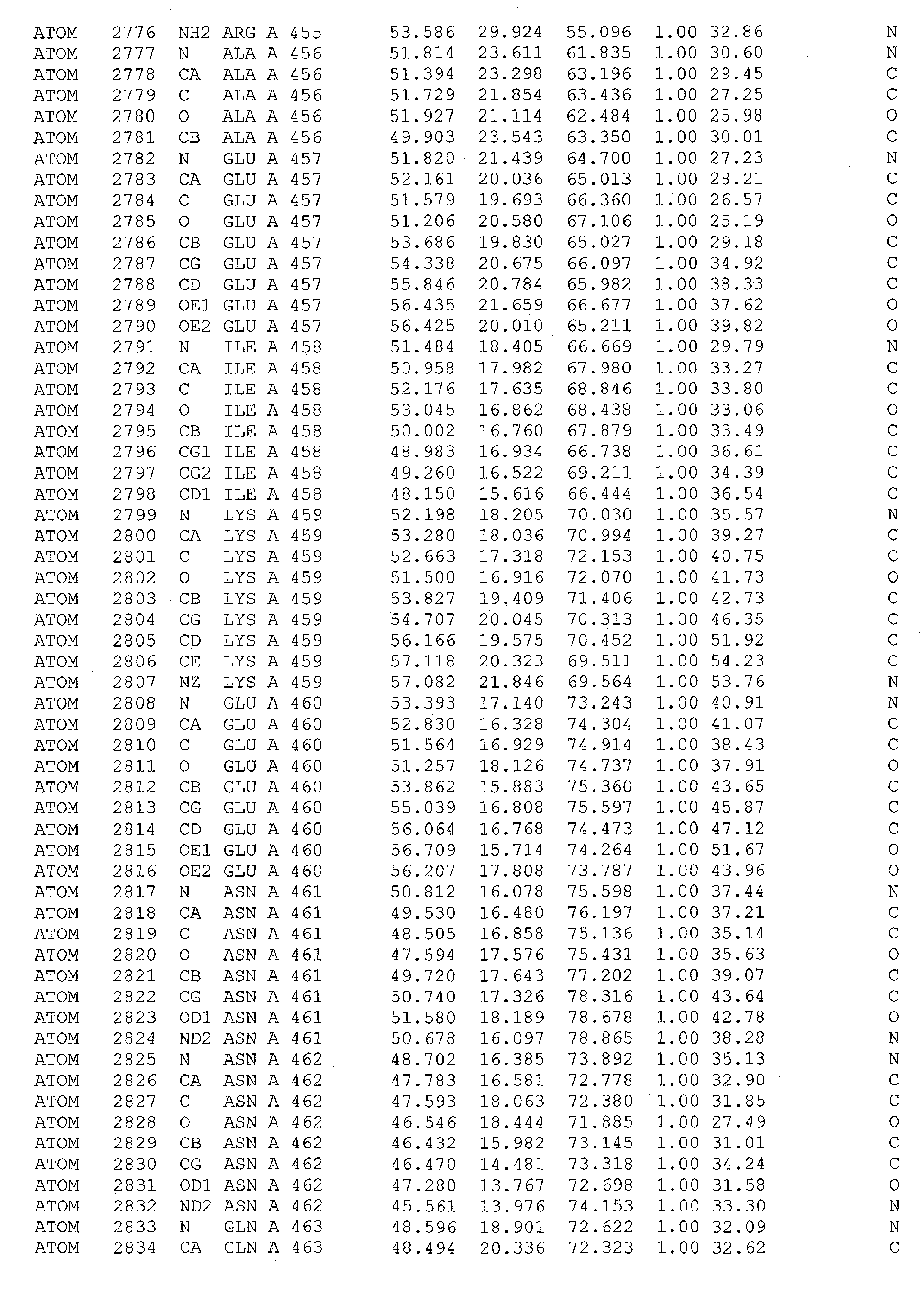

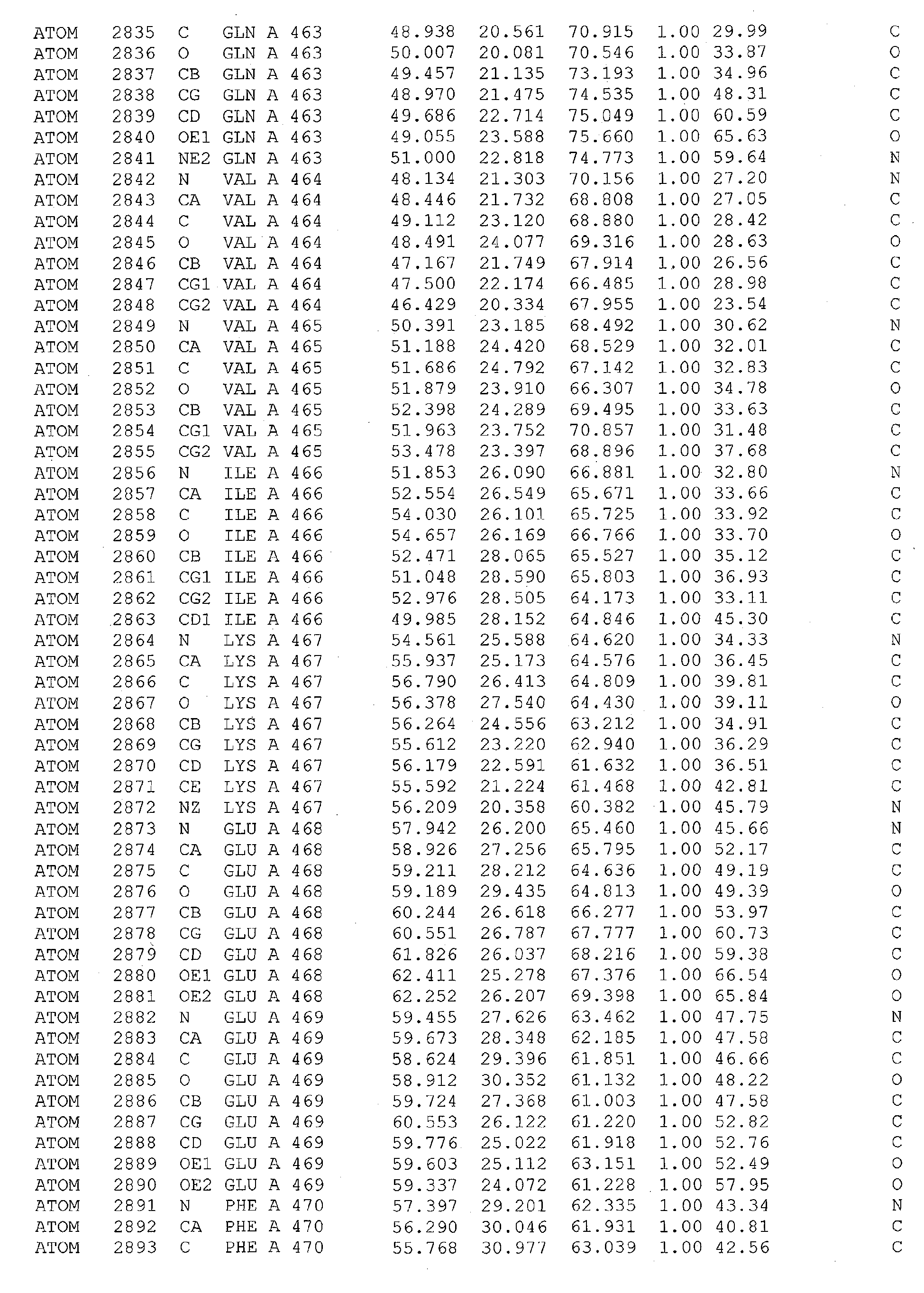

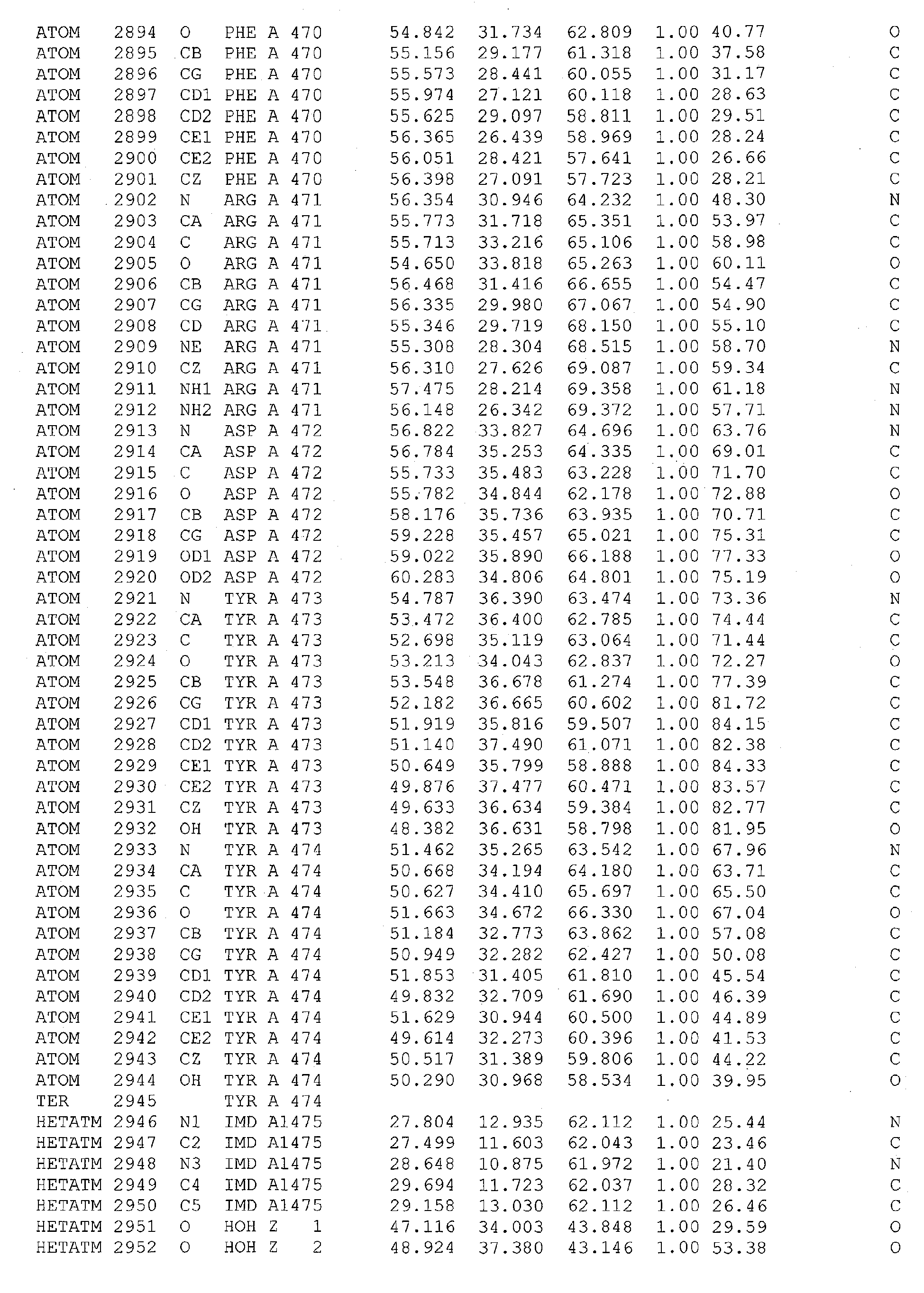

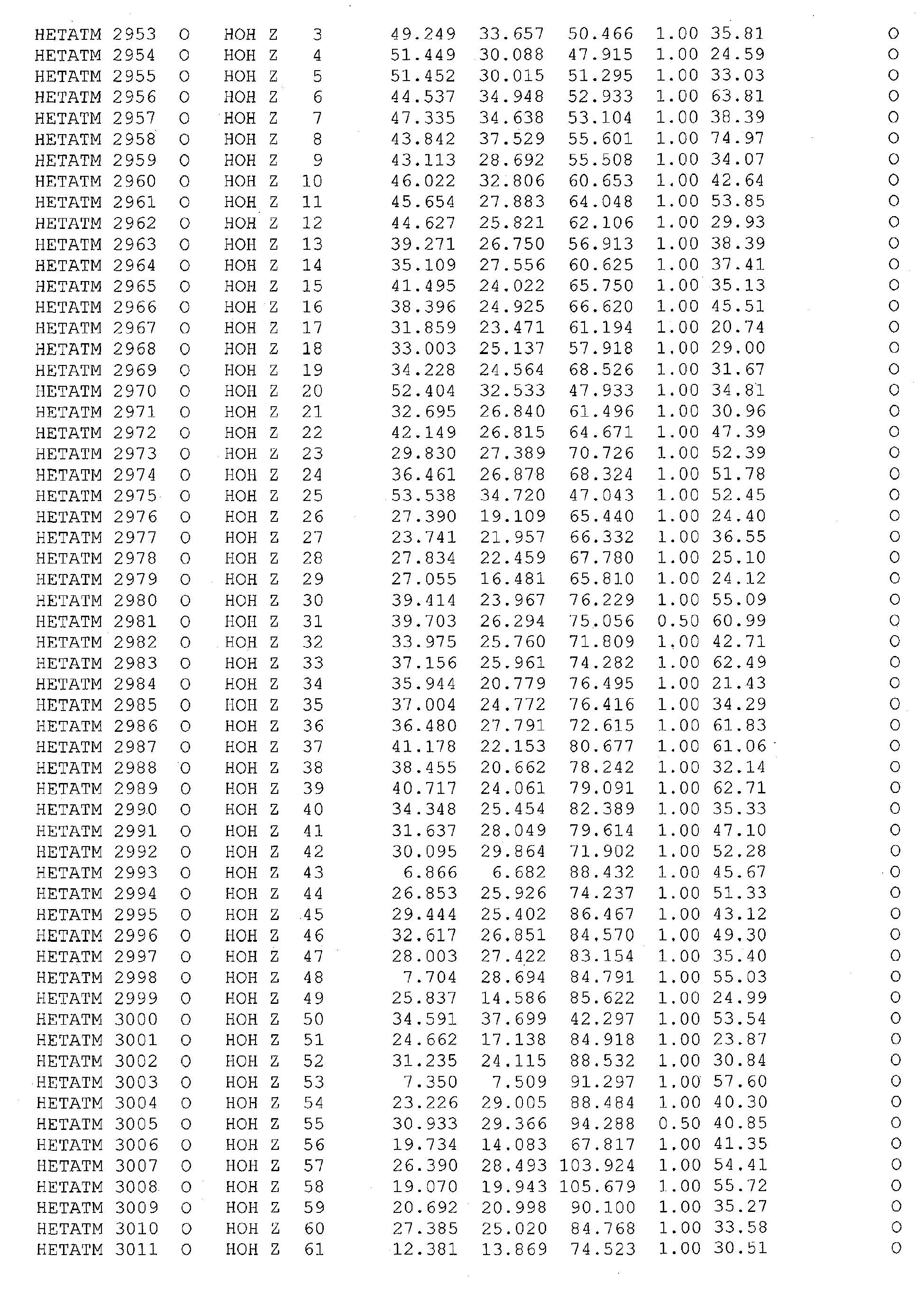

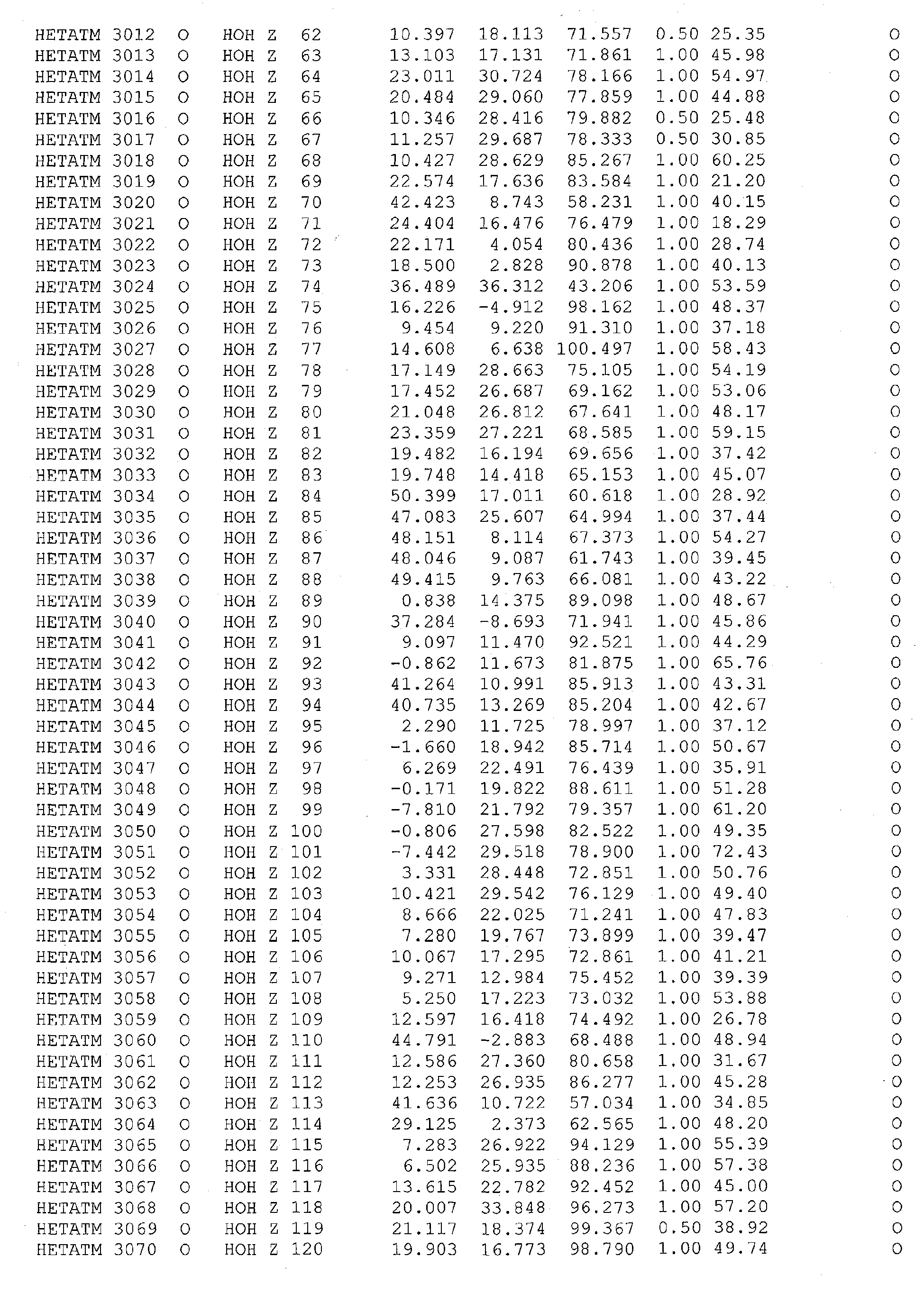

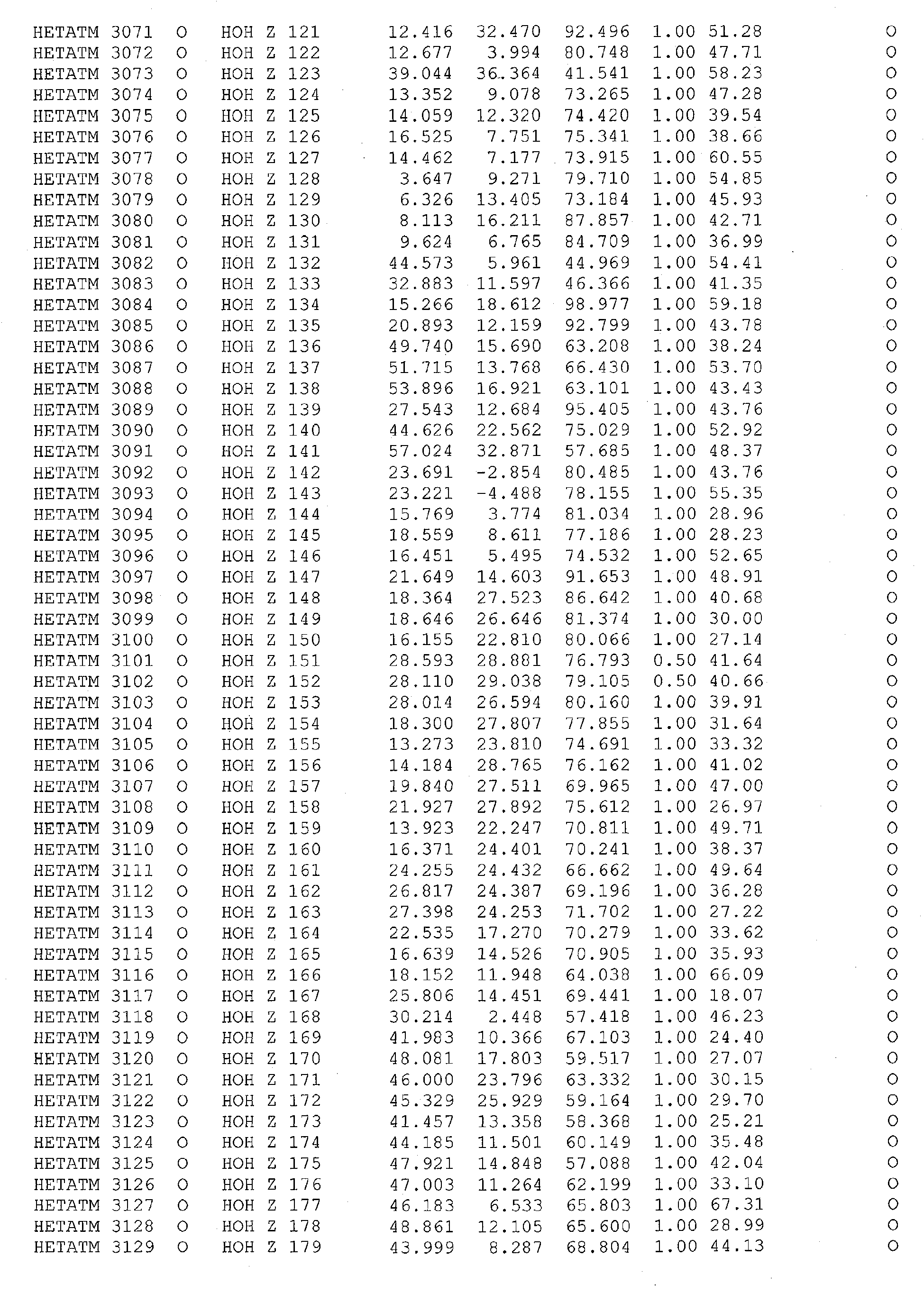

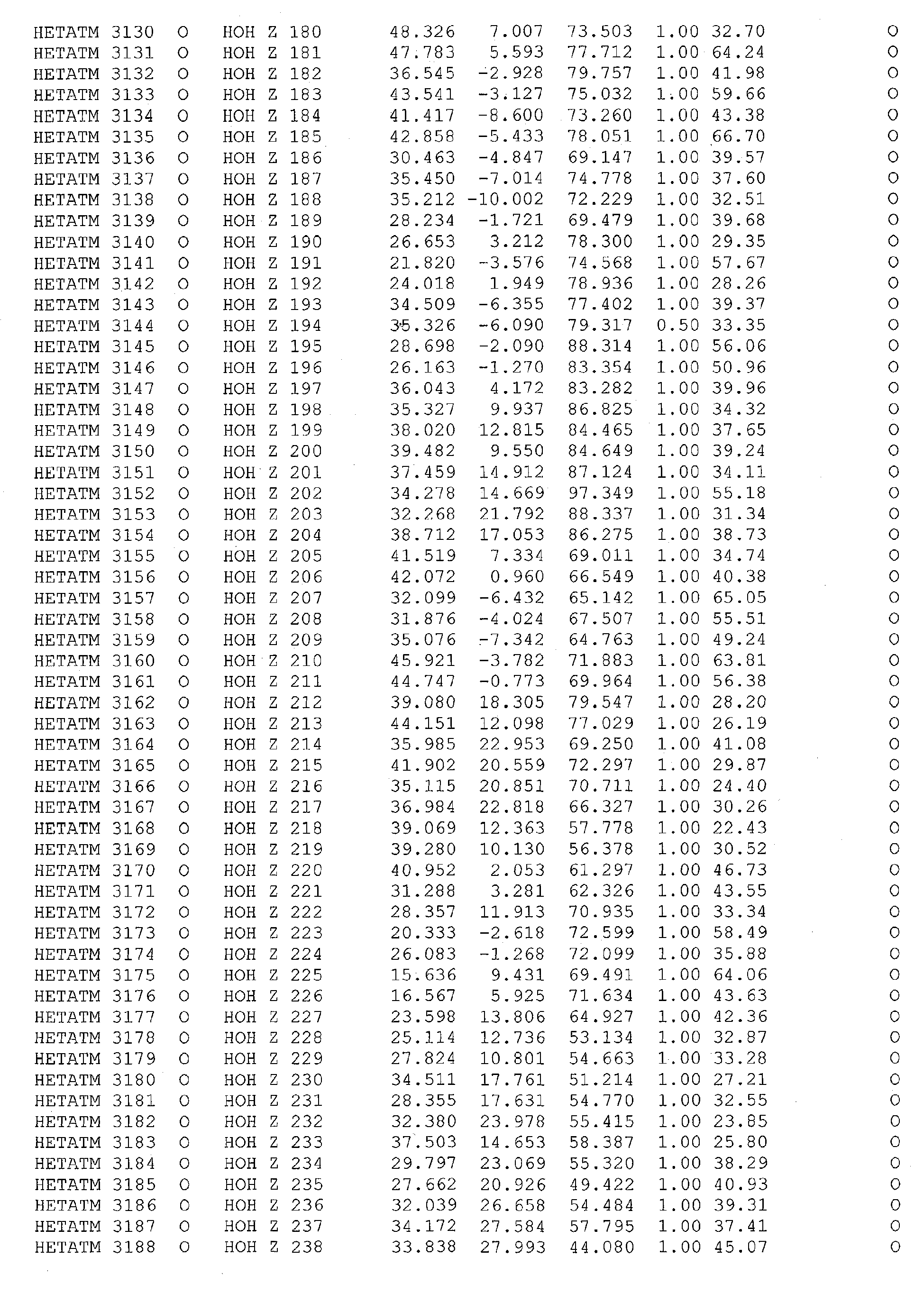

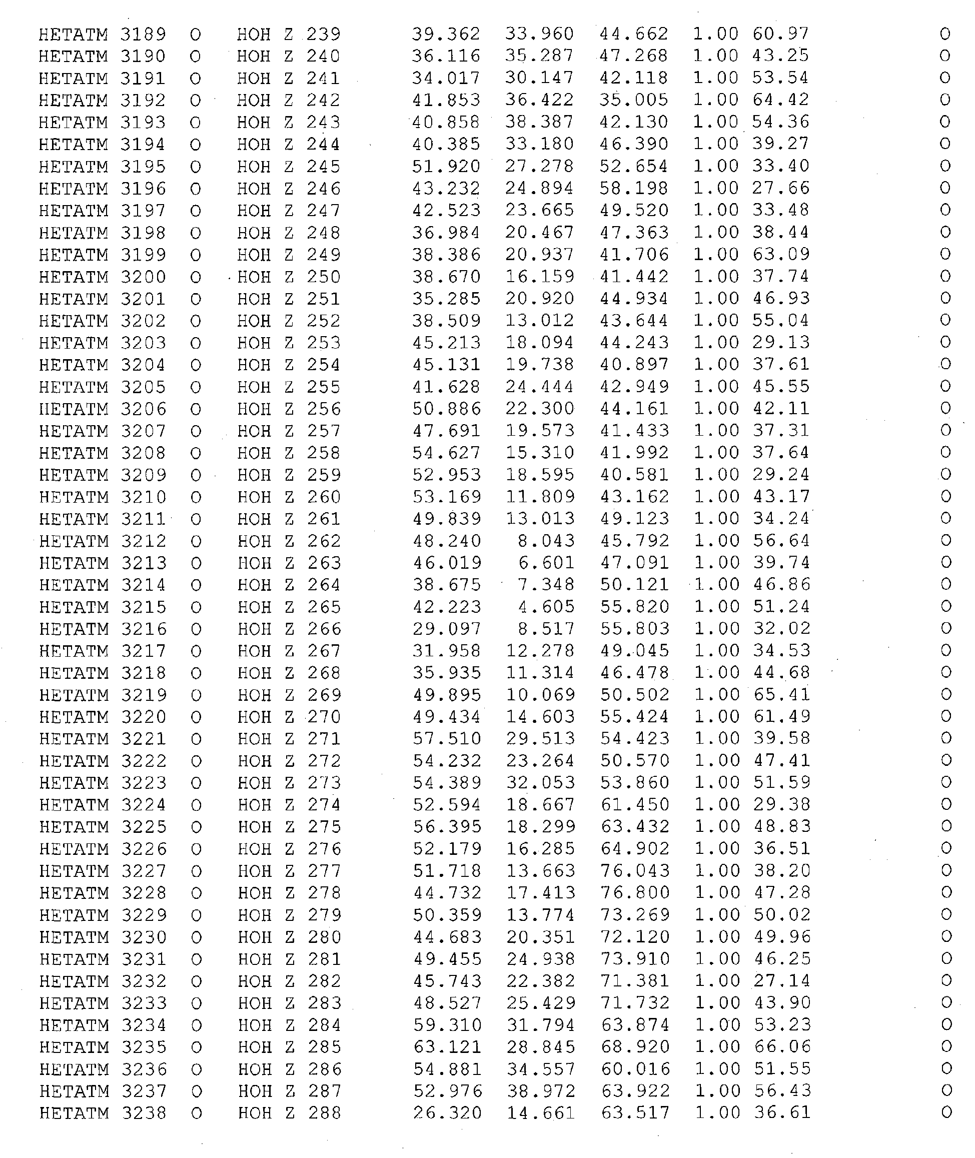

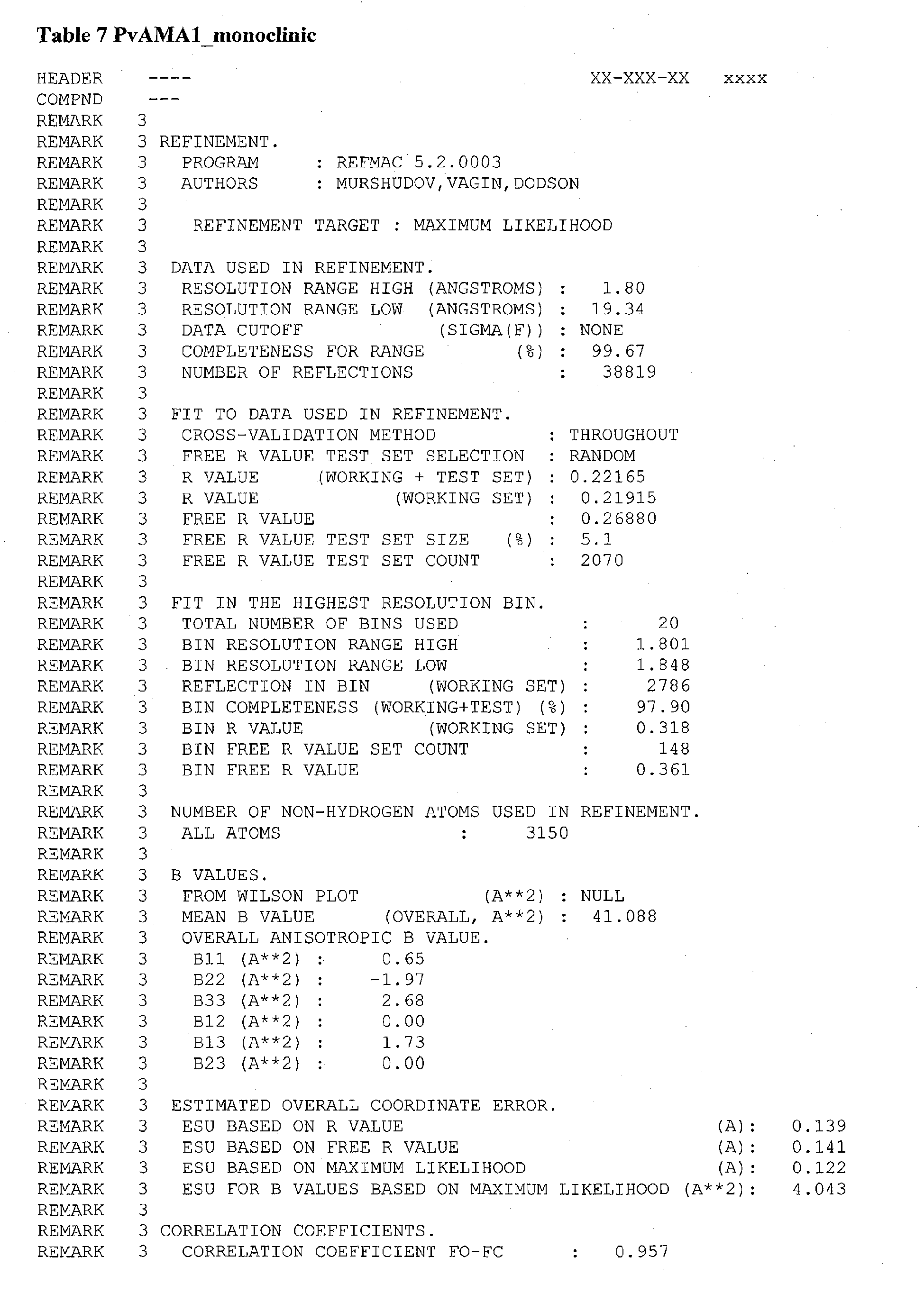

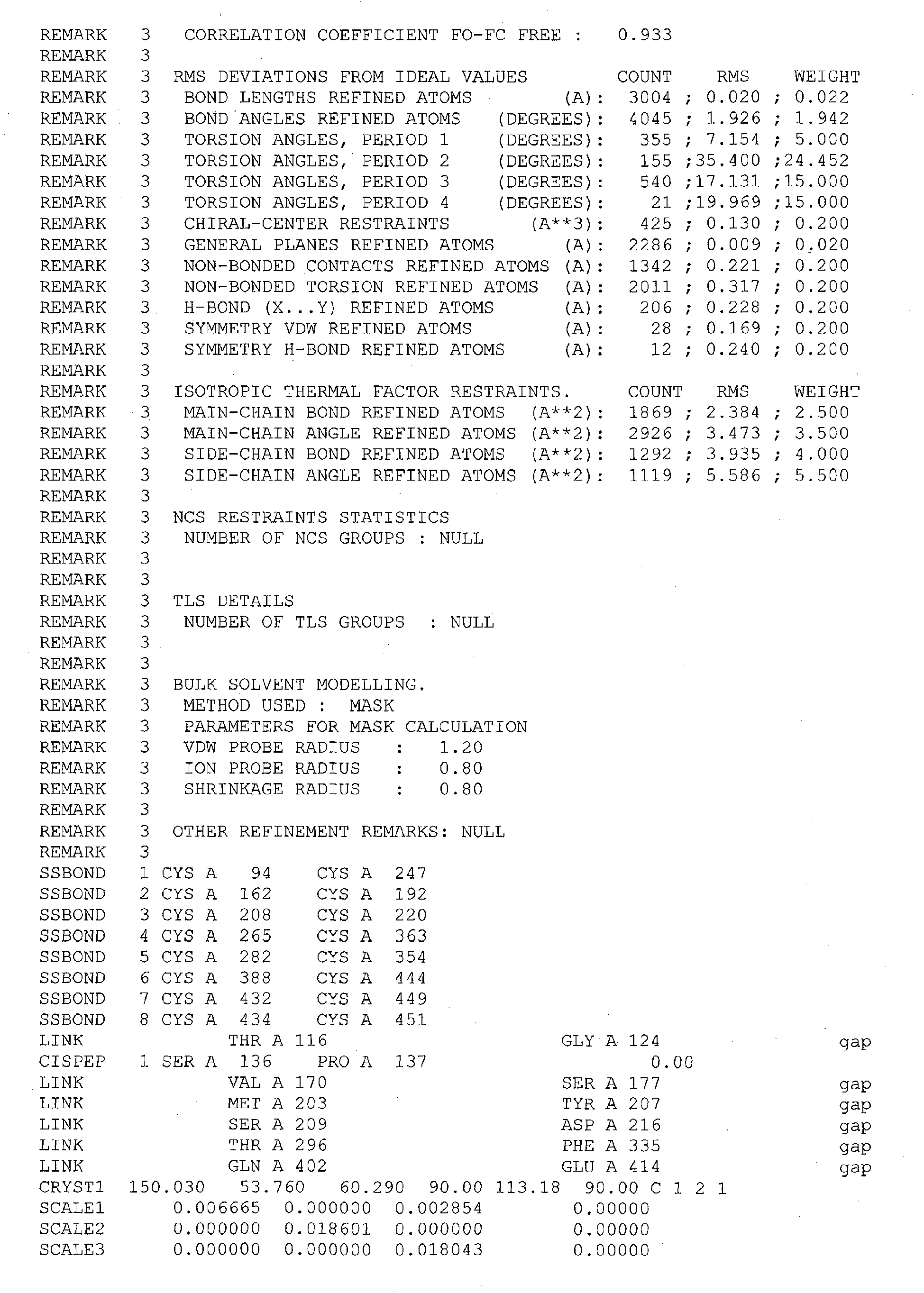

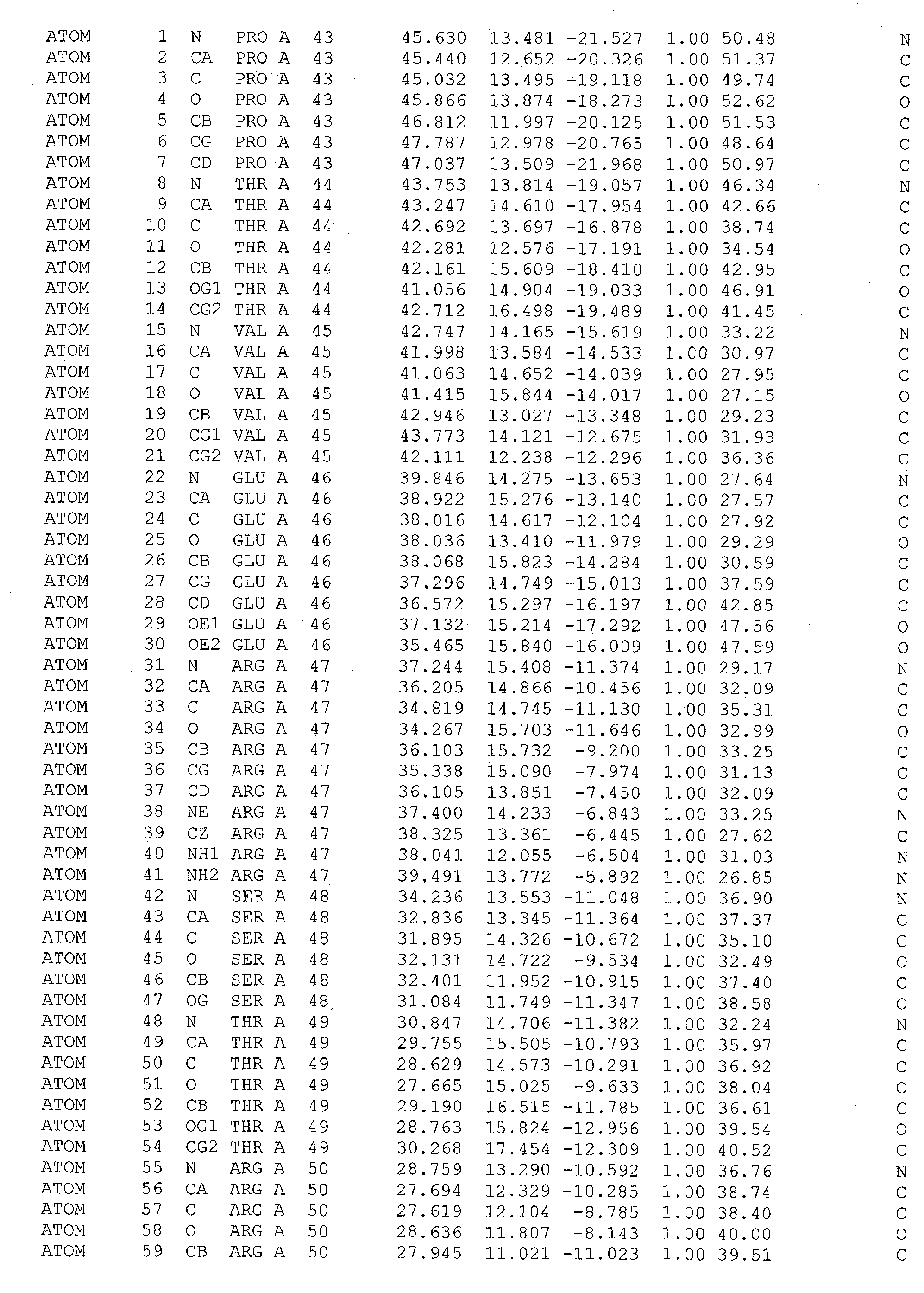

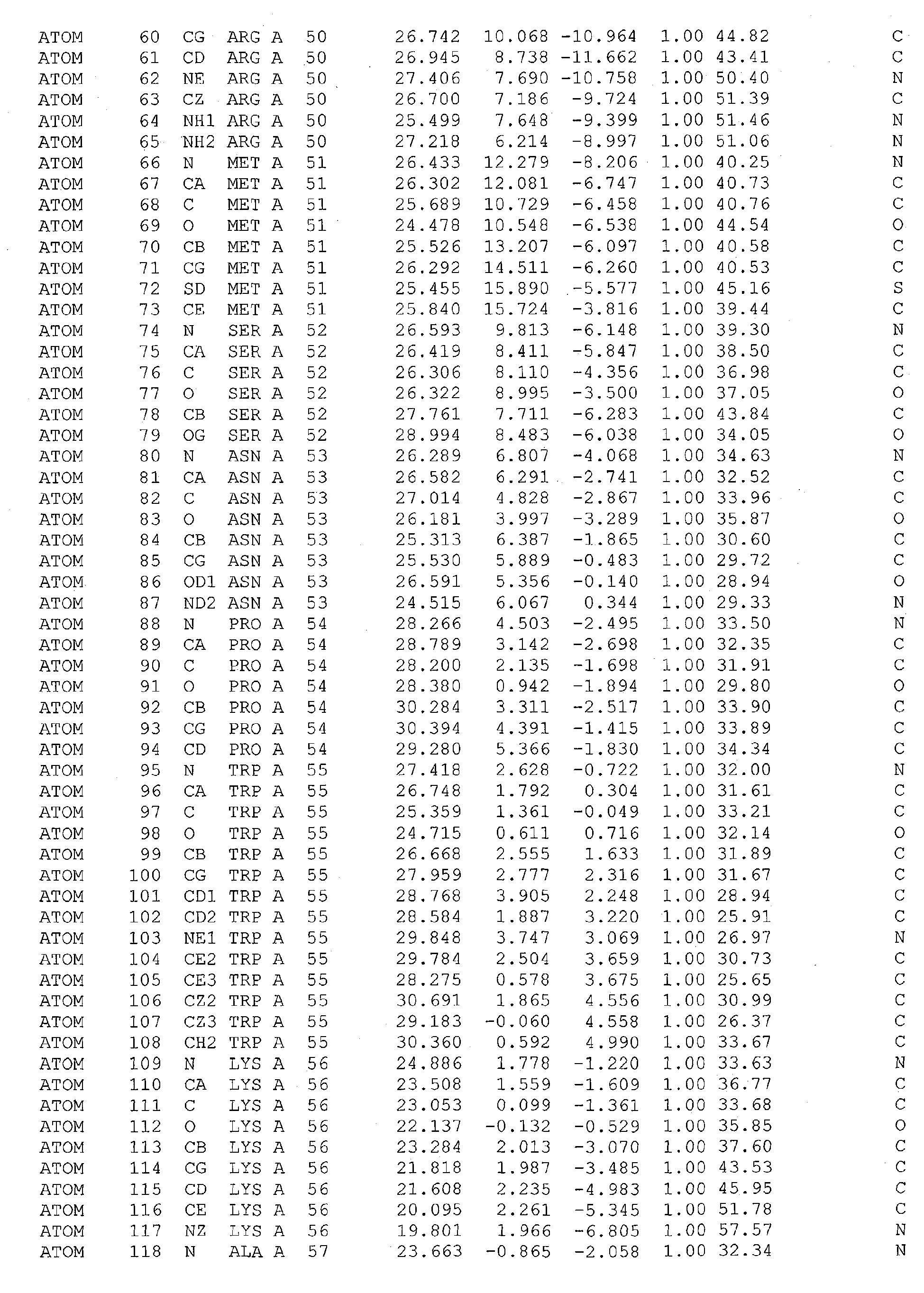

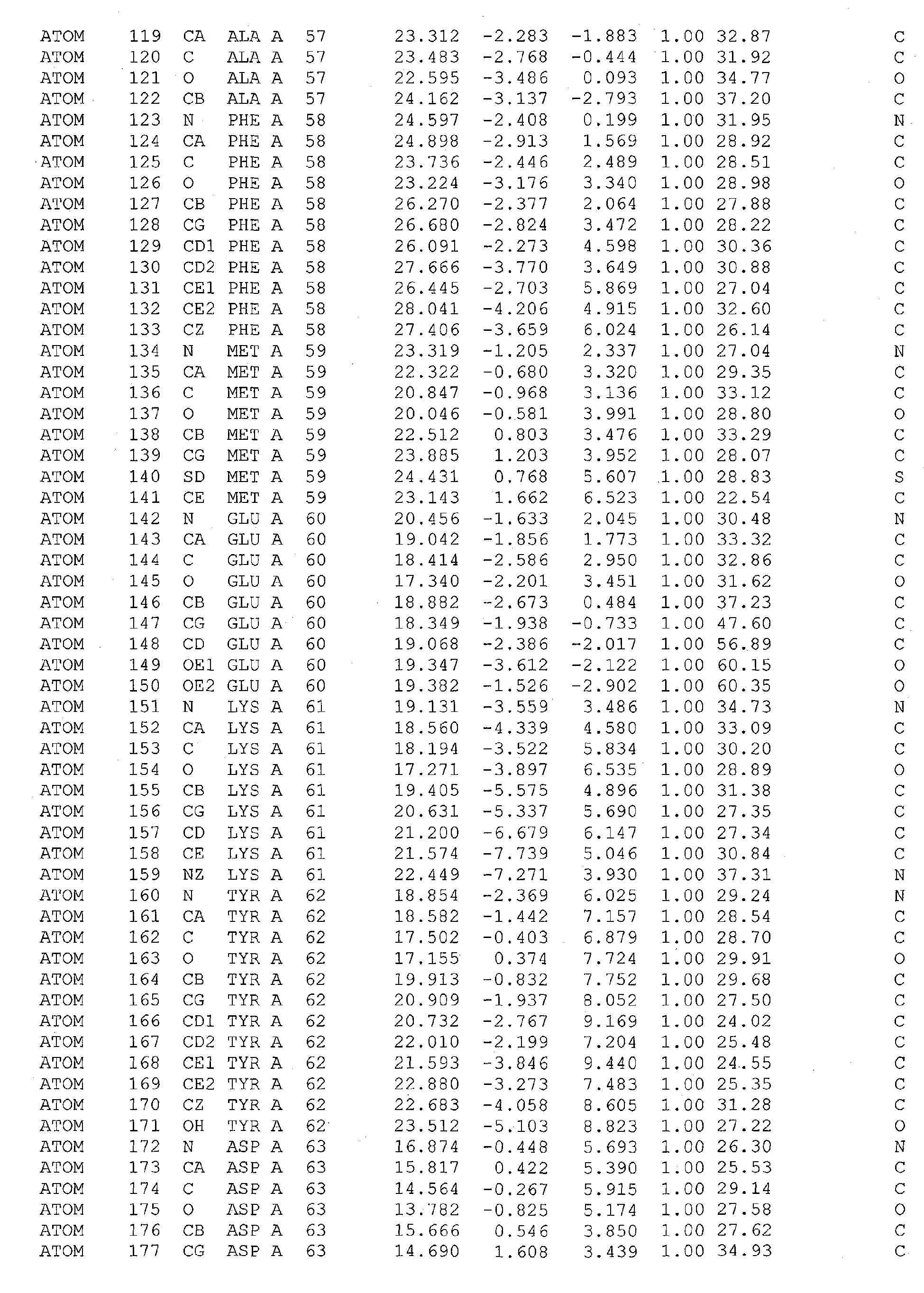

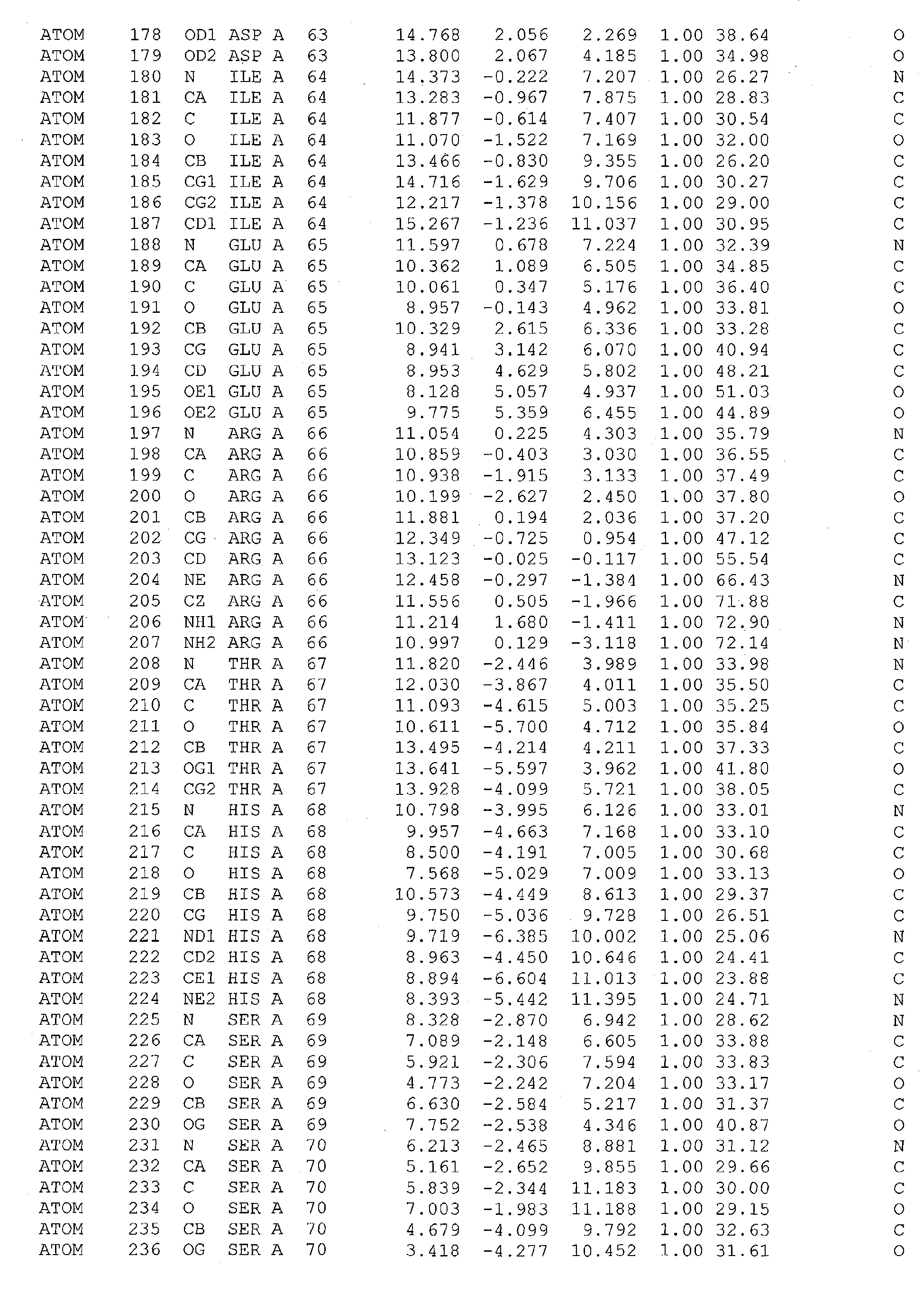

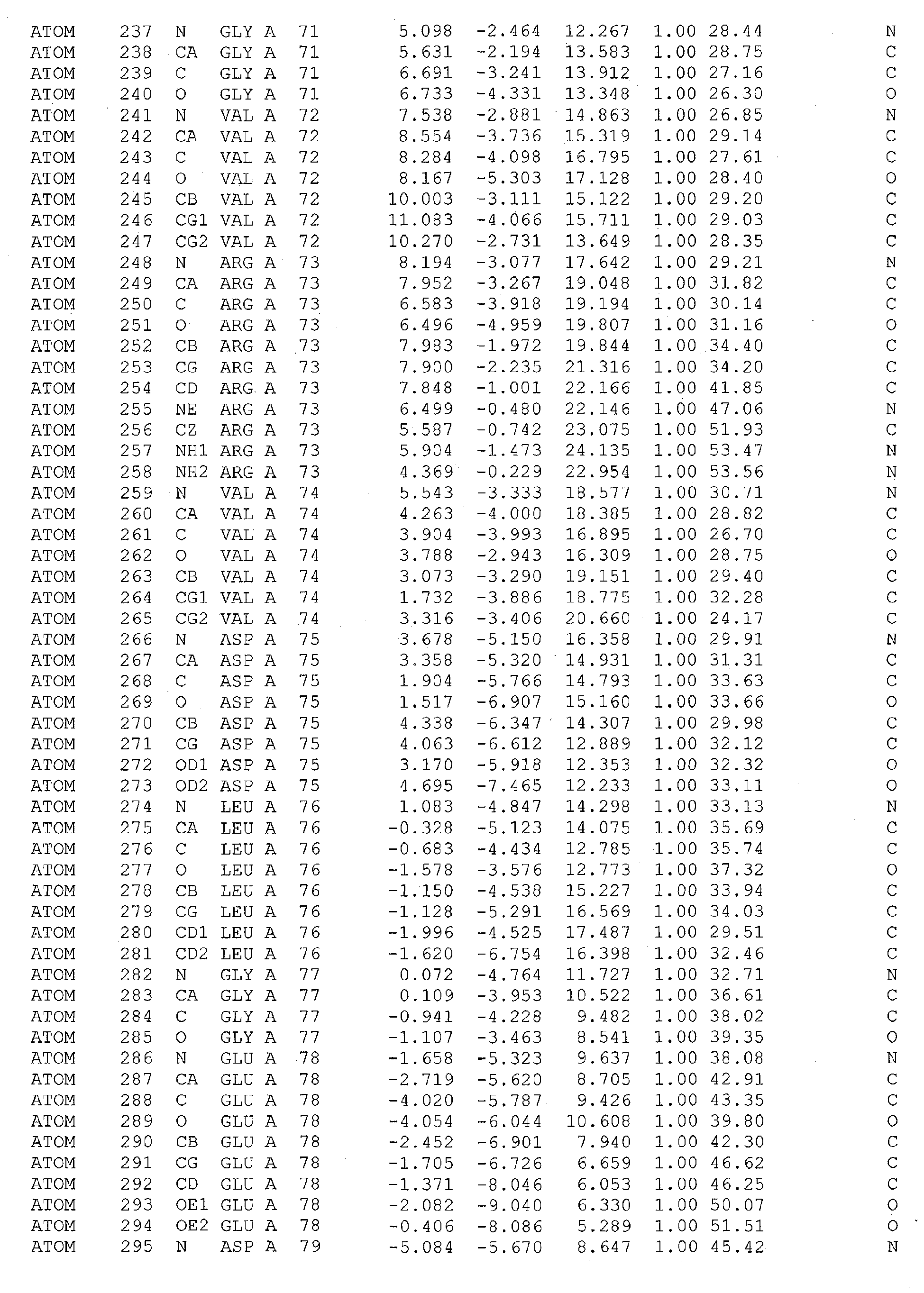

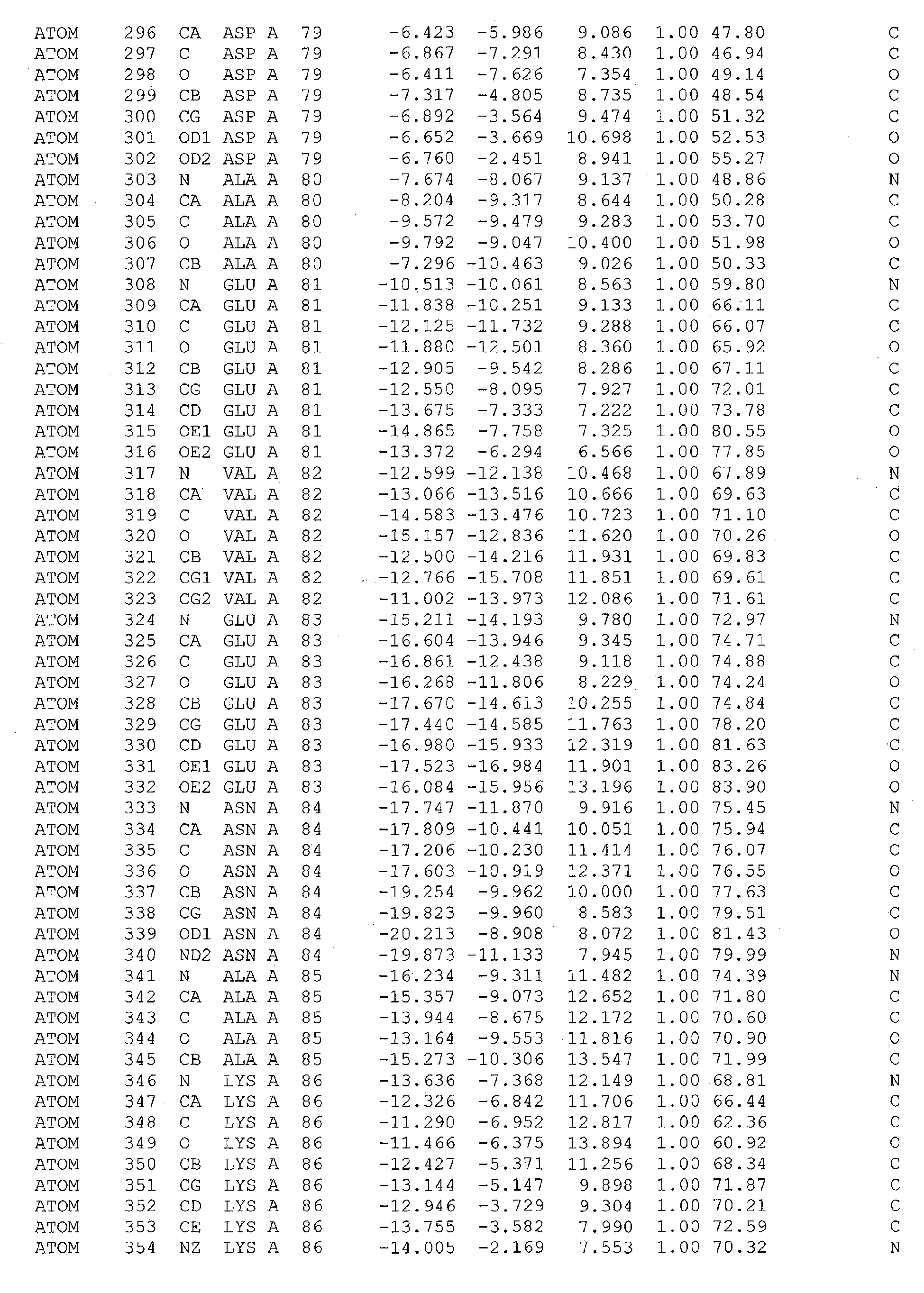

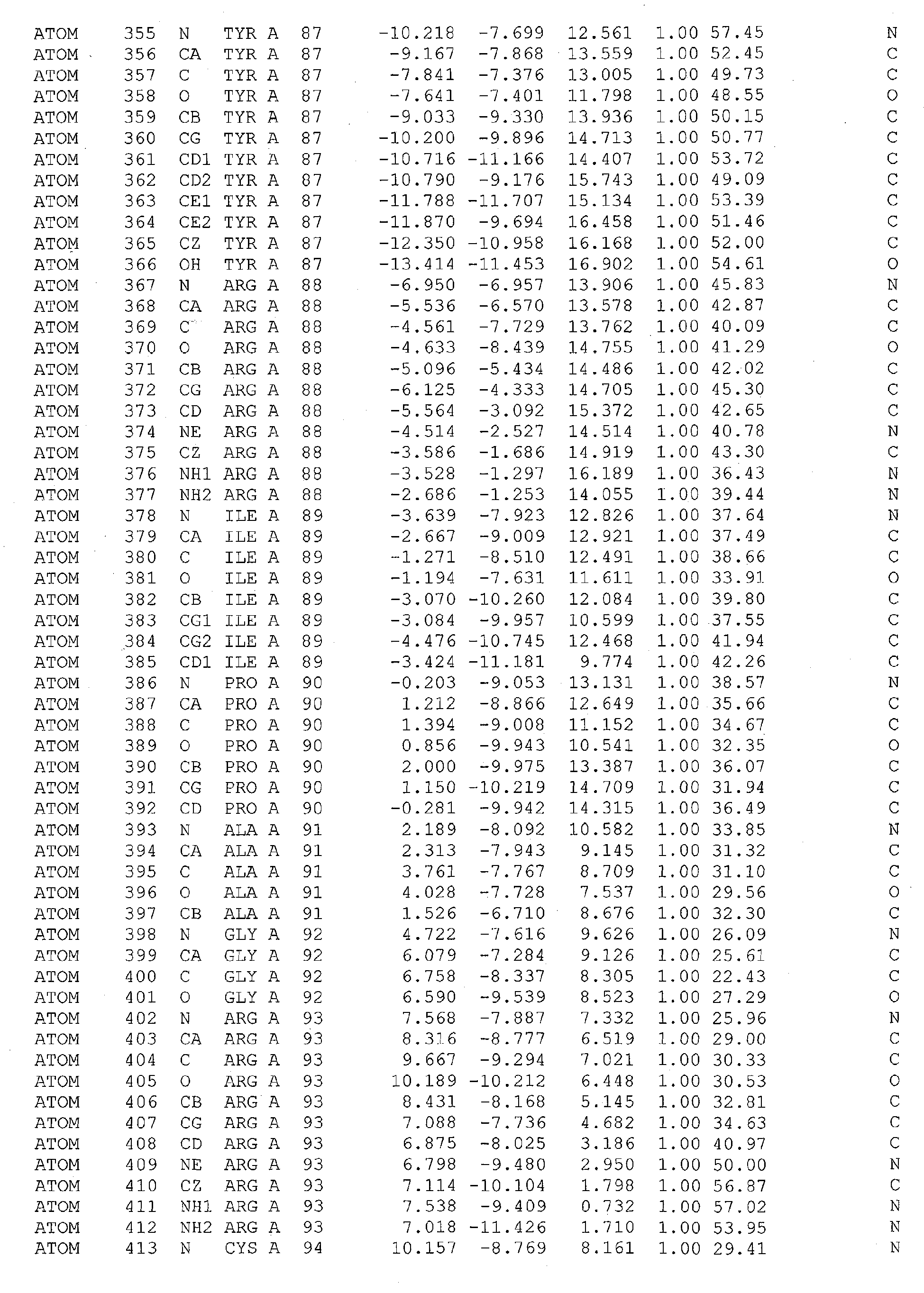

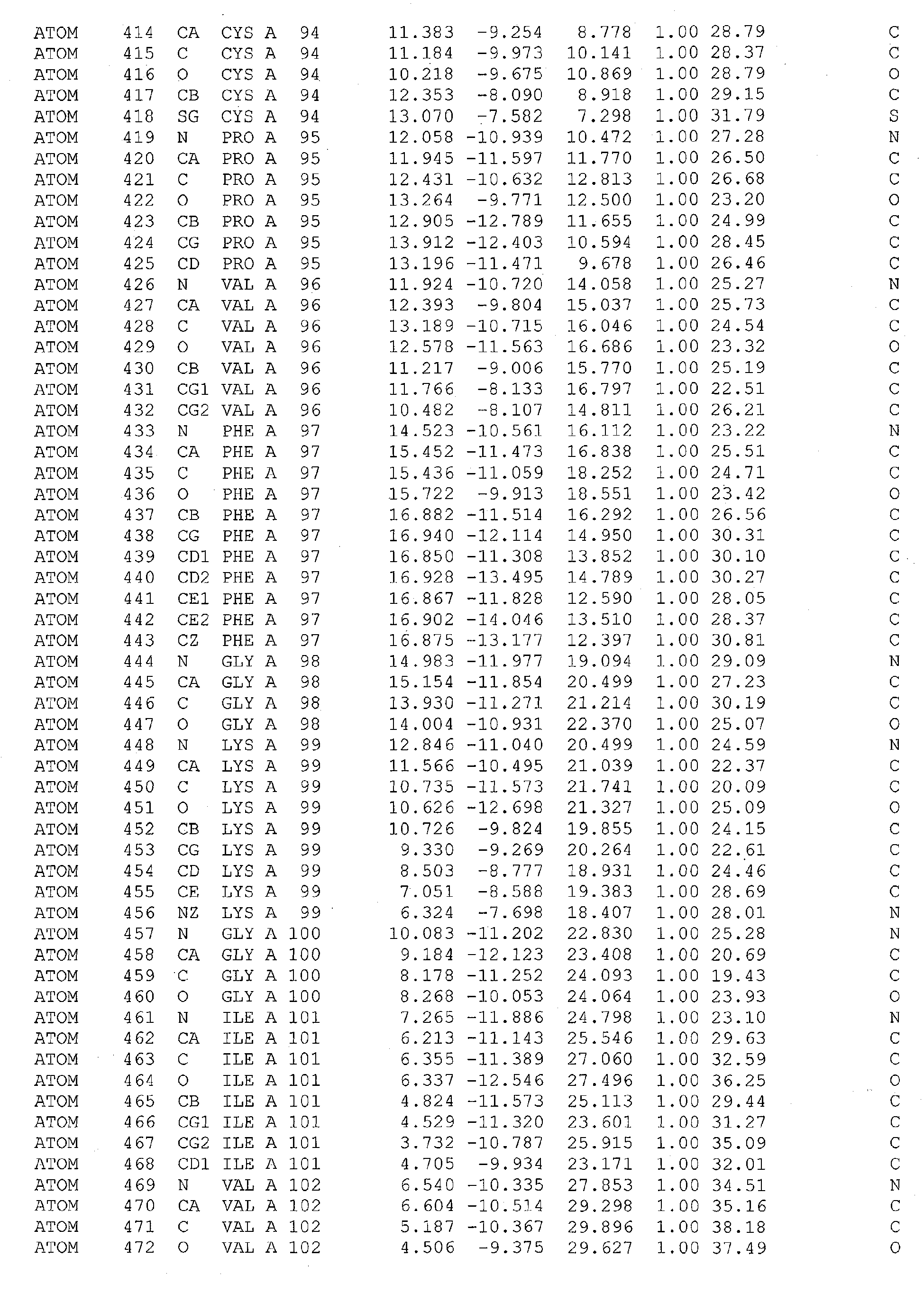

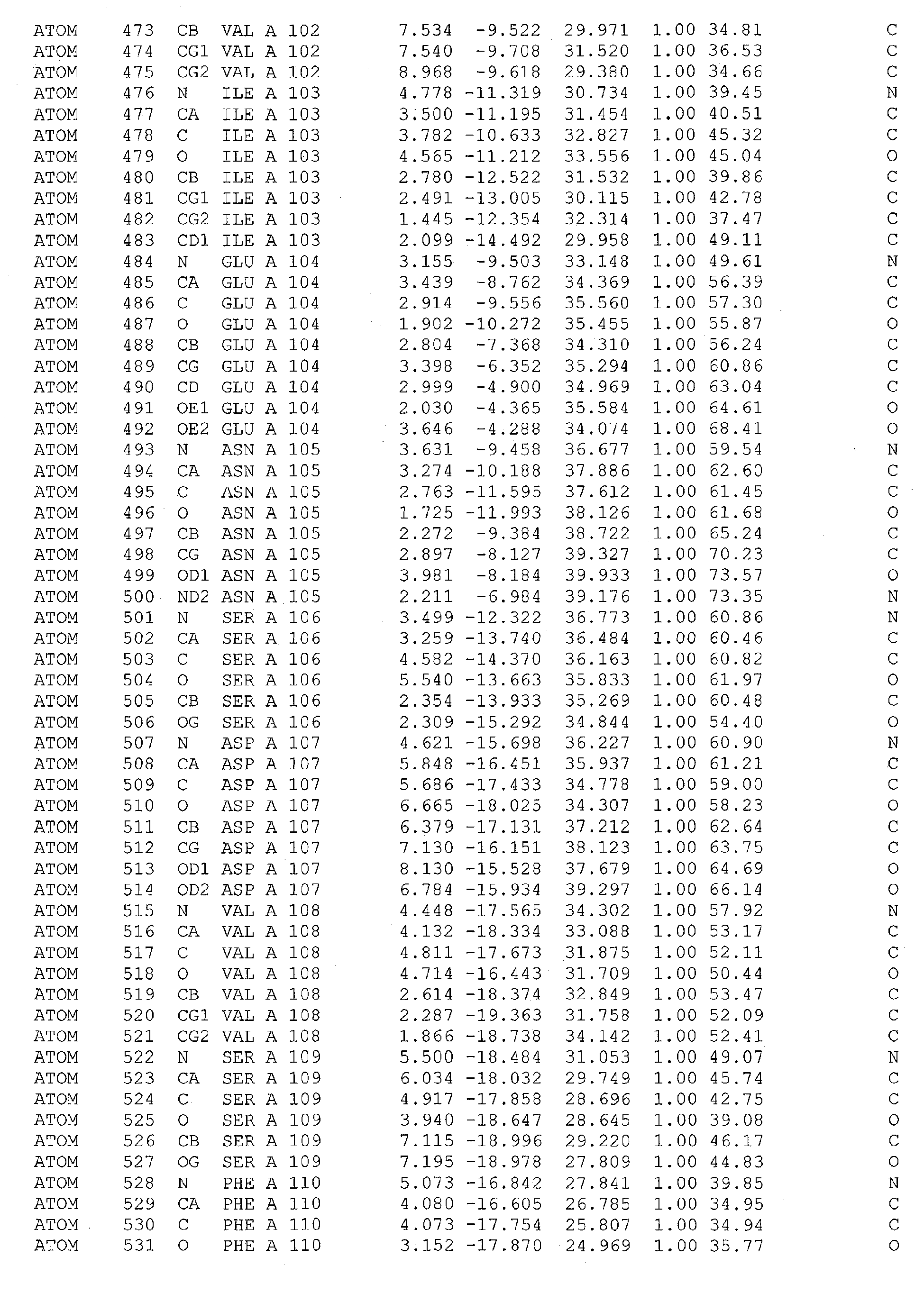

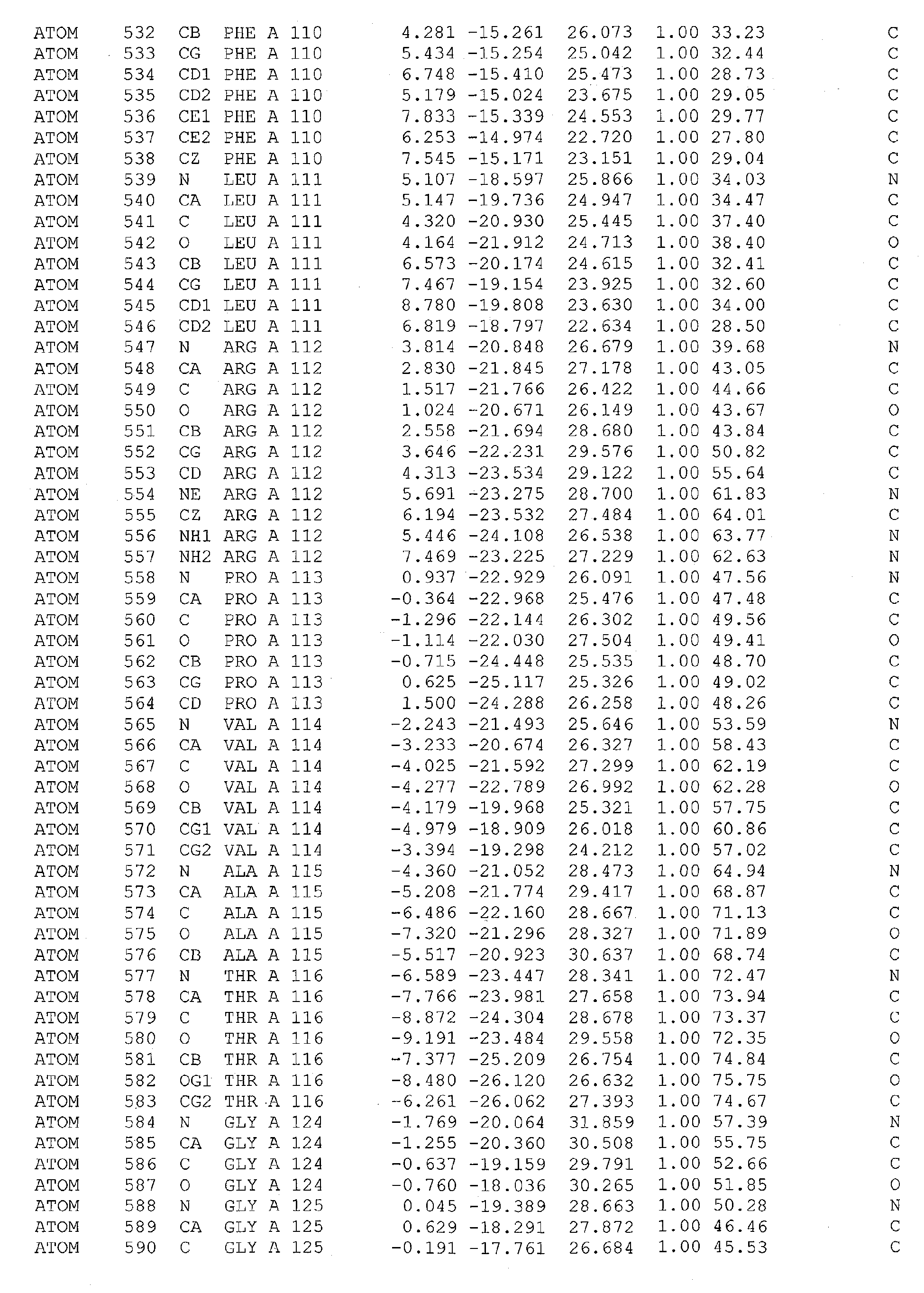

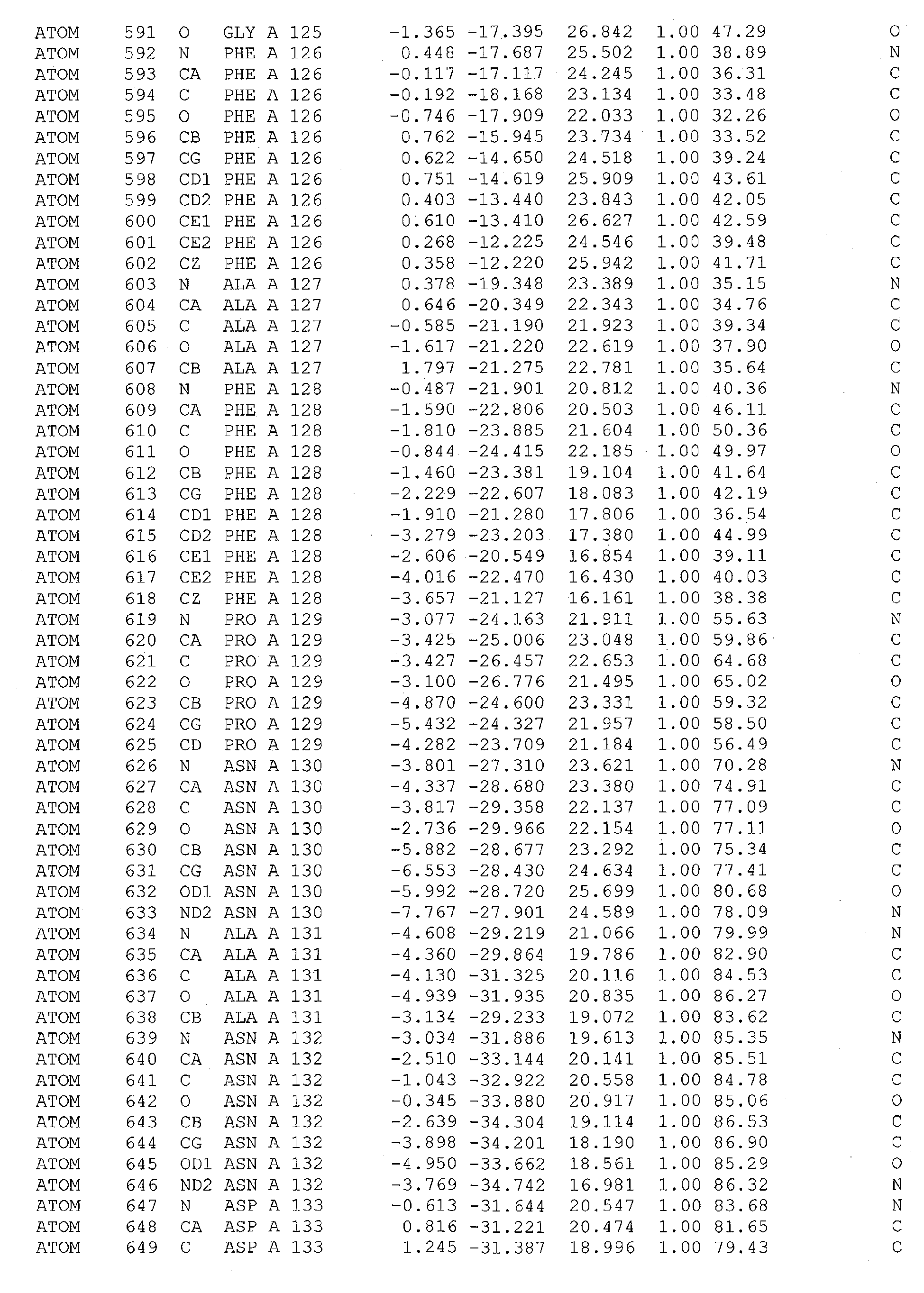

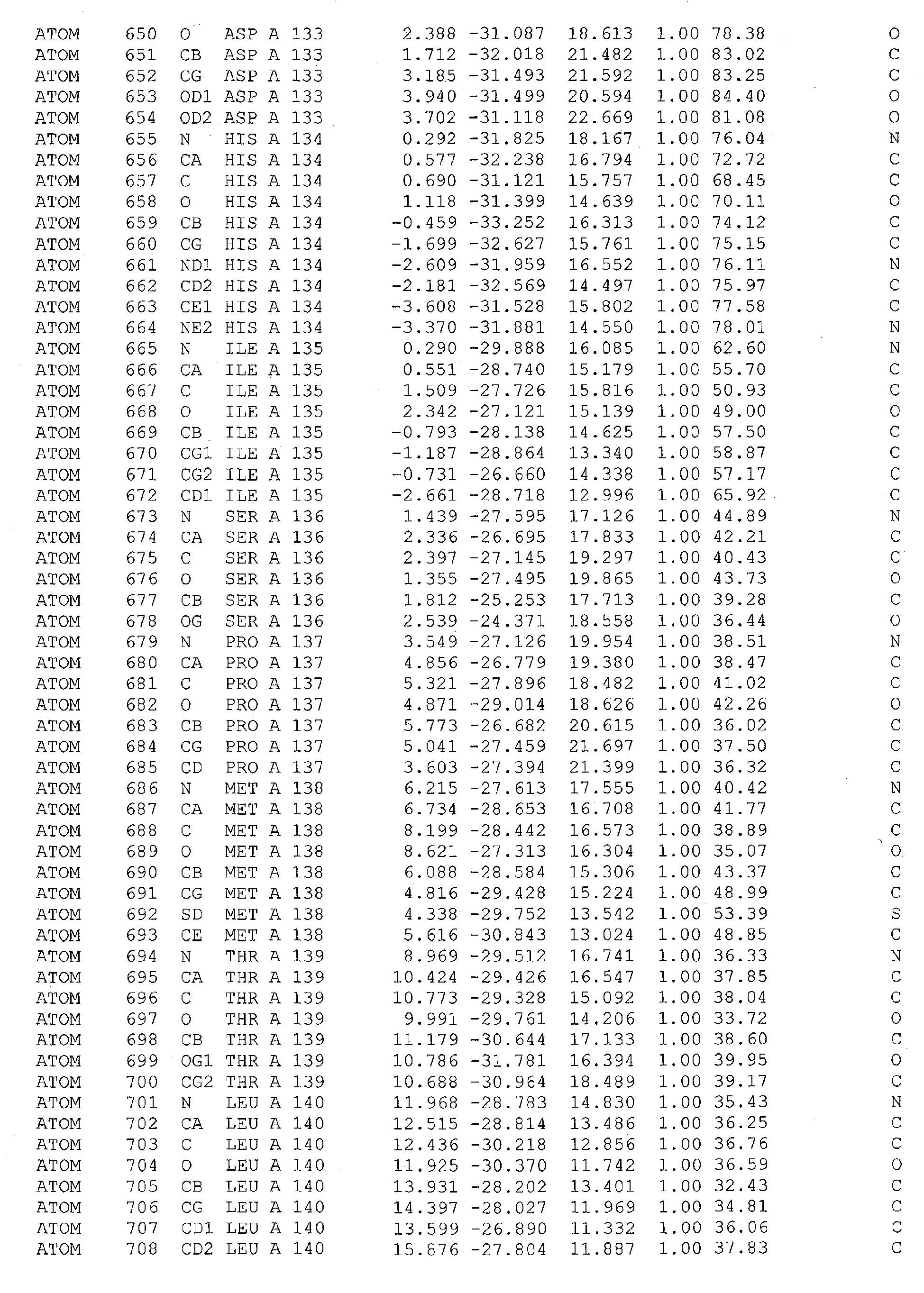

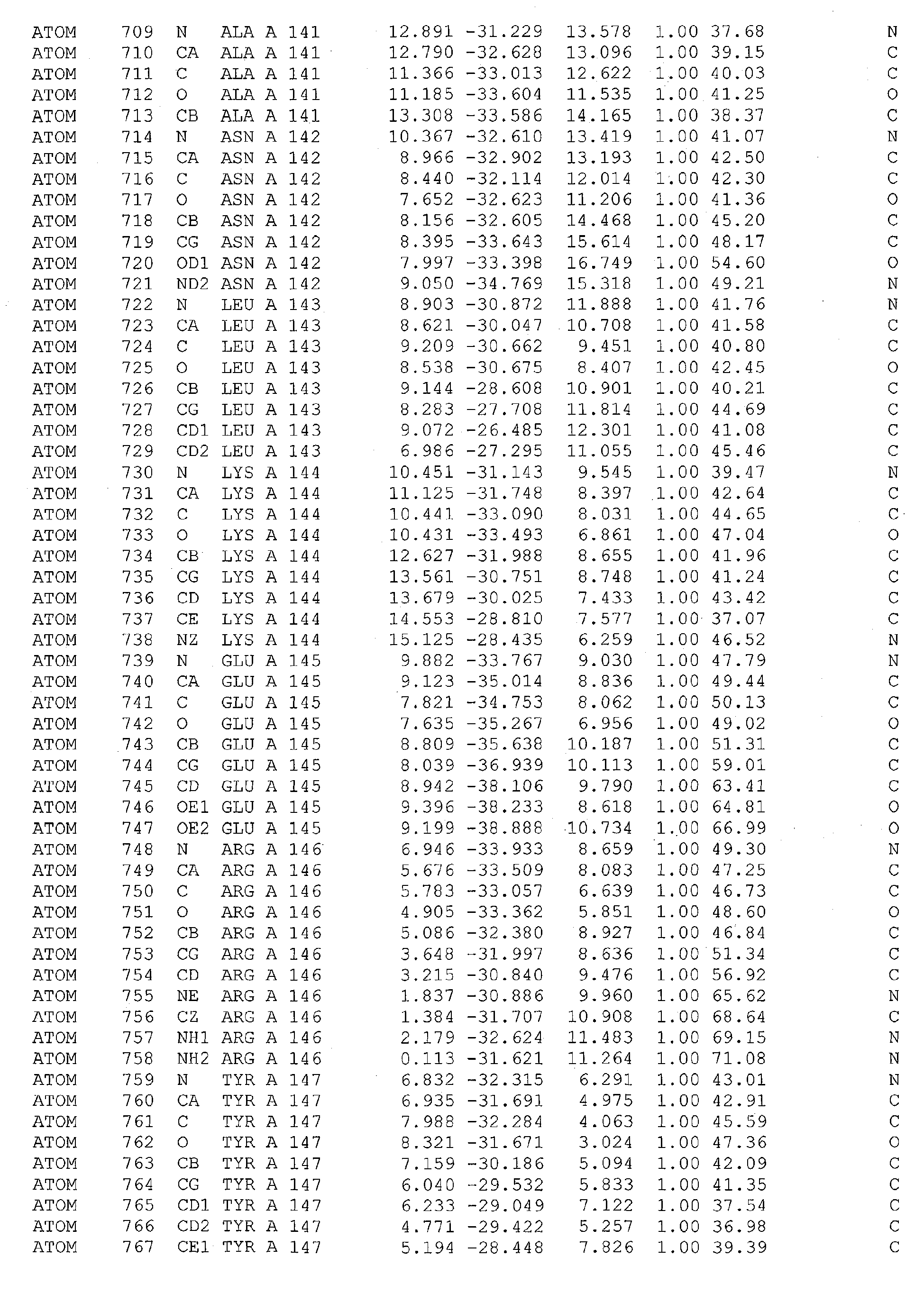

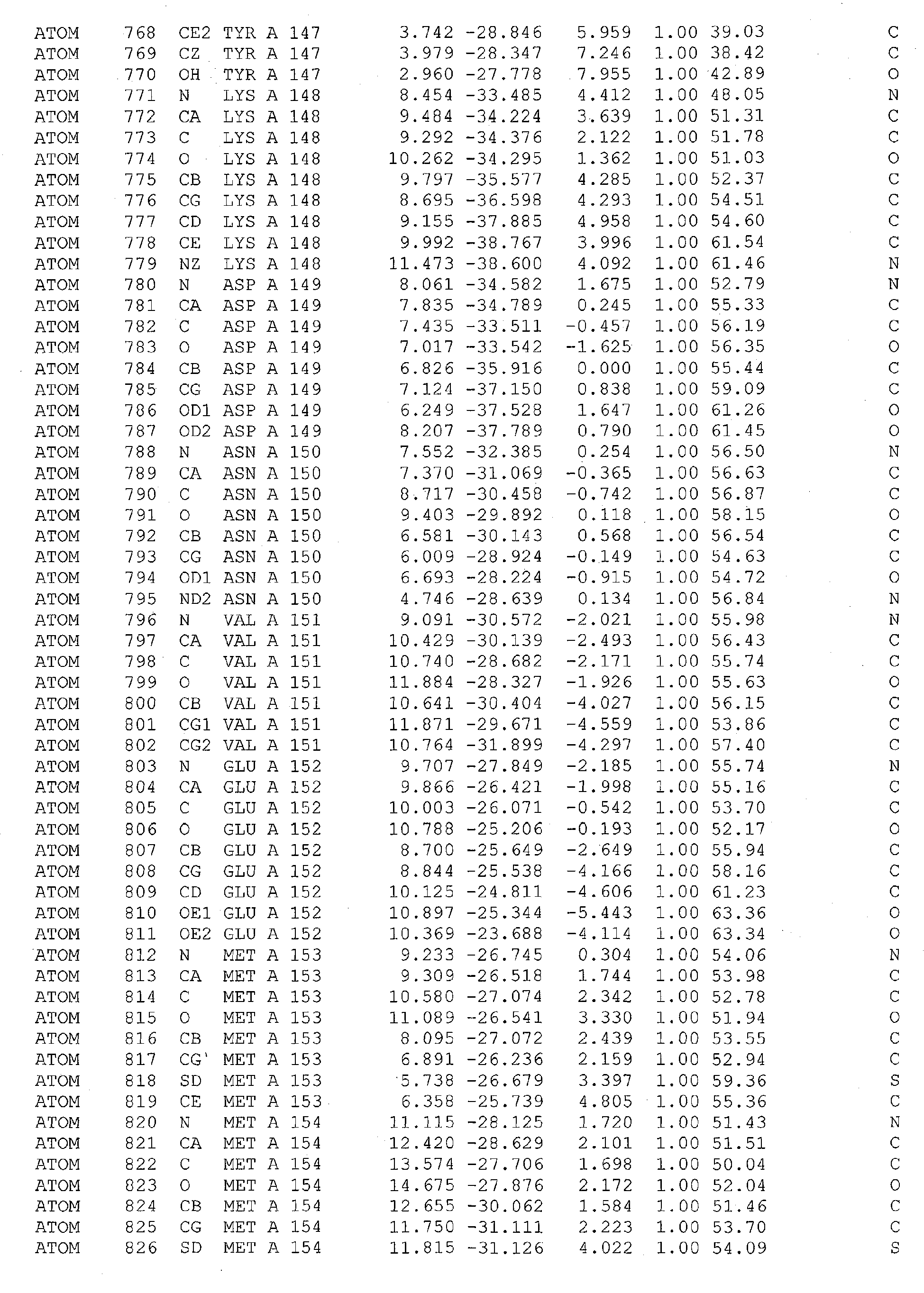

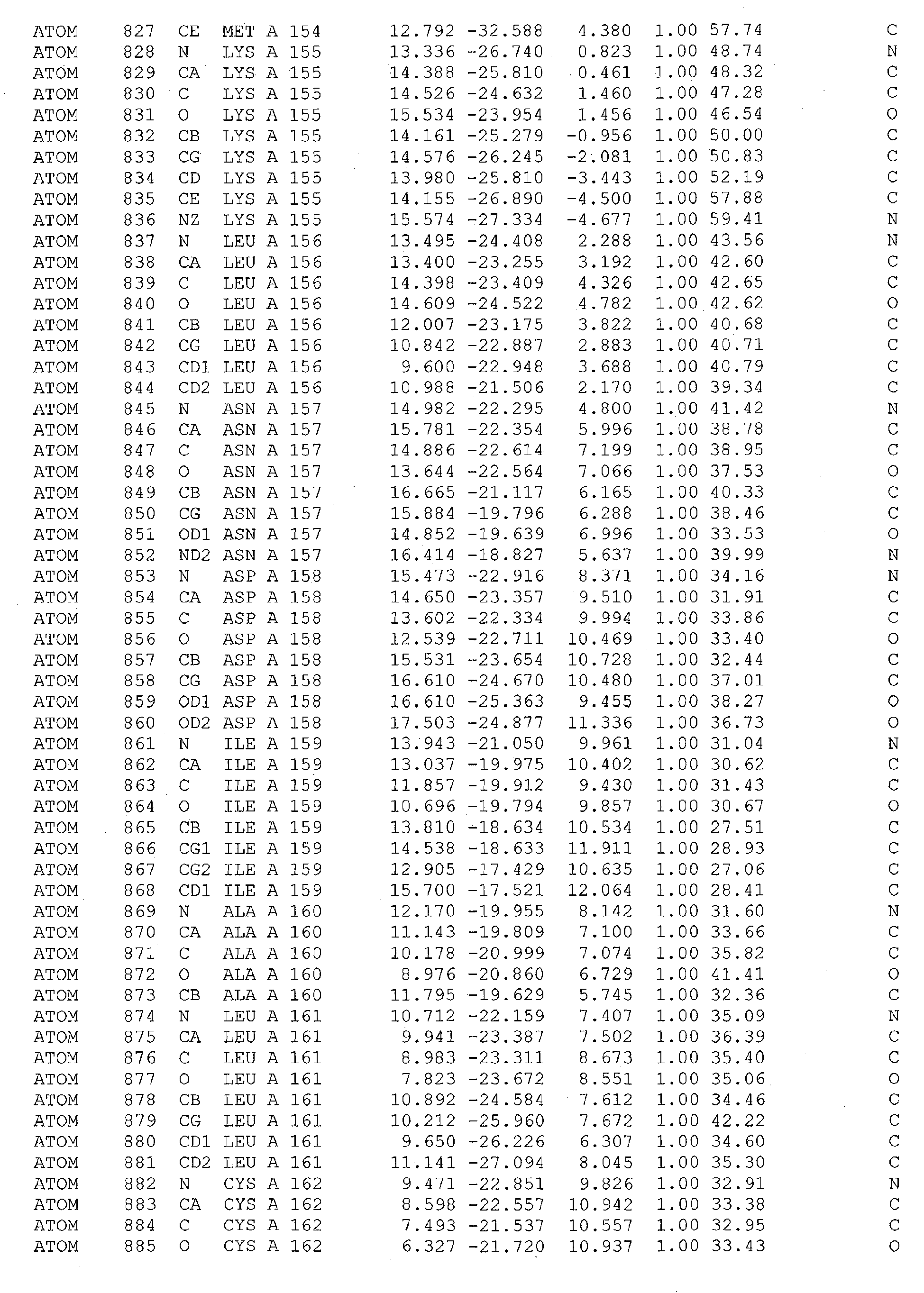

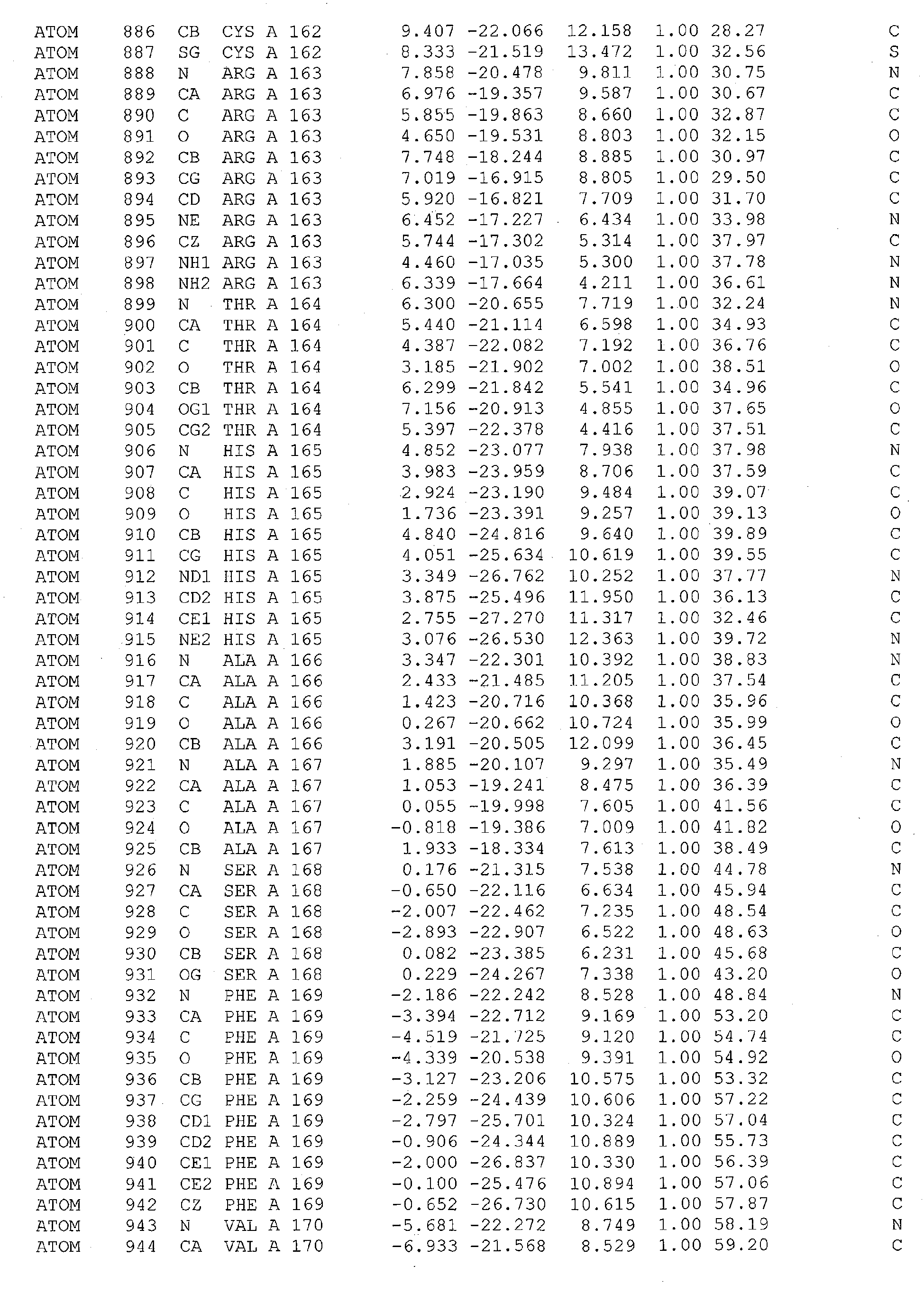

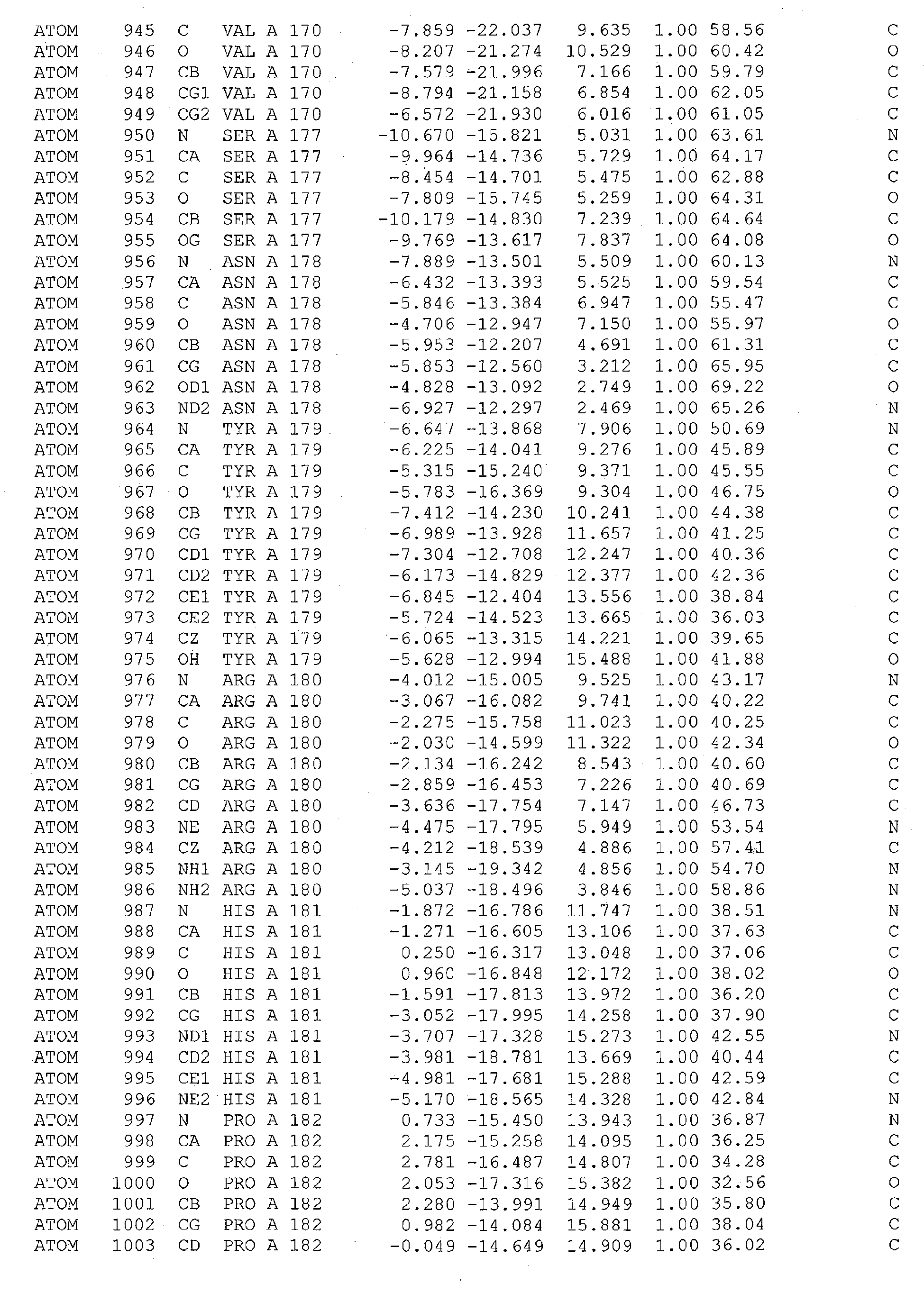

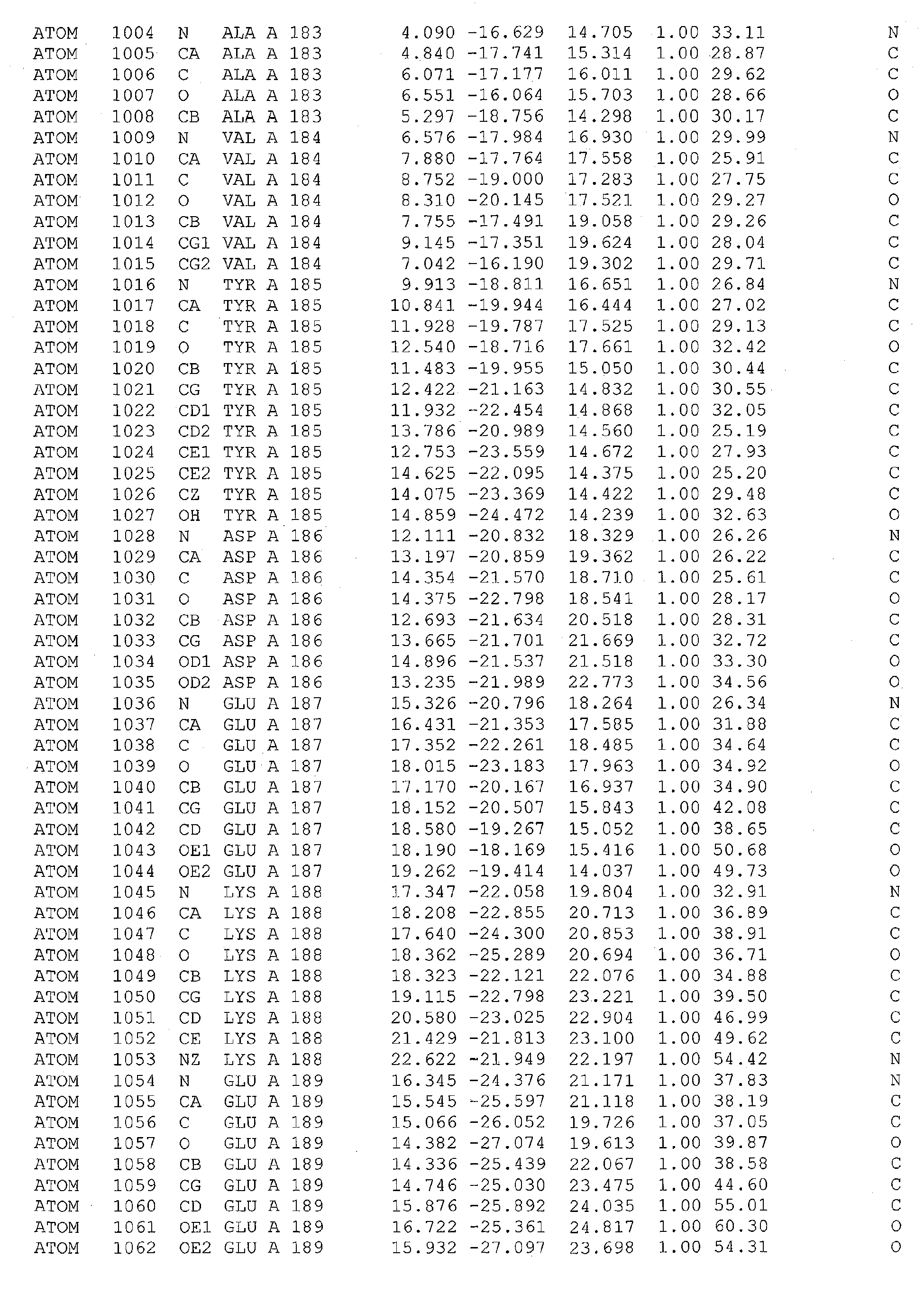

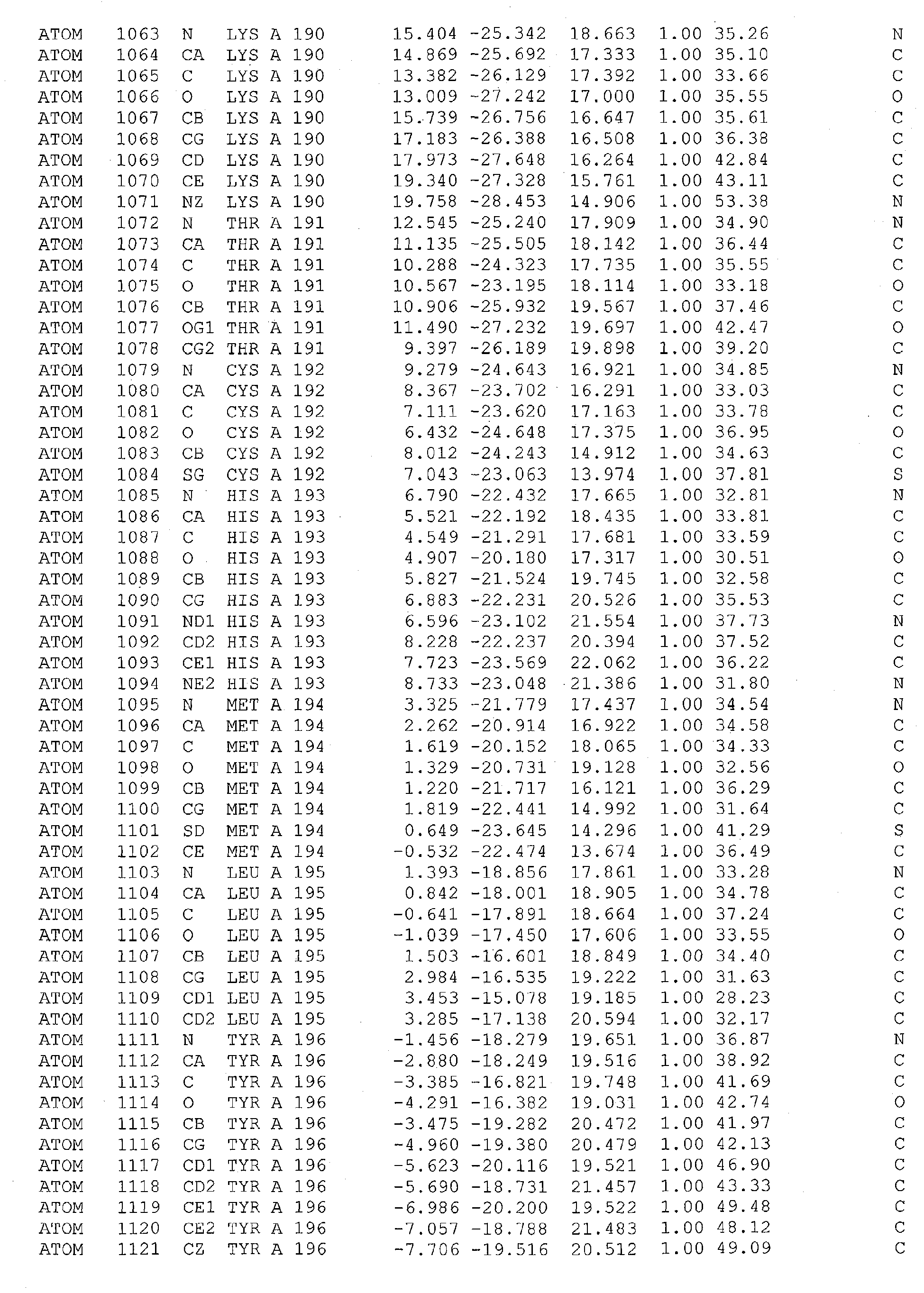

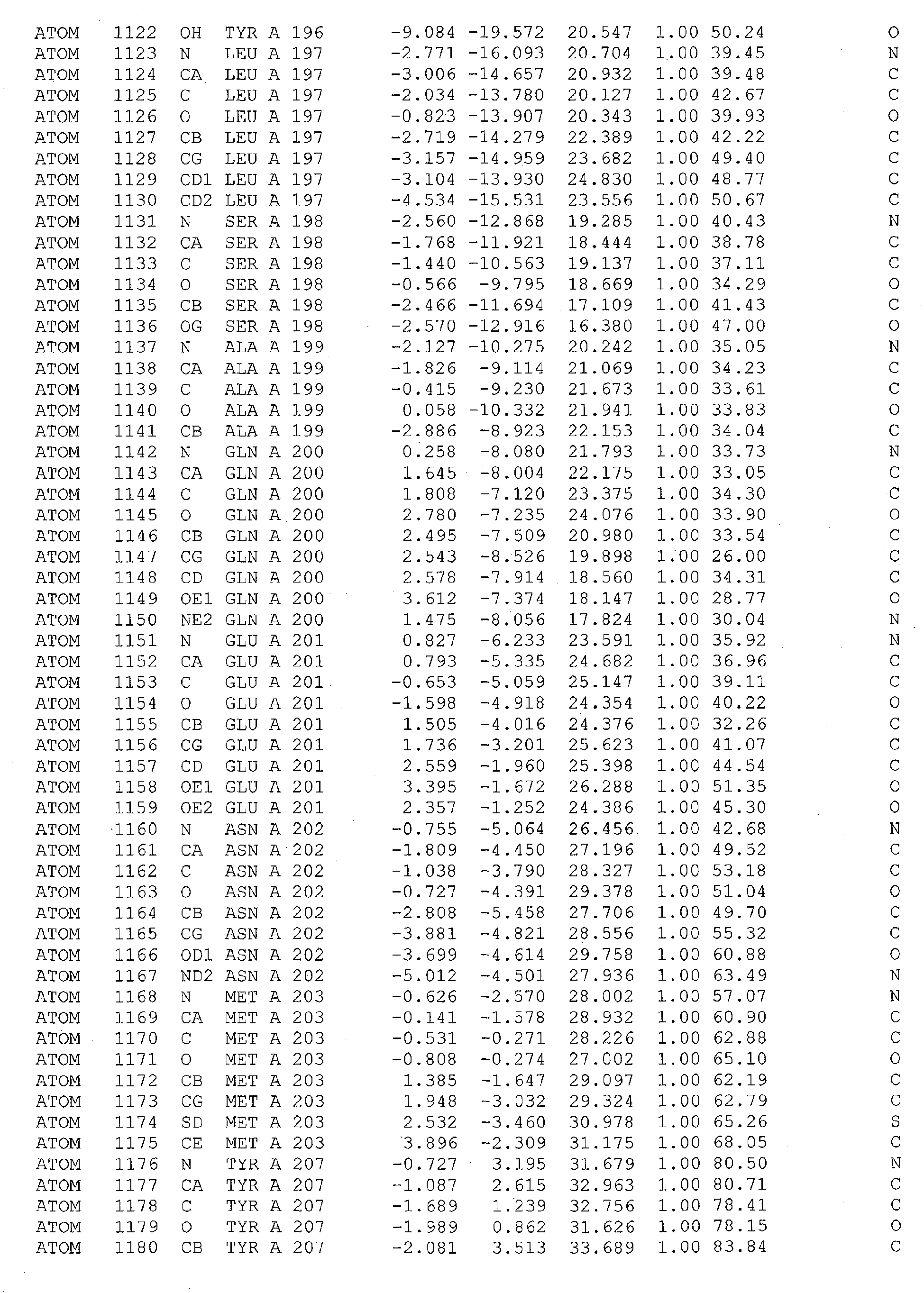

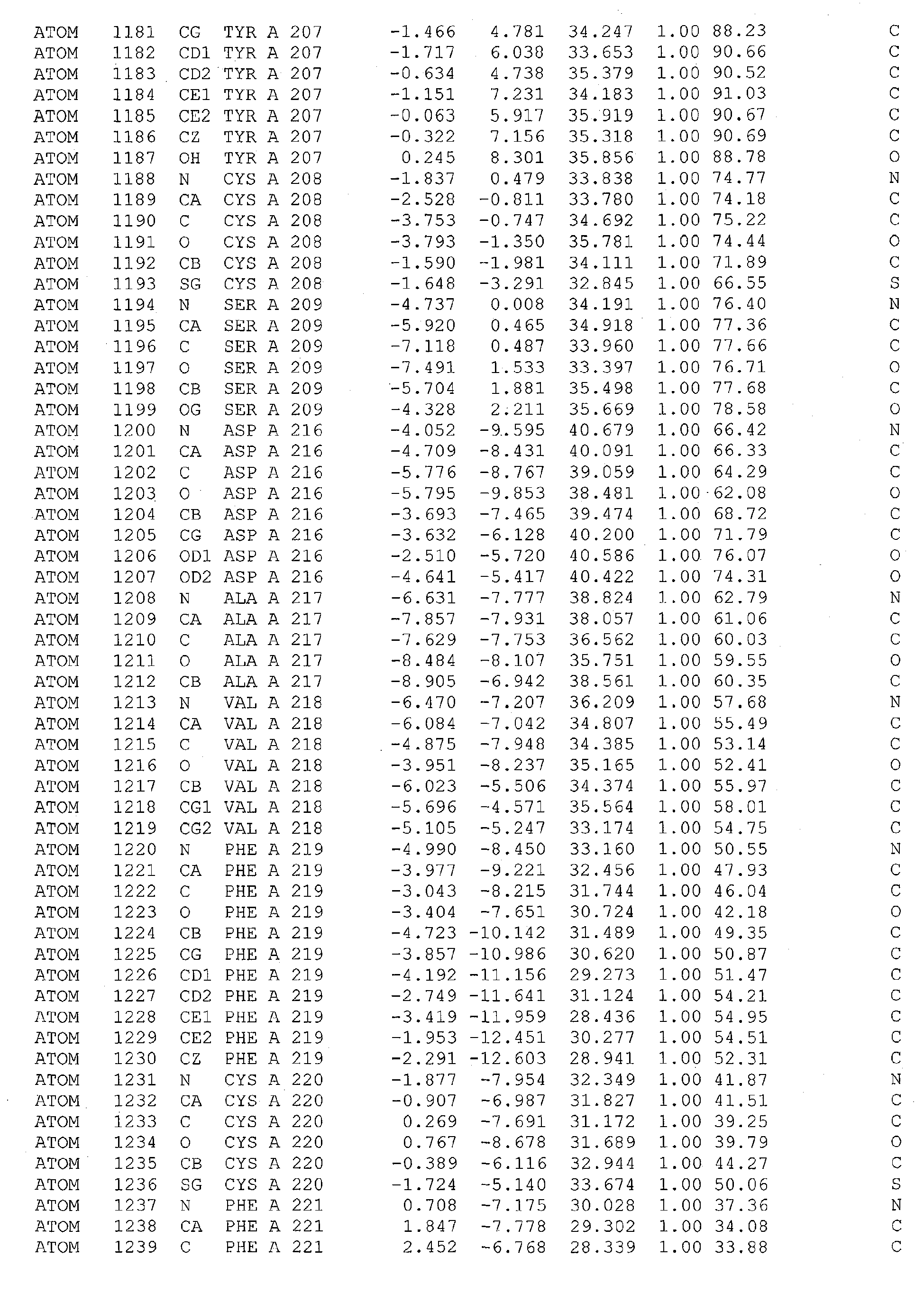

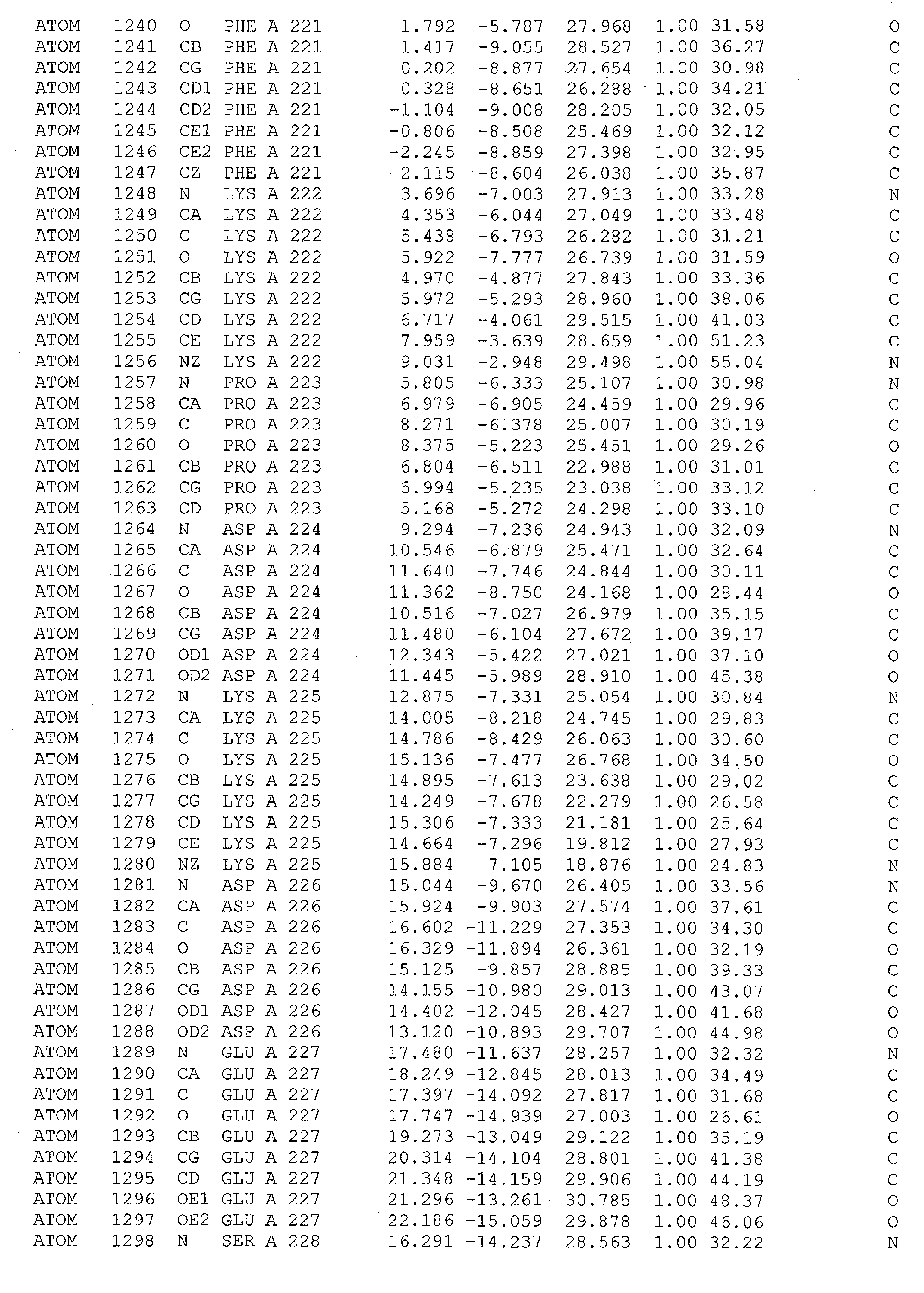

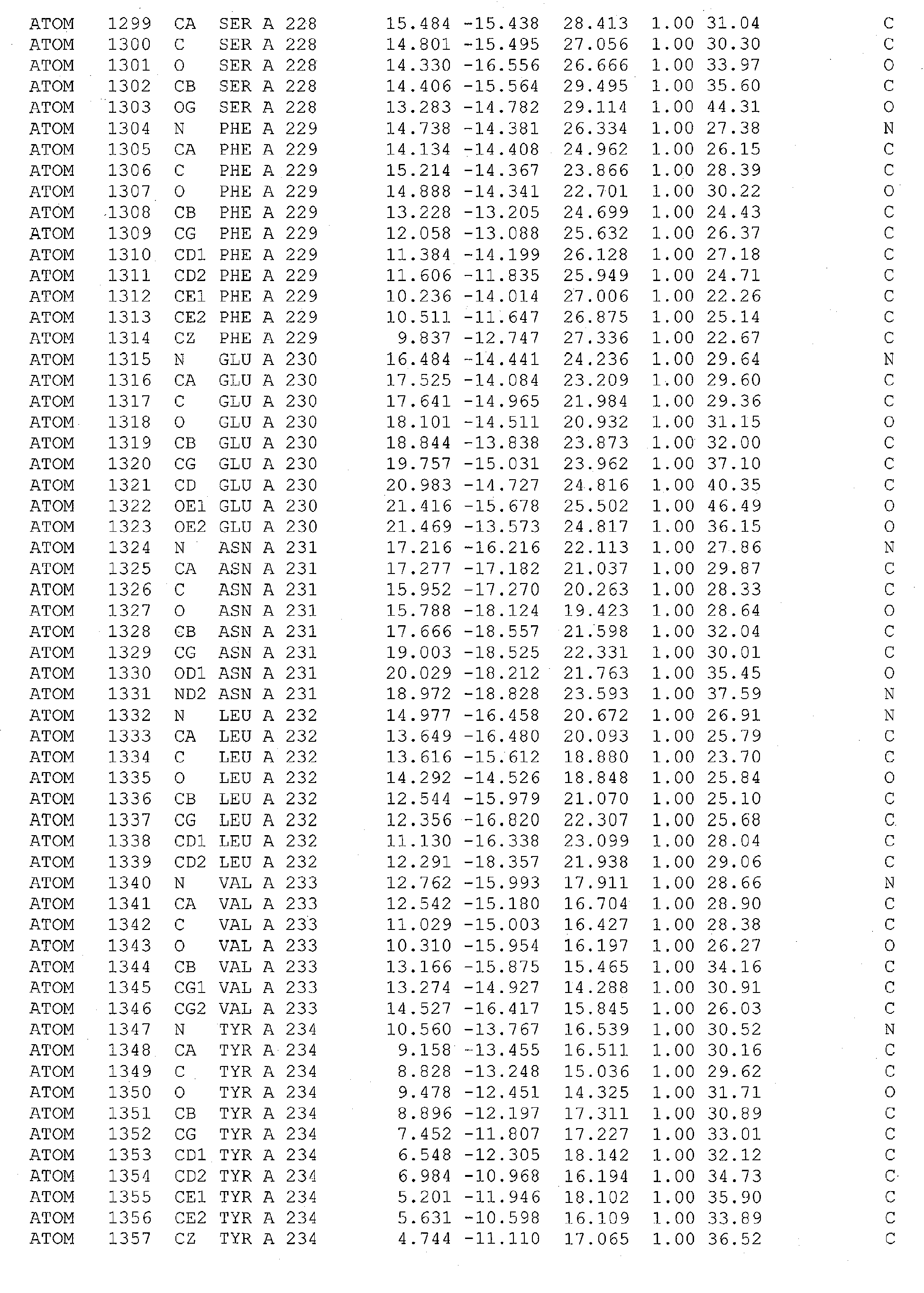

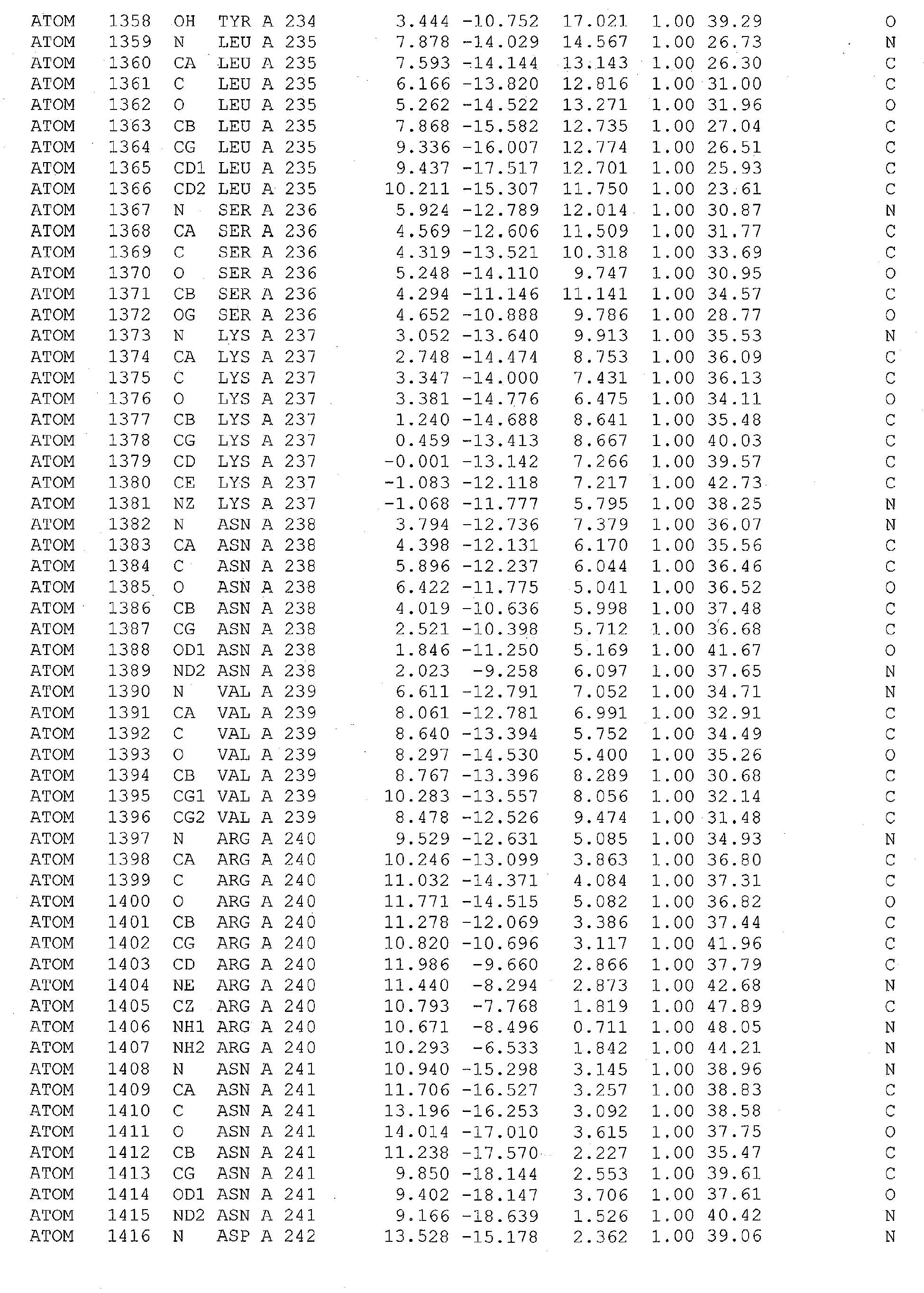

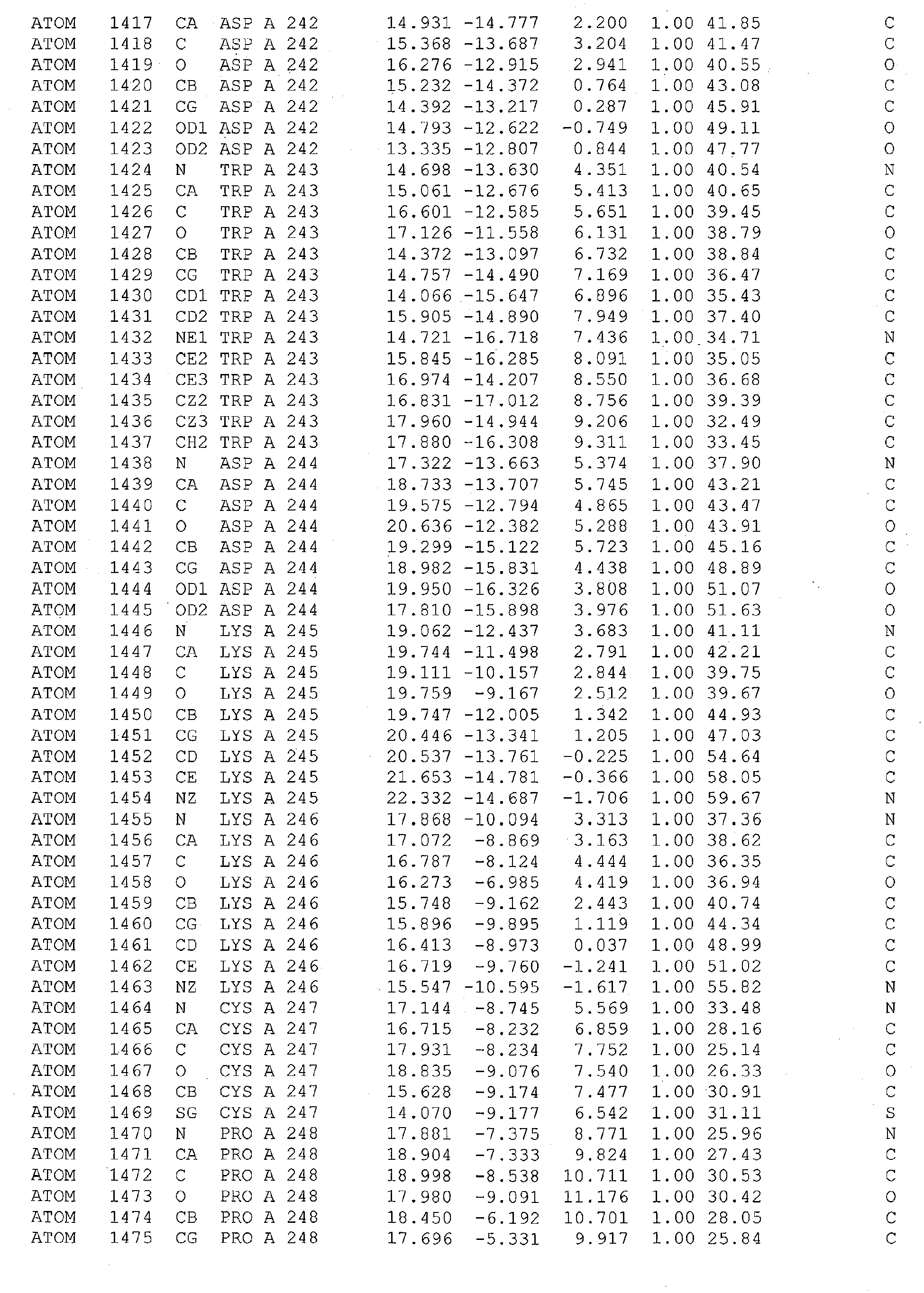

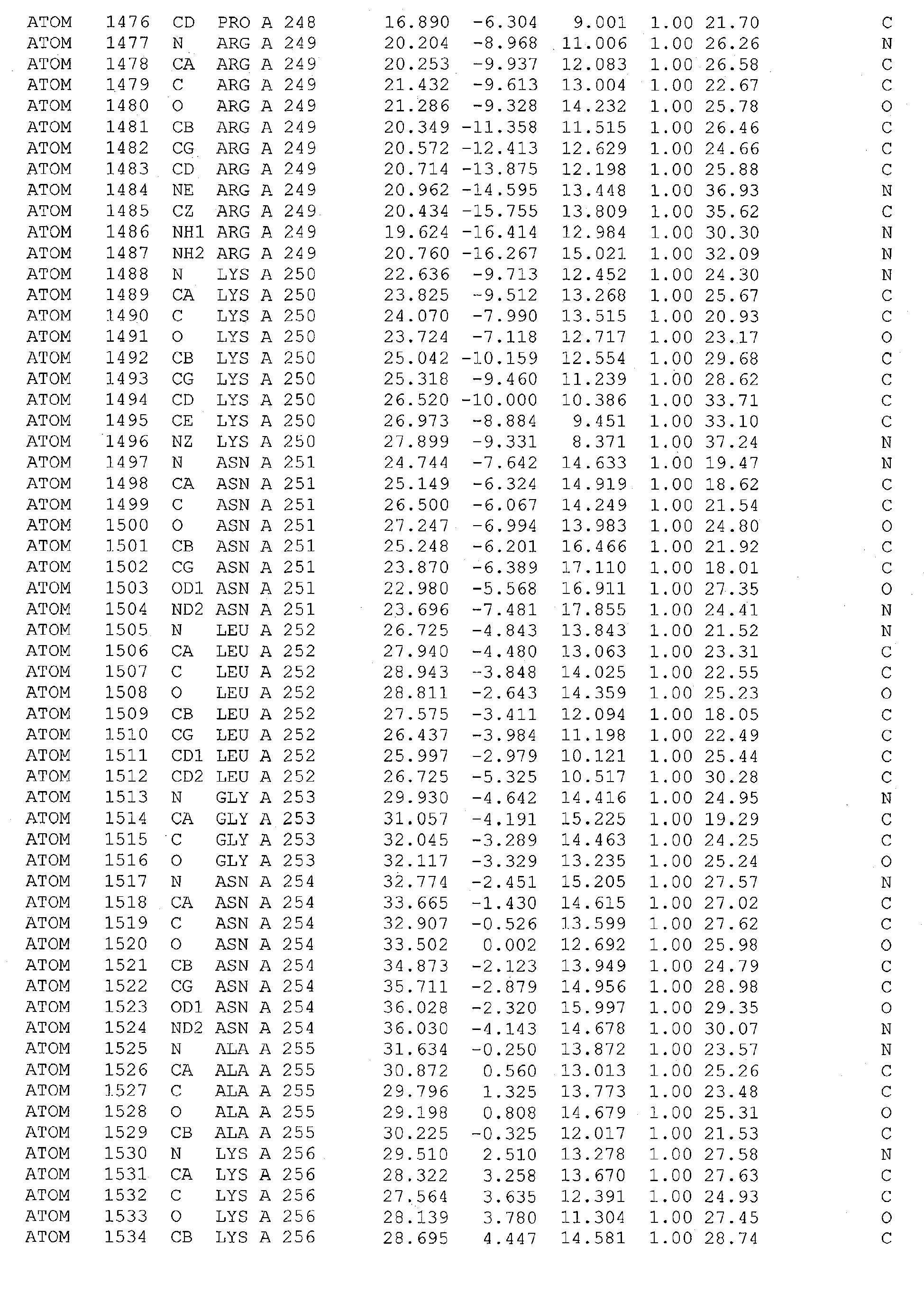

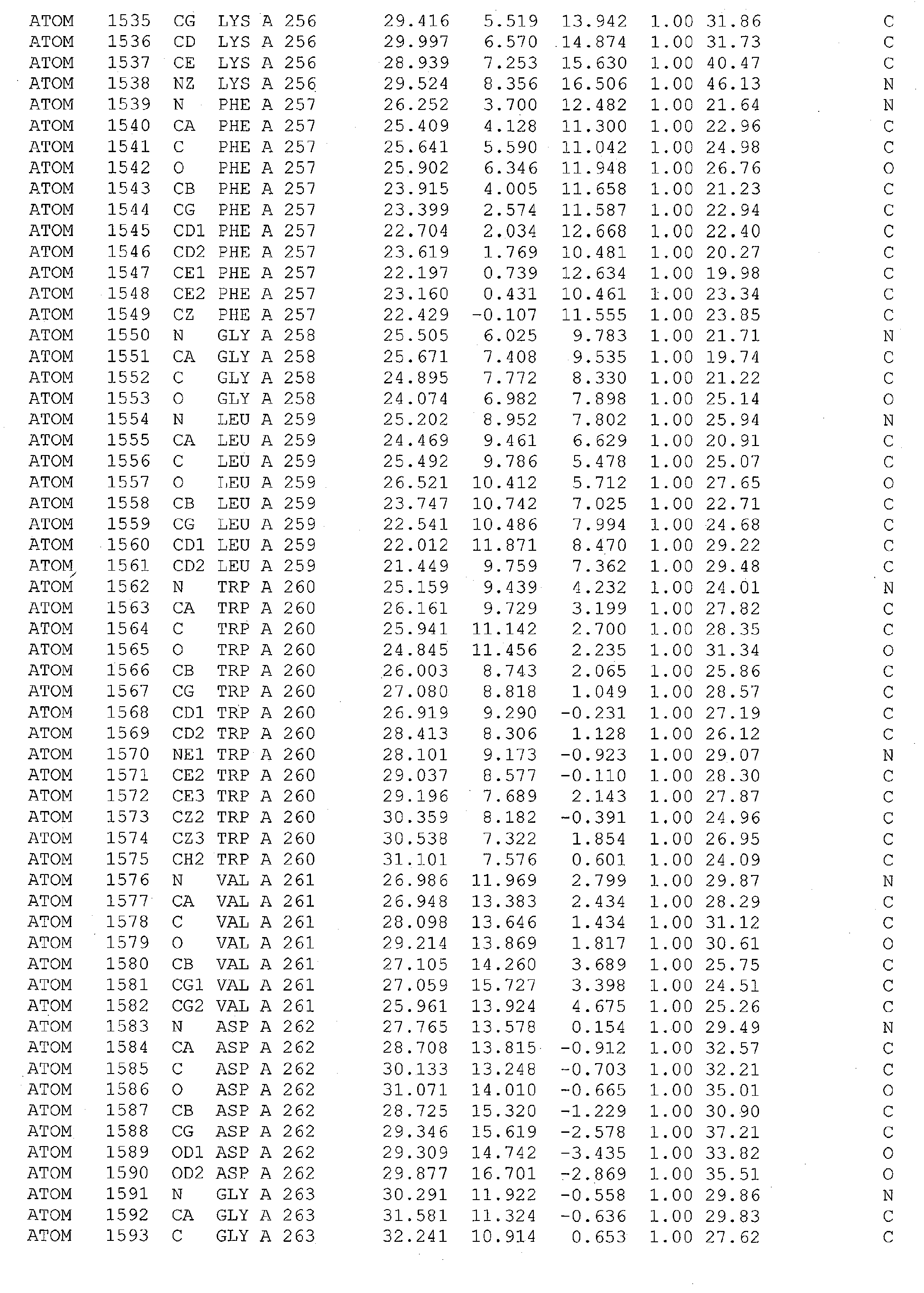

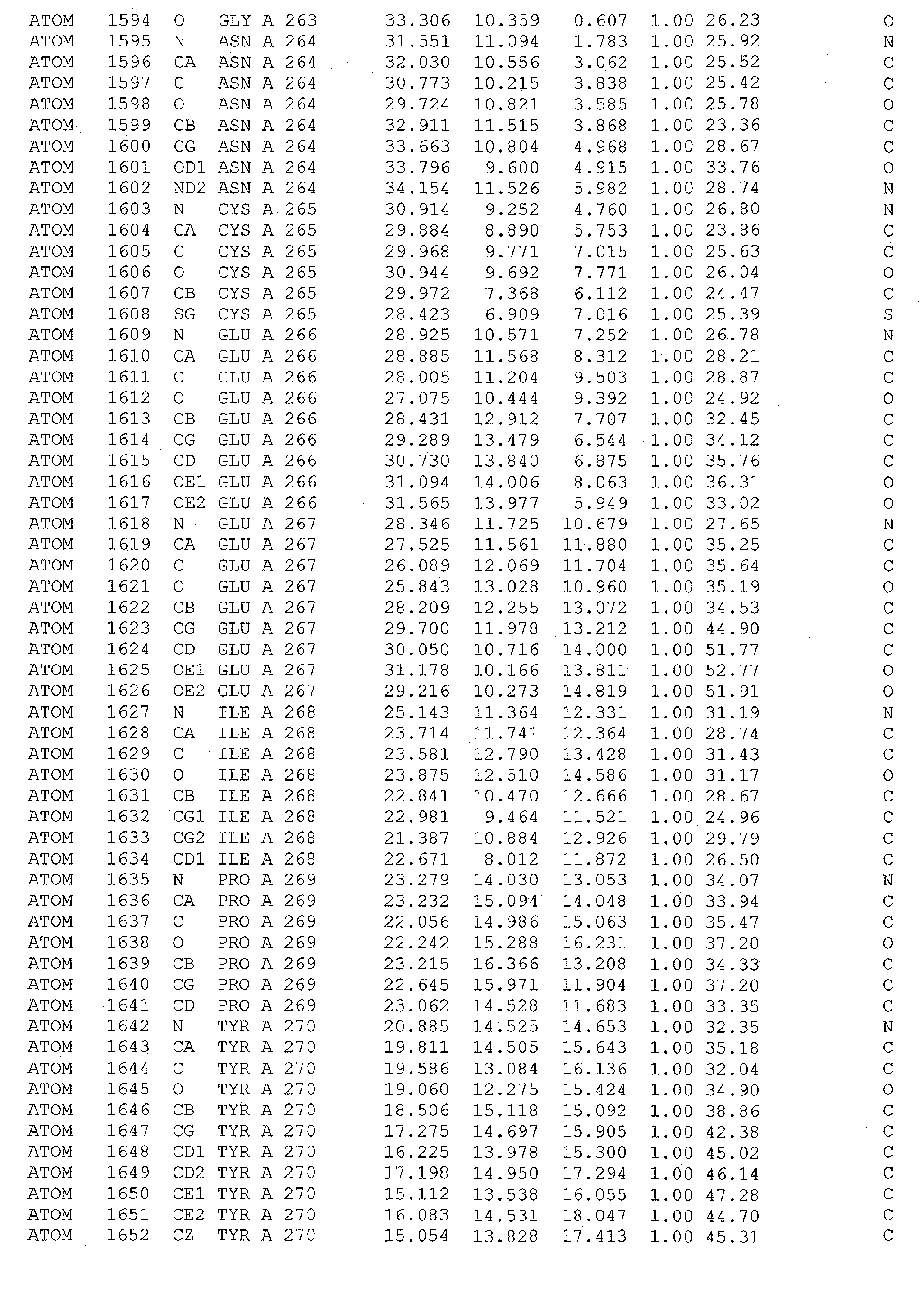

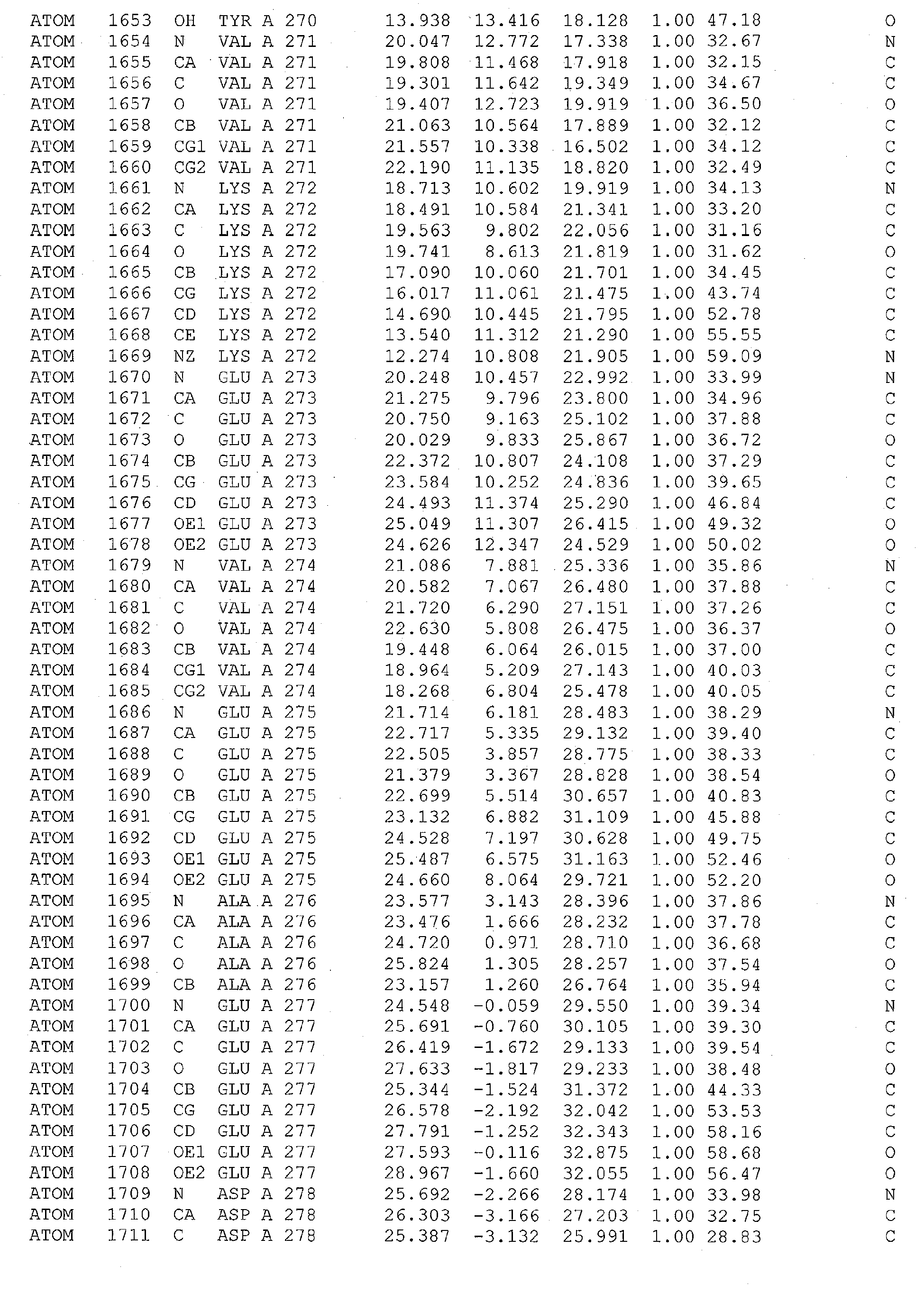

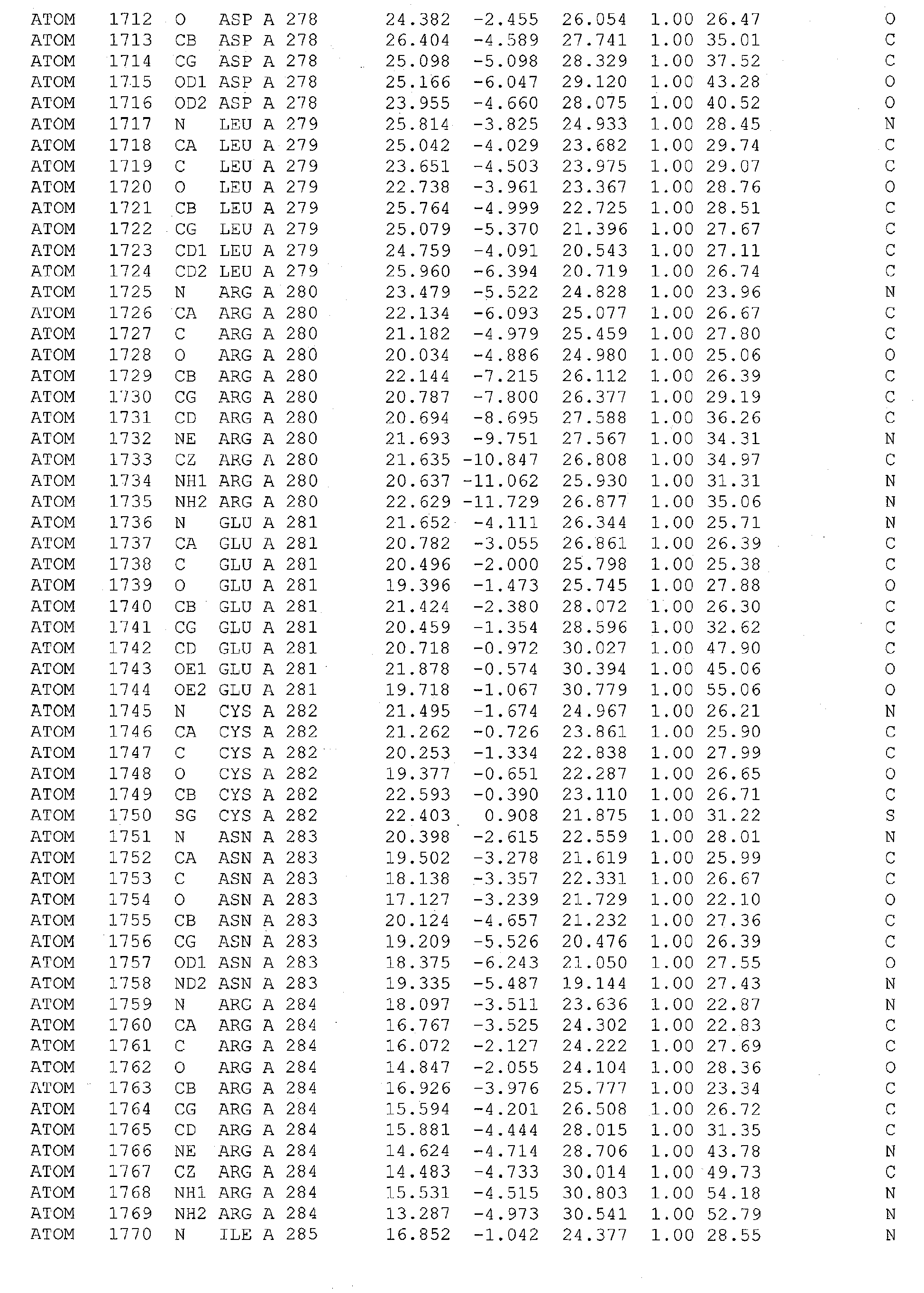

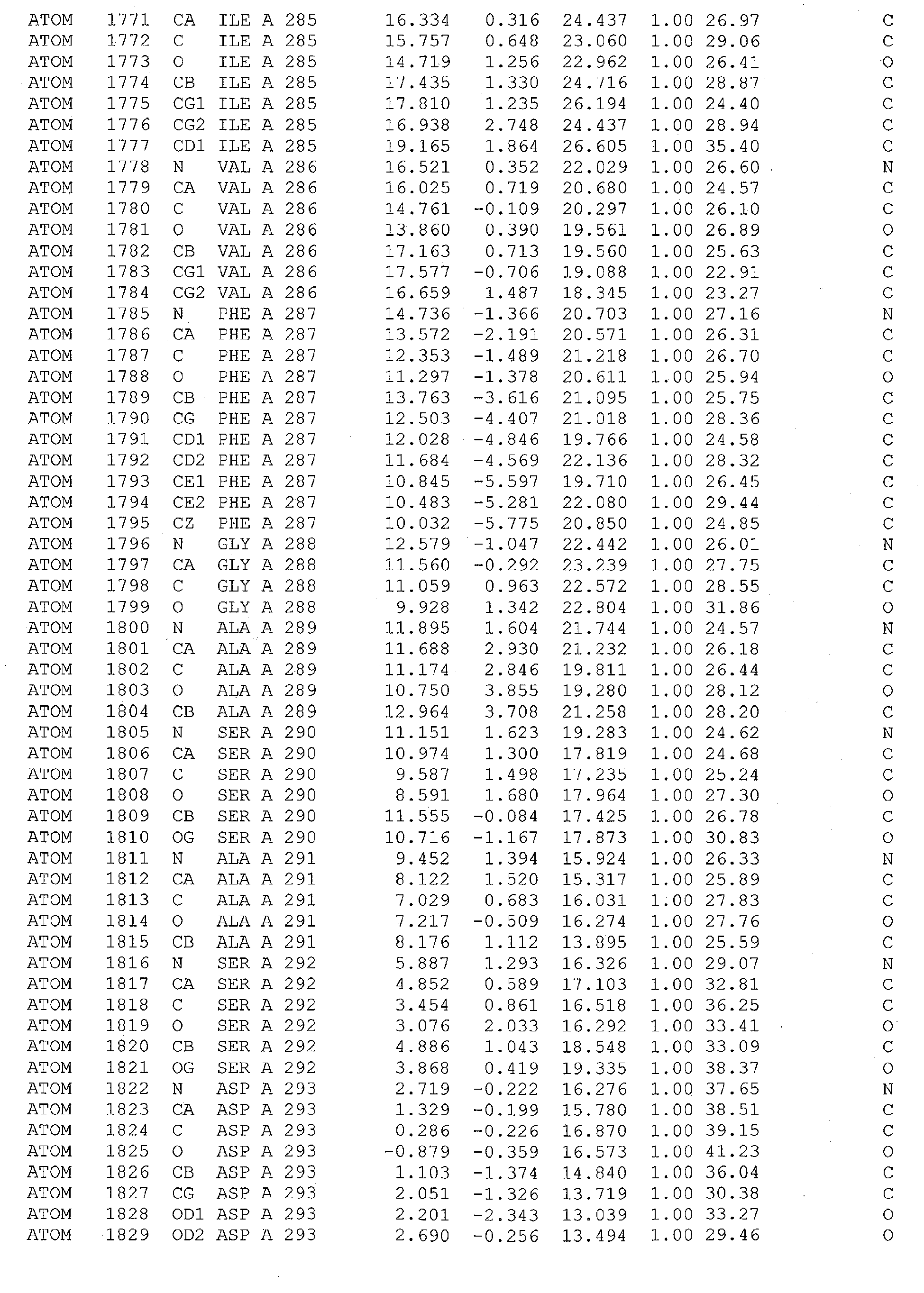

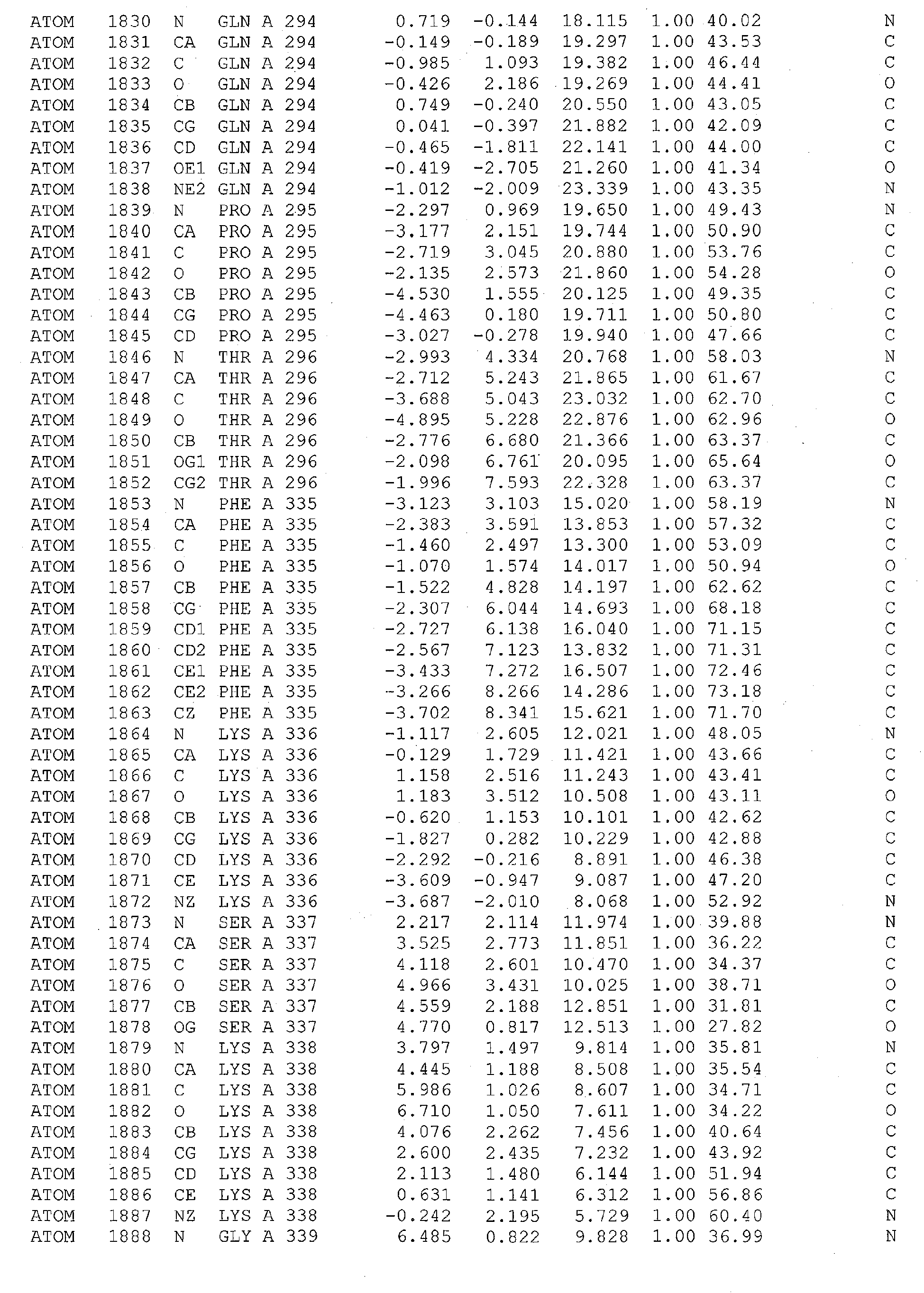

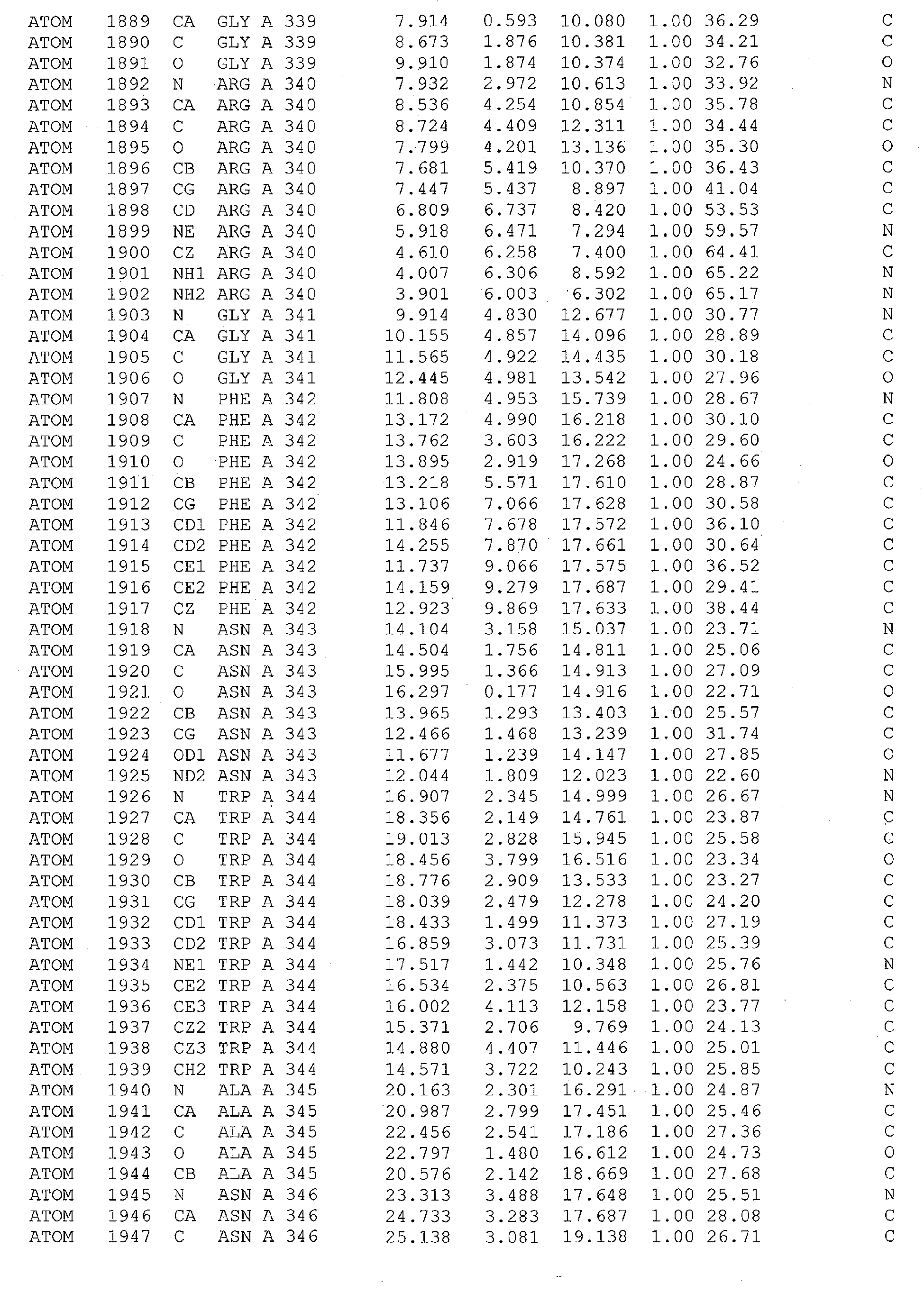

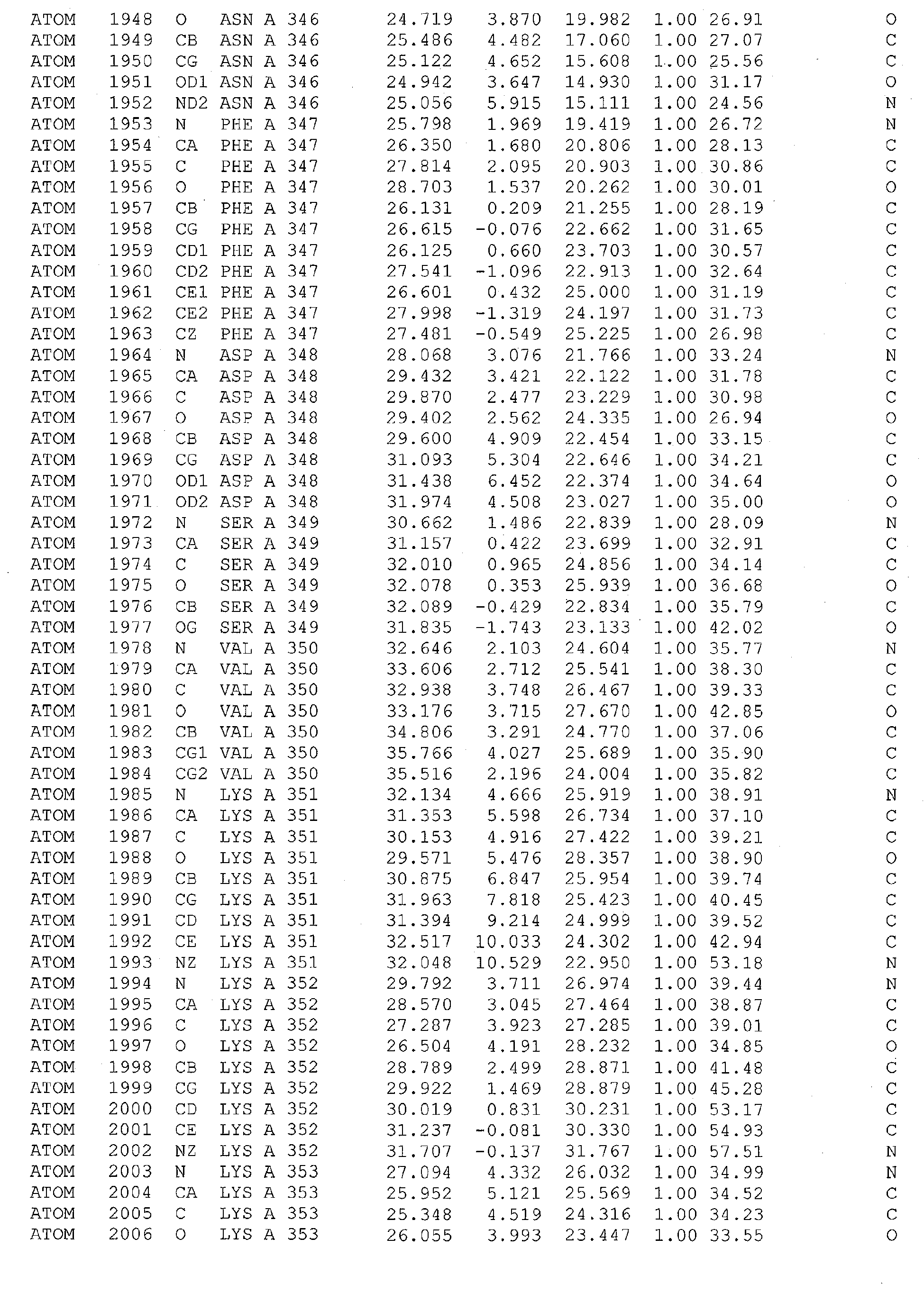

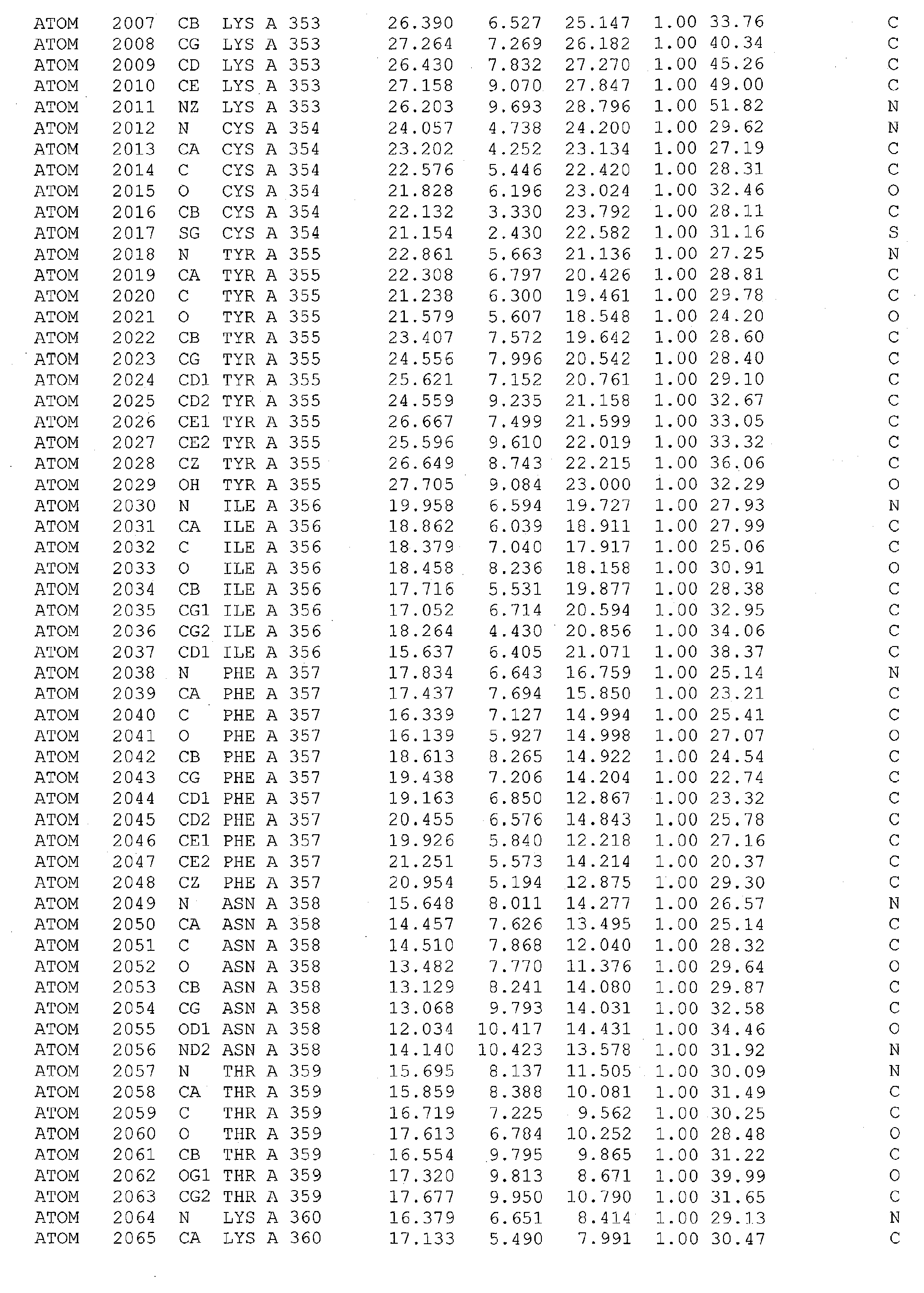

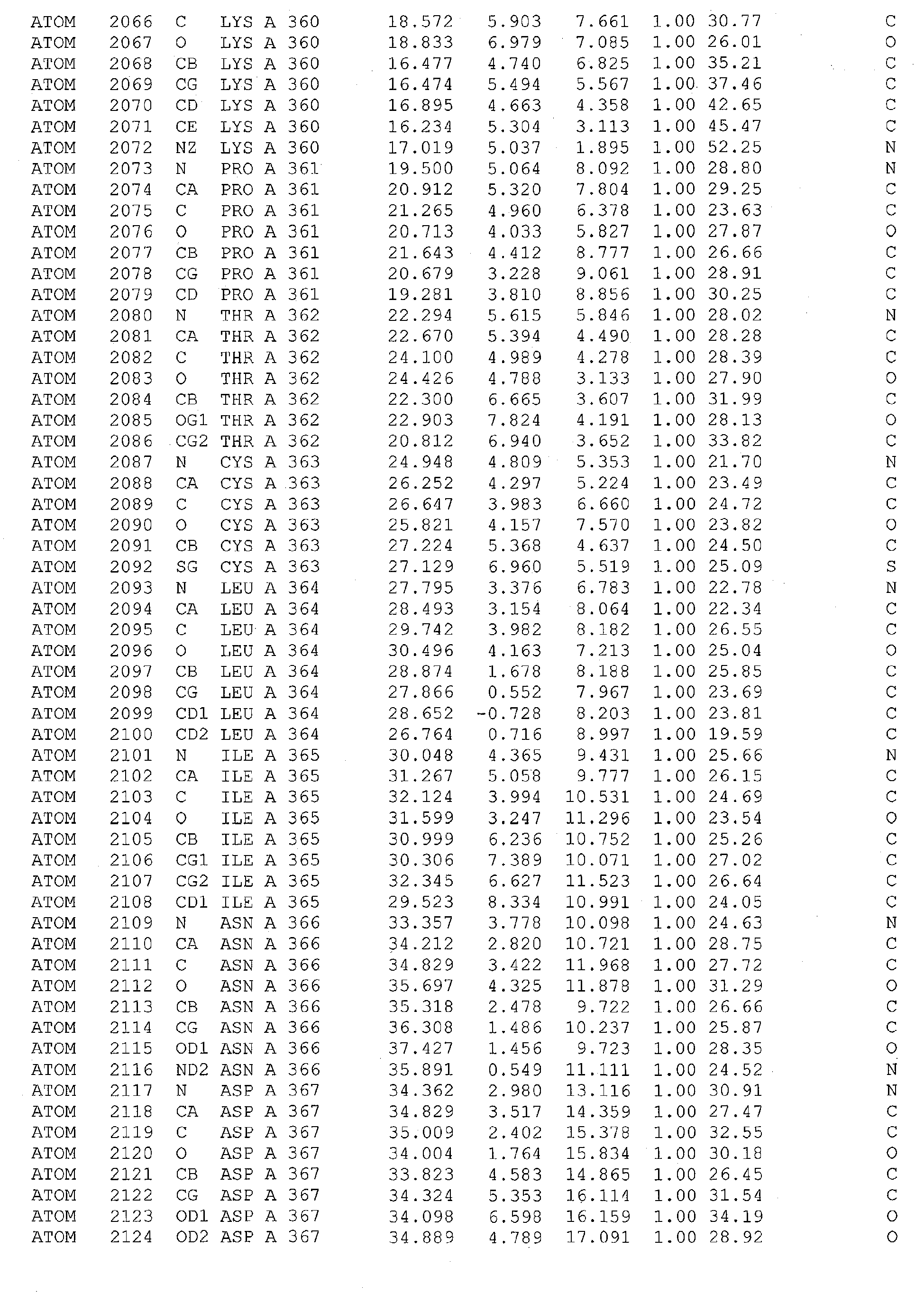

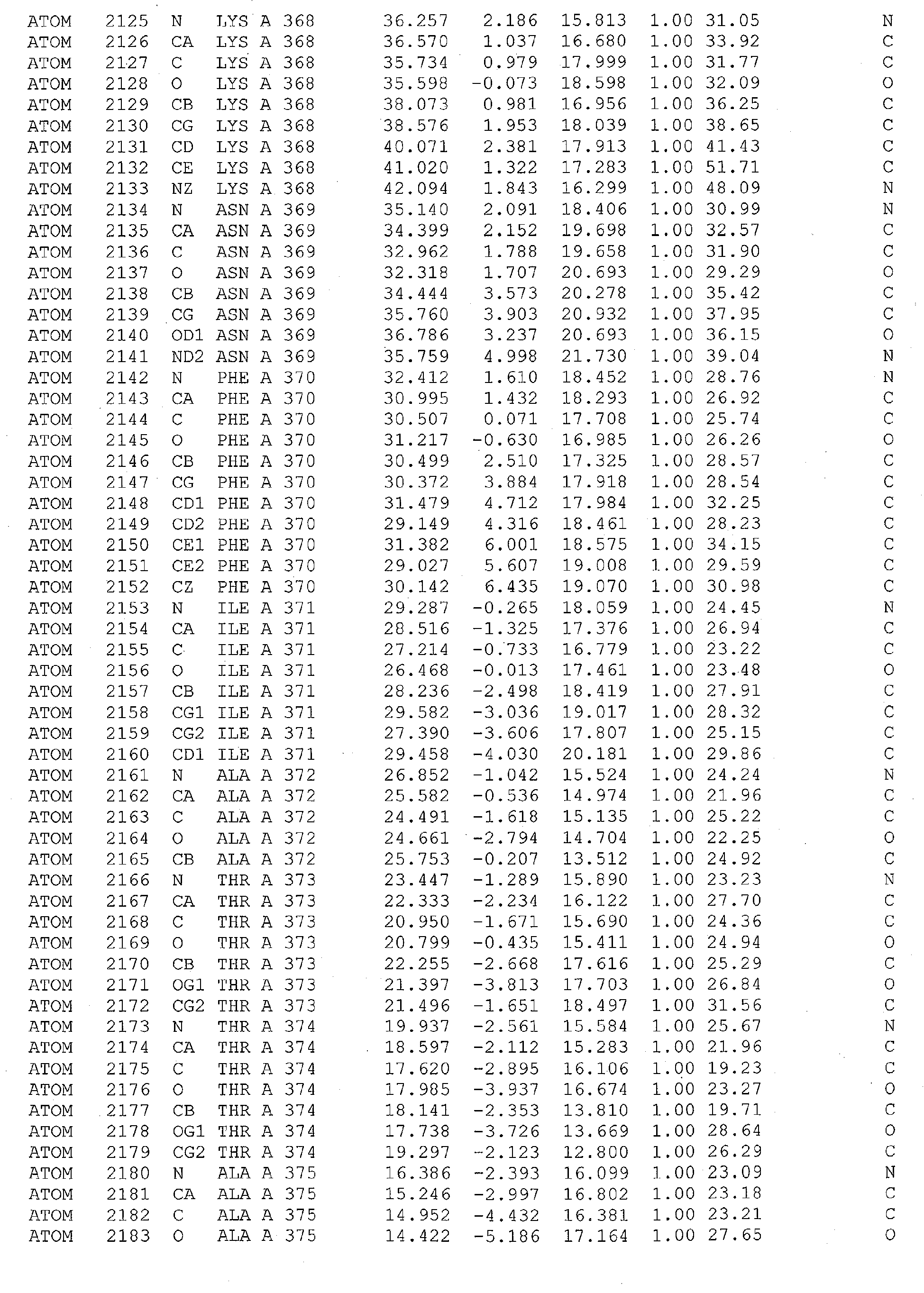

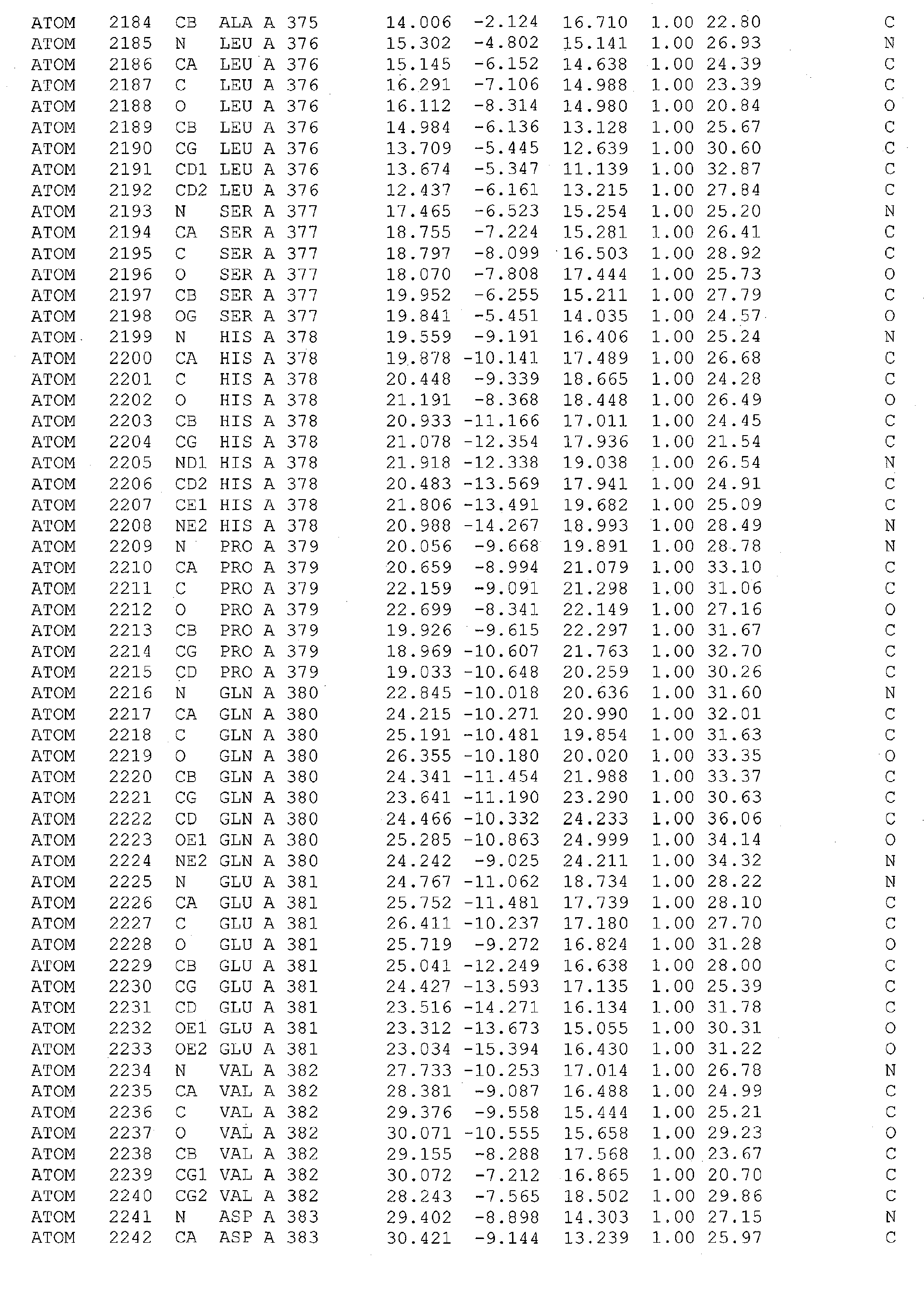

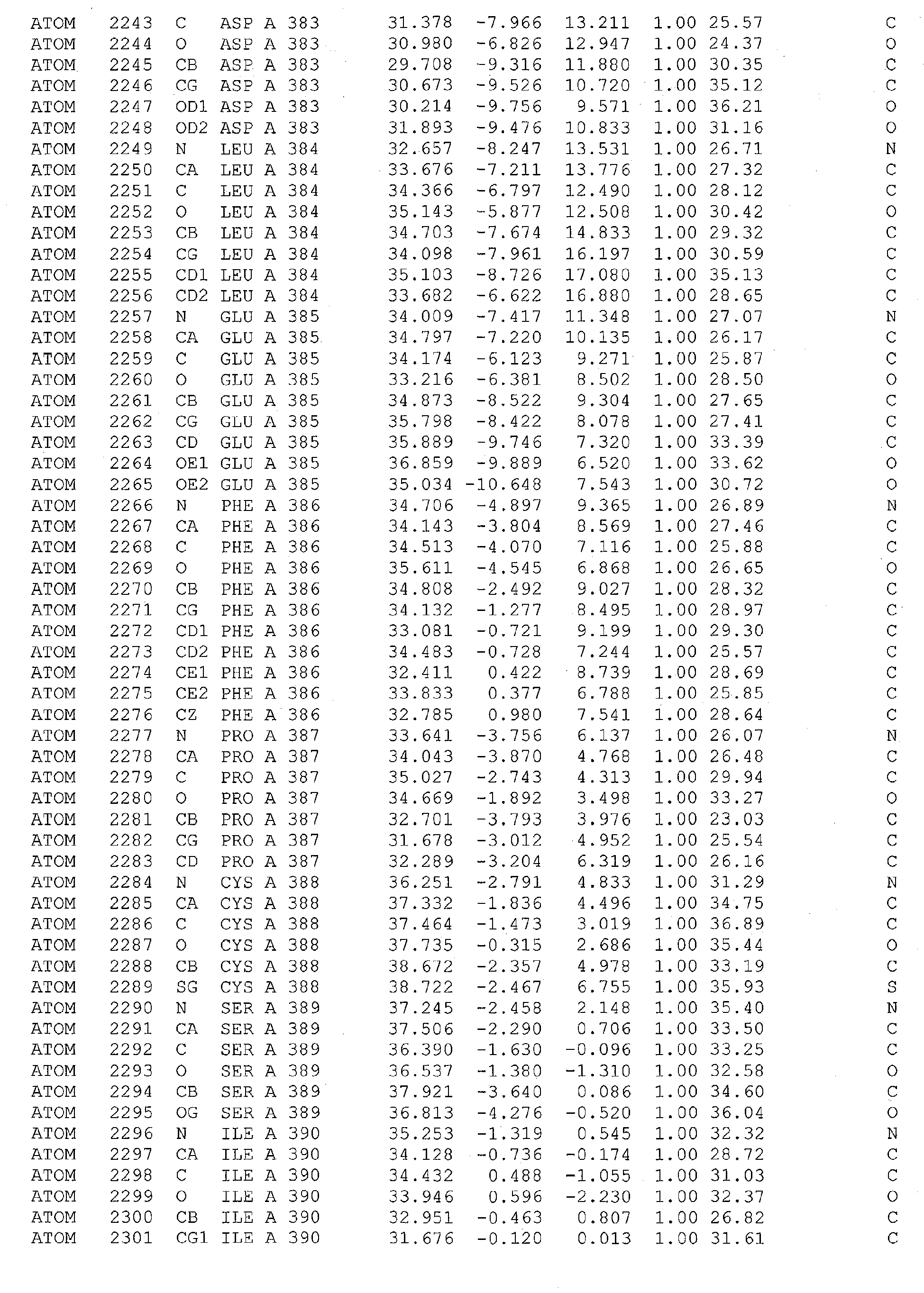

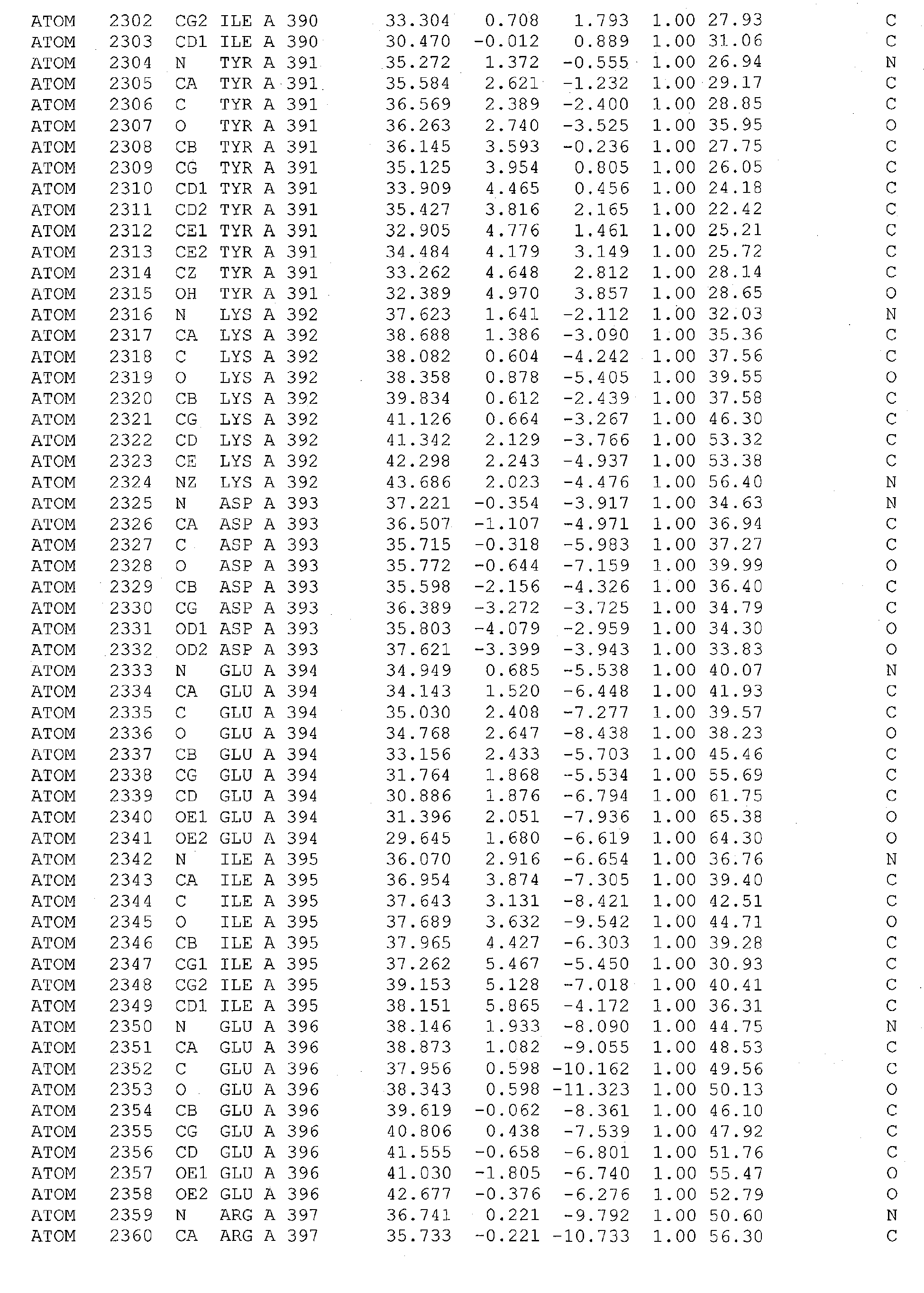

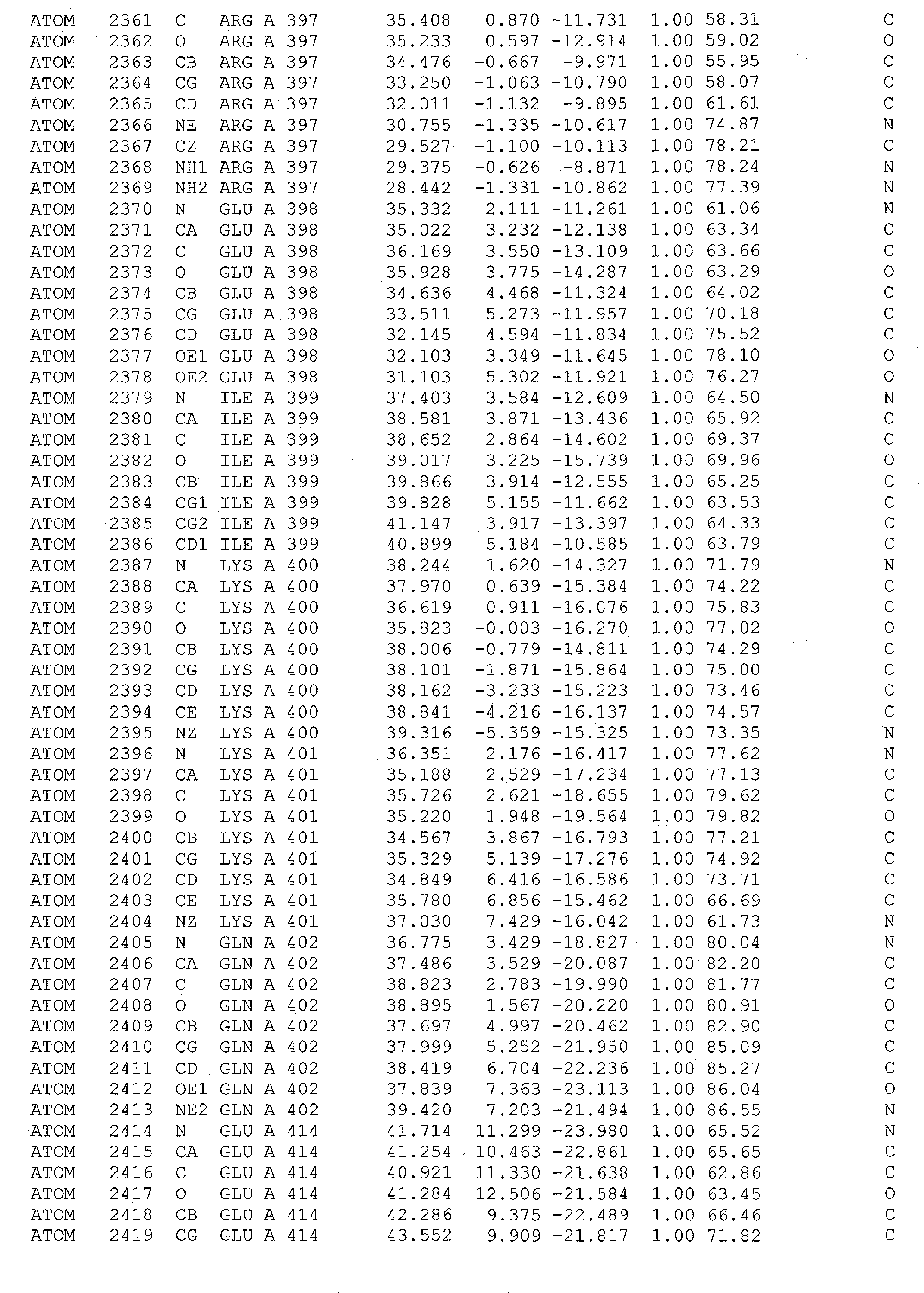

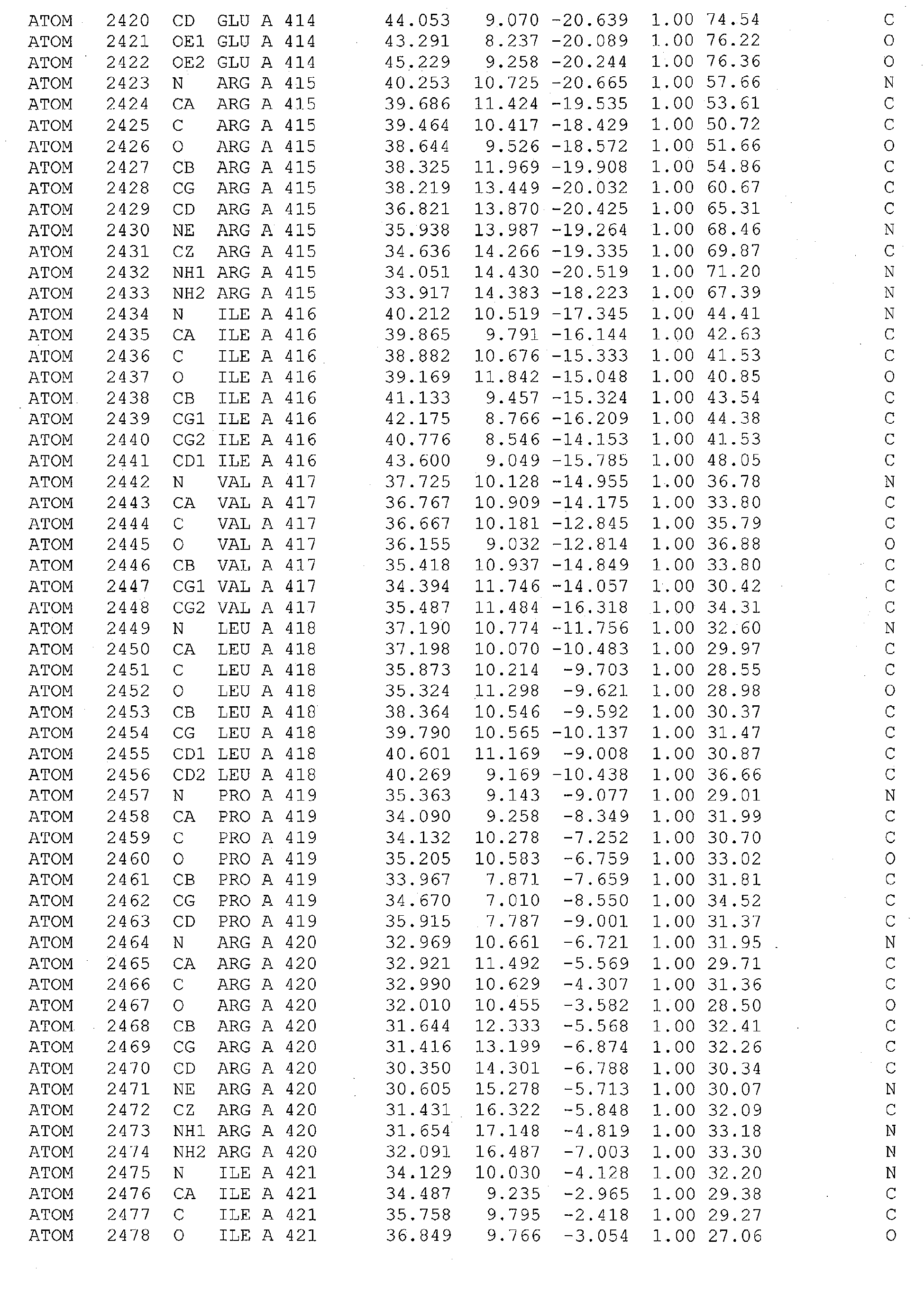

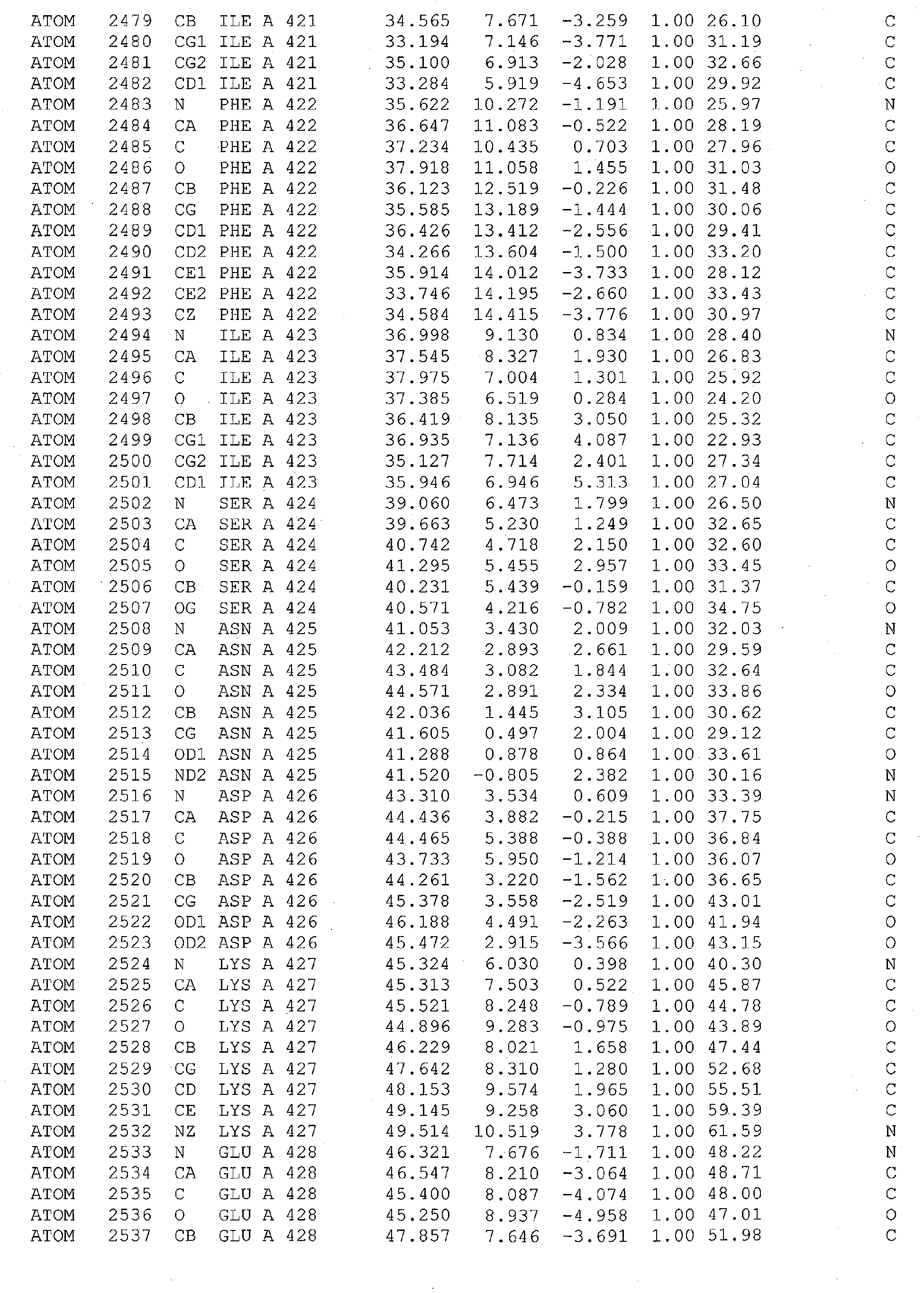

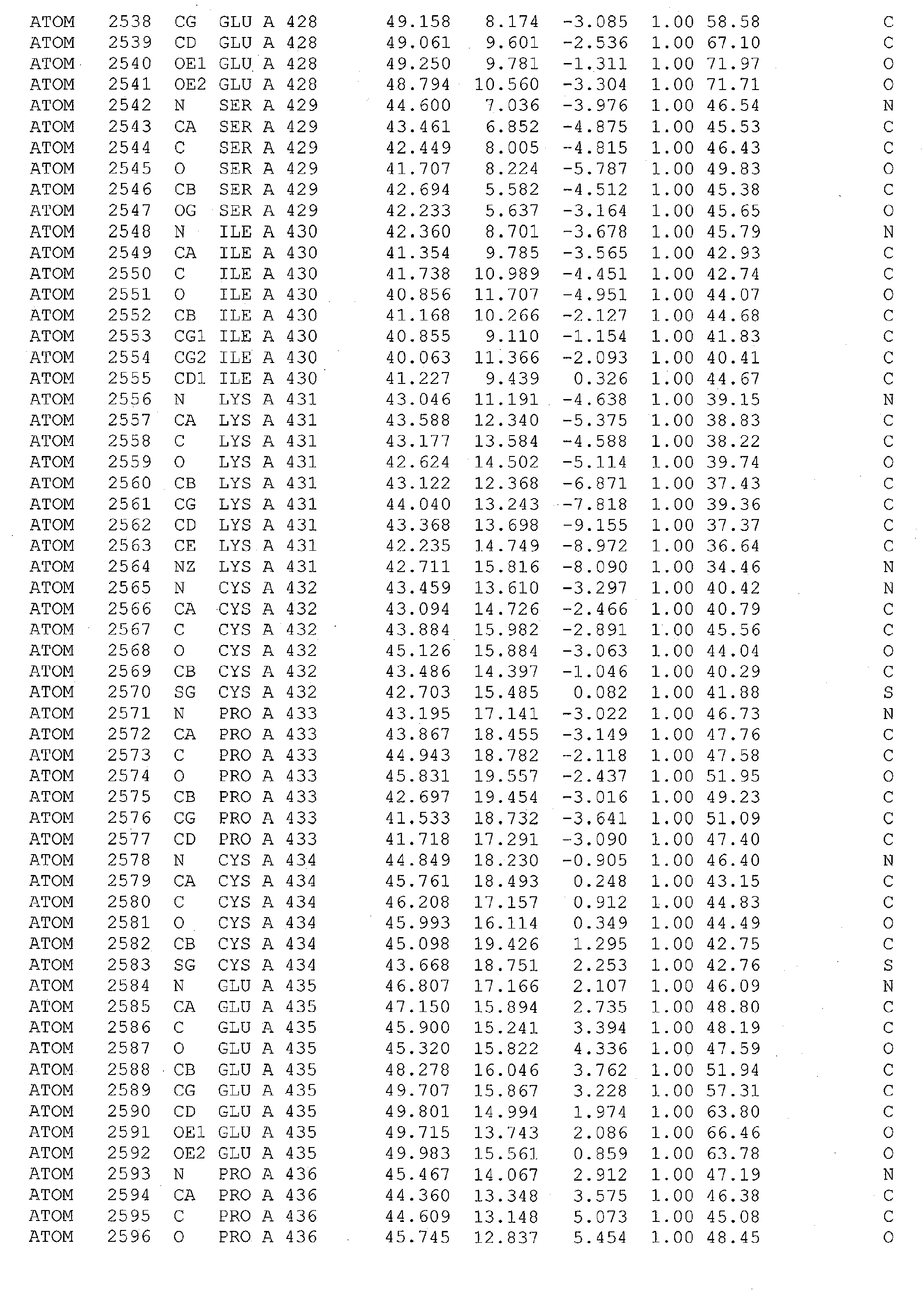

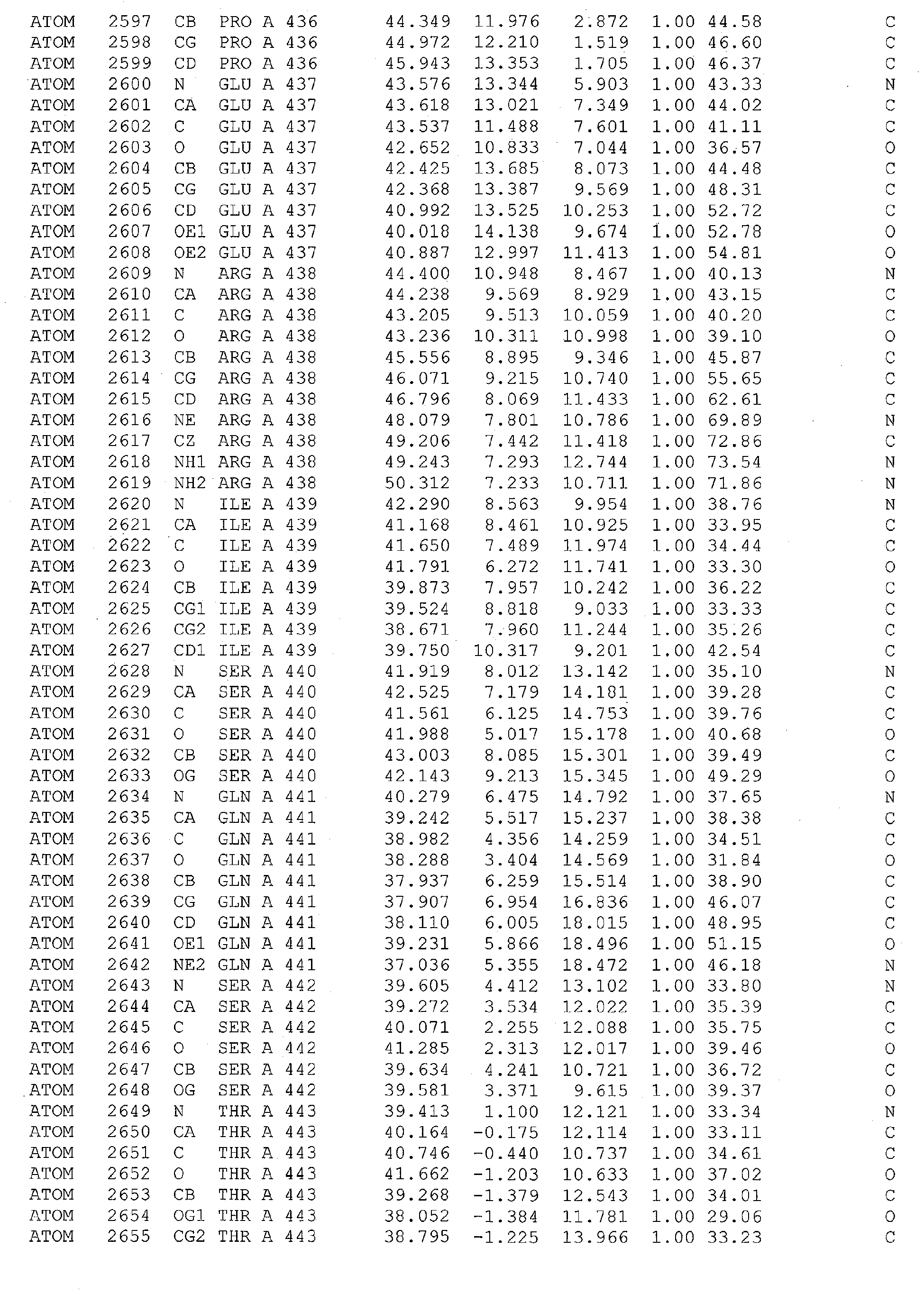

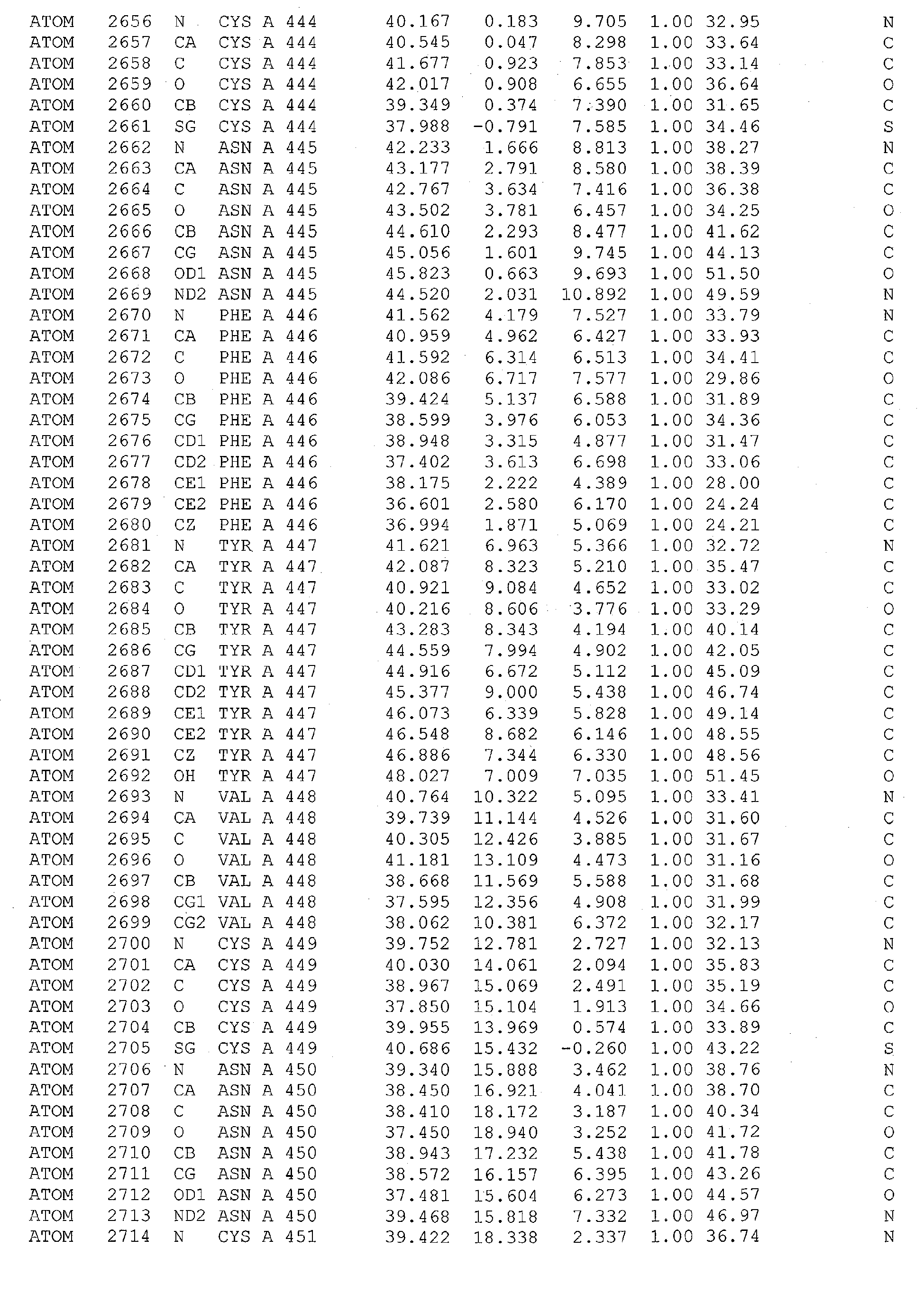

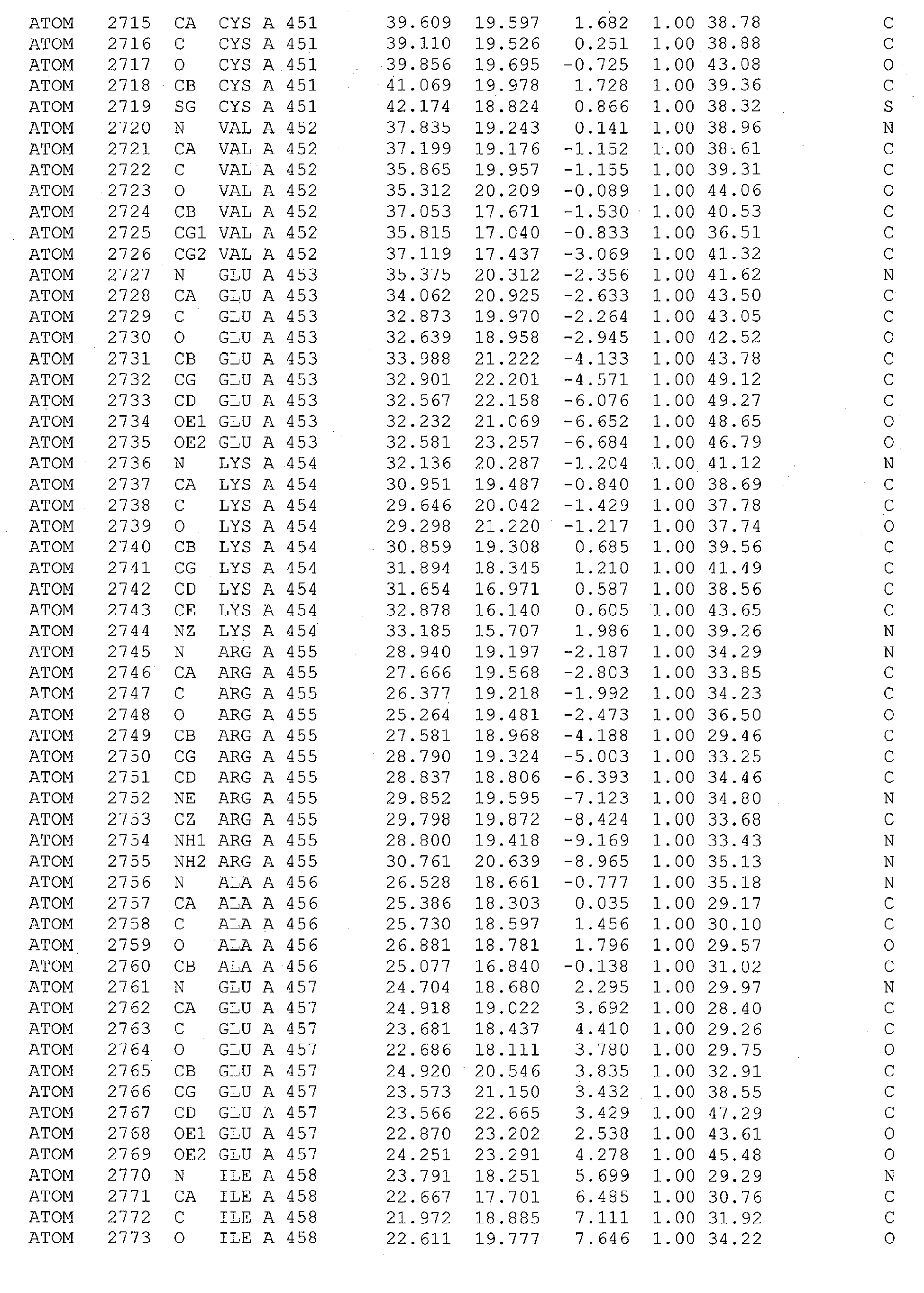

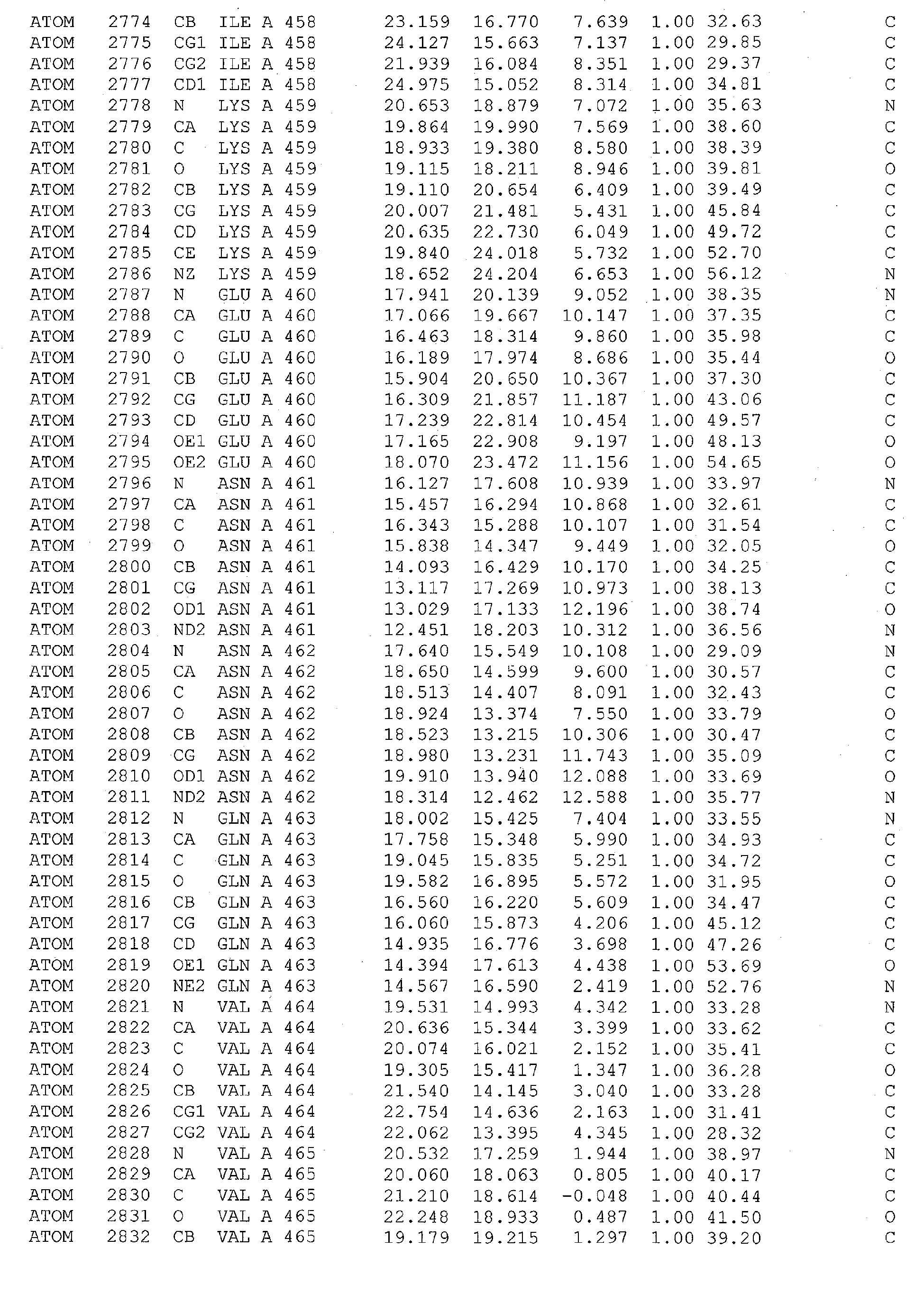

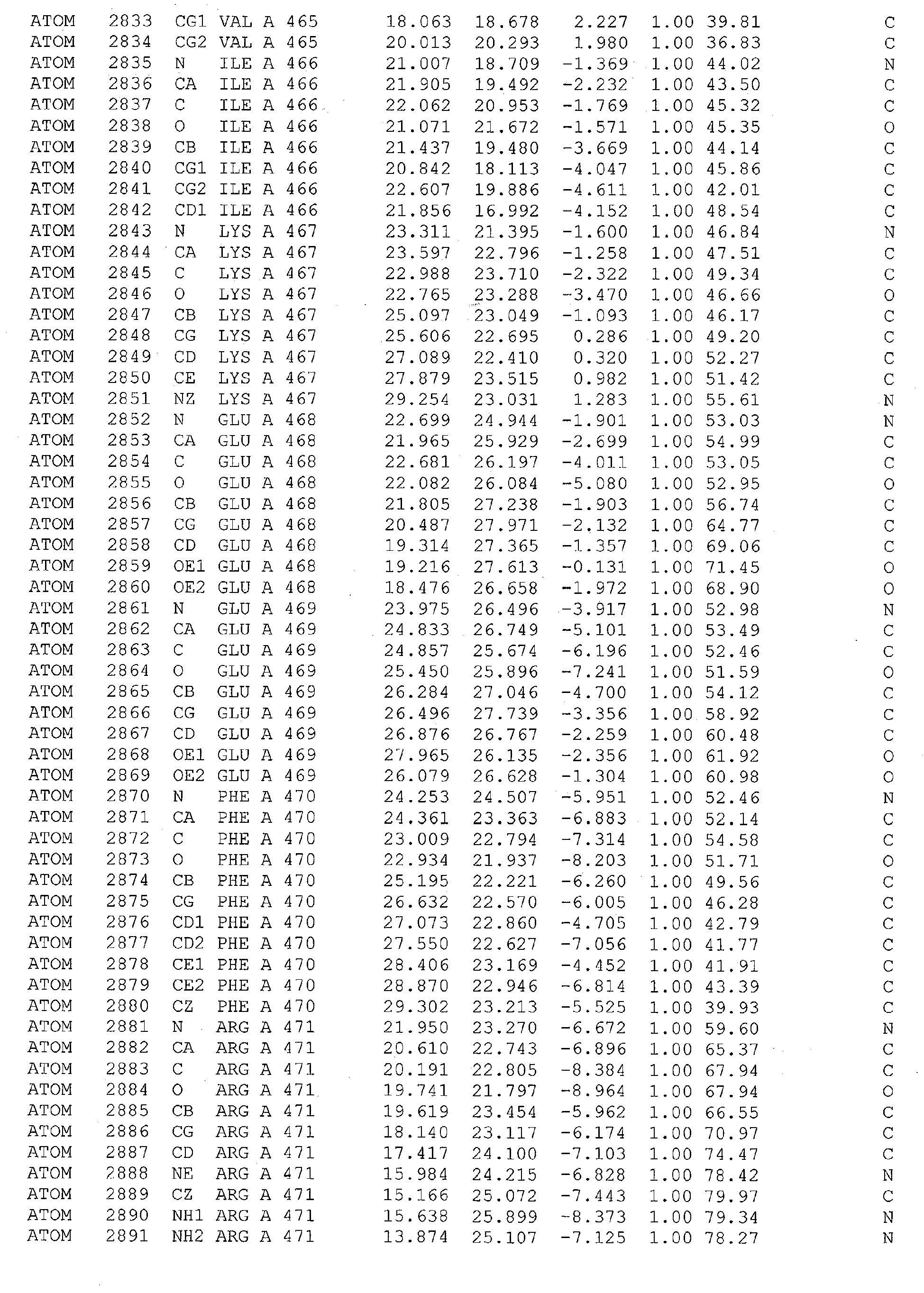

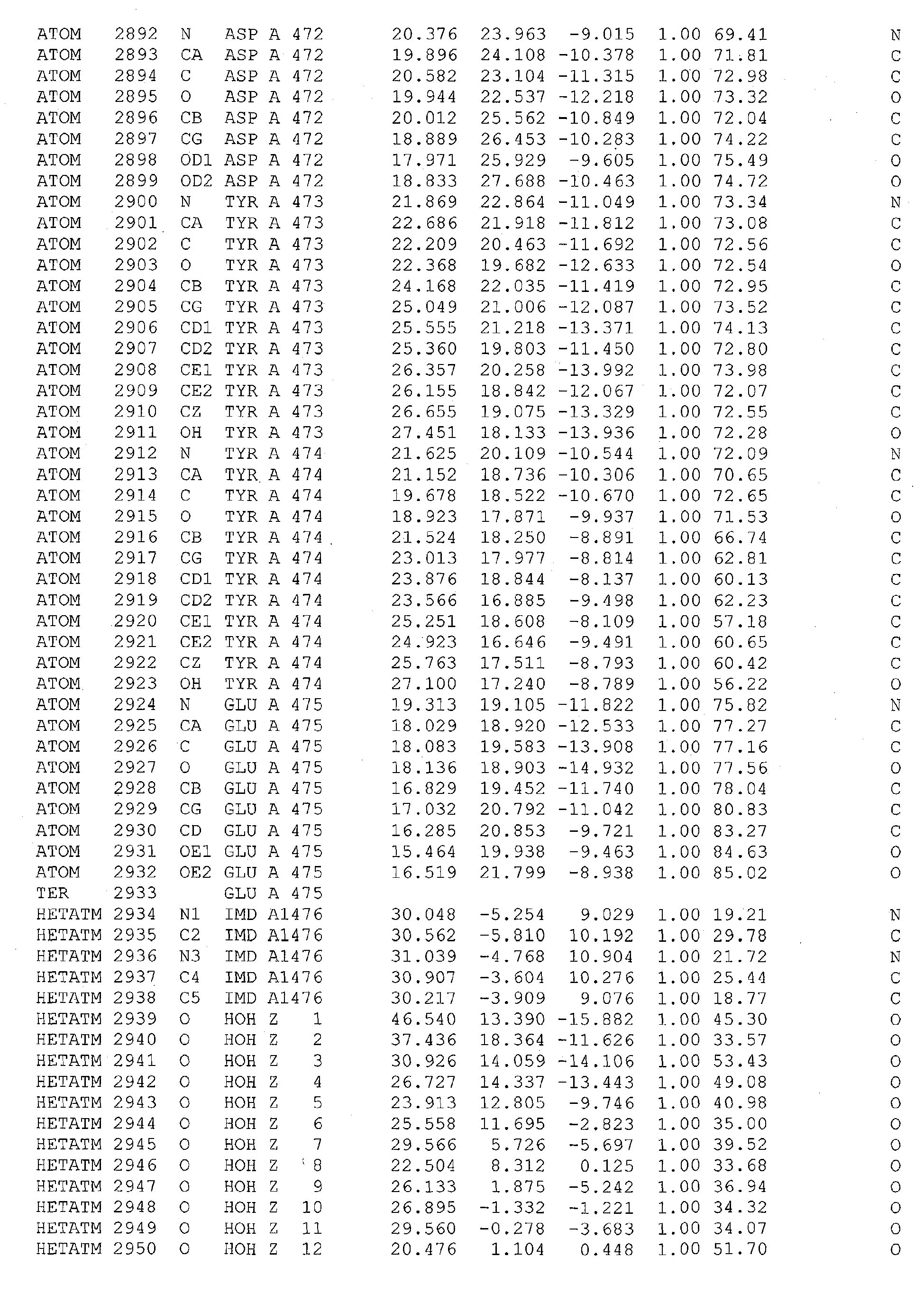

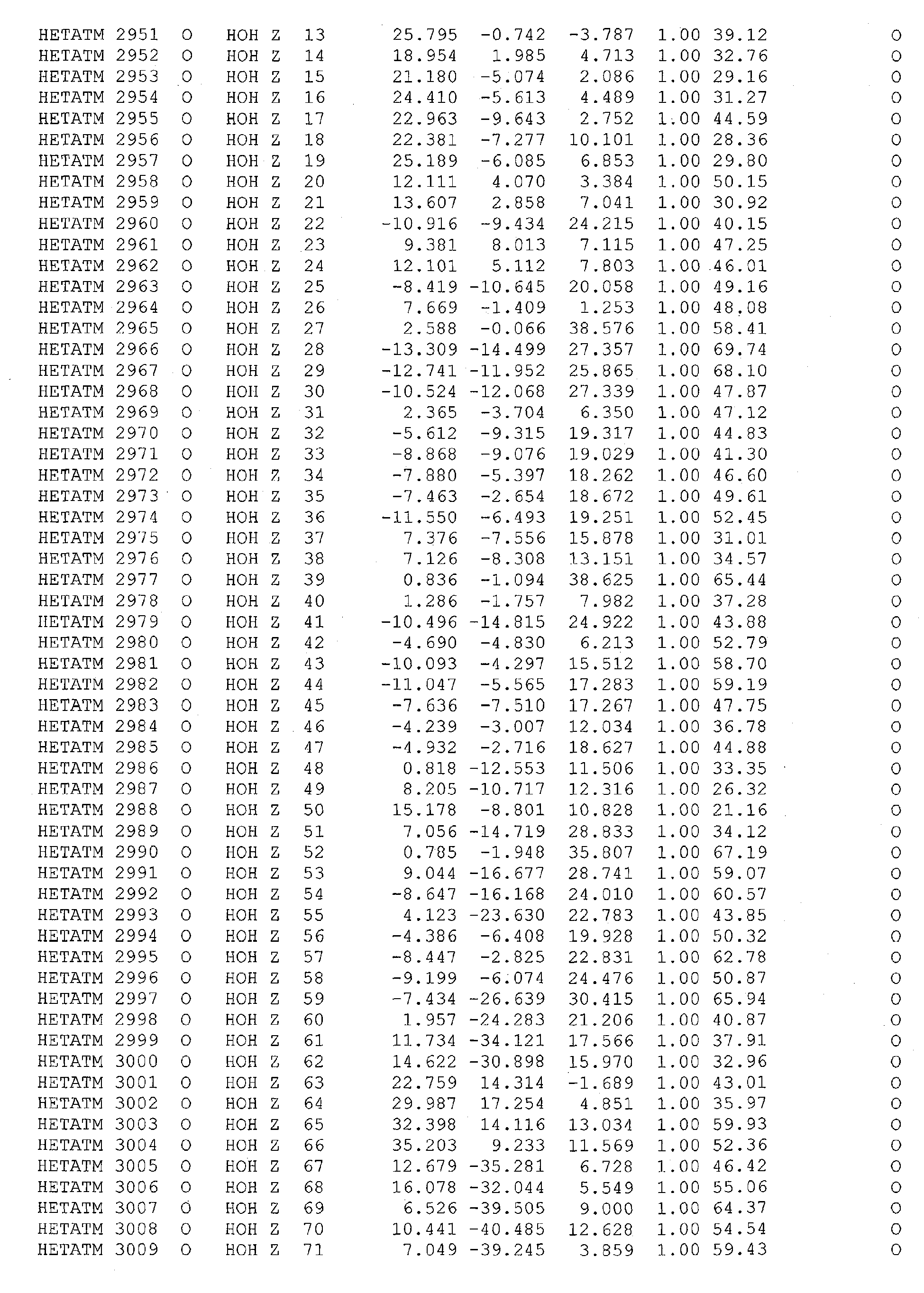

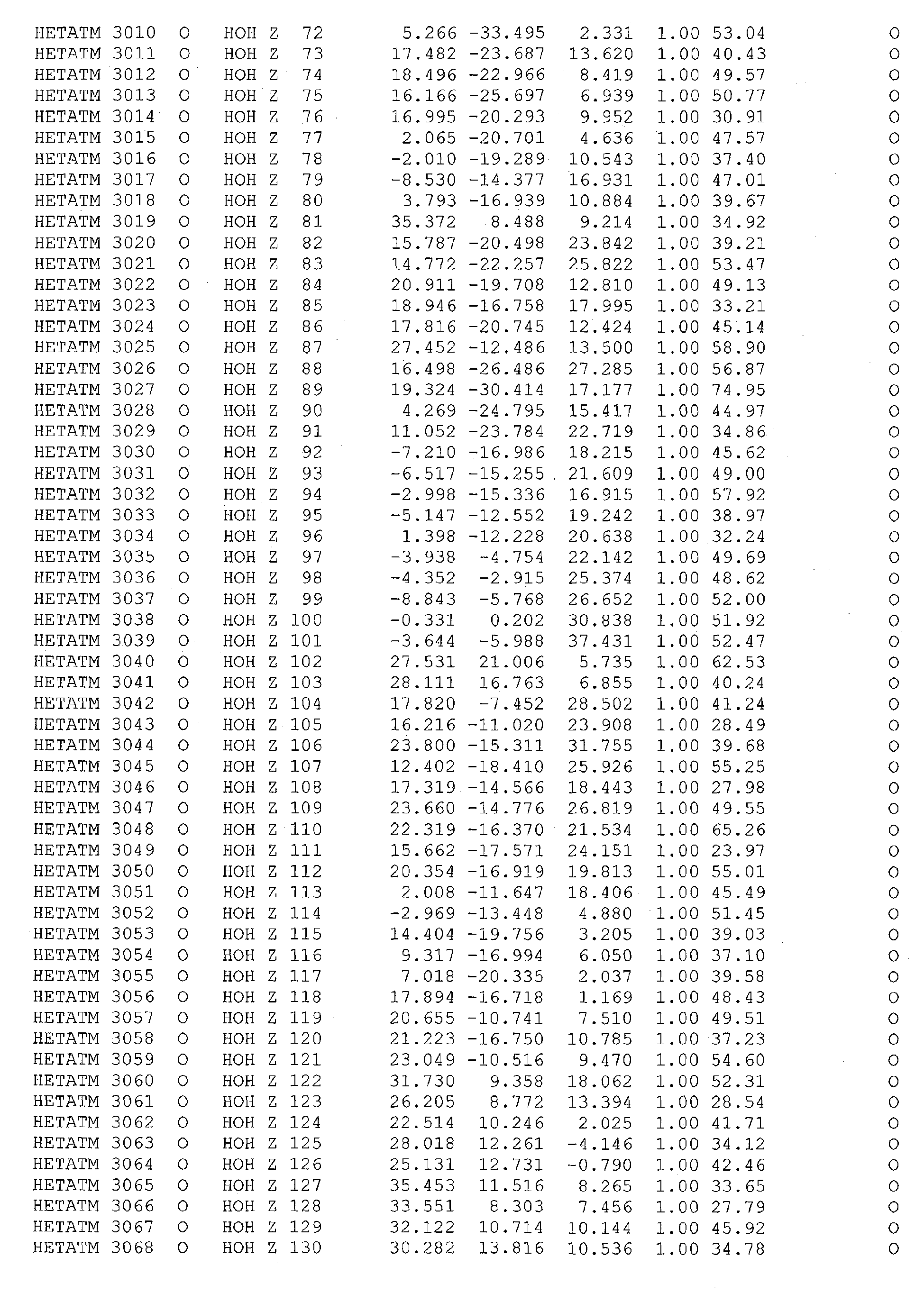

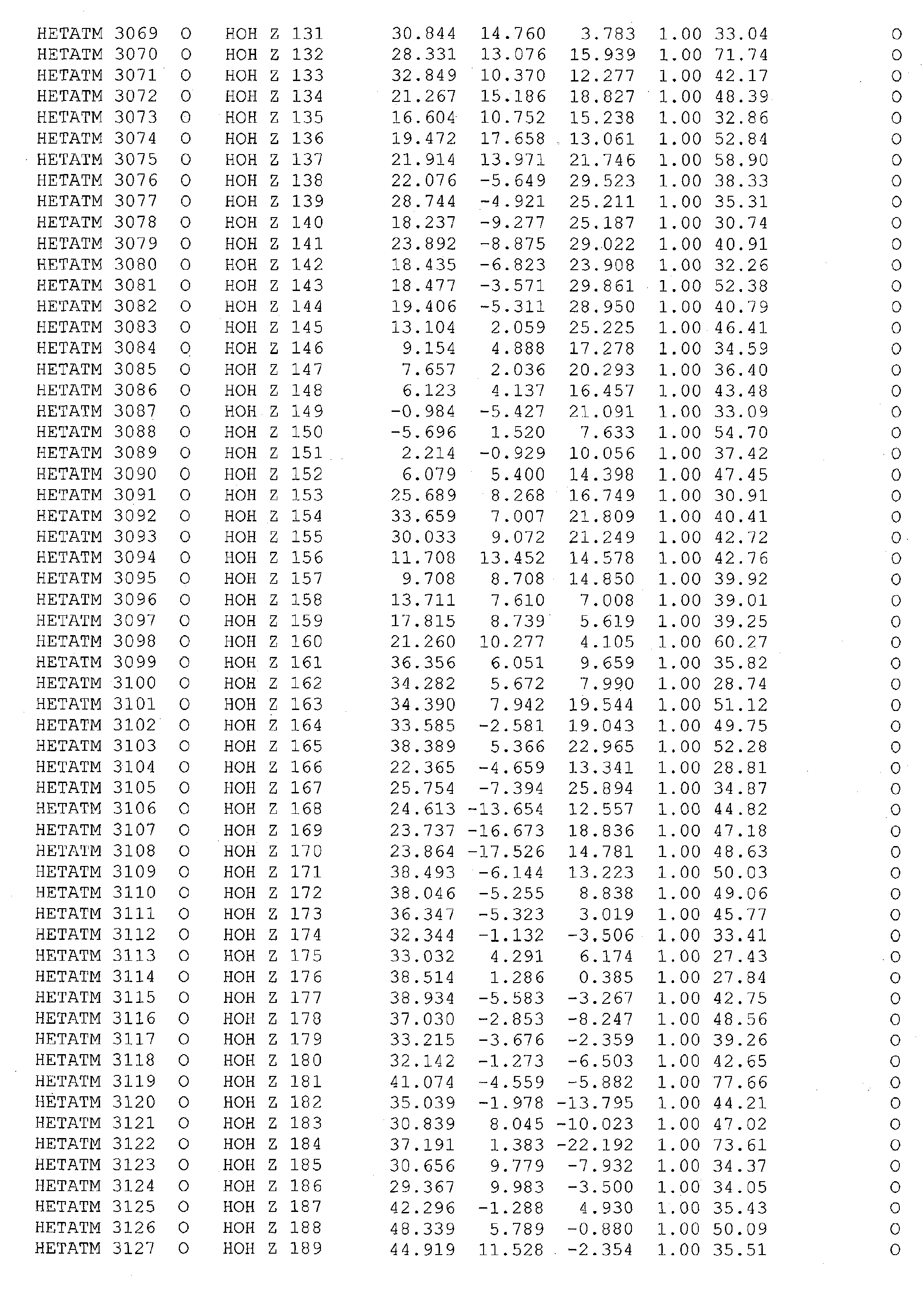

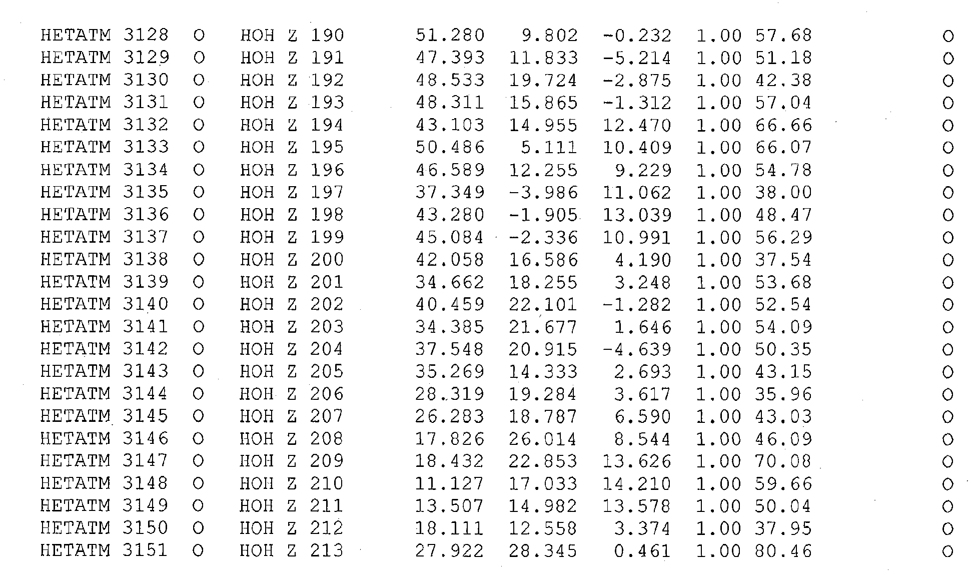

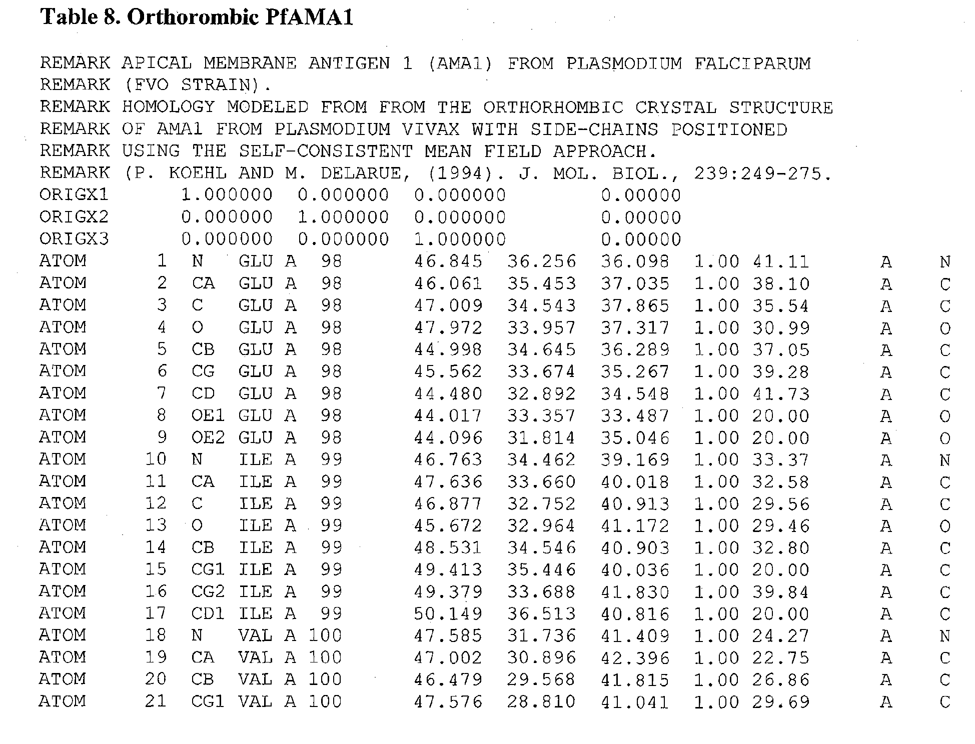

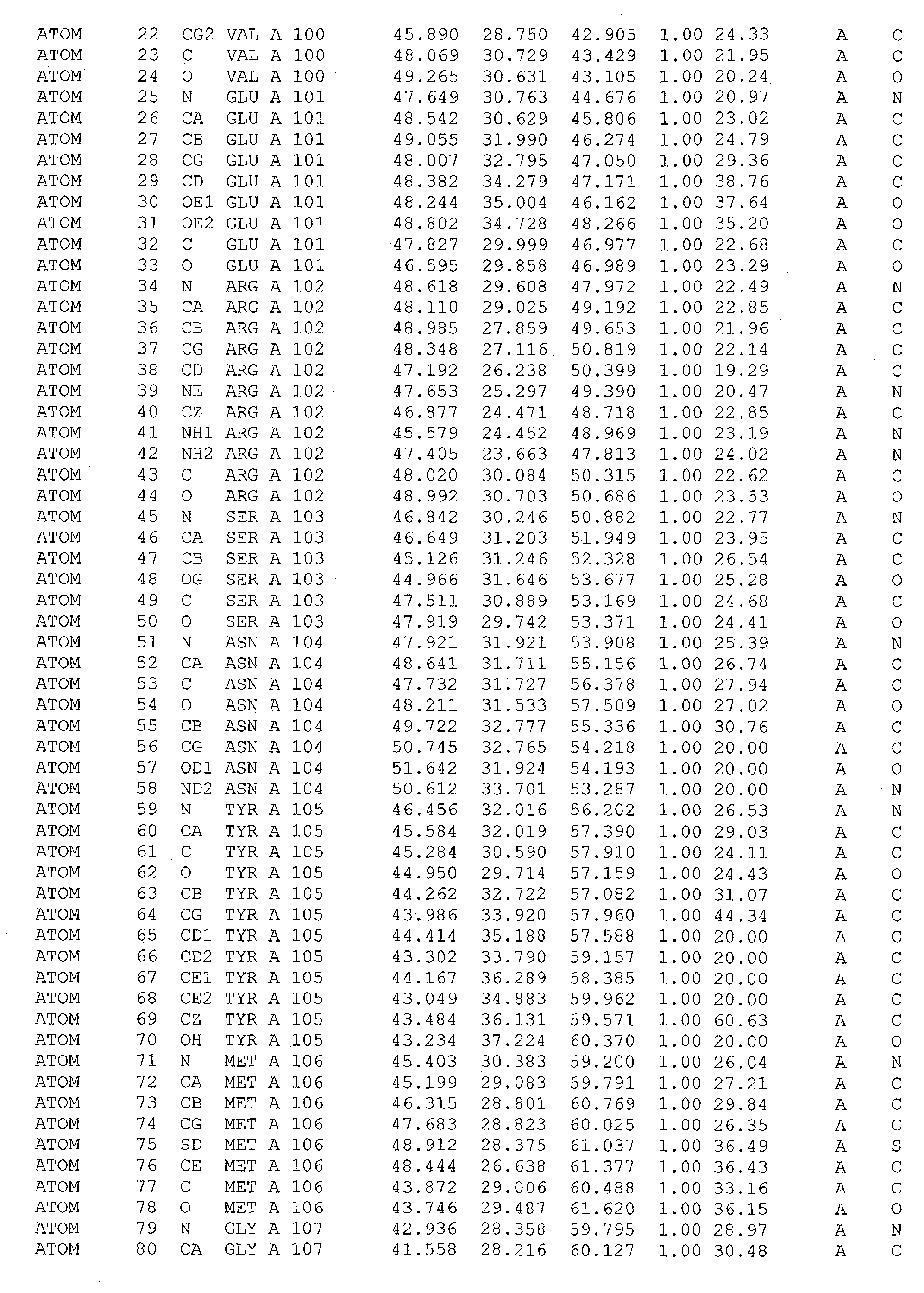

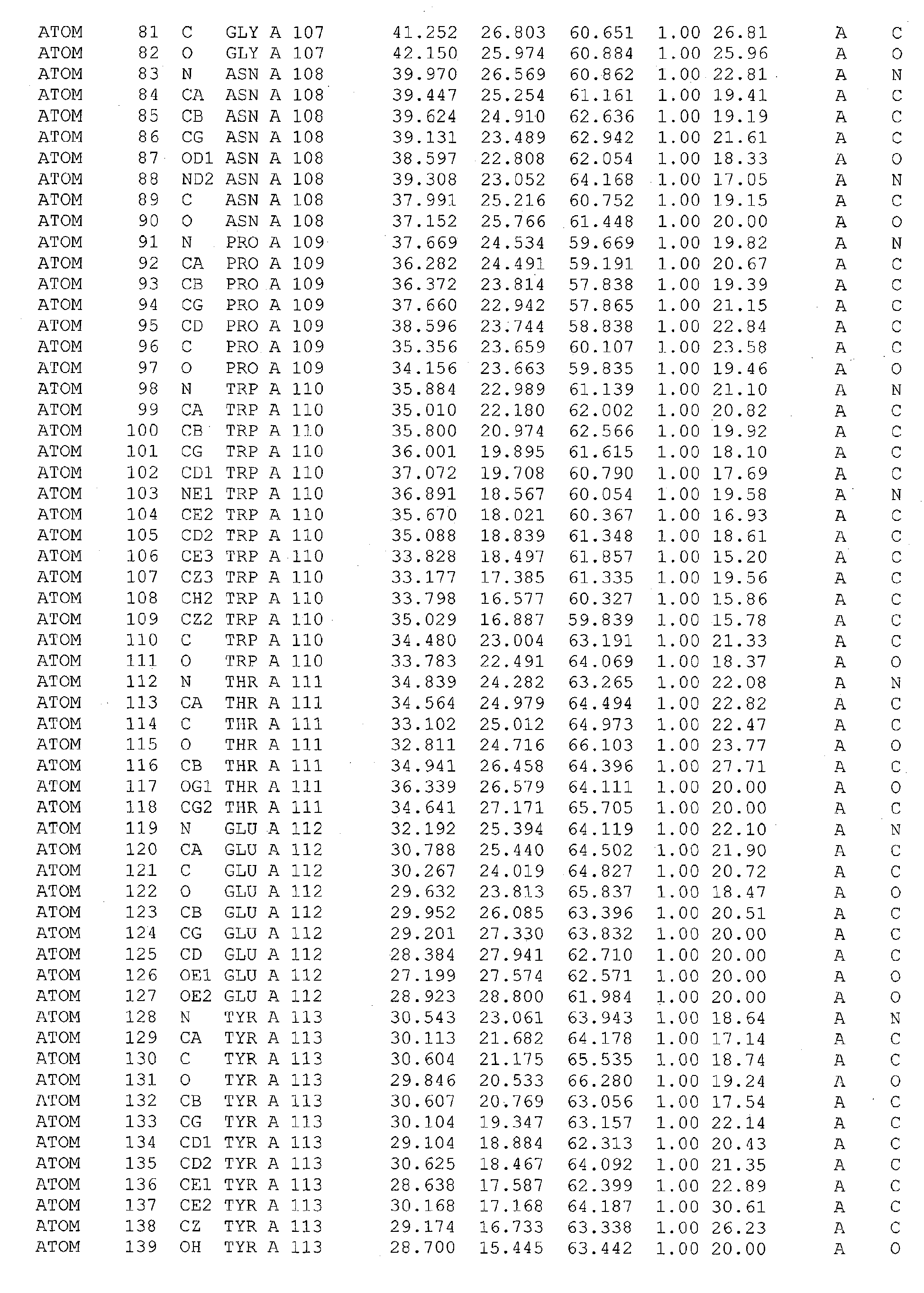

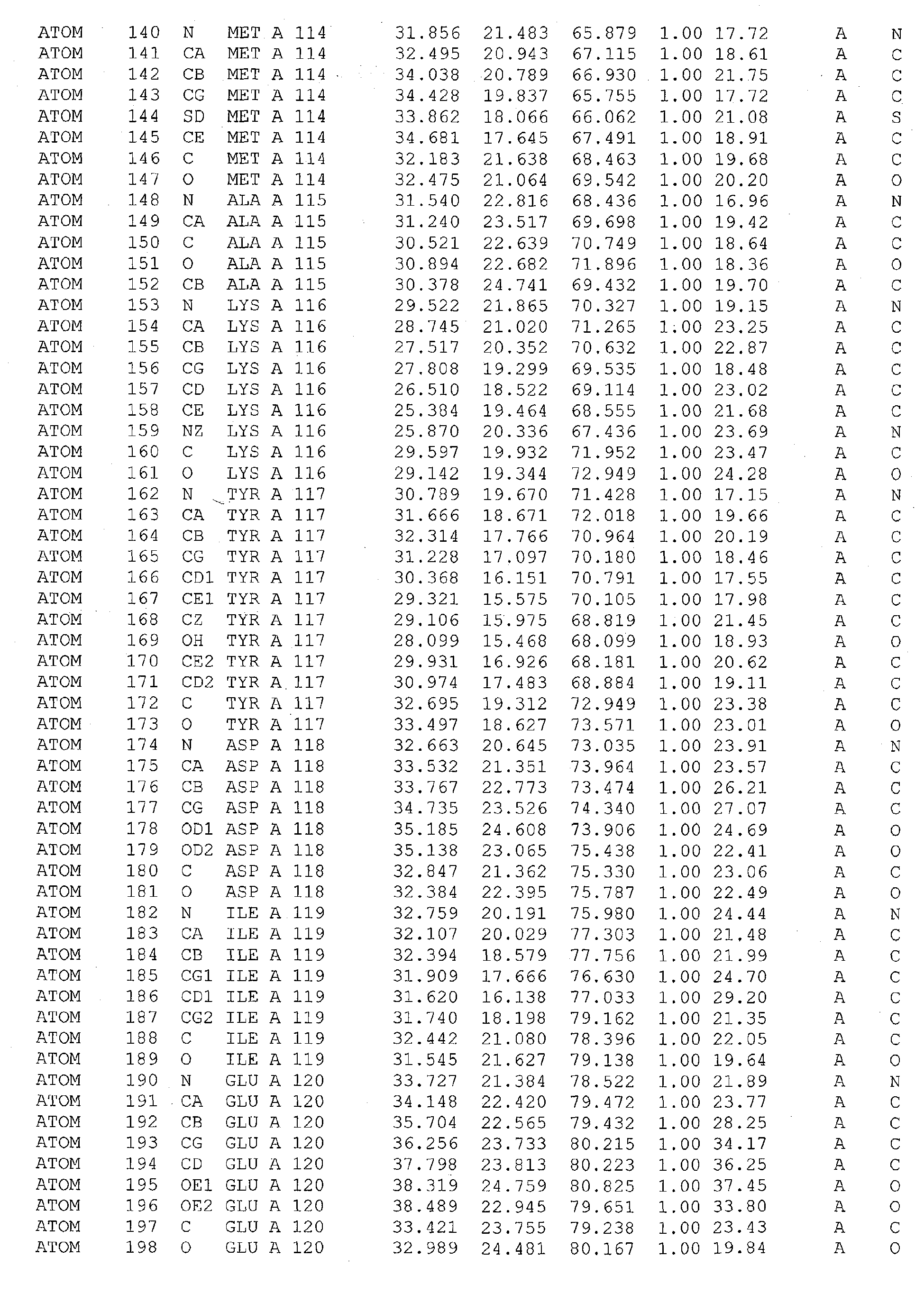

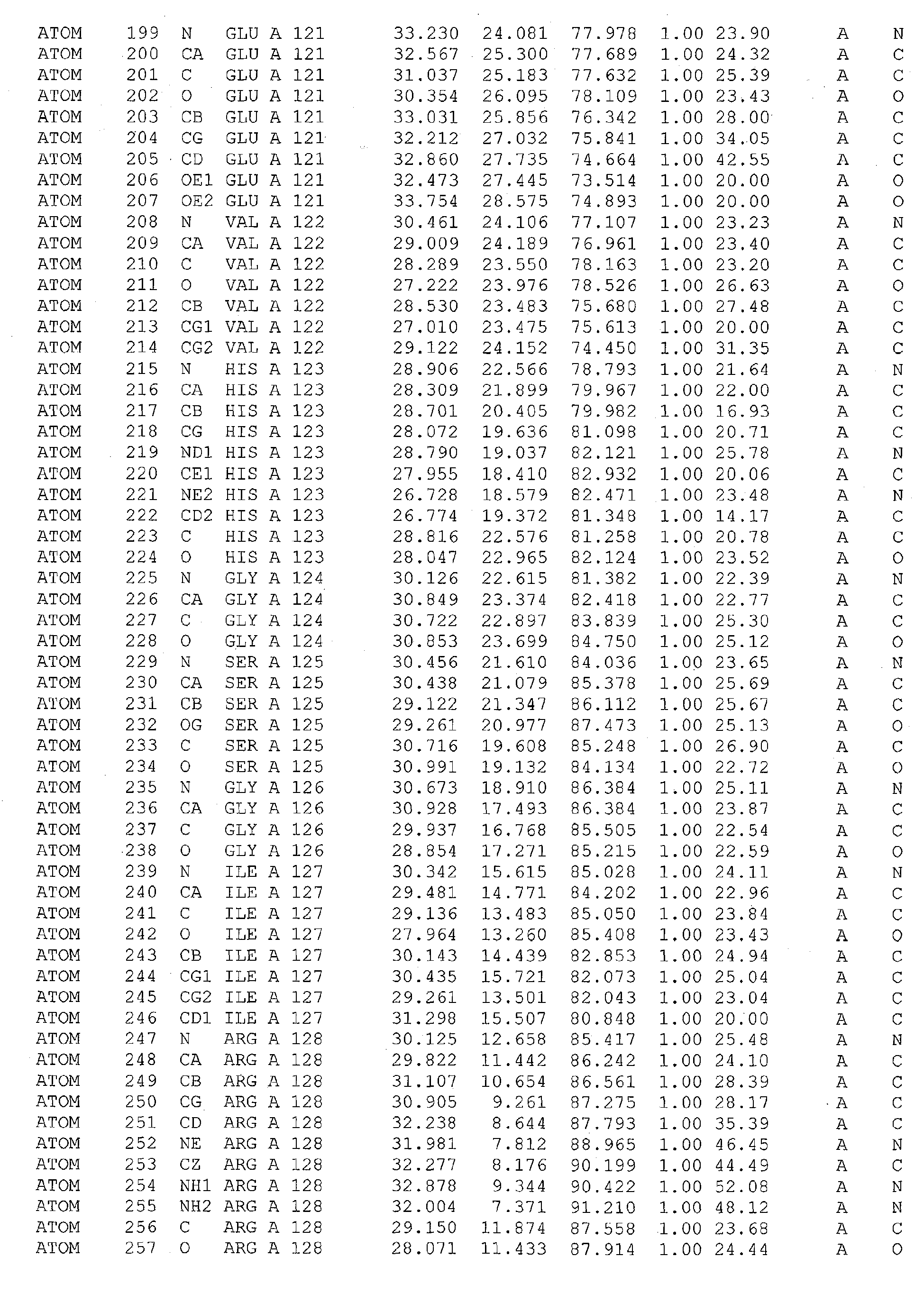

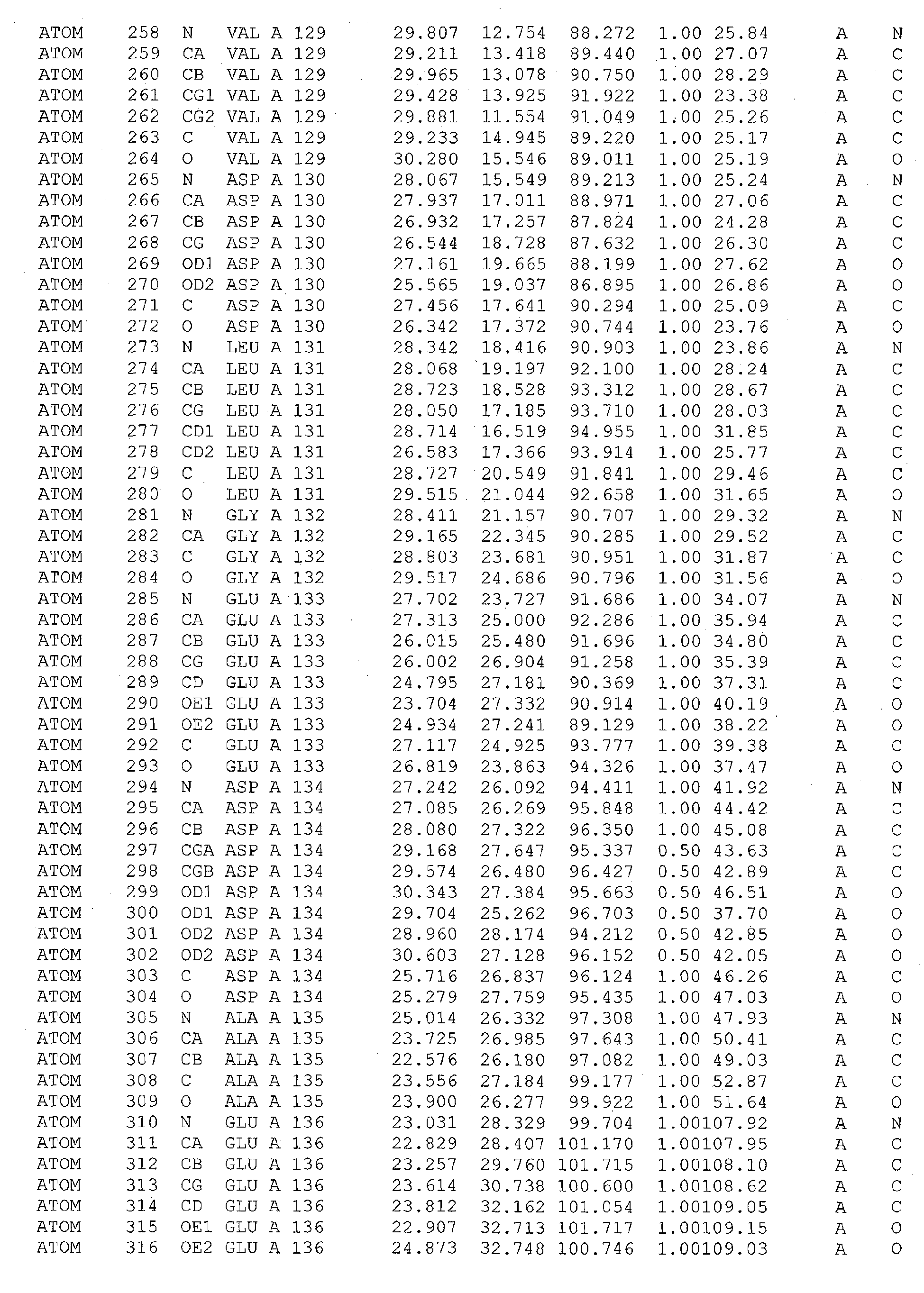

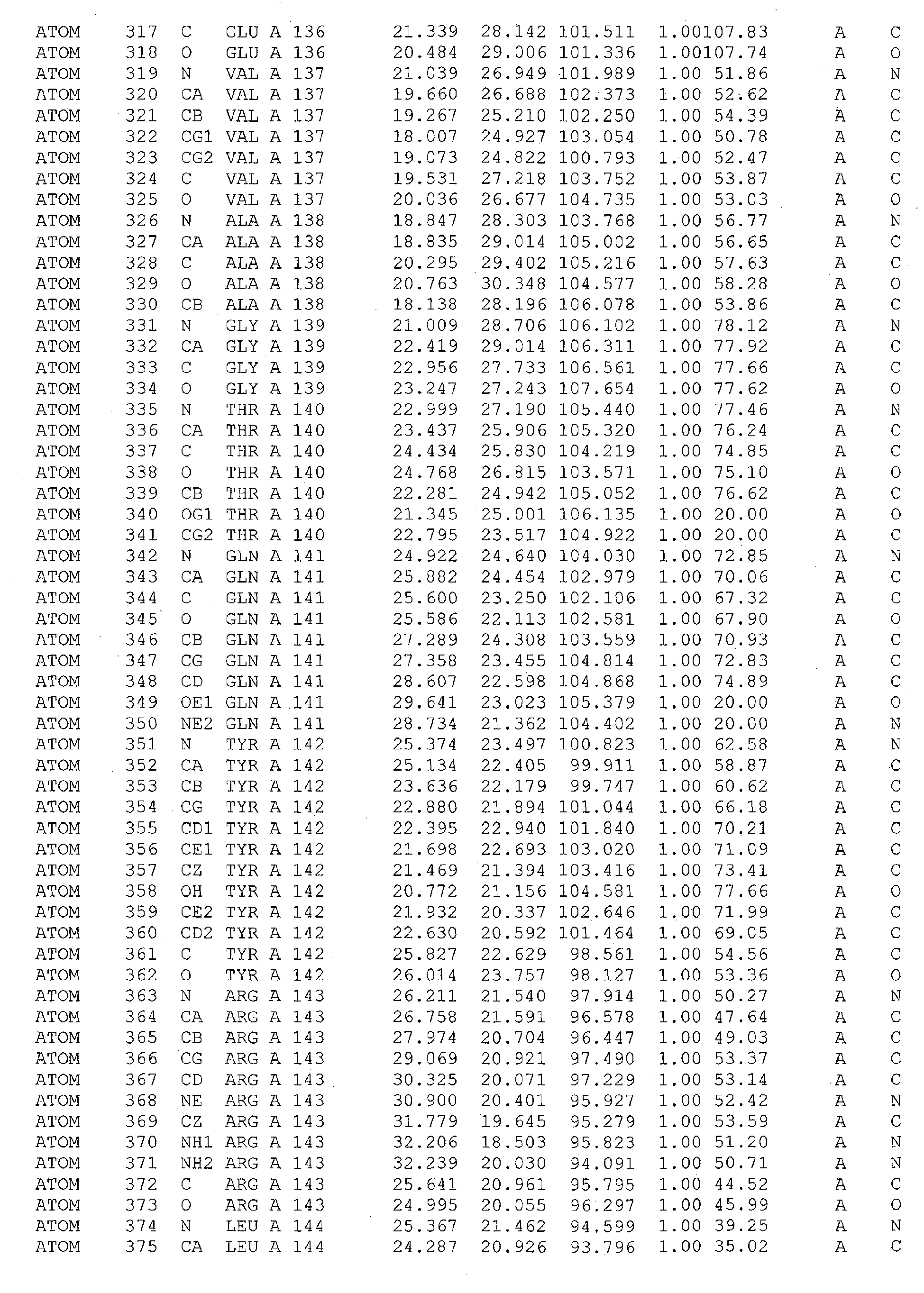

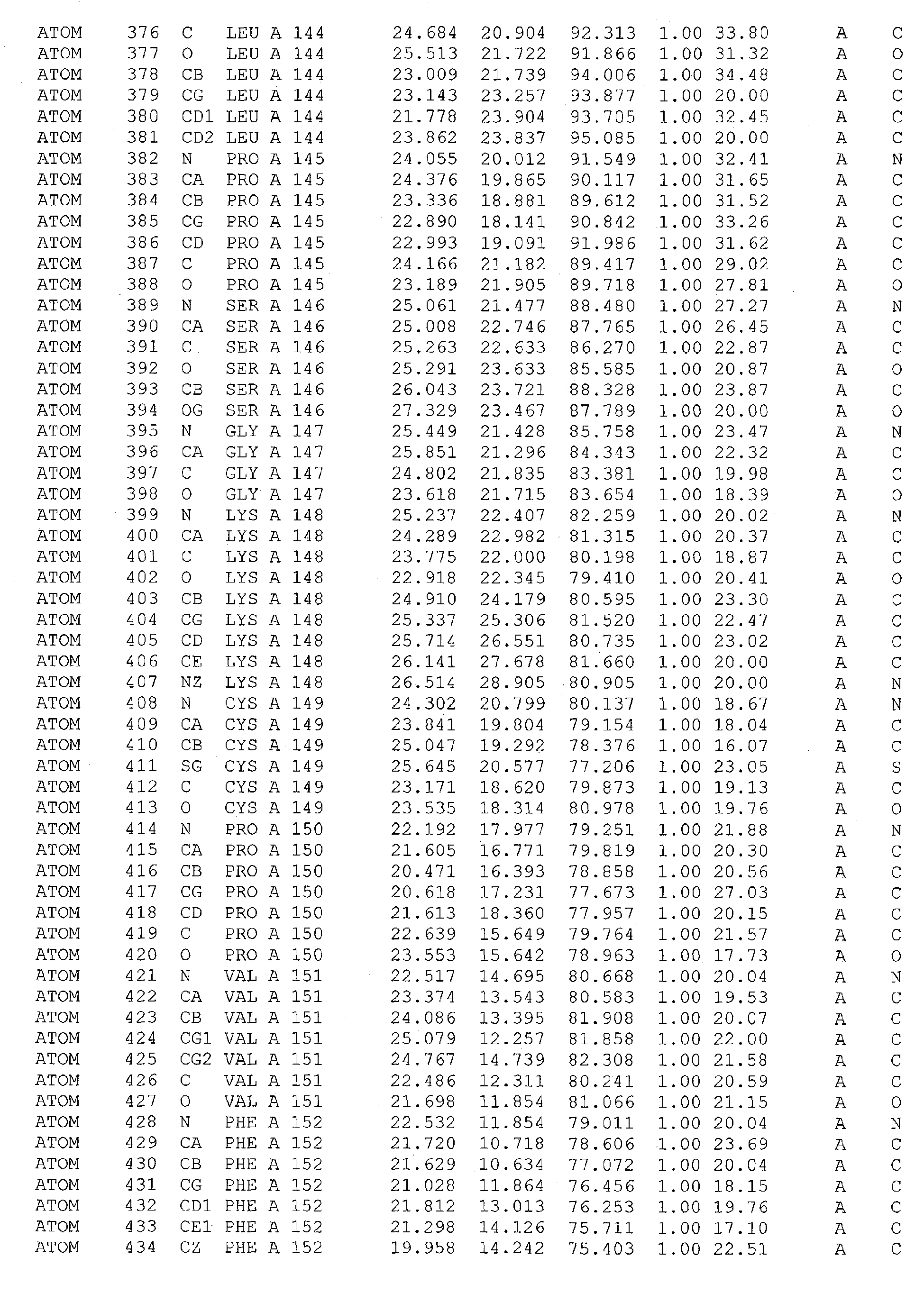

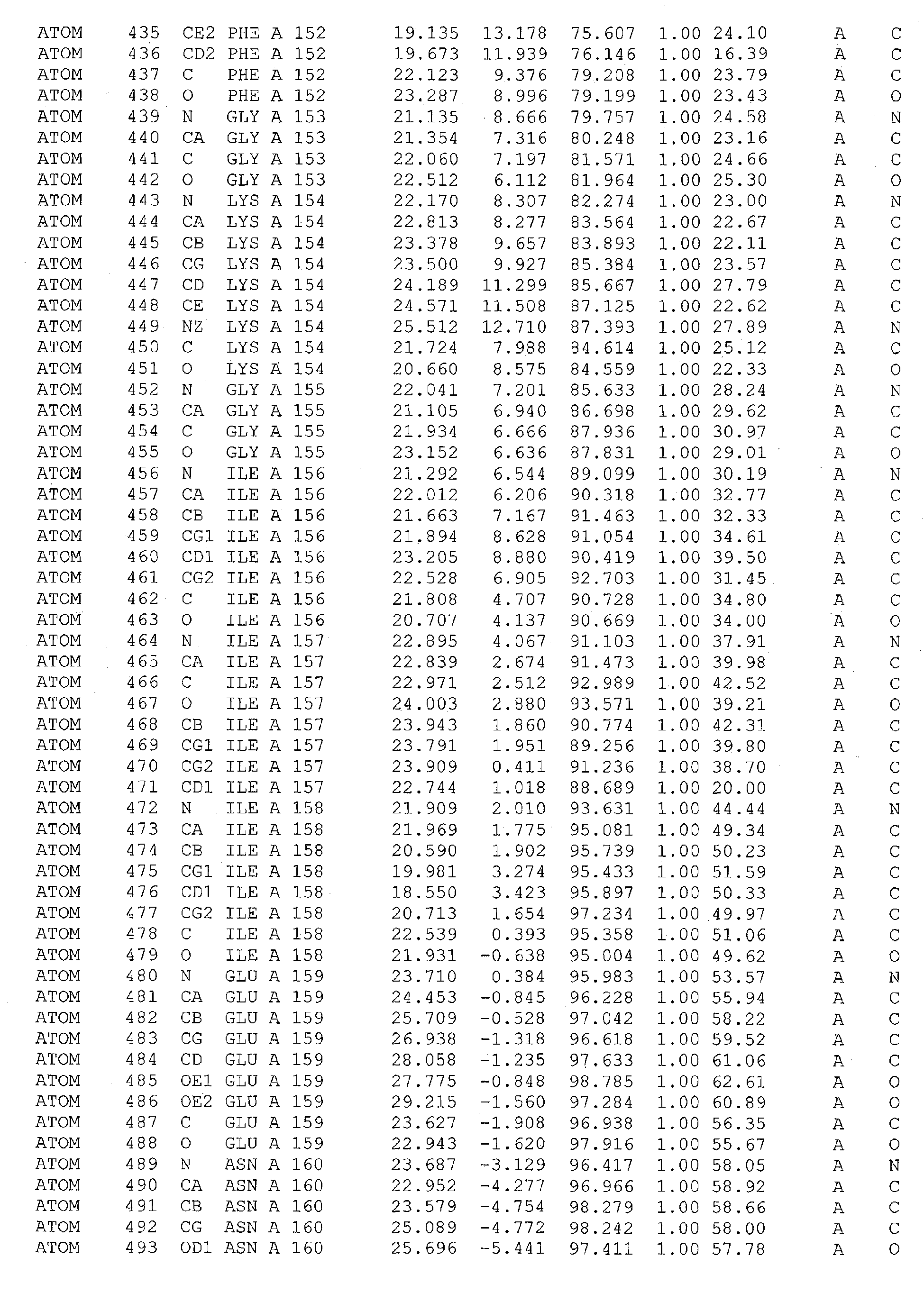

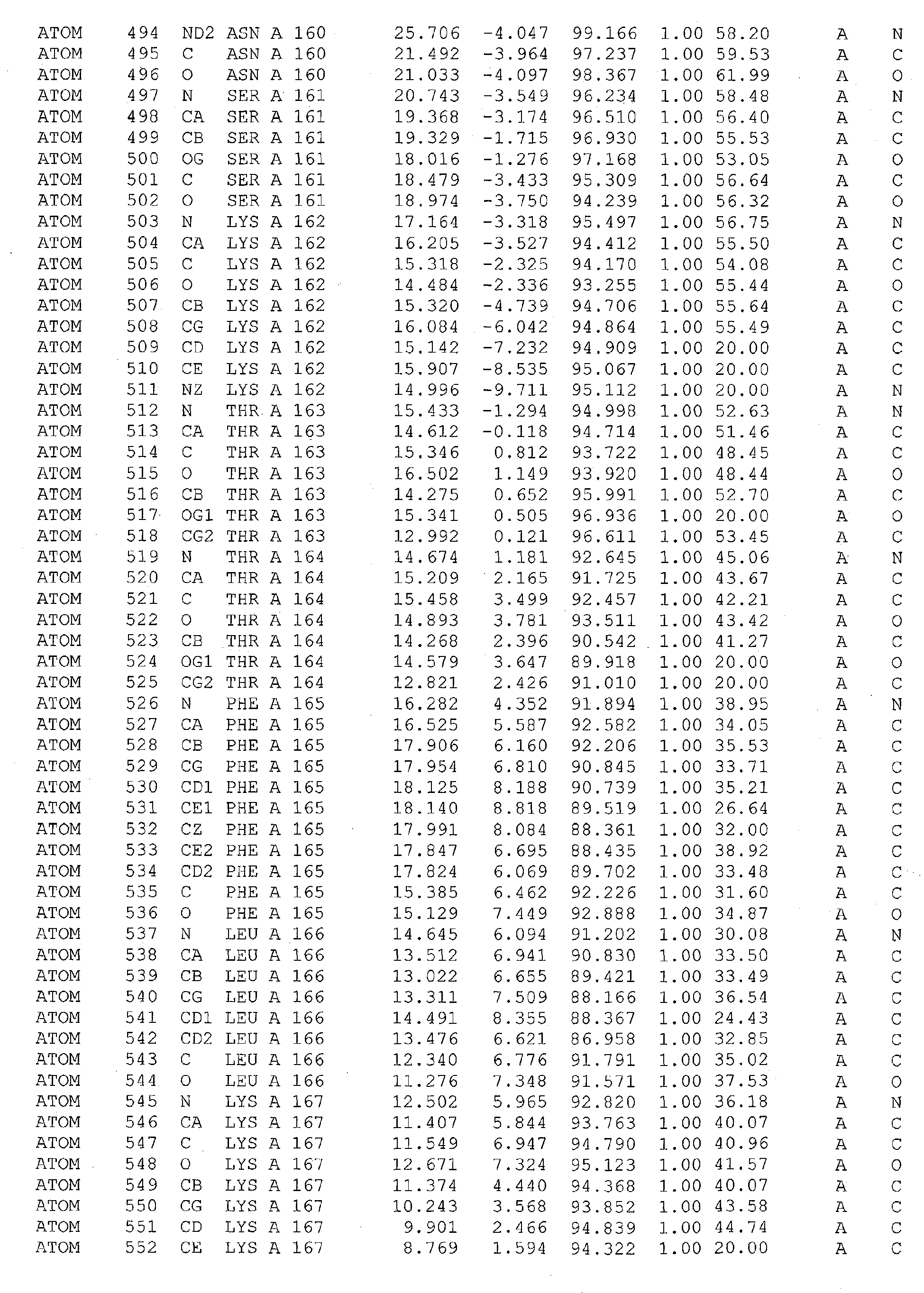

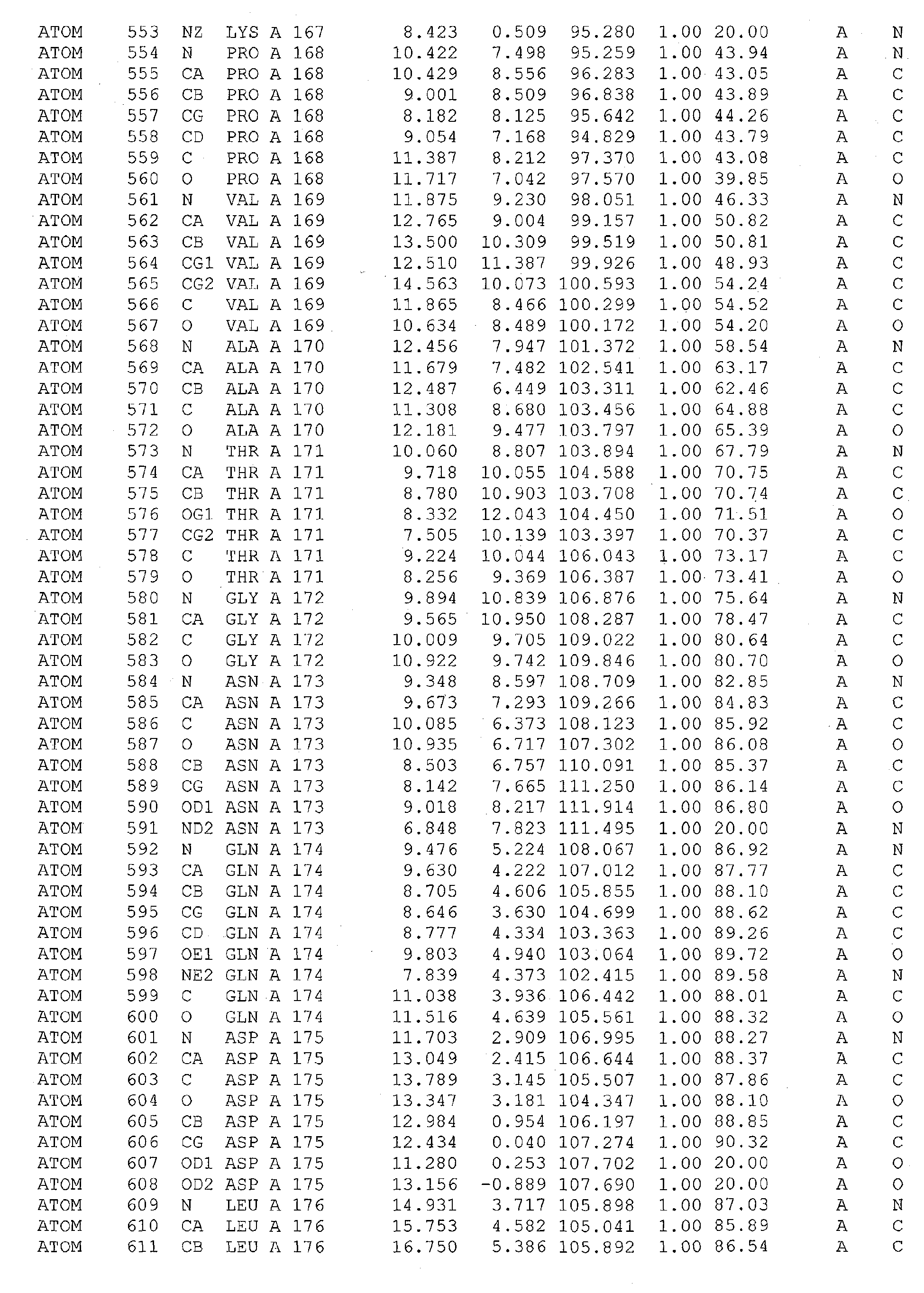

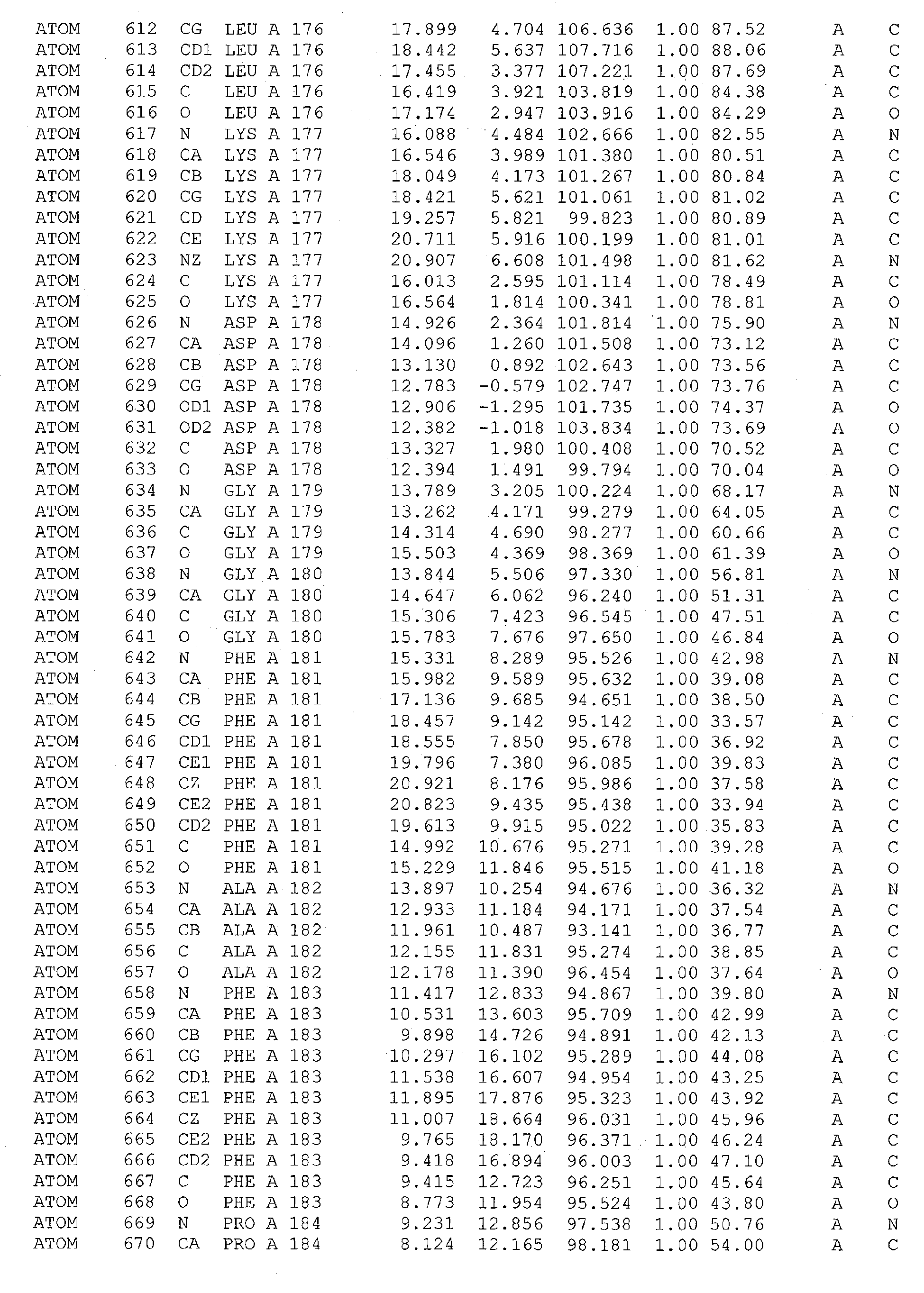

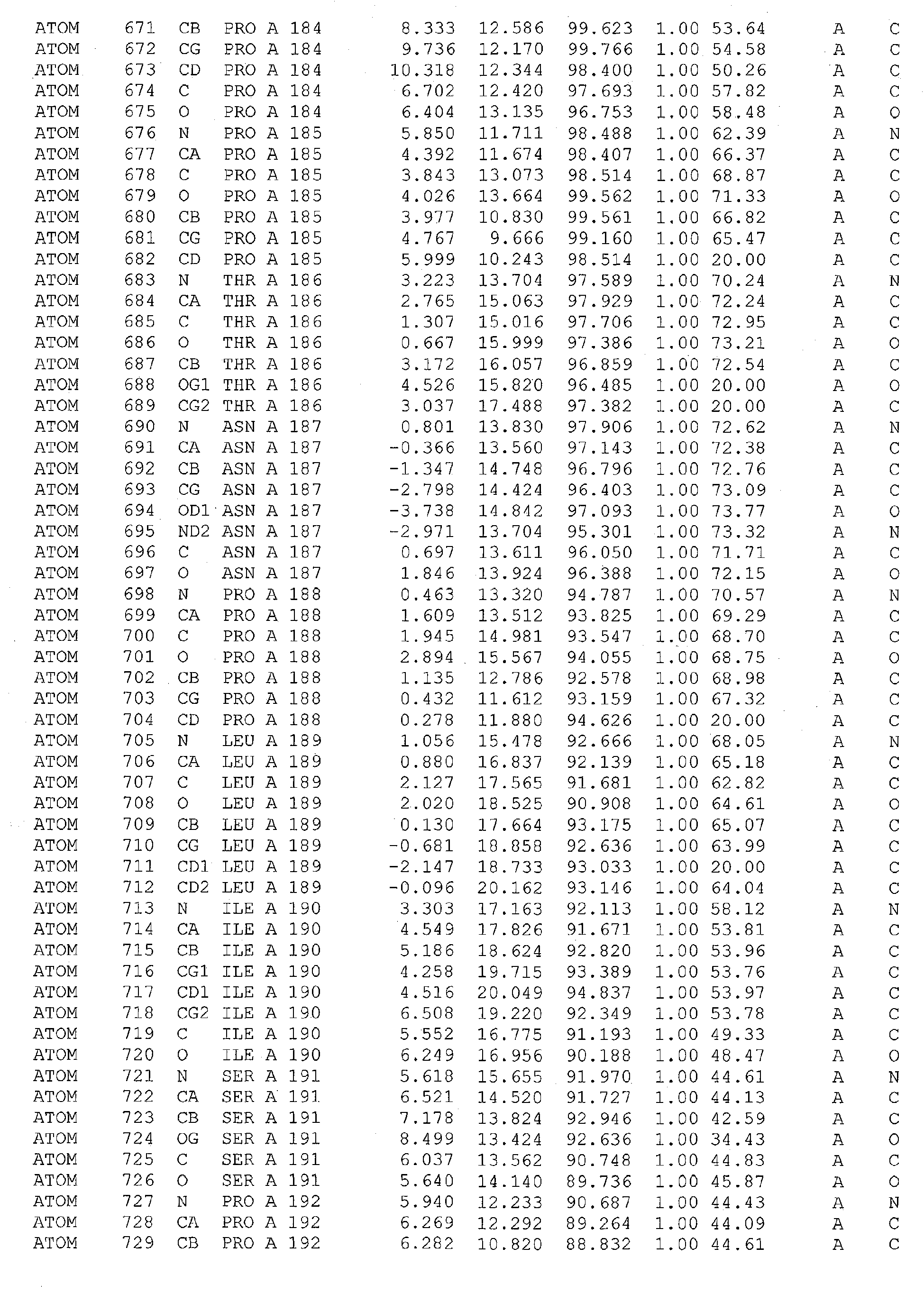

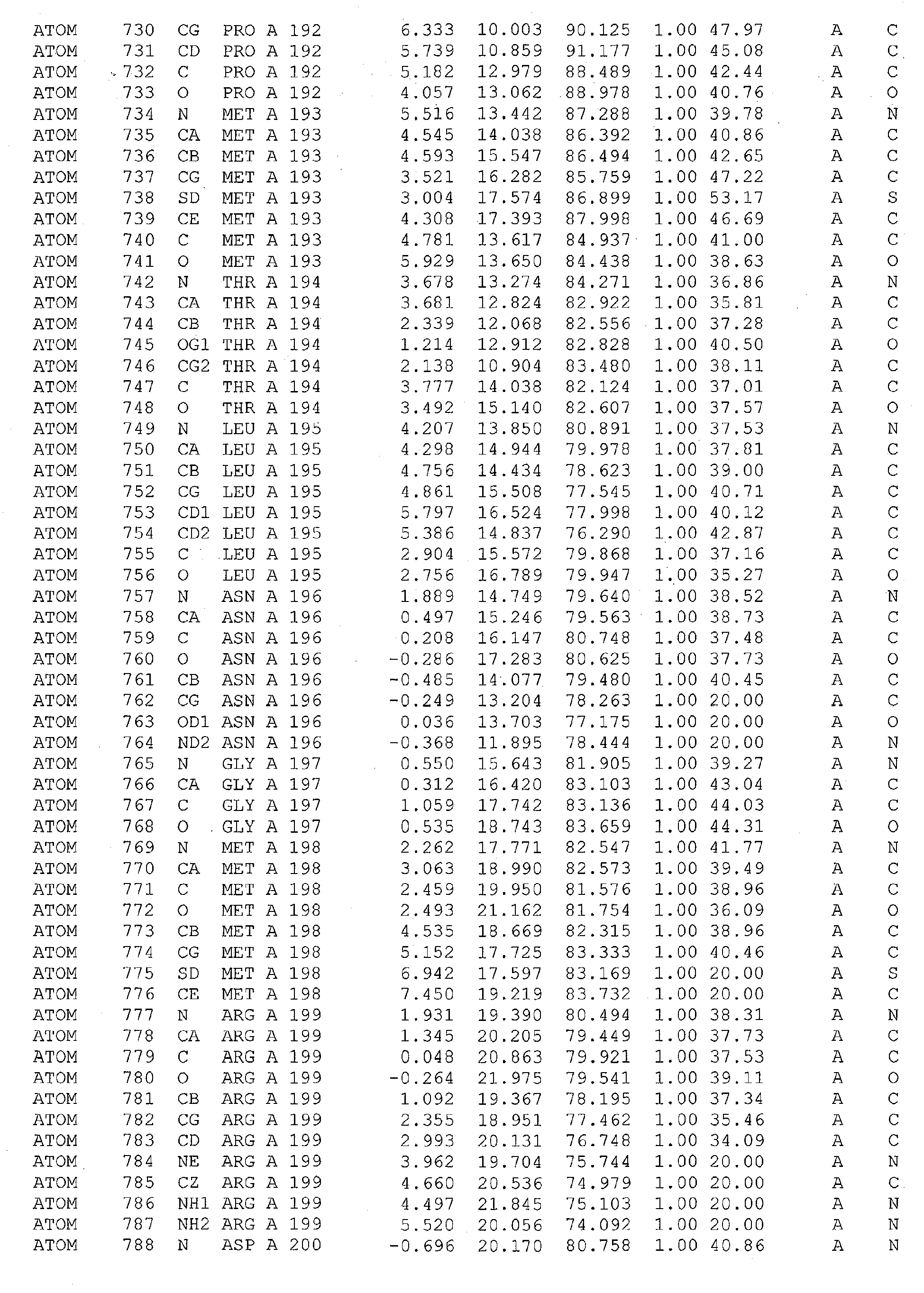

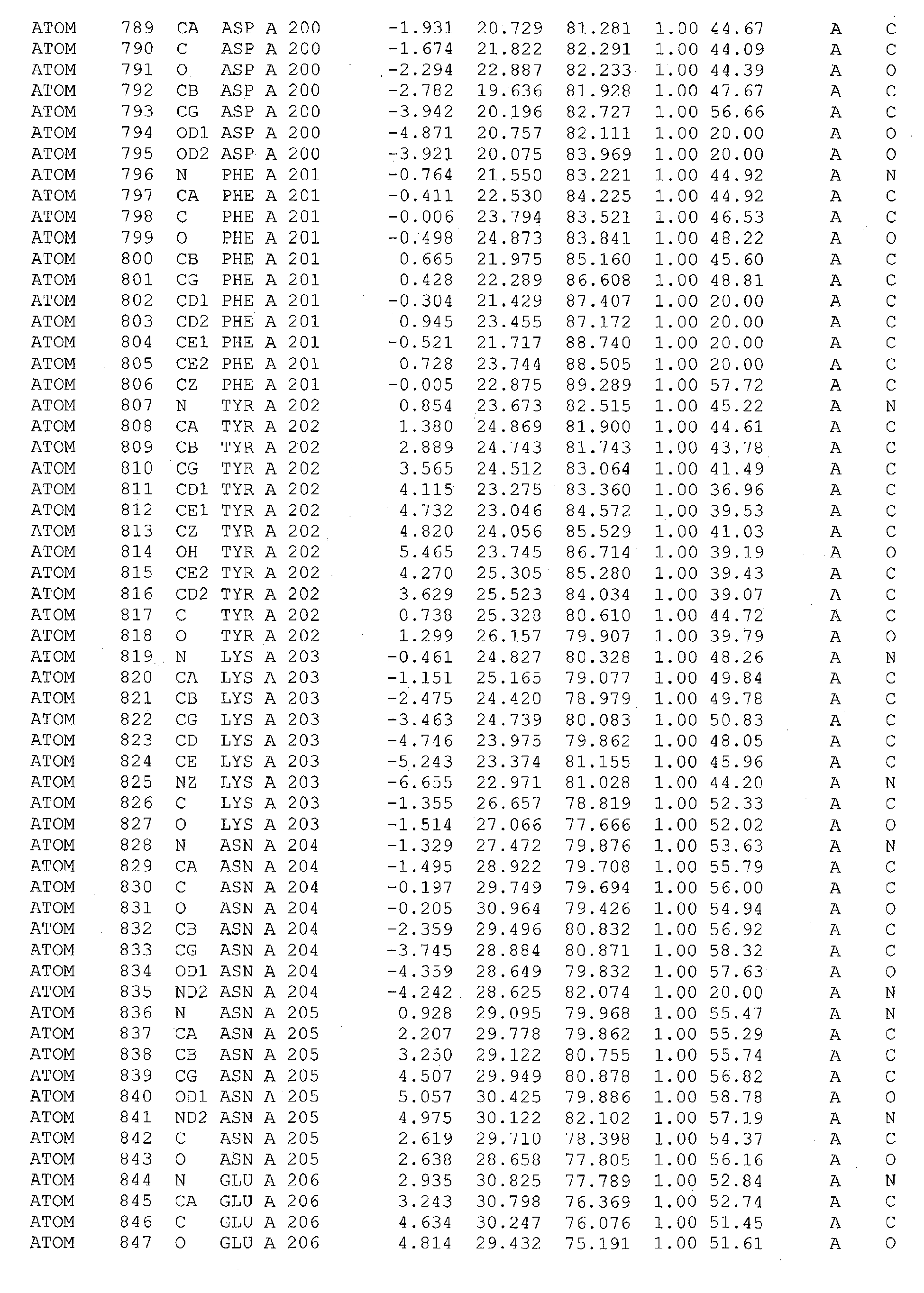

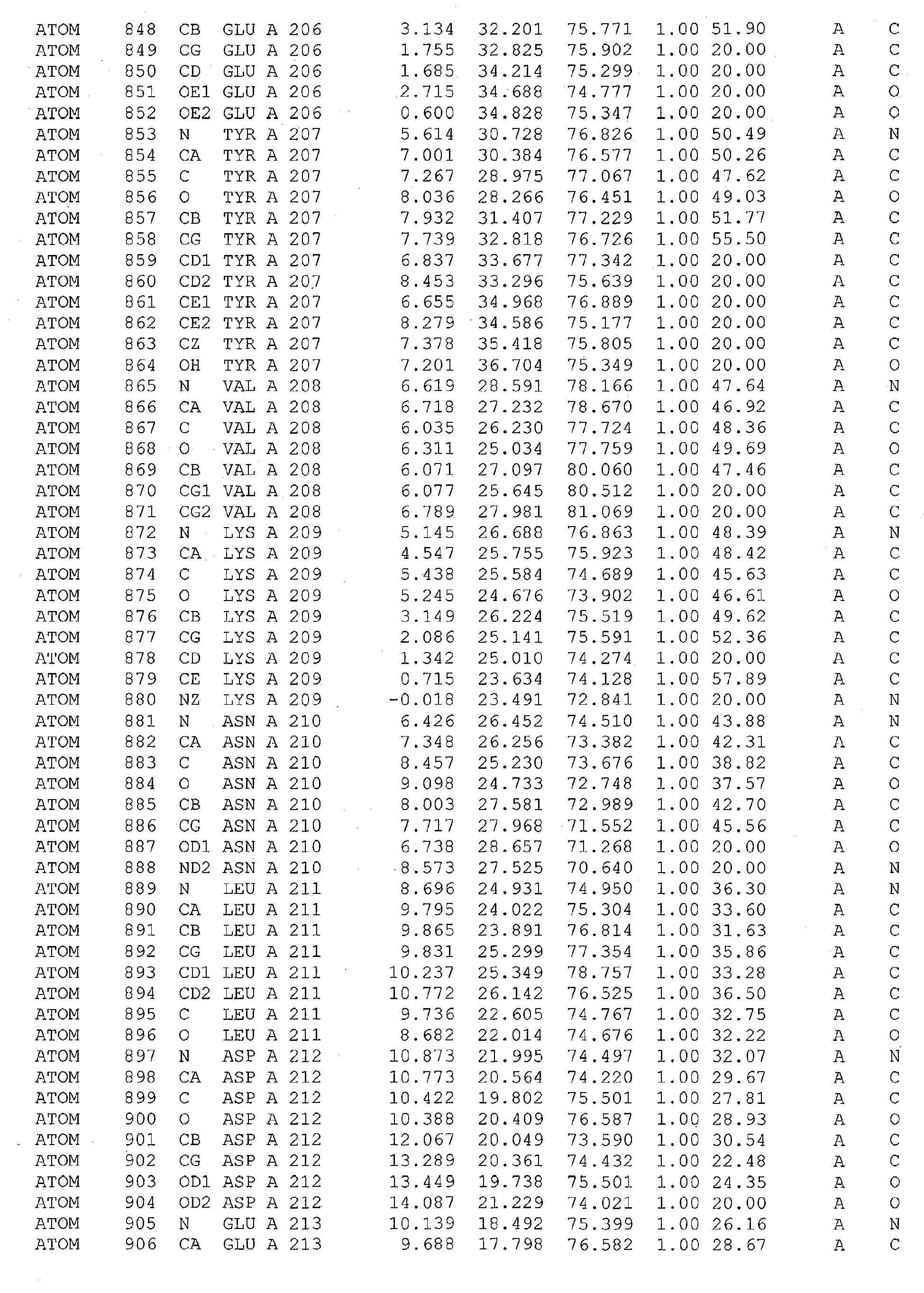

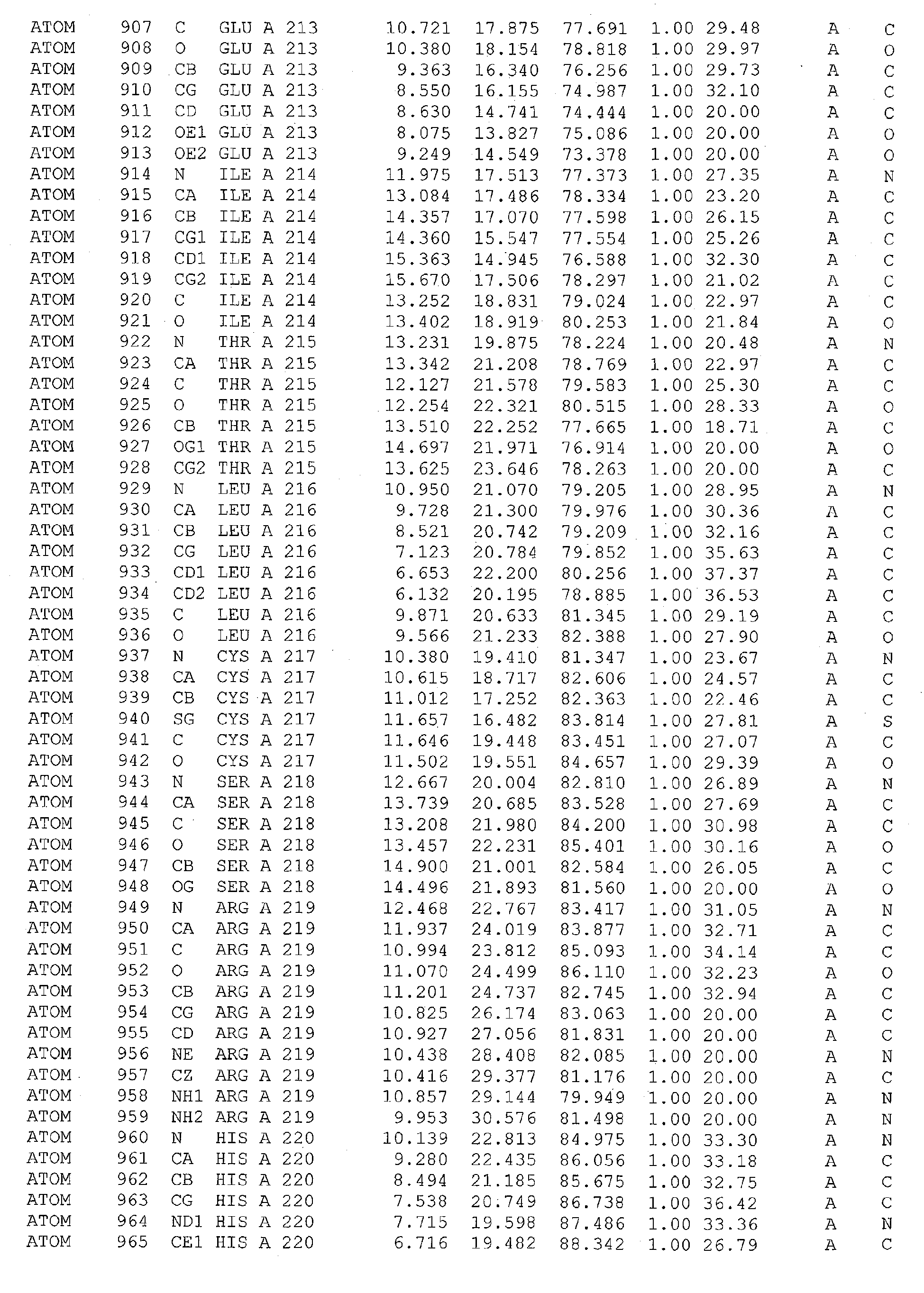

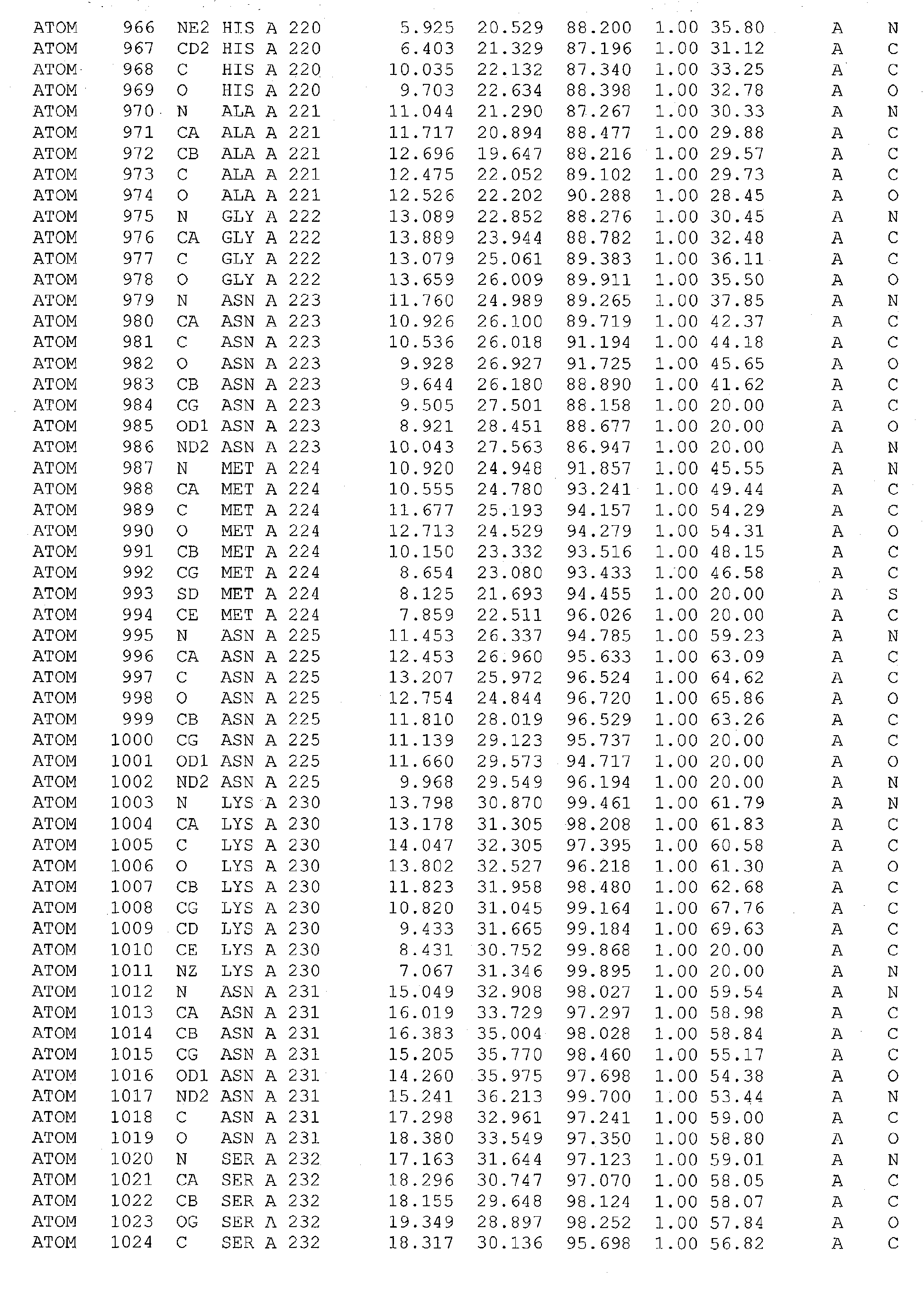

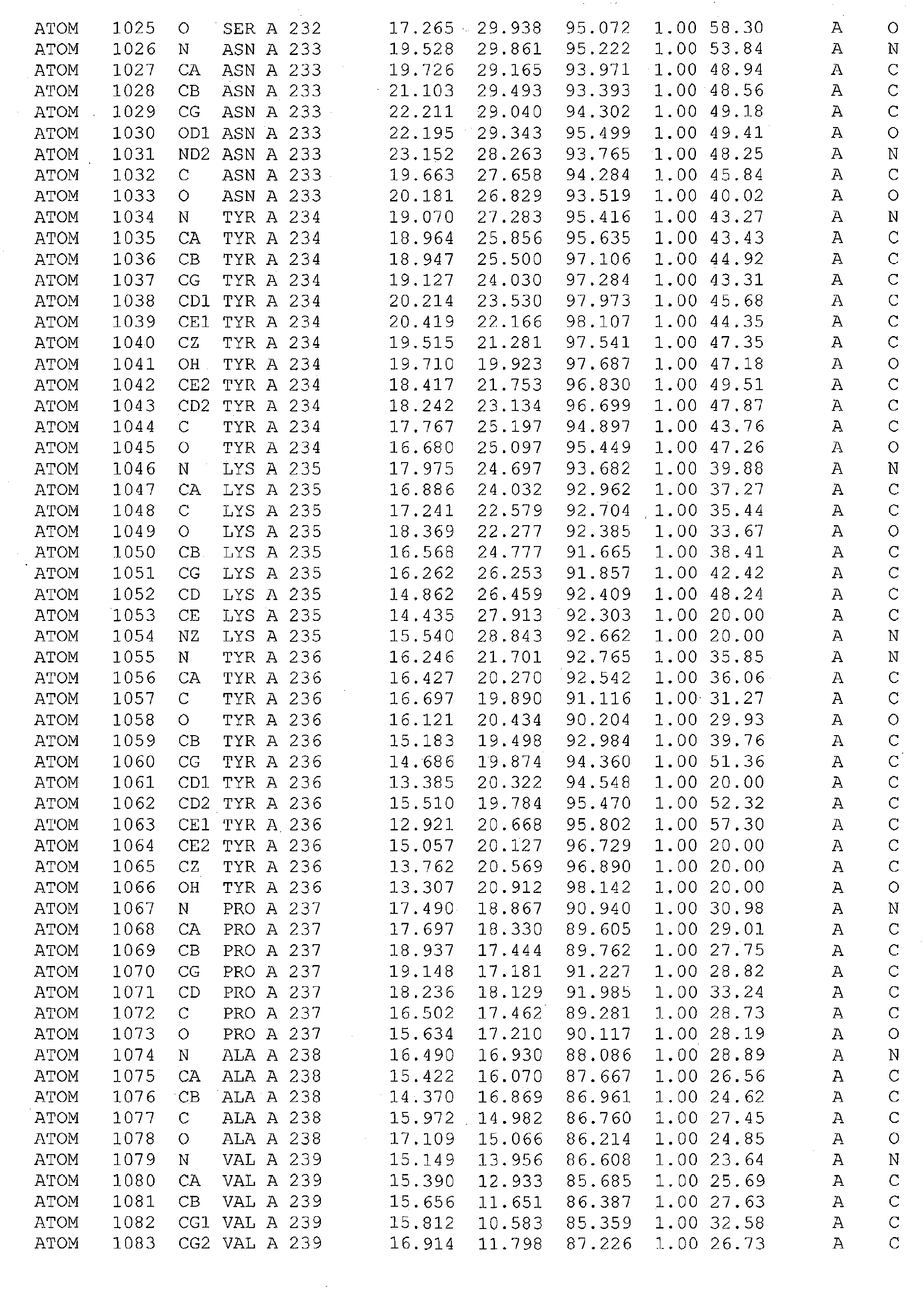

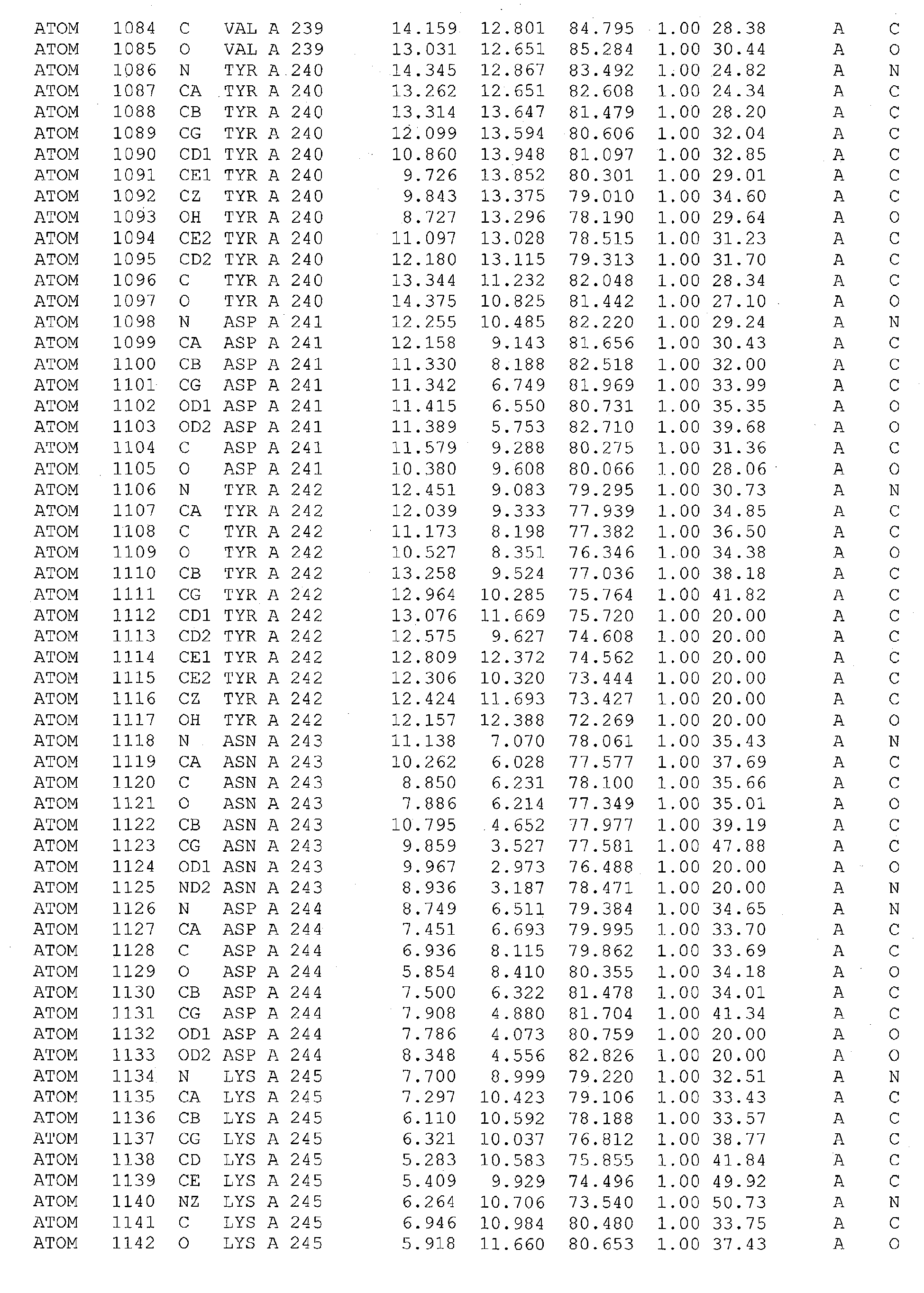

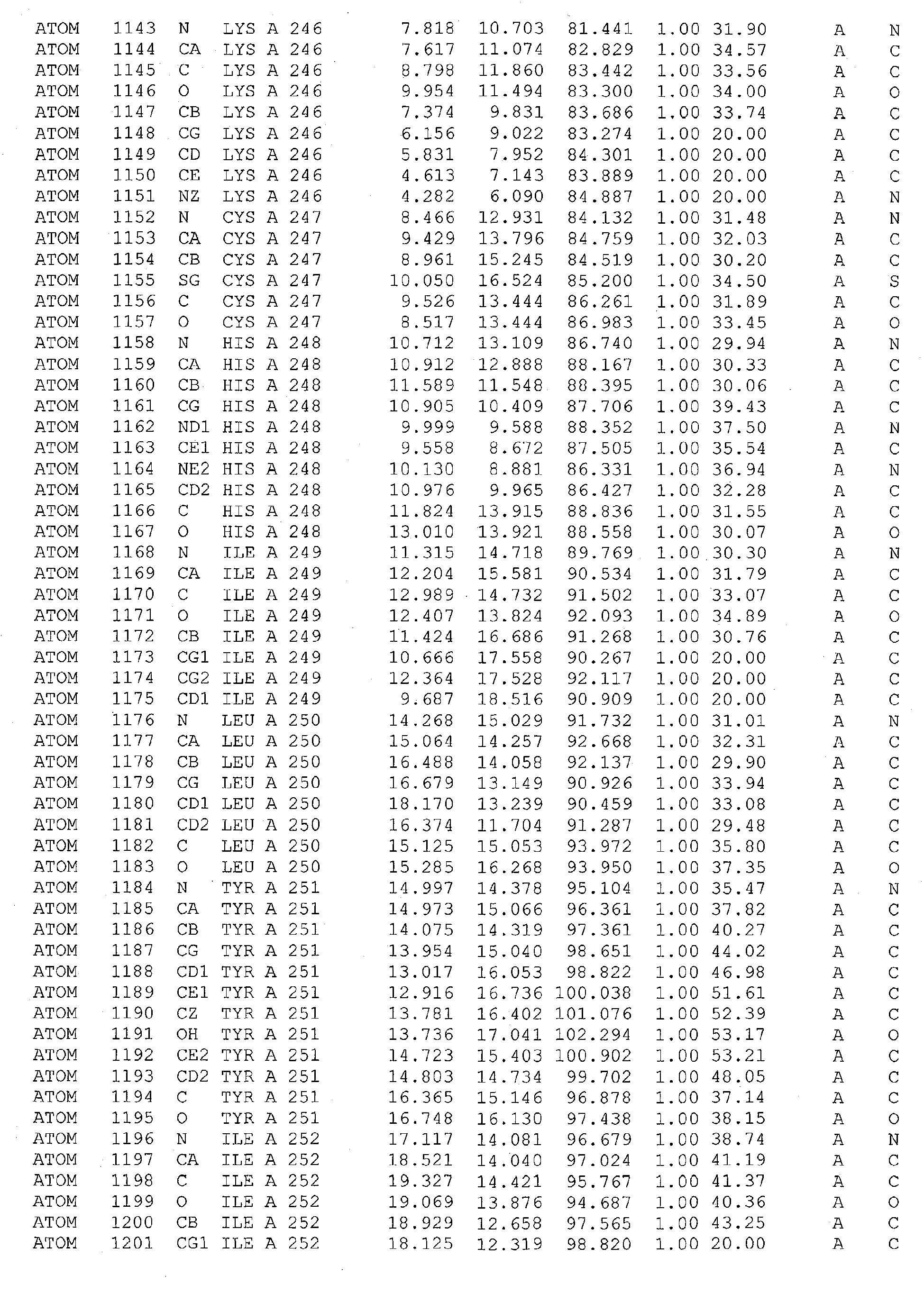

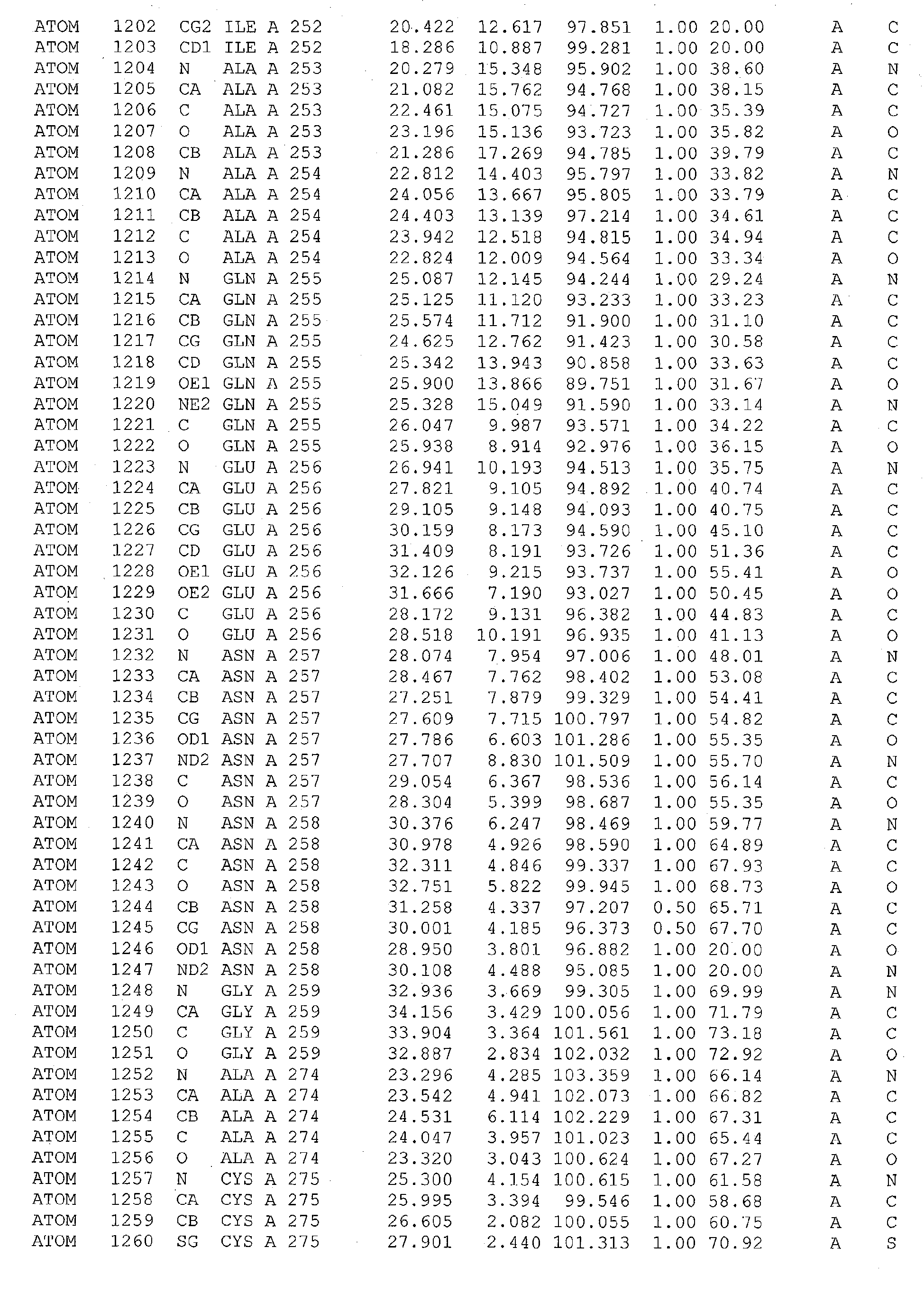

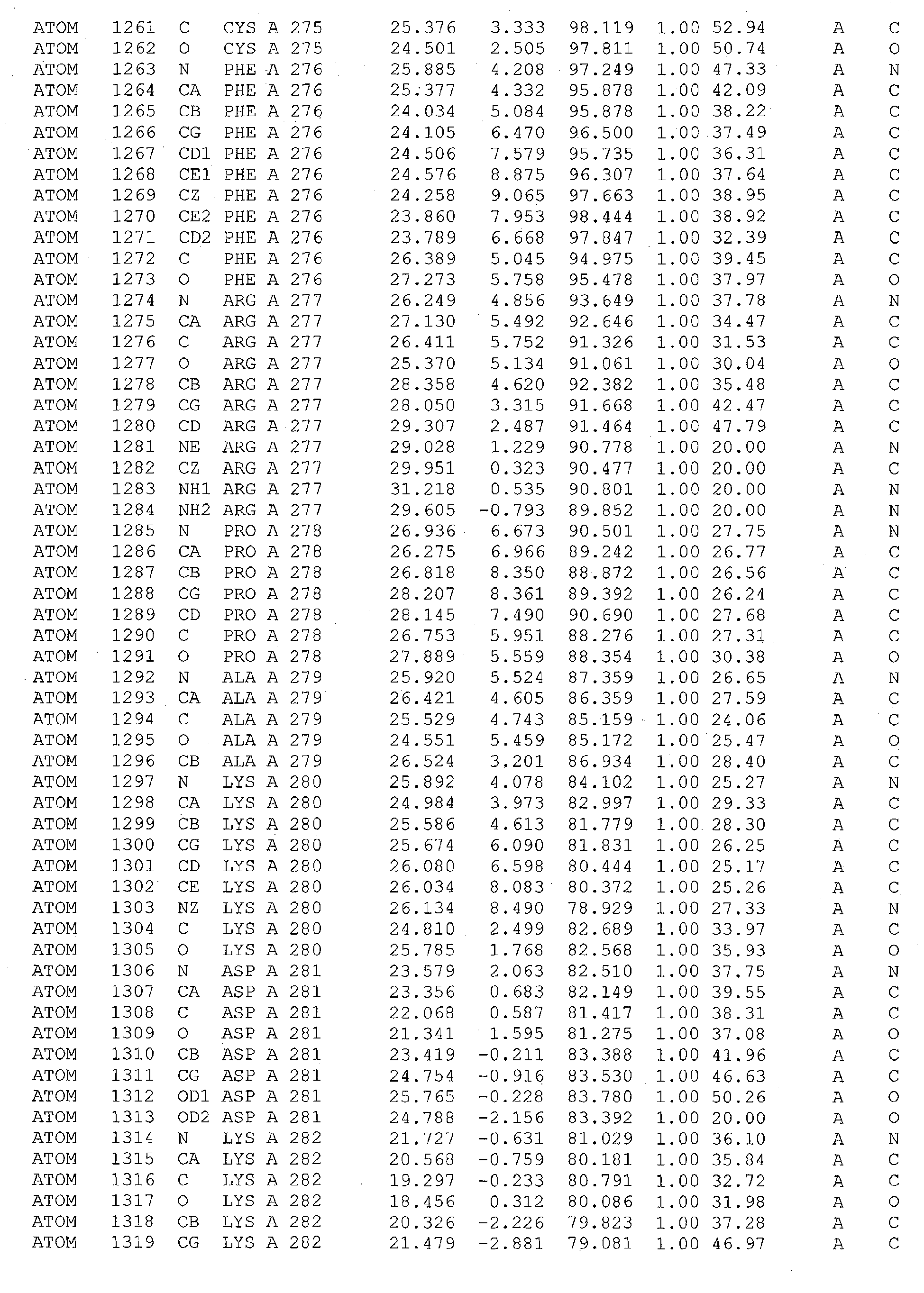

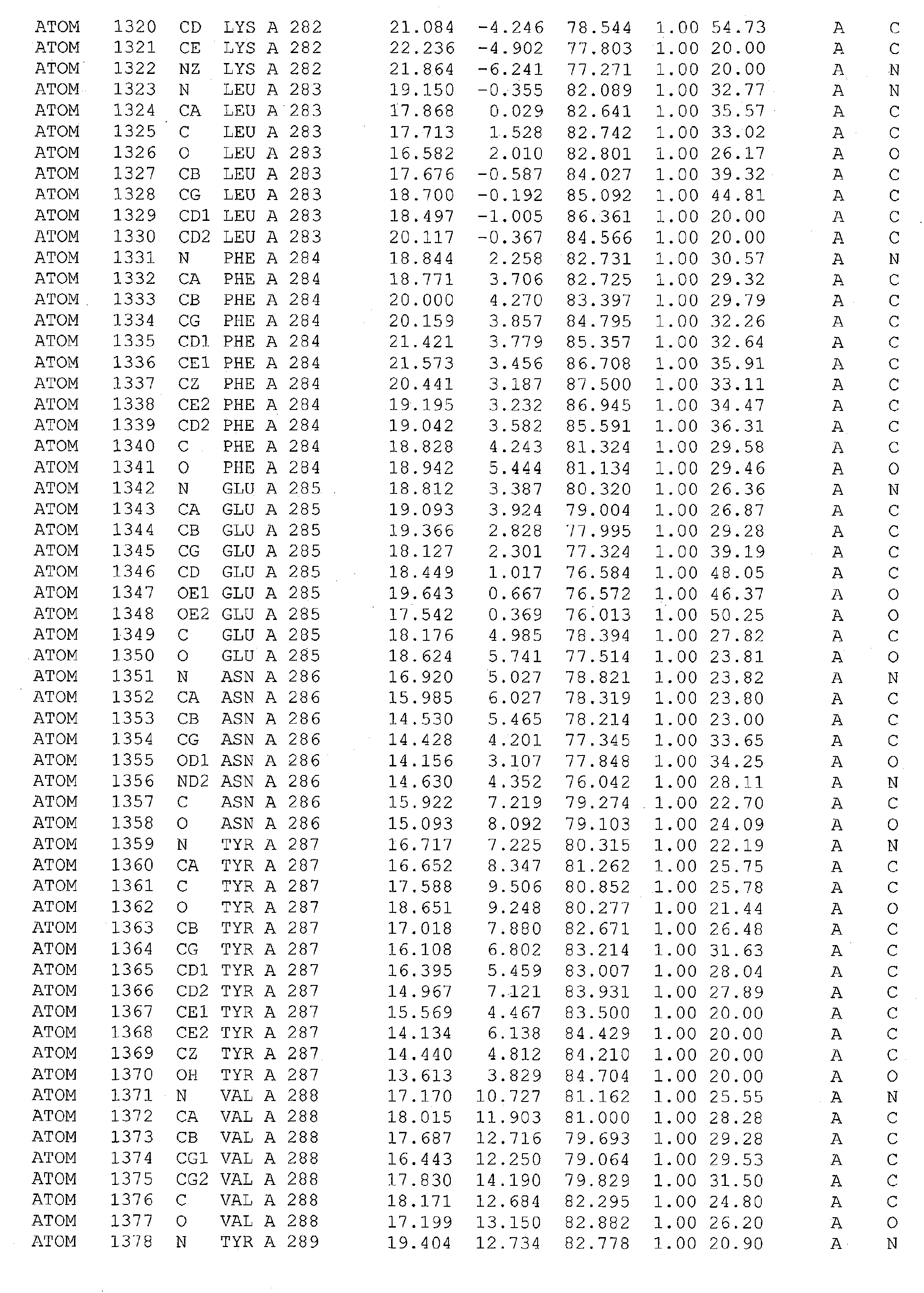

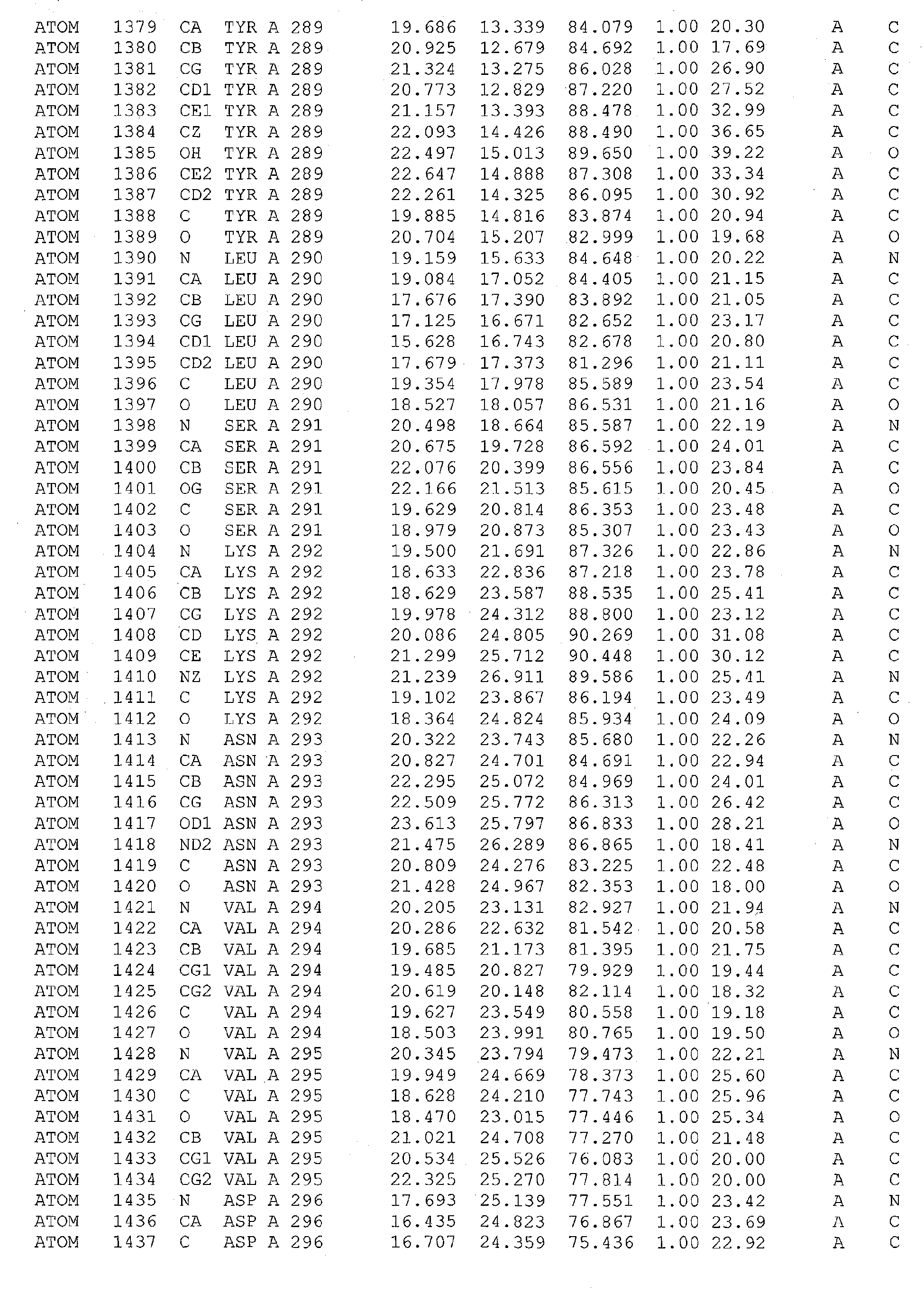

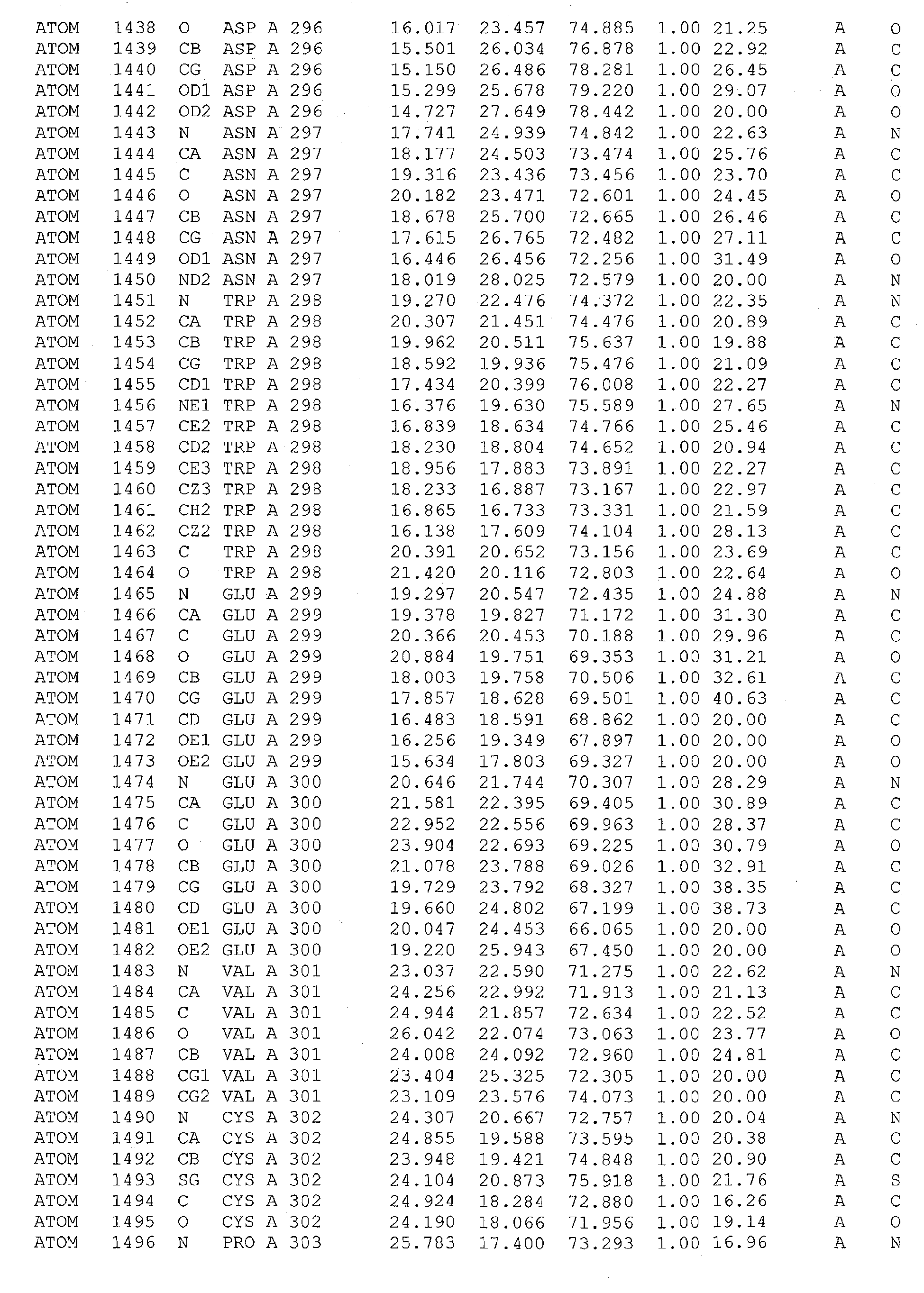

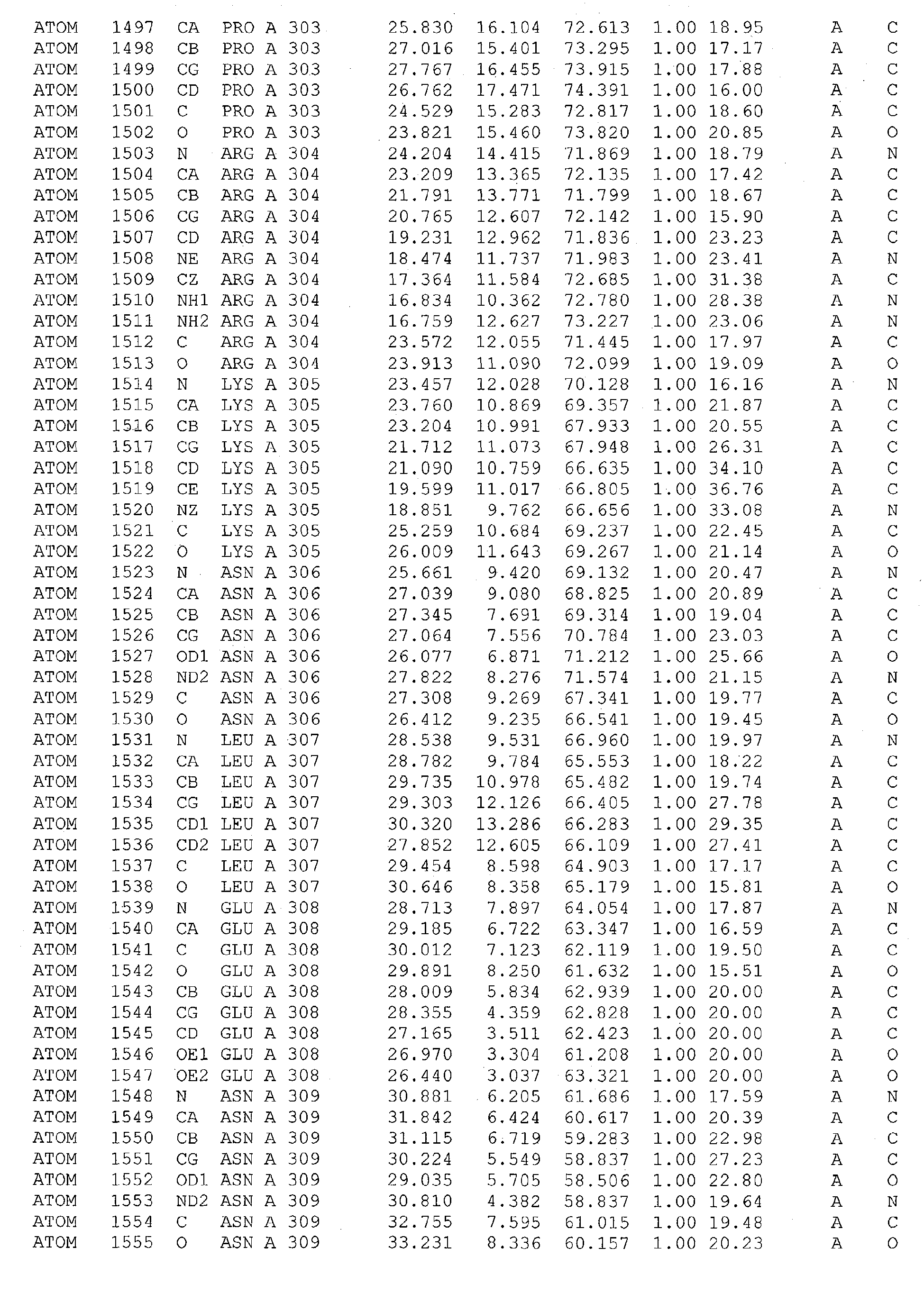

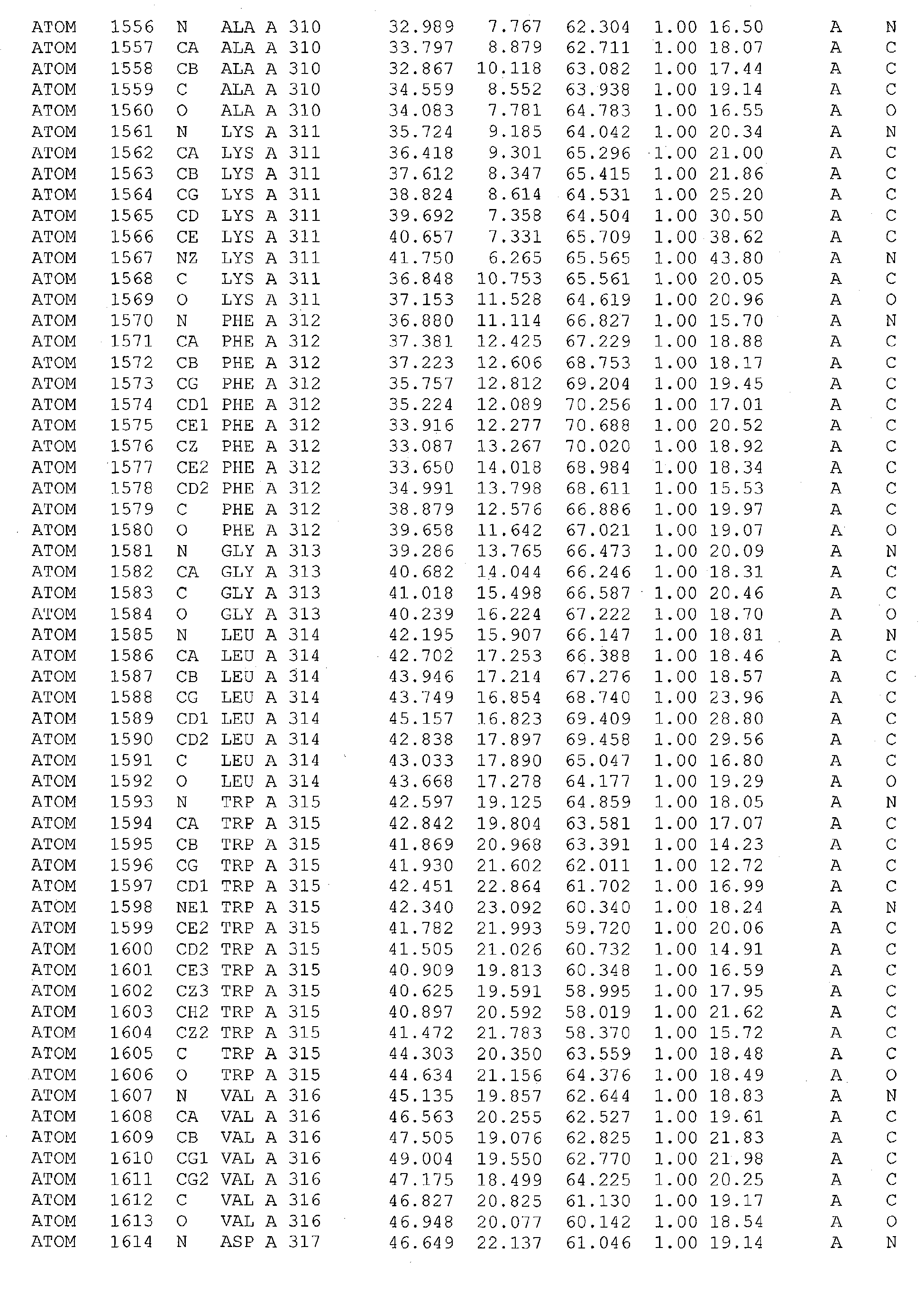

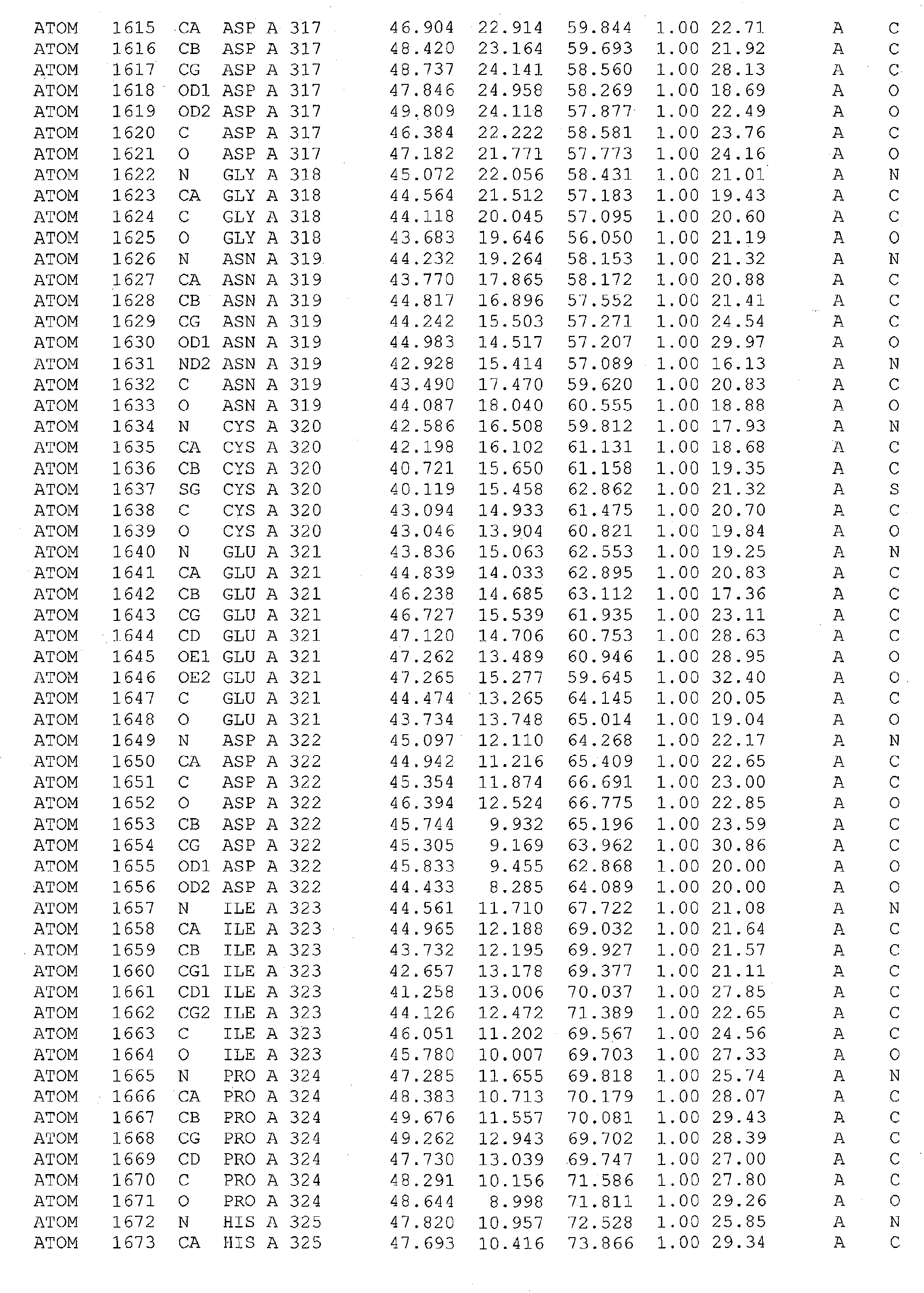

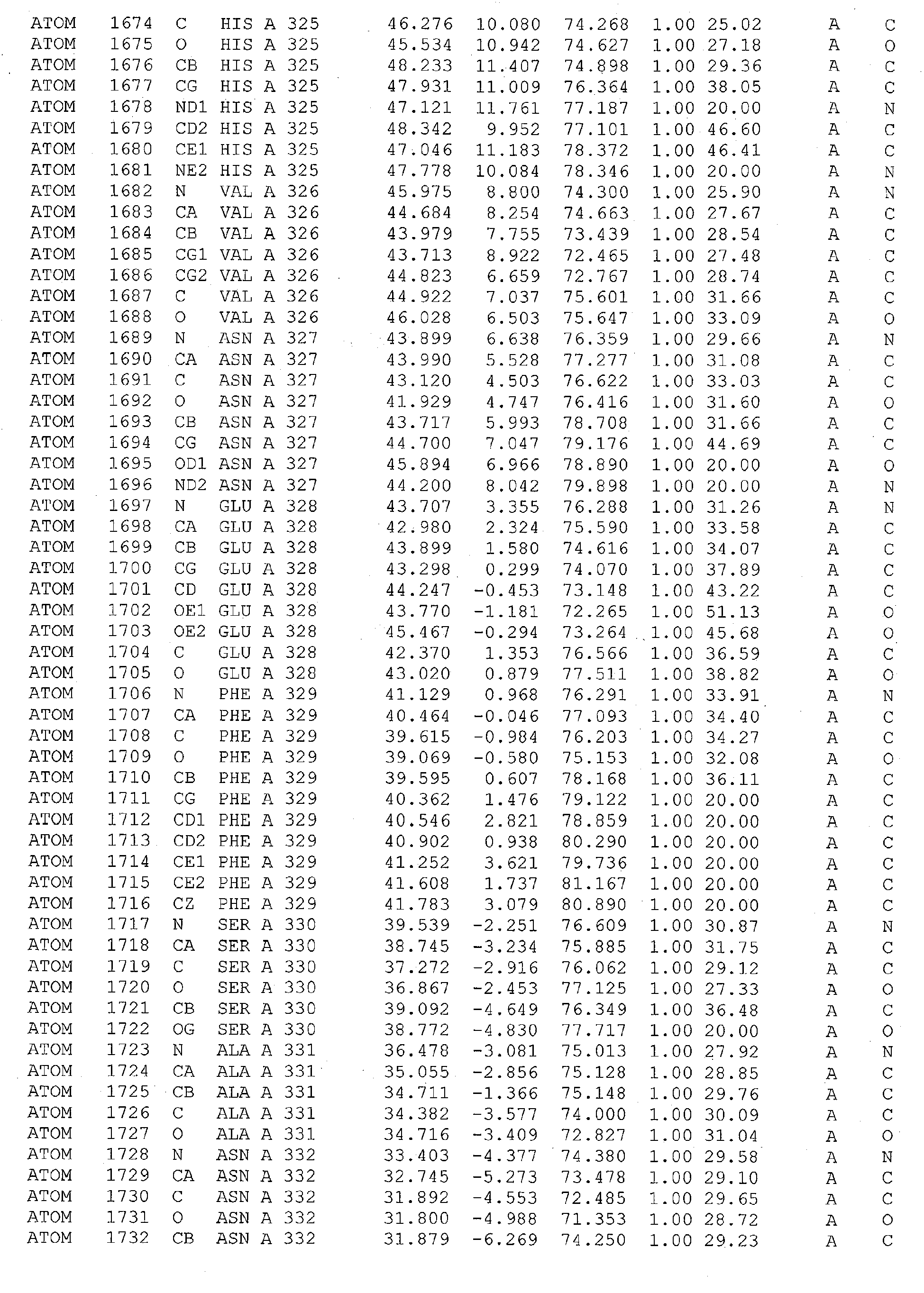

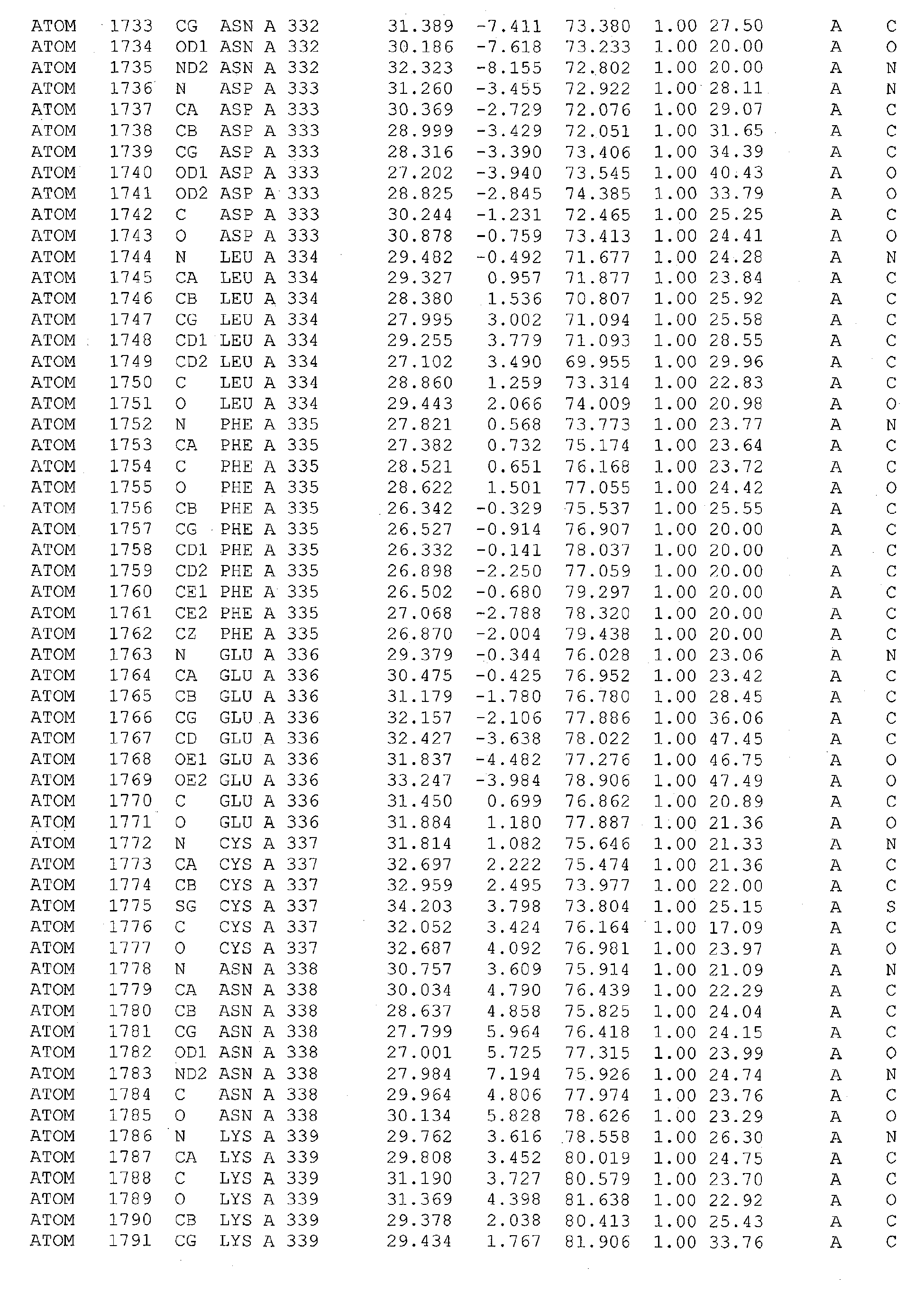

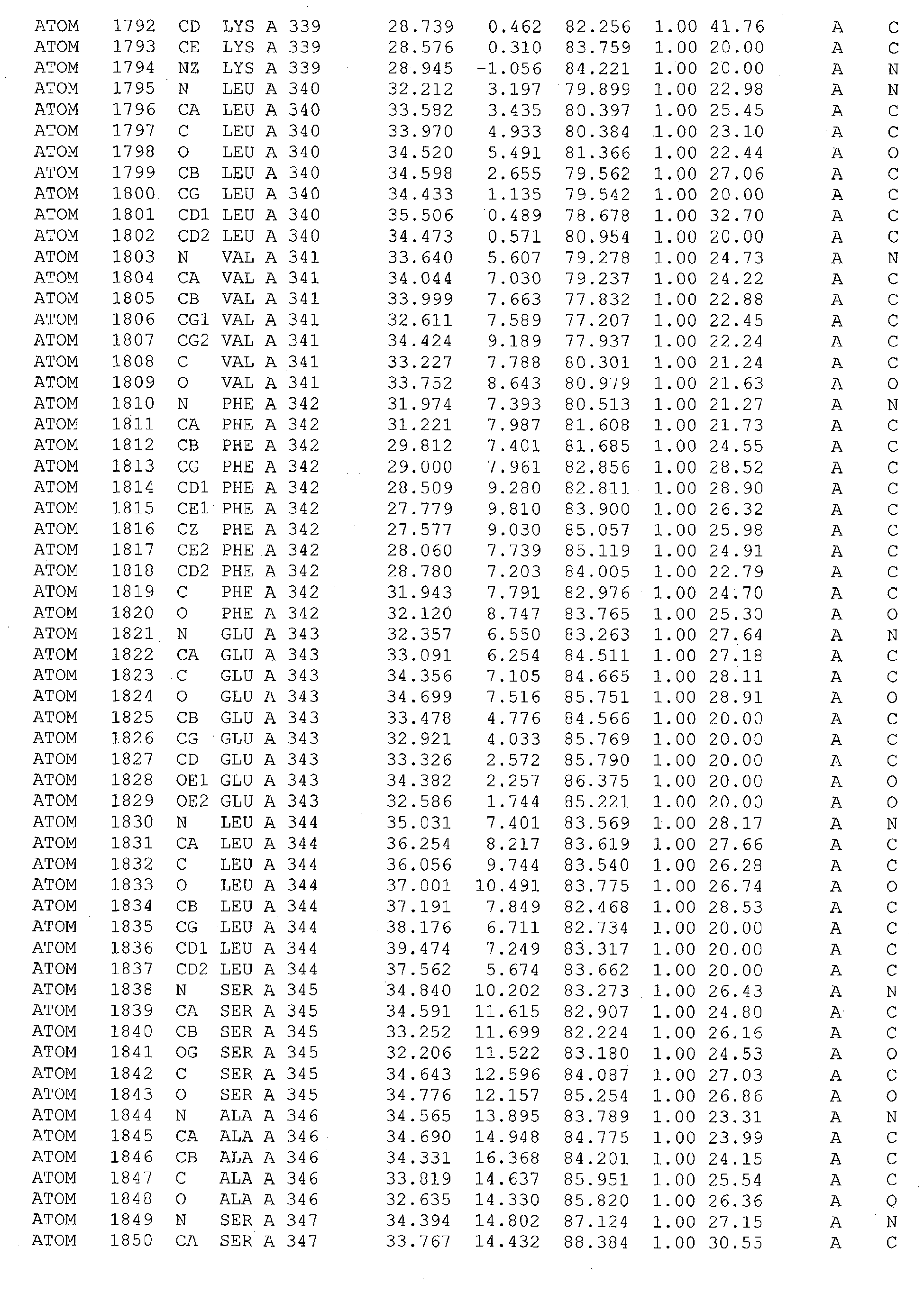

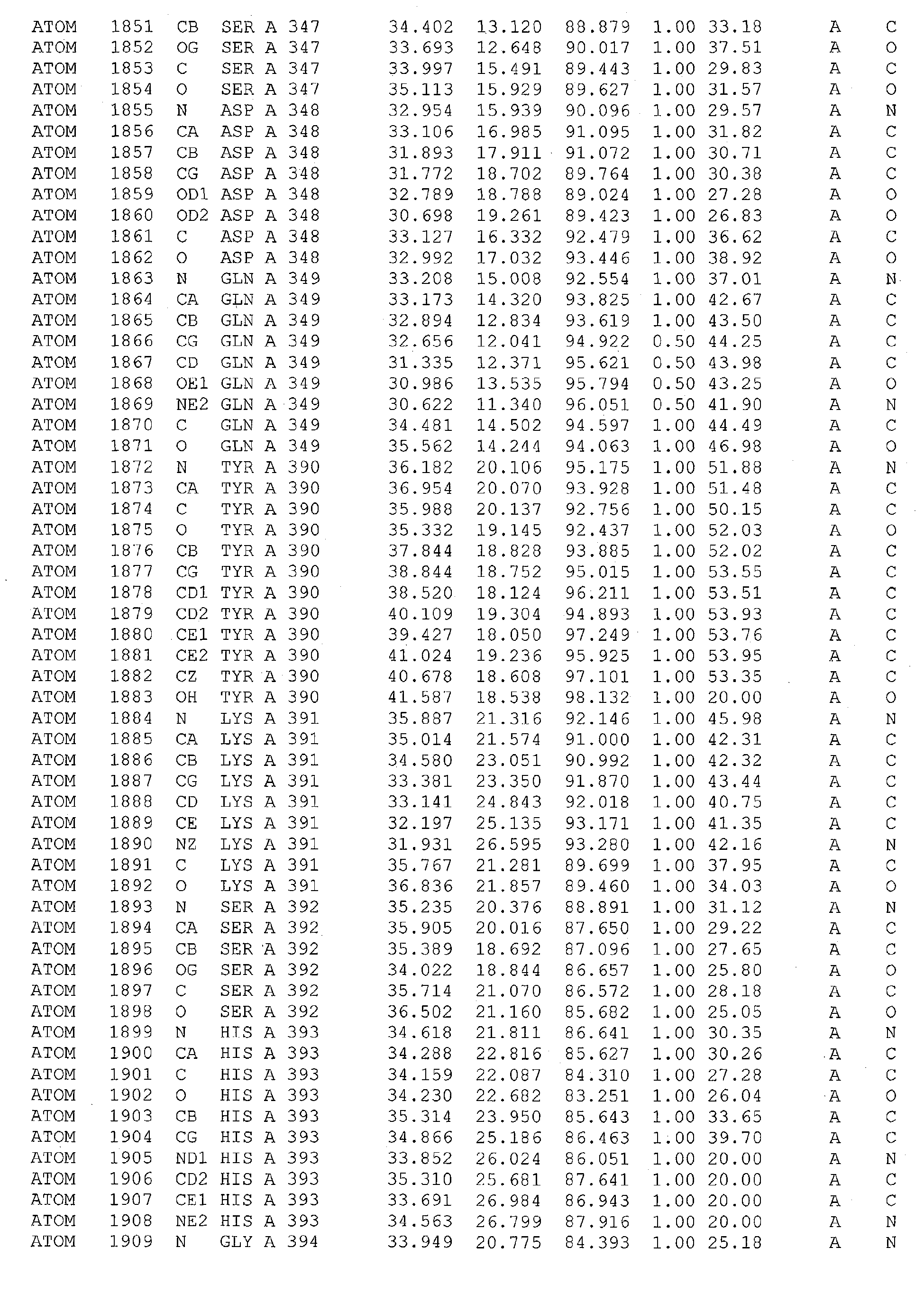

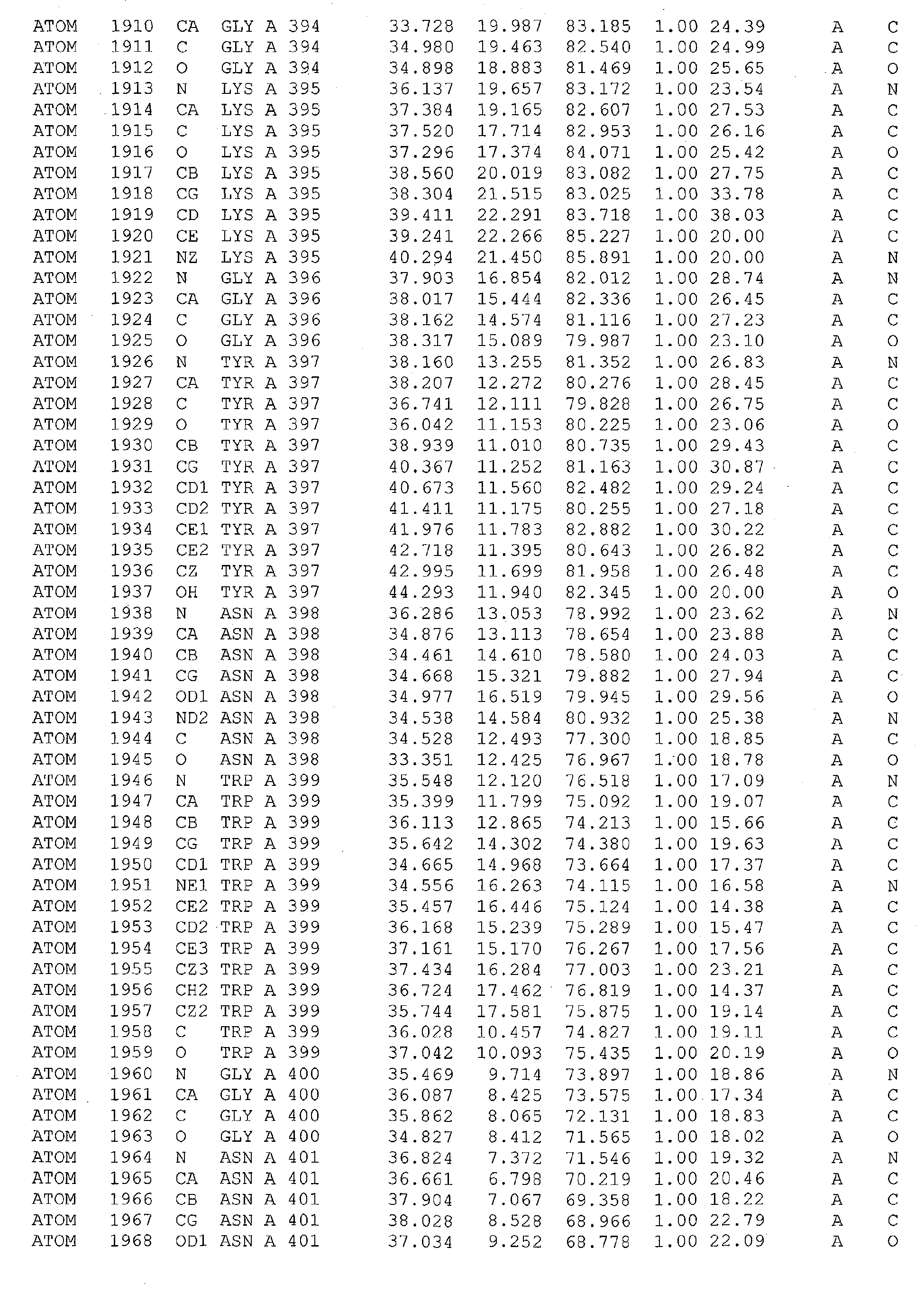

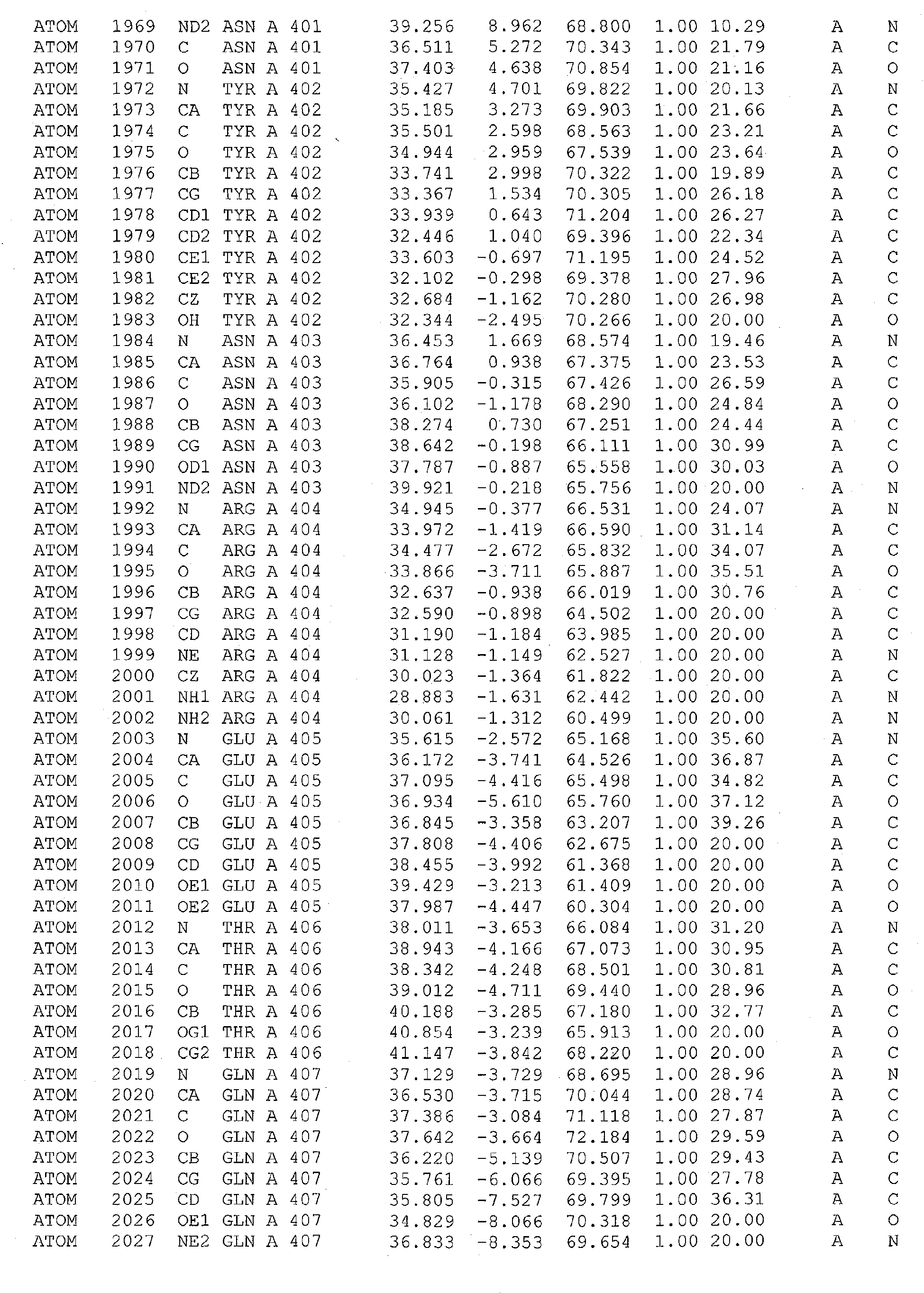

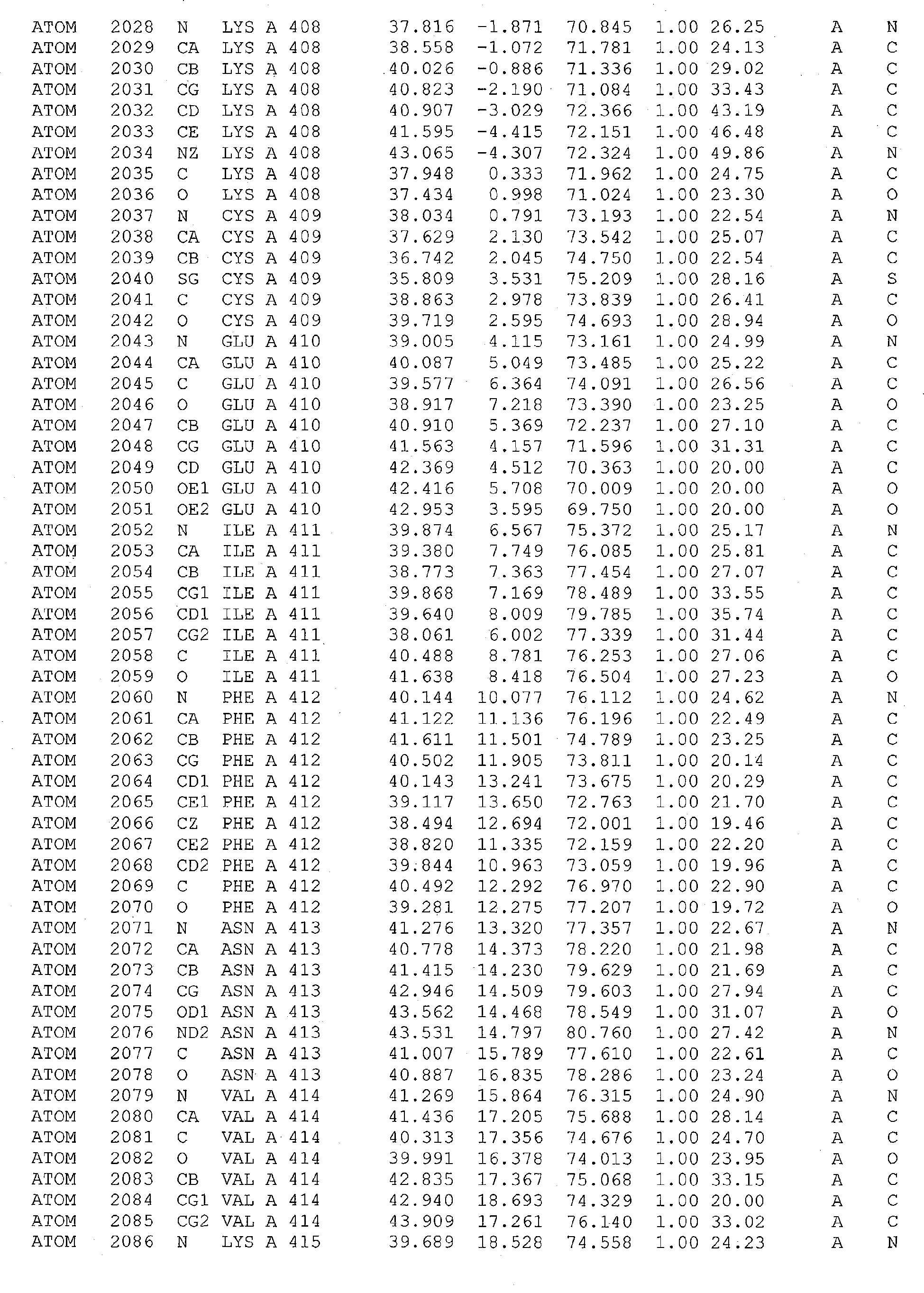

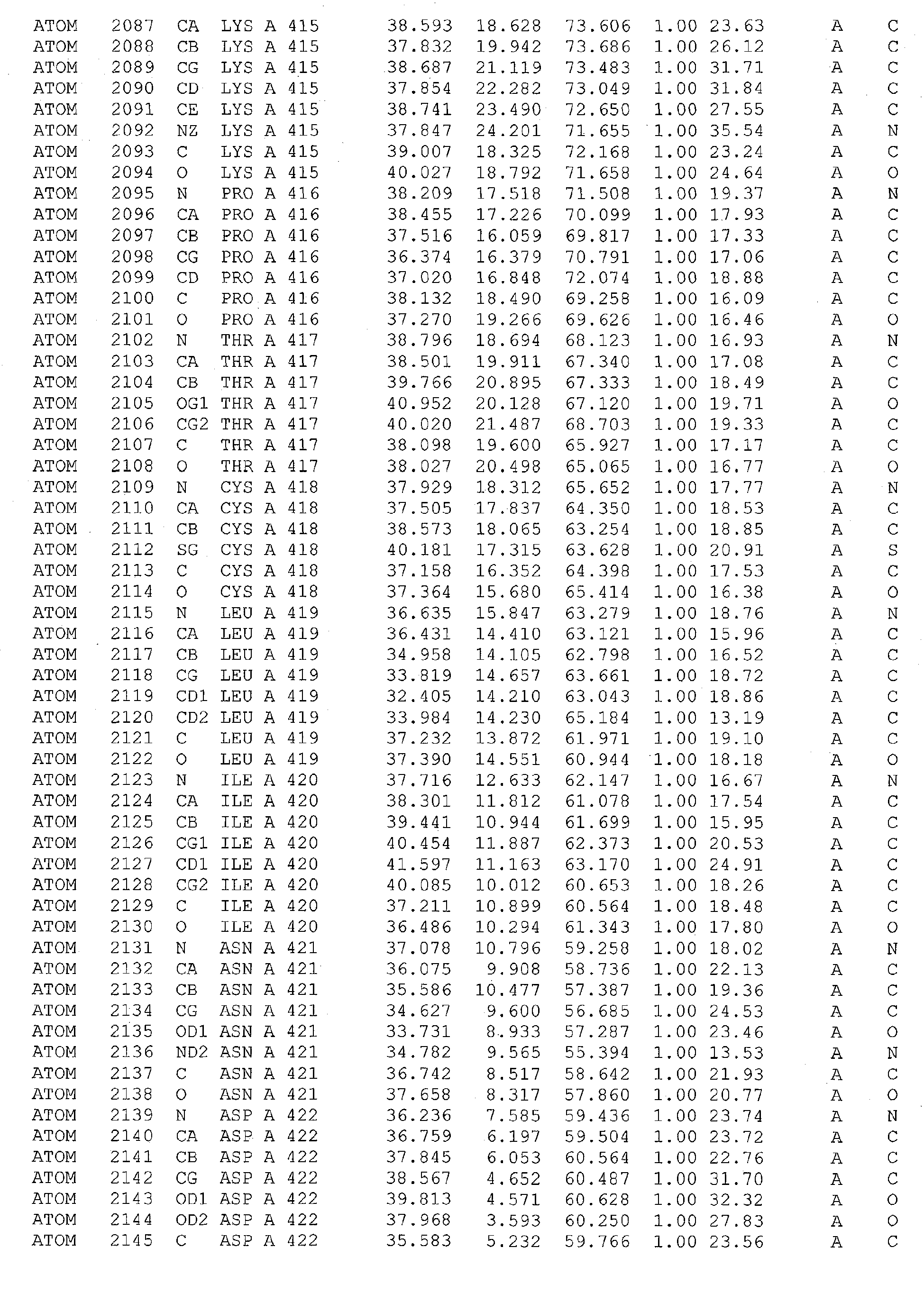

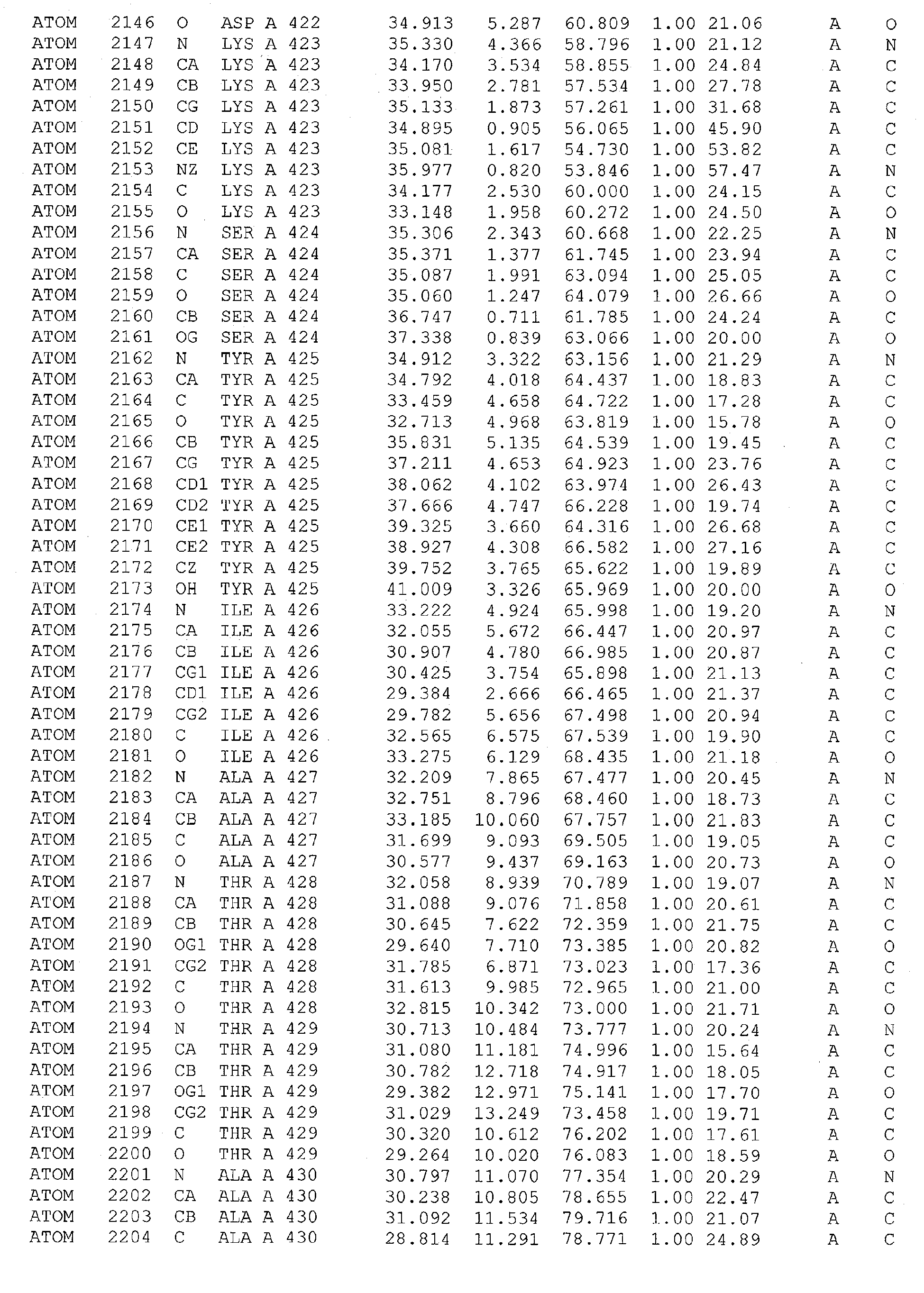

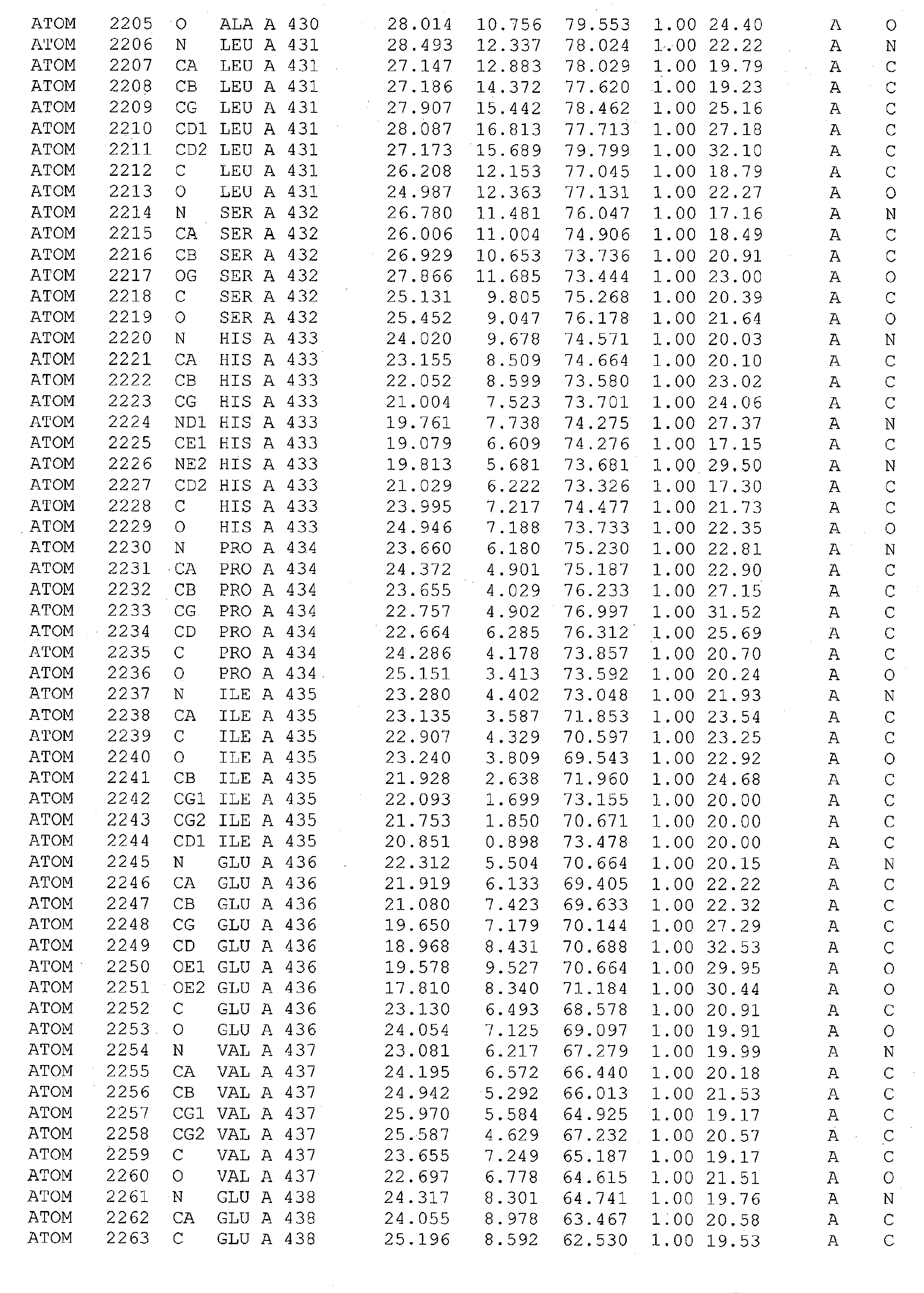

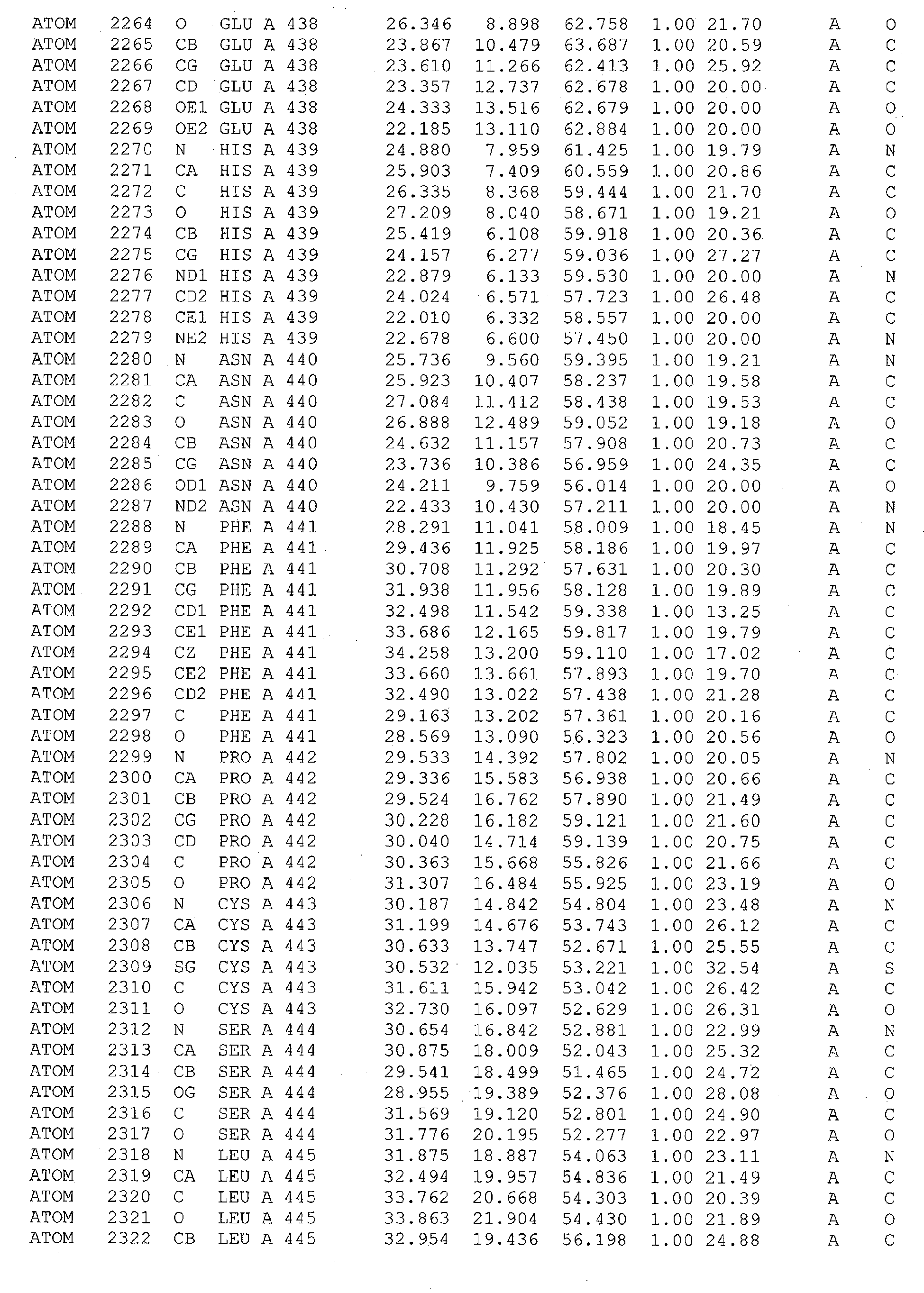

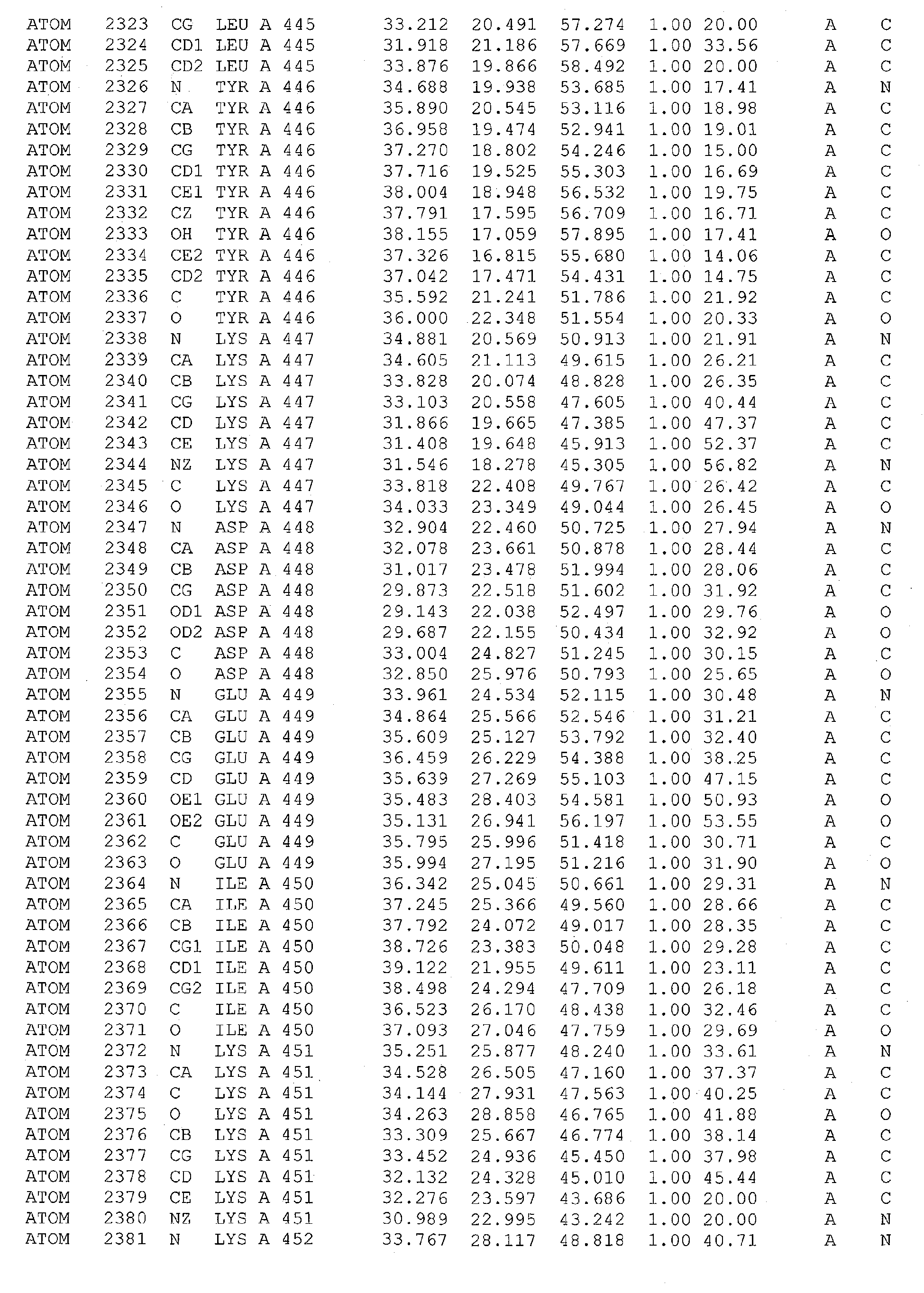

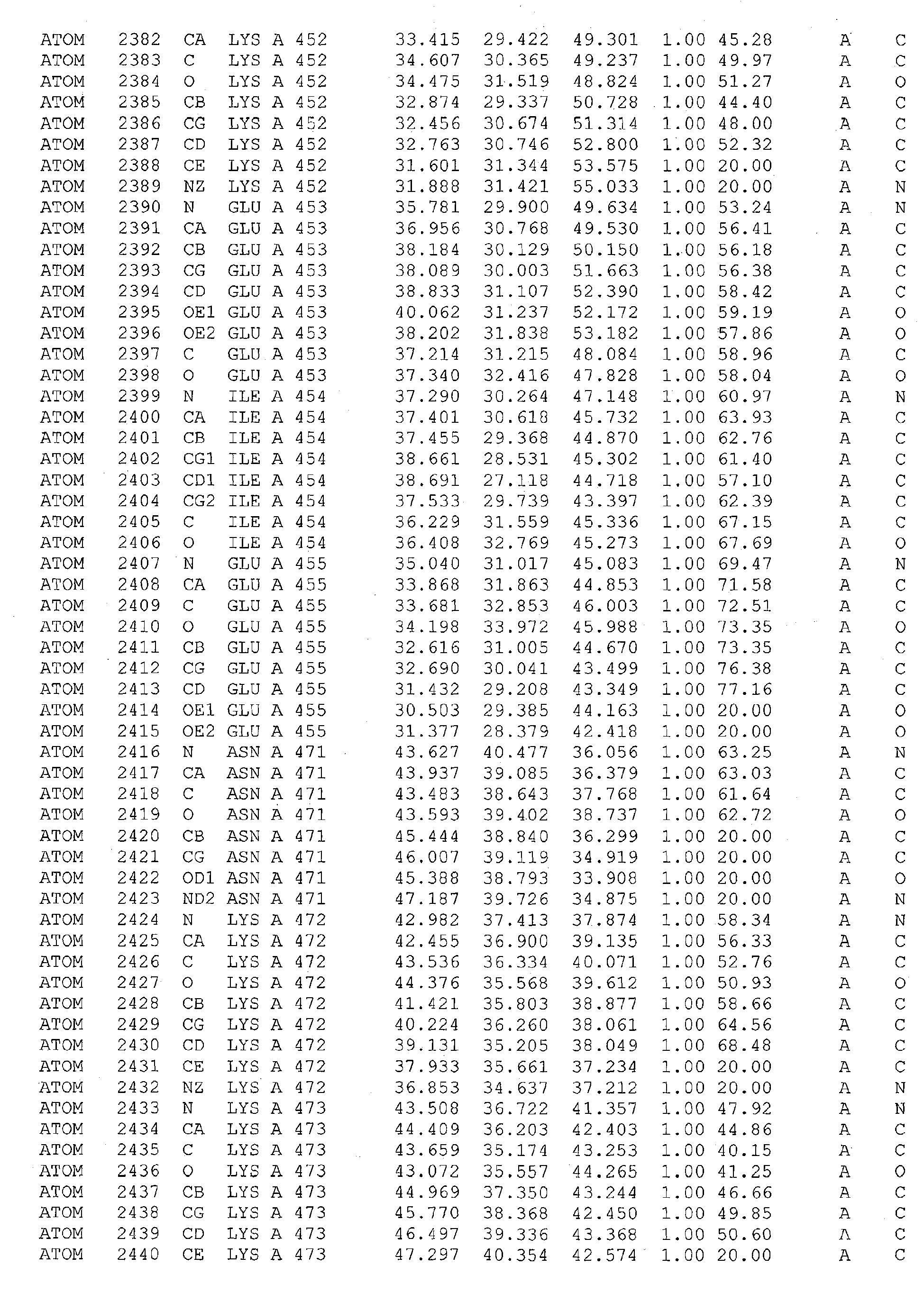

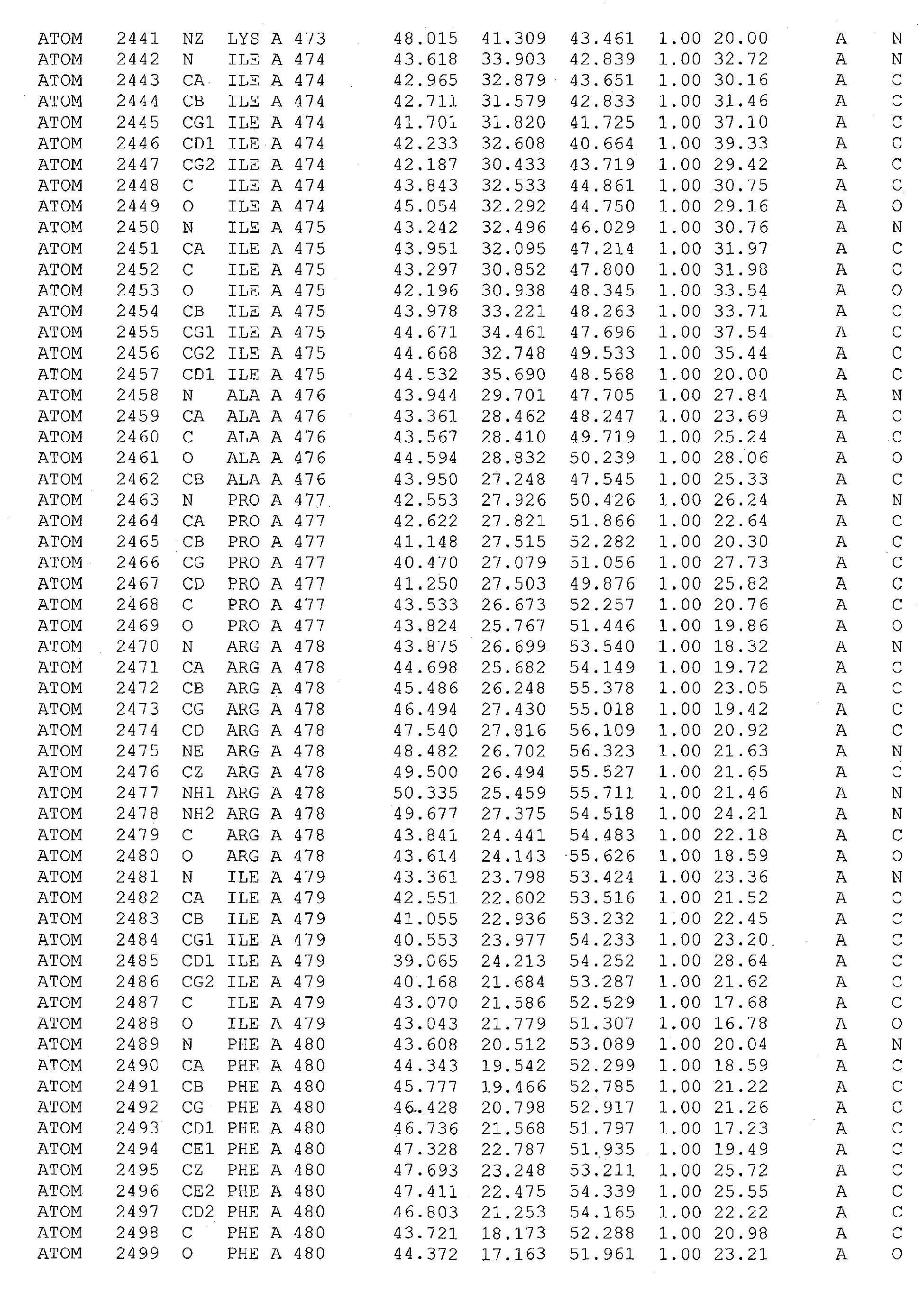

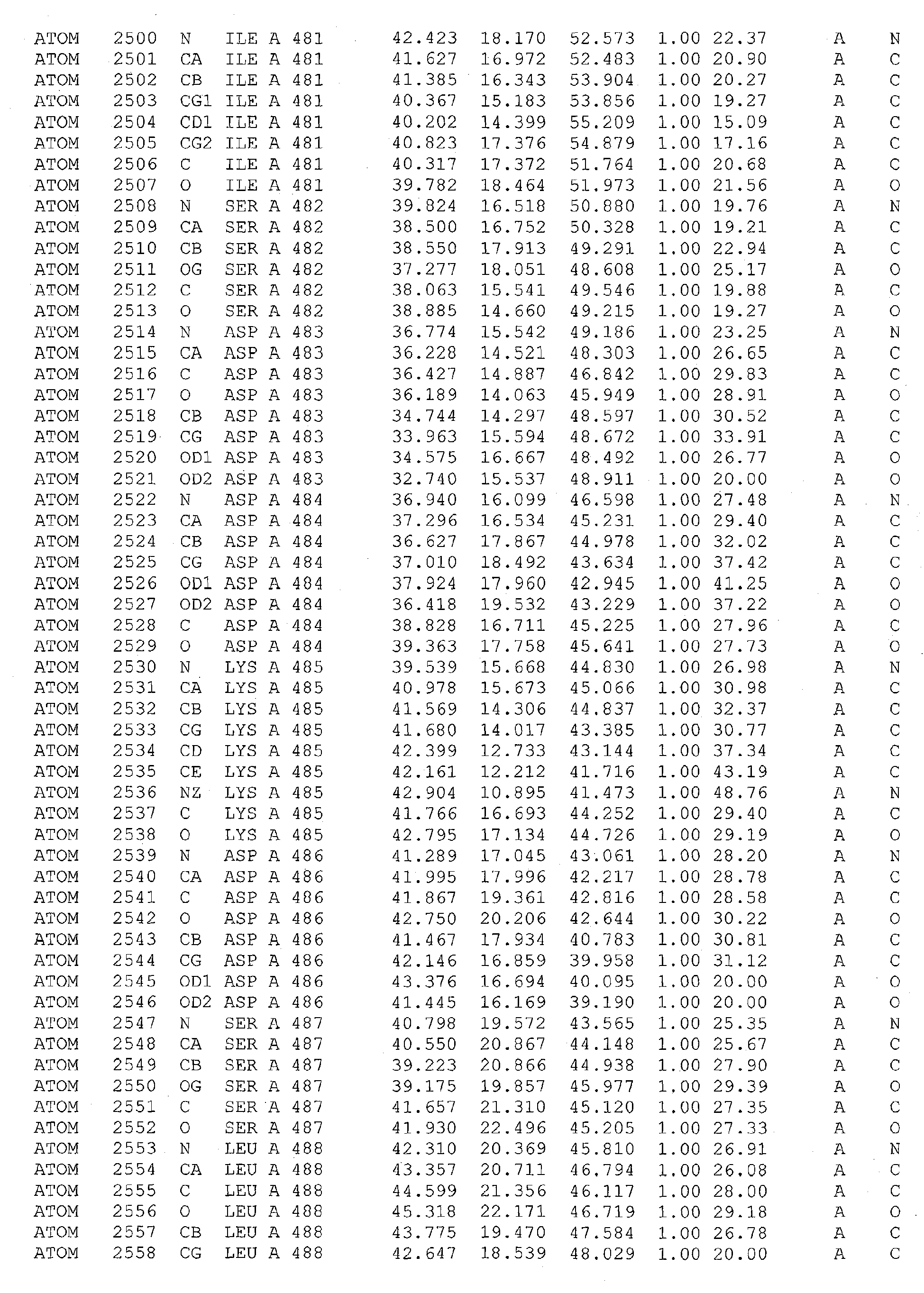

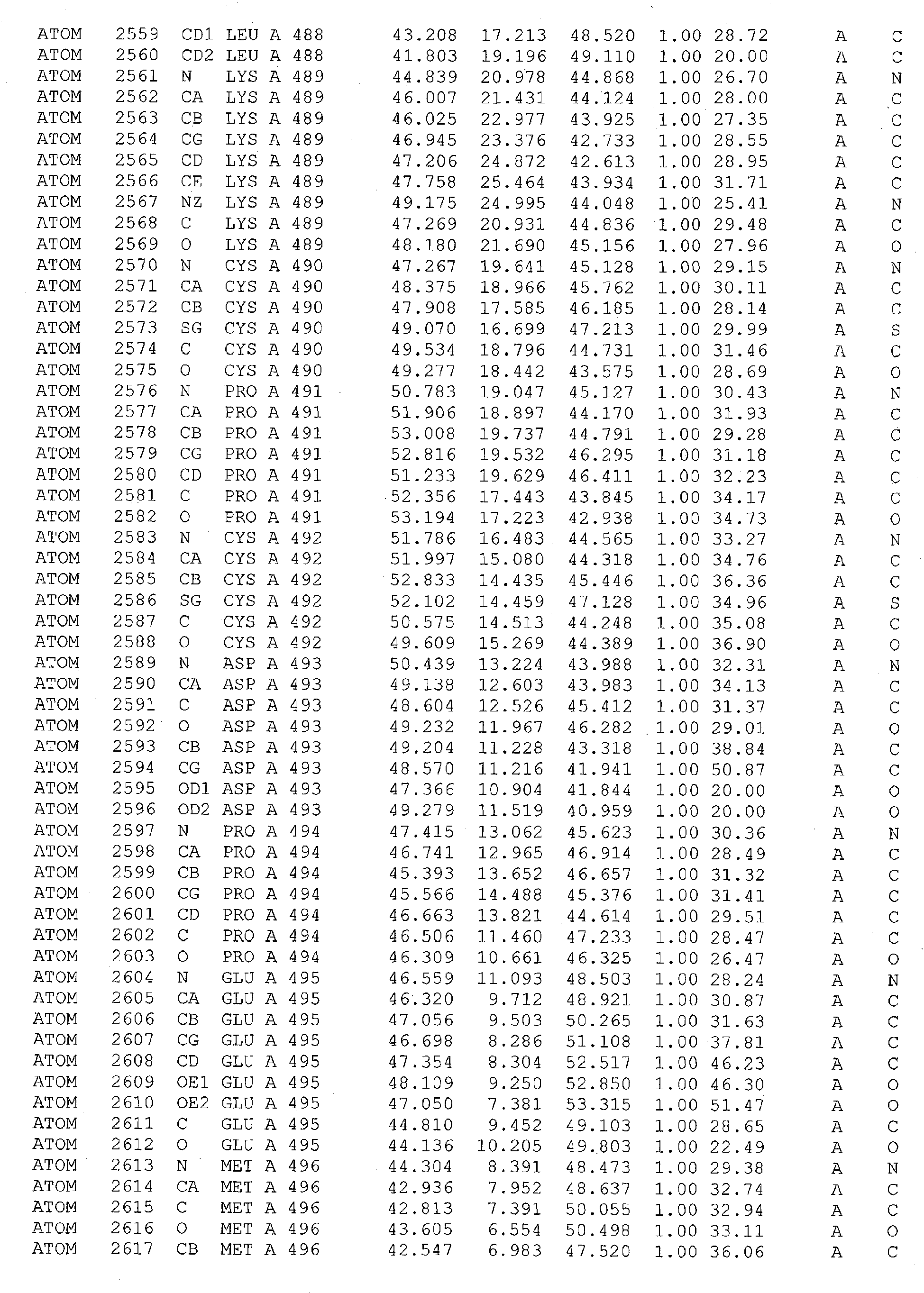

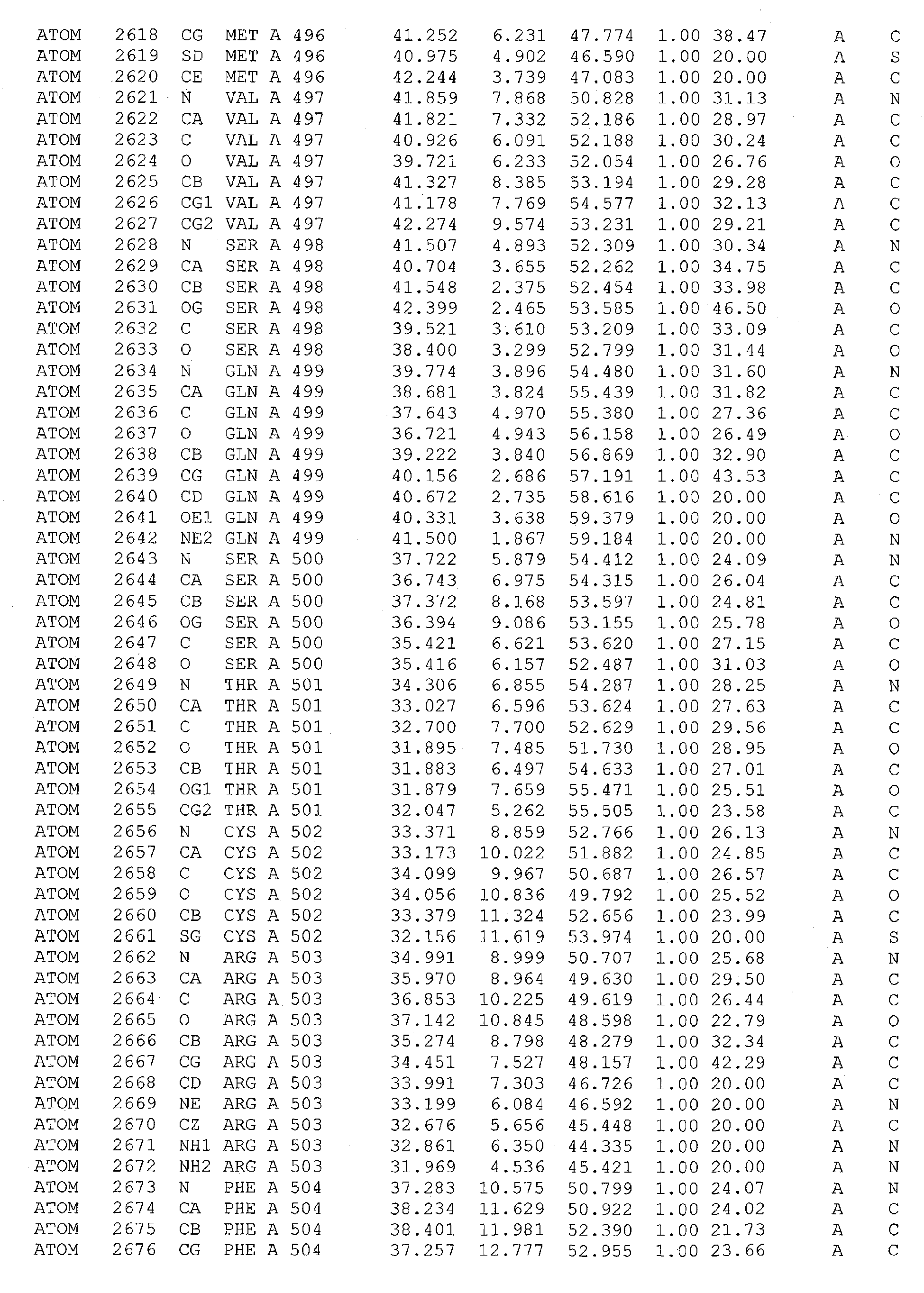

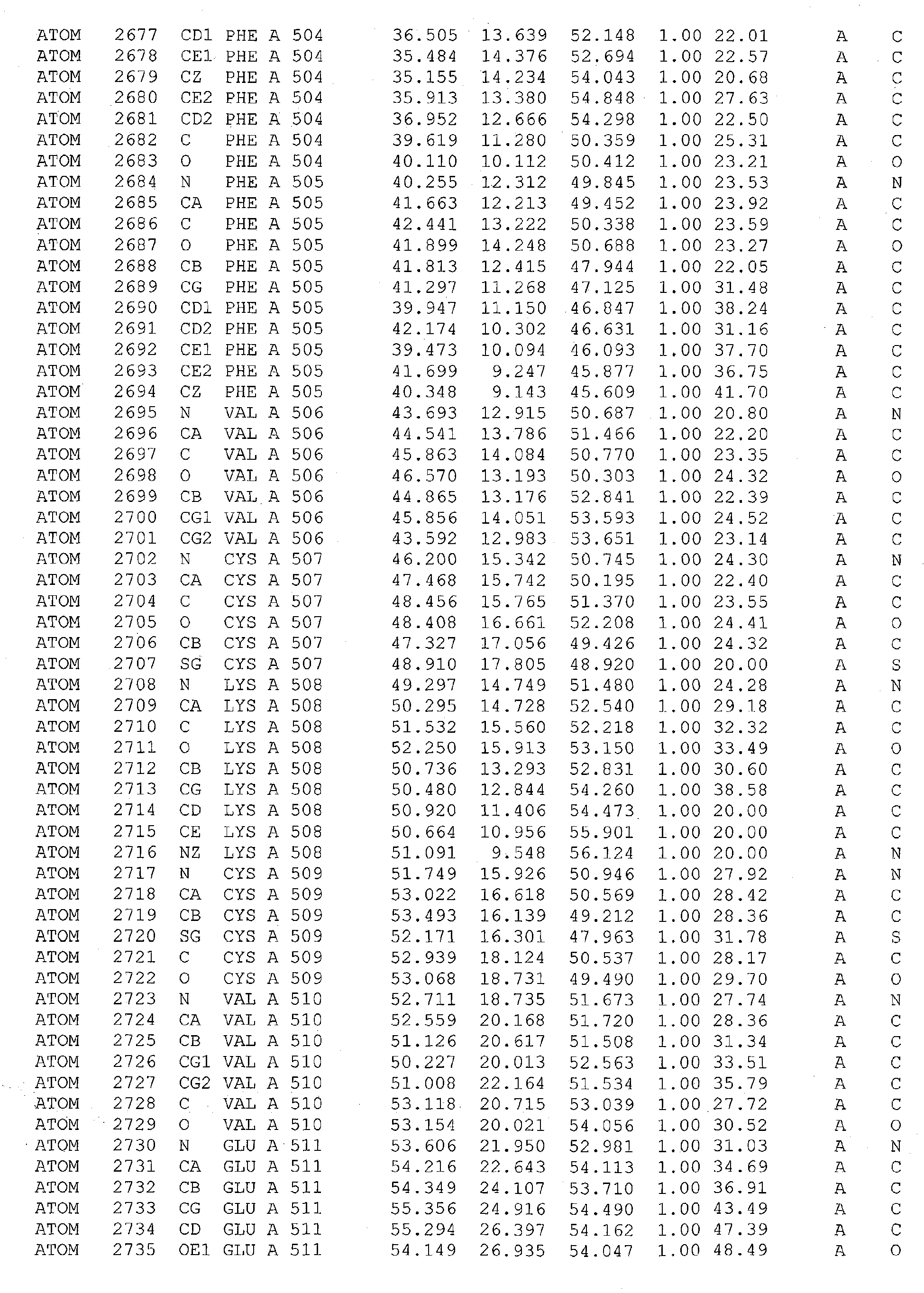

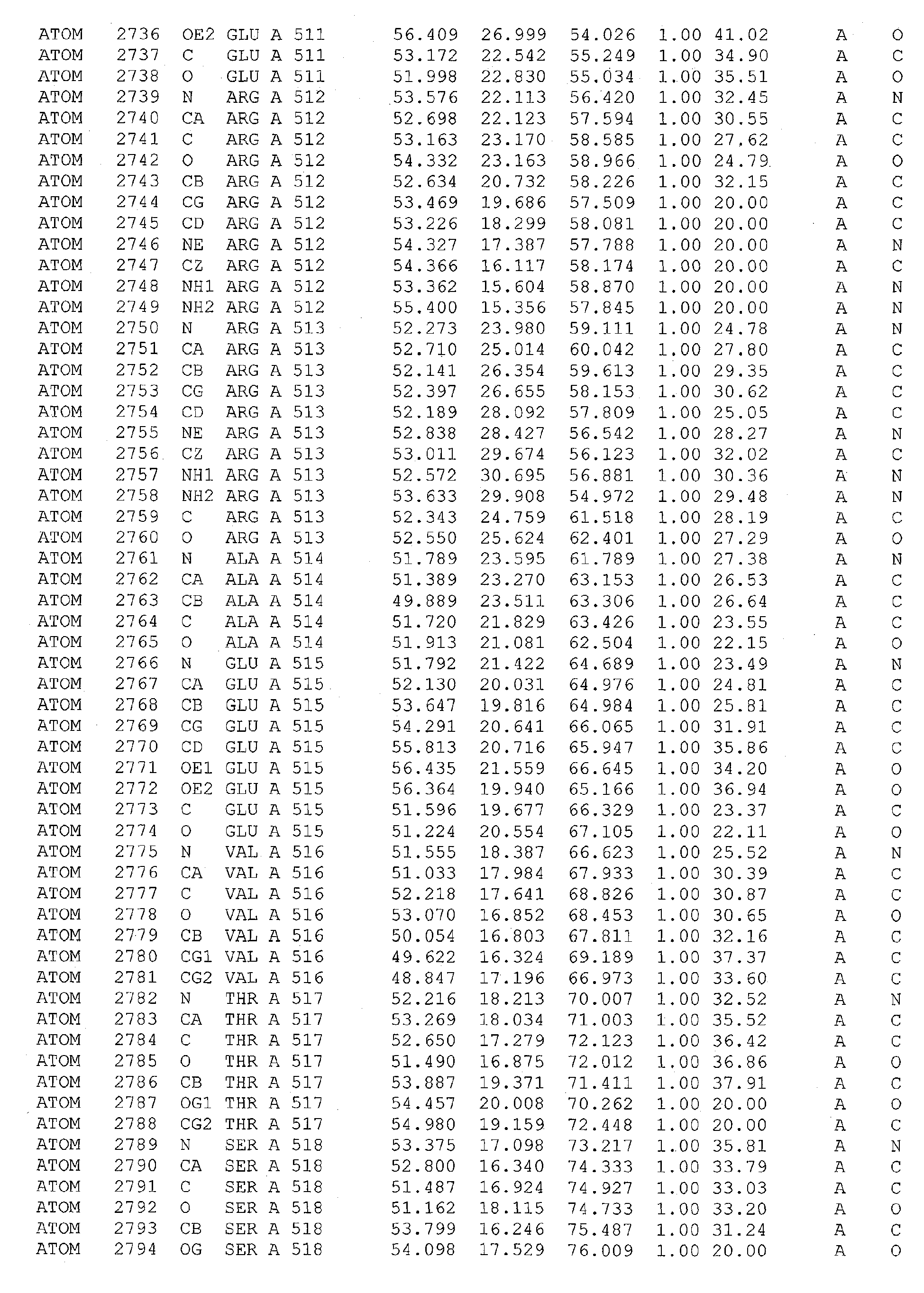

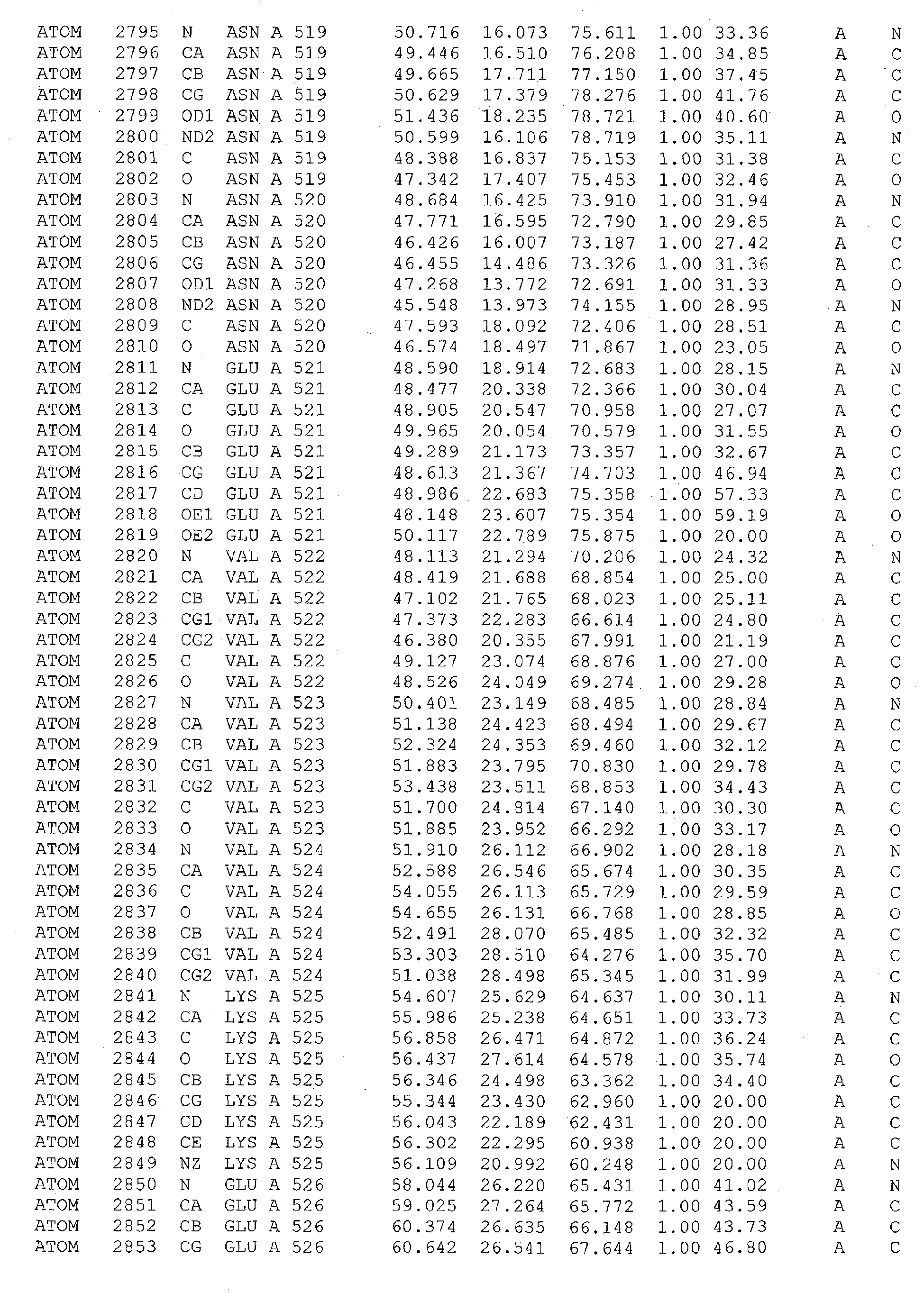

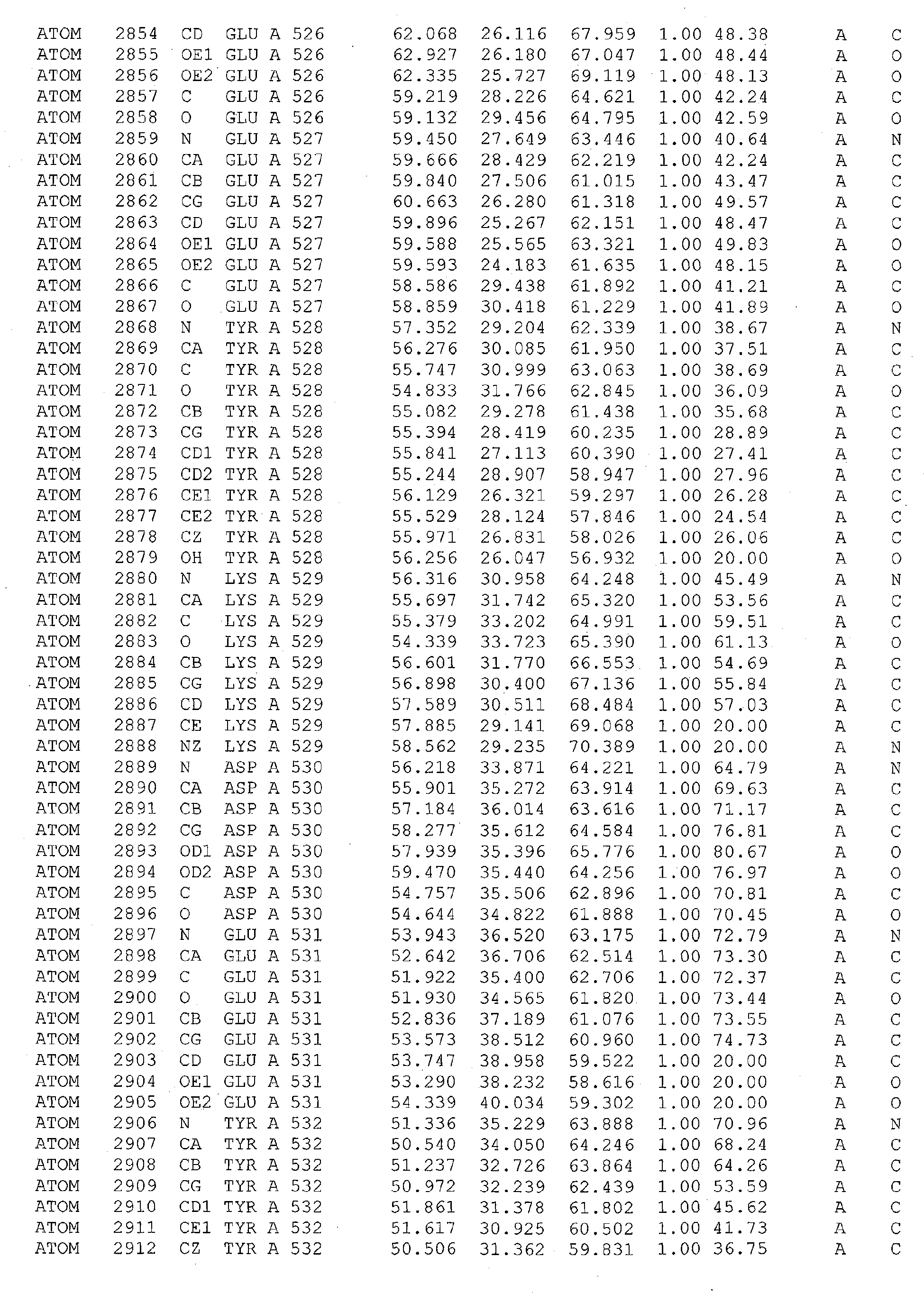

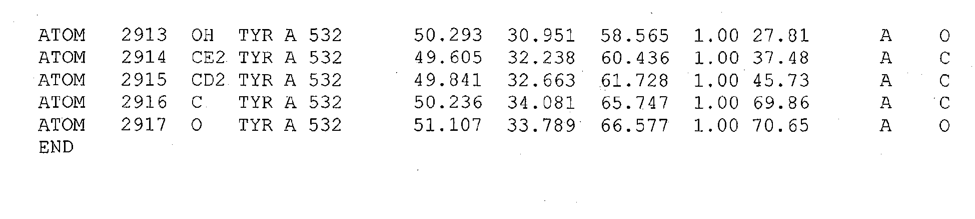

### References

Abrahams, J.P. (1997). Acta Cryst., D53, 371-376.
Bannister, L.H. *et al*. (2003). J. Cell. Sci., 116,3825-3834.
Brown, P.J. *et al*. (2001). FEBS Lett., 497, 31-38.
CCP4 (1994). Acta Cryst., D50, 760-763.
Cowtan, K. (1994). Joint CCP4 and ESF-EACBM Newsletter on Protein
Crystallography 31, 34-38.
Cheng, Q.et *al.*(1994) Mol Biochem Parasitol 65: 183-187.
Cuff, *J.A.et al.* (2000) Proteins 40: 502-511.
Deans, *J.A. et al.* (1982). Clin. Exp. Immunol., 49, 297-309.
Deans, J.A. *et al.* (1984). Mol. Biochem. Parasitol., 11, 189-204.
Donahue, G.G. *et al.* (2000). Mol. Biochem. Parasitol., 111, 15-30.
Emini, E.A. *et al.* (1985) J Virol 55: 836-839.
Gaffar, F.R. *et al.* (2004). Infect. Immun., 72, 2947-2955.
Healer, J. *et al.* (2002). Infect. Immun., 70, 5751-5758.
Hehl, A.B. *et al.* (2000). Infect. Immun., 68, 7078-7086.
Hodder, A.N. *et al.* (1996). J. Biol. Chem., 271, 29446-29452.
Hodder, A.N. *et al.* (1991). J. Appl. Cryst., 24, 409-411.
Holm, L., Sander, C. (1993) J.Mol.Biol. 233,123-138.
Howell, S.A. *et al.* (2001) J Biol Chem 276: 31311-31320.
Howell, S.A*. et al.* (2003) J Biol Chem 278: 23890-23898.
Huizinga, E.G. *et al.,* (2001). Acta Crystallogr. Sec. D. 57, 1071-1078.
Kappe, S.H.I. *et al.,* (1998). Proc. Natl. Acad. Sci. USA, 95, 1230-1235.
Kocken, C.H.M. *et al.* (1999). Infect. Immun., 67, 43-49.
Kocken, C.H.M. *et al.* (1998). J. Biol. Chem., 273, 15119-15124.
Kocken, C.M. *et al.* (2002). Infect. Immun., 70, 4471-4476.
Koehl, P. & Delarue, M. (1994). J. Mol. Biol. 239:249-275 (1994).
Lietha, D. *et al*., (2001). EMBO J., 20, 5543-5555.
Marshall, V.M. *et al*. (1996). Mol. Biochem. Parasitol., 77, 109-113.
Morris, R.J. *et al*. (2002). Acta Cryst., D58, 968-975.
Murshudov, G. N. *et al*. (1997). Acta Cryst., D53, 240-255.
Narum, D.L. & Thomas, A.W. (1994). Mol. Biochem. Parasitol., 67, 59-68.
Otwinowski, Z. & Minor, W. (1997). Methods Enzymol., 276, 307-326.
Polley, S.D. & Conway, D.J. (2001). Genetics, 158, 1505-1512.
Polley, *S.D.et al* (2003). Genetics 165: 555-561.
Schneider, T.R. & Sheldrick, G.M. (2002). Acta Cryst., D58, 1772-1779.
Thomas, A.W. *et al*. (1984). Mol. Biochem. Parasitol., 13, 187-199.
Thomas A.W. *et al*.. (1990). Mol. Biochem. Parasitol., 42, 285-288.
Tomley, F.M. & Soldati, D.S. (2001). Trends Parasitol., 17, 81-88.
Tordai, H. *et al.* (1999). FEBS Lett., 461, 63-67.
Waters, A.P. *et al.* (1990). J. Biol. Chem., 265, 17974-17979.
Zhou, H. *et al.* (1998). Structure, 6,109-116.

## Claims

1. A crystal of a protein wherein the said protein comprises or consists of the ectoplasmic domain of Apical Membrane Protein 1 (AMA1) of apicomplexan parasites, preferably selected from the group consisting of *Plasmodium, Toxoplasma* and *Babesia.*

2. The crystal of a protein according to claim 1, wherein said crystallized protein does not contain the signal sequence, the pro-sequence of the N-terminal region, the transmembrane region and the cytoplasmic domain of the AMA1 protein.

3. The crystal according to claim 1 or 2, wherein the said crystal has a monoclinic form or an orthorombic form.

4. The crystal according to one of the claims 1 to 3, wherein the said *Plasmodium* is selected from the group consisting of *Plasmodium vivax* or *Plasmodium falciparum*.

5. The crystal according to claim to 3, wherein the said *Plasmodium vivax* is the Sa1 I strain.

6. The crystal according to one of the claims 1 to 5, wherein the said crystal effectively diffracts X-rays for the determination of the atomic coordinates of the crystallized protein.

7. A crystal of a protein according to one of the claims 1 to 6, wherein the ectoplasmic domain of the AMA1 protein from *Plasmodium* is a recombinant protein, preferably expressed in *Pichia pastoris.*

8. A crystal of a protein according to one of the claims 1 to 7, wherein the ectoplasmic domain of AMA1 protein from *Plasmodium* is a recombinant protein which has been purified by a method comprising a step of purification on an anion-exchange column and/or a step of metallo-affinity purification.

9. A crystal of a protein according to claims 7 or 8, **characterized in that** the said ectoplasmic domain of the AMA1 protein is a recombinant protein wherein the potentiel N-glycosylation sites of the wild type AMA1 protein have been mutated.

10. A crystal of a protein according to one of the claims 1 to 9, wherein the buffer used for the step of crystallisation comprises or consists of 10-12% (w/v) PEG3350, 100-200 mM imidazole, pH 7.0, 5-10 % (v/v) isopropanol and 1 % (v/v) DMF or 3 % (v/v) t-butanol.

11. A crystal of a protein according to one of the claims 1 to 10, wherein the said crystallized protein comprises or consists in:
a) the protein having the sequence of the ectoplasmic domain of AMA1 protein from *Plasmodium vivax* strain Sa1 I depicted in the figure 3 or encoded by the nucleic sequence depicted in figure 3, or a protein having an identity of sequence of at least 50 % with the ectoplasmic domain of AMA1 protein from *Plasmodium vivax* strain Sal I depicted in the figure 3 ;
b) the protein having the sequence of the ectoplasmic domain of the AMA1 protein from *Plasmodium falciparum*, the said sequence consisting of the residues between Glu98 and Met545 of the sequence depicted in GenBank under the the Accession Number U84348, or a protein having a sequence with an identity of at least 50 % with the sequence of the ectoplasmic domain of *Plasmodium falciparum* strain FVO AMA1 protein.

12. A crystal of a protein according to one of the claims 1 to 11, wherein said crystal has the followings parameters for the native crystal:
a) Space group: P2₁2₁2₁,
Dimensions of the unit cell:
a (Å): 54.10,
b (Å): 76.10,
c (Å):103.93, wherein a variability of ±2.5% in the said dimensions can be anticipated depending on the conditions under which the crystal is manipulated, and
Resolution (Å): better than 3.0 Å,
when the crystallized protein is the ectoplasmic domain of AMA1 protein from *Plasmodium vivax* strain Sal I which has been crystallized in the orthorhombic form; or
b) Space group: C2,
Dimensions of the unit cell:
a (Å): 150.03,
b (Å): 58, 83,
c (Å): 60.29,
β (°): 113.2 °, wherein a variability of ±2.5% in the said dimensions can be anticipated depending on the conditions under which the crystal is manipulated, and
Resolution (Å): better than 3.0 Å,
when the crystallized protein is the ectoplasmic domain of AMA1 protein from *Plasmodium vivax* strain Sa1 I which has been crystallized in the monoclinic form.

13. A crystal of a protein or a modelled structure based on the crystal of a protein according to one of the claims 1 to 12, wherein said crystal or modelled crystal has:
- the characteristics as set forth in the Table 6 when the crystallized protein is the ectoplasmic domain of AMA1 protein from *Plasmodium vivax* strain Sa1 I which has been crystallized in the orthorhombic form;
- the characteristics as set forth in the Table 7 when the crystallized protein is the ectoplasmic domain of AMA1 protein from *Plasmodium vivax* strain Sa1 I which has been crystallized in the monoclinic form ; or
- the characteristics as set forth in the Table 8 when a model of the ectoplasmic domain of AMA1 from *Plasmodium falciparum* is based on the crystallized protein of the ectoplasmic domain of AMA1 protein from *Plasmodium vivax.*

14. Method for obtaining a crystal of a protein having a monoclinic or an orthorhombic form wherein the said protein comprises or consists in a recombinant ectoplasmic domain of AMA1 from *Plasmodium* comprising the step of:
a) purifying the said recombinant ectoplasmic domain of AMA1 by a method comprising a step of purification on an anion-exchange column and/or a step of metallo-affinity purification;
b) growing the crystal comprising or consisting in the said recombinant ectoplasmic domain of AMA1 protein by hanging drop technique wherein the crystallising buffer used for the step of crystallisation comprises or consists of 10-12% (w/v) PEG3350, 100-200 mM imidazole, pH 7.0, 5-10 % (v/v) isopropanol and 1 % DMF (v/v) or 3 % (v/v) t-butanol.

15. Method for preparing a crystal for diffraction measurements, the said crystal comprising or consisting in a recombinant ectoplasmic domain of AMA1 protein of *Plasmodium,* **characterized in that** the said method comprises the step of:
a) obtaining the said crystal by a method according to claim 14; and
b) flash-freezing the said crystal in liquid nitrogen after a brief transfer to a cryo-protecting buffer consisting of the said crystallising buffer of claim 14 in which the PEG 3350 has a concentration of 30 % (w/v).

16. Method for obtaining a platinium derivative of a crystal of a protein wherein said protein comprises or consists in a recombinant ectoplasmic domain of AMA1 protein from *Plasmodium,* **characterized in that** the said method comprises the step of:
a) obtaining said crystal by a method according to claim 14; and
b) soaking said crystal in a solution of potassium tetrachloroplatinate(II) at a concentration of 2 mg/ml in a buffer of 30 % (w/v) PEG 3350, 100 mM sodium cacodylate, 10 % (v/v) isopropanol, pH 7.0, for 24 hours; and
c) flash-freezingthe crystals in liquid nitrogen without back-washing, using a cryo-buffer containing PEG 3350 at 30 % (w/v).

17. Method for obtaining a crystal according to claim 14 or method for preparing a crystal for diffraction measurements according to claim 15 or method for obtaining a platinium derivative of a crystal according to claim 16 wherein:
- the crystallized protein does not contain the signal sequence, the pro-sequence of the N-terminal region, the transmembrane region and the cytoplasmic domain of the AMA1 protein; and/or
- the said *Plasmodium* is selected from the group consisting of *Plasmodium vivax* or *Plasmodium falciparum;* and/or
- wherein the said *Plasmodium* is *Plasmodium vivax* strain Sa1 I
- wherein the said ectoplasmic domain of the AMA1 recombinant protein from *Plasmodium* is a recombinant protein expressed in *Pichia pastoris;* and/or
- **characterized in that** the said ectoplasmic domain of the AMA1 recombinant protein is a recombinant protein wherein the potentiel N-glycosylation sites of the wild type AMA1 protein have been mutated; and/or
- wherein said crystallized recombinant protein has the sequence of the ectoplasmic domain of AMA1 protein from *Plasmodium vivax* strain Sa1 I depicted in the figure 3 or encoded by the nucleic sequence depicted in figure 3, or a protein having an identity of sequence of at least 50 % with the ectoplasmic domain of AMA1 protein from *Plasmodium vivax* strain Sa1 I depicted in the figure 3, or the protein having the sequence of the ectoplasmic domain of the *Plasmodium falciparum* AMA1 protein, the said sequence consisting of the residues between Glu98 and Met545 of the sequence depicted in GenBank under the Accession Number U84348, or a protein having an identity of sequence of at least 50 % with the this ectoplasmic domain of *Plasmodium falciparum* AMA1 protein.

18. A crystal that has been obtained or obtainable by the method according to one of claims 14 to 17.

19. Method to derive a model of the three-dimensional structure of a target structure of the ectoplasmic domain of AMA1 protein from Apicomplexan parasites, preferably from *Toxoplasma, Babesia* or plasmodial parasite species other than *Plasmodium vivax*, the method comprising the steps of:
a) providing the amino acid sequence of the target ectoplasmic domain of AMA1 protein from Apicomplexan parasite species other than *Plasmodium vivax*;
b) identifying structurally conserved regions shared between the amino acid sequence of the ectoplasmic domain of AMA1 protein from *Plasmodium vivax,* such as the amino acid sequence depicted in figure6, and the amino acid sequence of said target ectoplasmic domain of AMA1 protein;
c) determining the atomic coordinates for the target ectoplasmic domain of AMA1 protein by assigning the said structurally conserved regions of the target ectoplasmic domain of AMA1 protein to a three-dimensional structure using a three-dimensional structure of the ectoplasmic domain of AMA1 protein from *Plasmodium vivax* based on atomic coordinates that substantially conform to the atomic coordinates as set forth in the Table 6 or 7, to derive a model of the three-dimensional structure of the target ectoplasmic domain of AMA1 protein from the said from Apicomplexan parasites species other than *Plasmodium vivax,*
preferably, the target ectoplasmic domain of AMA1 protein is the AMA1 of *Plasmodium falciparum* or the protein MAEBL which contains two consecutive segments having structurally conserved regions compared with the ectoplasmic domain of AMA1 protein of *Plasmodium vivax.*

20. A computer-readable data storage material encoded with computer-readable data comprising atomic structure coordinates of the proteins in the crystal according to one of the claims 1 to 18 or of the modelled crystal obtained by the method of claim 19.

21. A computer-readable data storage material encoded with computer-readable data comprising atomic structure coordinates as set forth in the Tables 6 to 8.

22. A method for identifying or an assay for screening a potential inhibitor for inhibiting the erythrocyte invasion of mammal by *Plasmodium* comprising:
a) selecting a potential inhibitor by performing rational drug design with the three-dimensional structure of proteins determined for the crystal of the said proteins according to one of the claims 1 to 13 and 18, or by using the computer-readable data storage material according to claims 20 or 21, the said selecting being optionally performed in conjunction with computer modeling.

23. A method for identifying or an assay for screening a potential inhibitor according to claim 22, further comprising:
b) growing a supplemental crystal comprising a protein-ligand complex formed between the ectoplasmic domain of AMA1 protein from *Plasmodium* and the said potential inhibitor from step (a), wherein the supplemental crystal effectively diffracts X-rays for the determination of the atomic coordinates of the protein-ligand complex to a suitable resolution; and
c) determining the three-dimensional structure of the said protein-inhibitor complex in the supplemental crystal.

24. A method for identifying or an assay for screening a potential inhibitor according to claim 23, further comprising:
d) determining from the three-dimensional structure of the said protein-inhibitor complex in the supplemental crystal obtained in step c) whether this potential inhibitor is bound to the said ectoplasmic domain AMA1 protein.

25. A method for identifying or an assay for screening a potential inhibitor according to claim 24, wherein in step d), it is determined whether this potential inhibitor is bound to at least one the amino-acid residue of the said ectoplasmic domain AMA1 protein selecting from the group of residues consisting of:
- the amino acid residues situated in positions aa225, aa293, aa296, aa297, aa301, aa309, aa333, aa334 and residues within the segment aa298 to 322 of the ectoplasmic domain of the *Plasmodium vivax* strain Sa1 I AMA1 protein when the protein of the said protein-ligand complex is derivated from *Plasmodium vivax* AMA1 protein; and
- the amino acid residues situated in positions aa280,aa348, aa351, aa352, aa356, aa364 aa388, aa389, and within the segment aa353-377 of the ectoplasmic domain of the *Plasmodium falciparum* AMA1 protein when the protein of the said protein-ligand complex is derivated from *Plasmodium falciparum* AMA1 protein; and
- the amino acid residues situated at a position corresponding to the positions aa280, aa348, aa351, aa352, aa356, aa364 aa388, aa389, and within the segment aa353-377 of the ectoplasmic domain of the *Plasmodium falciparum* AMA1 protein, when the ectoplasmic domain AMA1 protein of the said protein-ligand complex is derivated from another *Plasmodium* species than *Plasmodium falciparum* or *Plasmodium vivax.*

26. A method for identifying or an assay for screening a potential inhibitor according to one of claims 22 to claim 25, further comprising:
e) administrating the potential inhibitor to a non-human animal model which has been infected by *Plasmodium* or contacting *in vitro* the potential inhibitor with a cell model by using a cellular-based assay capable of reproducing the invasion of erythrocytes by *Plasmodium;* and
f) detecting the ability of the potential inhibitor for inhibiting the erythrocyte invasion and/or parasite growth.

27. A method according to one of claims 22 to 26, wherein in step (a) the selection or the design of a potential inhibitor is performed by a rational drug design with the three-dimensional structure coordinates of any of Tables 6 to 8, optionally in conjunction with computer modeling.

28. The method according to one of claims 22 to claim 27, wherein the potential inhibitor is designed *de novo* or designed from a known inhibitor.

29. Purified or isolated polypeptide selected from the group of polypeptides constisting of the following polypeptides:
a) - the polypeptide (reference polypeptide) having the sequence of the domains I and II of the ectoplasmic domain of Apical Membrane Protein 1 (AMA1) from *Plasmodium,* wherein the signal sequence, the pro-sequence of the N-terminal region, the transmembrane region and the cytoplasmic domain of the AMA1 protein have been deleted, or
- a homologous polypeptide therof having a sequence presenting an identity of at least 50 % with that domain I and II sequence and wherein at least the 8 amino-acids residues of the reference polypeptide defined in b) are conserved; and
b) a fragment of polypeptide as defined in a), the said fragment having at least 10 amino-acids residues and comprising at least one of the amino-acid residues of said ectoplasmic domain AMA1 protein selecting from the group of residues consisting of:
- the amino acid residues situated in position aa225, aa293, aa296, aa297, aa301, aa309, aa333; aa334 and residues within the segment aa298 to 322 of the ectoplasmic domain of the *Plasmodium vivax* AMA1 protein when the AMA1 protein is derivated from *Plasmodium vivax* AMA protein; and
- the amino acid residues situated in position aa280, aa348, aa351, aa352, aa356, aa364 aa388, aa389, and within the segment aa353-377 of the ectoplasmic domain of the *Plasmodium falciparum* AMA1 protein when the protein is derivated from *Plasmodium falciparum* AMA1 protein; and
- the amino acid residues situated at a position corresponding to the position aa280, aa348, aa351, aa352, aa356, aa364 aa388, aa389, and within the segment aa353-377 of the ectoplasmic domain of the *Plasmodium falciparum* AMA1 protein, when the ectoplasmic domain AMA1 protein is derivated from another apicomplexan parasite, preferably from another *Plasmodium* species than *Plasmodium falciparum* or *Plasmodium vivax*;
- a fragment comprised in the sequence of the domains I and II of the ectoplasmic domain of AMA1 of *Plasmodium vivax*, and having at least the 40-residue segment between aa295 and aa 324, inclusive, in Domain II, or equivalent fragments thereof derivated from other apicomplexan parasites, preferably derivated from another *Plasmodium* species than *Plasmodium vivax,* preferably from derivated from *Plasmodium falciparum.*

30. Polypeptide according to claim 29, **characterized in that** it is a recombinant protein, and, optionally, wherein at least one the potentiel N-glycosylation sites of the wild type AMA1 protein ectoplasmic domains I and II have been mutated.

31. Polypeptide according to claim 29 or 30, **characterized in that** the said polypeptide is a fragment of the ectoplasmic domains I and II and wherein said fragment comprises at least one of:
- the amino acid residues situated in position aa293, aa296, aa297, aa301, aa309, aa333, aa334 and within the segment aa298 to 322, optionally, aa225, of the ectoplasmic domain of a *Plasmodium vivax* AMA1 protein, when the AMA1 protein is derivated from *Plasmodium vivax* AMA1 protein; and
- the amino acid residues situated in positions aa348, aa351, aa352, aa356, aa364, aa388, aa389, and within the segment aa353-377, and, optionally, aa280, of the ectoplasmic domain of a *Plasmodium falciparum* AMA1 protein, when the protein is derivated from *Plasmodium falciparum* AMA1 protein; and
- the amino acid residues situated at a position corresponding to the position, aa348, aa351, aa352, aa356, aa364 aa388, aa389, and within the segment aa353-377, and, optionally, aa280,of the ectoplasmic domain of the *Plasmodium falciparum* AMA1 protein, when the ectoplasmic domain AMA1 protein is derivated from another *Plasmodium* species than *Plasmodium falciparum* or *Plasmodium vivax*.

32. Purified or isolated nucleic acid encoding the polypeptide according to one of claims 29 to 31.

33. Cloning or expression vector encoding the polypeptide according to one of claims 29 to 31.

34. Host cell transformed by a vector of claim 33.

35. Recombinant polypeptide according to one of claims 29 to 31.

36. Polypeptide or recombinant polypeptide according to one of claims 29 to 35 as a medicament, as an immunogen or as a vaccine.

37. Use of a polypeptide according to one of claims 29 to 31 or 35, for the manufacture of a pharmaceutical composition for the prevention or the treatment of malaria.


SEQUENCE LISTING

<110> INSTITUT PASTEUR
      CENTRE NATIONAL DE LA RECHERCHE SCIENTIFIQUE (CNRS)

<120> IDENTIFICATION OF REGIONS OF APICAL MEMBRANE ANTIGEN 1 OF
      APICOMPLEXAN PARASITES SUITABLE FOR TARGETING BY VACCINS AND
      DRUGS BY DERIVATION AND ANALYSIS OF THE THREE-DIMENTIONAL
      STRUCTURE

<130> D22677

<140> EP 04/292 378
<141> 2004-10-06

<160> 3

<170> PatentIn version 3.3

<210> 1
<211> 1338
<212> DNA
<213> Plasmodium vivax

<220>
<223> Nucleotide sequence of ectoplasmic domain of
      Plasmodium vivax AMA1

<400> 1
cctaccgttg agagaagcac acgaatgggt aacccctgga aagcgttcat ggaaaaatac      60
gacatcgaaa gaacacacag ttctggggtt cgagtggatt taggggaaga tgcagaagtg     120
gaaaatgcaa agtacagaat tccagctgga agatgtcctg tttttggaaa gggtatcgtt     180
atagagaatt ctgacgttag cttcttaaga cctgtggcta caggagatca gaagctgaag     240
gatggaggtt tcgccttccc caatgcgaat gaccatatct ccccaatgac attagcgaac     300
cttaaggaaa ggtataaaga caatgtagag atgatgaagt taaacgatat agctttgtgc     360
agaacccacg cagctagctt tgtcatggca ggggatcaaa attcgaacta cagacaccca     420
gctgtatacg acgaaaagga aaaaacatgc cacatgttgt atttatcagc gcaggaaaat     480
atgggtccga ggtactgcag cccagatgca caaaatagag atgccgtgtt ctgcttcaag     540
ccagataaag atgaaagctt tgaaaacctg gtgtatttga gcaaaaatgt gcgtaatgat     600
tgggataaaa aatgcccccg taaaaattta ggaaacgcca agttcggatt atgggtggat     660
gggaactgcg aagaaattcc atacgttaaa gaagtggagg cagaggatct gcgcgaatgc     720
aaccgaatcg ttttcggagc gagtgcctca gatcaaccaa ctcagtatga agaagaaatg     780
acggattatc aaaaaataca acaagggttt agacaaaaca accgagagat gattaaaagt     840
gcctttcttc cagtgggtgc attcaactcg gataatttca aaagtaaagg aagaggattt     900
aactgggcaa atttcgattc tgtaaaaaag aagtgttaca tttttaatac caaaccgact     960
tgcctcatta atgacaaaaa ttttattgca acaacggcgt tatctcaccc acaagaagta    1020
gacctggagt tcccctgcag catatataaa gacgaaattg aaagagaaat taagaaacaa    1080
tcgaggaaca tgaatctgta cagtgttgat ggggaacgca ttgtcctgcc gaggatattt    1140
atctccaacg ataaggagag tatcaaatgt ccctgcgaac ctgagcgcat ttcccagagt    1200
acctgcaact tttacgtttg taactgtgta gagaaaaggg cggaaattaa ggaaaataac    1260
caagttgtta taaaggaaga atttaggaat tattacgaaa atggggagga aaaatcgaac    1320
aagcagatgc tactatag                                                  1338

<210> 2
<211> 445
<212> PRT
<213> Plasmodium vivax

<220>

<223> Protein sequence of ectoplasmic domain of Plasmodium vivax AMA1

<400> 2

Pro Thr Val Glu Arg Ser Thr Arg Met Gly Asn Pro Trp Lys Ala Phe
1               5                   10                  15

Met Glu Lys Tyr Asp Ile Glu Arg Thr His Ser Ser Gly Val Arg Val
            20                  25                  30

Asp Leu Gly Glu Asp Ala Glu Val Glu Asn Ala Lys Tyr Arg Ile Pro
        35                  40                  45

Ala Gly Arg Cys Pro Val Phe Gly Lys Gly Ile Val Ile Glu Asn Ser
    50                  55                  60

Asp Val Ser Phe Leu Arg Pro Val Ala Thr Gly Asp Gln Lys Leu Lys
65                  70                  75                  80

Asp Gly Gly Phe Ala Phe Pro Asn Ala Asn Asp His Ile Ser Pro Met
                85                  90                  95

Thr Leu Ala Asn Leu Lys Glu Arg Tyr Lys Asp Asn Val Glu Met Met
            100                 105                 110

Lys Leu Asn Asp Ile Ala Leu Cys Arg Thr His Ala Ala Ser Phe Val
        115                 120                 125

Met Ala Gly Asp Gln Asn Ser Asn Tyr Arg His Pro Ala Val Tyr Asp
    130                 135                 140

Glu Lys Glu Lys Thr Cys His Met Leu Tyr Leu Ser Ala Gln Glu Asn
145                 150                 155                 160

Met Gly Pro Arg Tyr Cys Ser Pro Asp Ala Gln Asn Arg Asp Ala Val
                165                 170                 175

Phe Cys Phe Lys Pro Asp Lys Asp Glu Ser Phe Glu Asn Leu Val Tyr
            180                 185                 190

Leu Ser Lys Asn Val Arg Asn Asp Trp Asp Lys Lys Cys Pro Arg Lys
        195                 200                 205

Asn Leu Gly Asn Ala Lys Phe Gly Leu Trp Val Asp Gly Asn Cys Glu
    210                 215                 220

Glu Ile Pro Tyr Val Lys Glu Val Glu Ala Glu Asp Leu Arg Glu Cys
225                 230                 235                 240

Asn Arg Ile Val Phe Gly Ala Ser Ala Ser Asp Gln Pro Thr Gln Tyr
                245                 250                 255

Glu Glu Glu Met Thr Asp Tyr Gln Lys Ile Gln Gln Gly Phe Arg Gln
            260                 265                 270

Asn Asn Arg Glu Met Ile Lys Gly Ala Phe Leu Pro Val Gly Ala Phe
        275                 280                 285

Asn Ser Asp Asn Phe Lys Ser Lys Gly Arg Gly Phe Asn Trp Ala Asn
    290                 295                 300

Phe Asp Ser Val Lys Arg Lys Cys Tyr Ile Phe Asn Thr Lys Pro Thr
305                 310                 315                 320

Cys Leu Ile Asn Asp Lys Asn Phe Ile Ala Thr Thr Ala Leu Ser His
                325                 330                 335

Pro Gln Glu Val Asp Leu Glu Phe Pro Cys Ser Ile Tyr Lys Asp Glu
            340                 345                 350

Ile Glu Arg Glu Ile Lys Lys Gln Ser Arg Asn Met Asn Leu Tyr Ser
        355                 360                 365

Val Asp Gly Glu Arg Ile Val Leu Pro Arg Ile Phe Ile Ser Asn Asp
    370                 375                 380

Lys Glu Ser Ile Lys Cys Pro Cys Glu Pro Glu Arg Ile Ser Gln Ser
385                 390                 395                 400

Thr Cys Asn Phe Tyr Val Cys Asn Cys Val Glu Lys Arg Ala Glu Ile
                405                 410                 415

Lys Glu Leu Asn Gln Val Val Ile Lys Glu Glu Phe Arg Leu Tyr Tyr
            420                 425                 430

Glu Leu Gly Glu Glu Lys Ser Asn Lys Gln Met Leu Leu
        435                 440                 445

<210> 3
<211> 22
<212> PRT
<213> Artificial sequence

<220>
<223> Peptide encoded by pPICZaC expression vector

<400> 3

Leu Glu Gln Lys Leu Ile Ser Glu Glu Asp Leu Asn Ser Ala Val Asp
1               5                   10                  15

His His His His His His
            20